(19) **Europäisches Patentamt / European Patent Office / Office européen des brevets**

(11) **EP 4 600 963 A1**

(12) **EUROPEAN PATENT APPLICATION**

(43) Date of publication:
13.08.2025   Bulletin 2025/33

(21) Application number: 25157250.9

(22) Date of filing: 11.02.2025

(51) International Patent Classification (IPC):
**G16B 20/20** (2019.01)   **C12Q 1/6886** (2018.01)

(52) Cooperative Patent Classification (CPC):
**G16B 20/20; C12Q 1/6886**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: 12.02.2024   US 202463552644 P
30.01.2025   US 202519041047

(71) Applicant: **Tempus AI, Inc.**
**Chicago, IL 60654-2282 (US)**

(72) Inventors:
• **Driessen, Terri M.**
  **Chicago, 60654 (US)**
• **Hui, Sandra C.**
  **Chicago, 60654 (US)**
• **Lee, Christine Y.**
  **Chicago, 60654 (US)**
• **Pereira, Tiana Marie**
  **Chicago, 60654 (US)**
• **Shi, Yue**
  **Chicago, 60654 (US)**
• **Sonnenschein, Anne Franklin**
  **Chicago, 60654 (US)**
• **Zhu, Wei**
  **Chicago, 60654 (US)**

(74) Representative: **Grund, Martin et al**
**Grund Intellectual Property Group**
**Patentanwälte und Solicitor PartG mbB**
**Steinsdorfstraße 2**
**80538 München (DE)**

(54) **METHODS AND SYSTEMS FOR DETERMINING BLOOD TUMOR MUTATIONAL BURDEN IN A LIQUID BIOPSY ASSAY**

(57) Systems and methods for determining a blood tumor mutational burden (bTMB) for a test subject are provided in which there is obtained, from a panel-enriched sequencing reaction, a plurality of nucleic acid sequences. The plurality of nucleic acid sequences comprises a corresponding sequence for each cell-free DNA fragment in a plurality of cell-free DNA fragments obtained from a liquid biopsy sample from the test subject. Each respective cell-free DNA fragment in the plurality of cell-free DNA fragments corresponds to a respective probe sequence in a plurality of probe sequences used to enrich cell-free DNA fragments in the liquid biopsy sample in the panel-enriched sequencing reaction. There is deteremined, using the panel-enriched sequencing reaction, that a circulating tumor fraction (ctFE) is above a threshold ctFE value. Responsive to this determination, the bTMB is calculated for the test subject from the panel-enriched sequencing reaction and reported.

**Description**

**CROSS REFERENCE TO RELATED APPLICATIONS**

This application claims priority to United States Provisional Patent Application No. 63/552,644, entitled "Methods and Systems for Determining Blood Tumor Mutational Burden in a Liquid Biopsy Assay," filed February 12, 2024, which is hereby incorporated by reference.

**TECHNICAL FIELD**

The present disclosure relates generally to the use of cell-free DNA sequencing data to provide clinical support for personalized treatment of cancer.

**BACKGROUND**

Precision oncology is the practice of tailoring cancer therapy to the unique genomic, epigenetic, and/or transcriptomic profile of an individual's cancer. Personalized cancer treatment builds upon conventional therapeutic regimens used to treat cancer based only on the gross classification of the cancer, *e.g.*, treating all breast cancer patients with a first therapy and all lung cancer patients with a second therapy. This field was borne out of many observations that different patients diagnosed with the same type of cancer, *e.g.*, breast cancer, responded very differently to common treatment regimens. Over time, researchers have identified genomic, epigenetic, and transcriptomic markers that improve predictions as to how an individual cancer will respond to a particular treatment modality.

There is growing evidence that cancer patients who receive therapy guided by their genetics have better outcomes. For example, studies have shown that targeted therapies result in significantly improved progression-free cancer survival. See, *e.g.,* Radovich et al., Oncotarget, 7(35):56491-500 (2016). Similarly, reports from the IMPACT trial-a large (n = 1307) retrospective analysis of consecutive, prospectively molecularly profiled patients with advanced cancer who participated in a large, personalized medicine trial-indicate that patients receiving targeted therapies matched to their tumor biology had a response rate of 16.2%, as opposed to a response rate of 5.2% for patients receiving non-matched therapy. Tsimberidou et al., ASCO 2018, Abstract LBA2553 (2018).

In fact, therapy targeted to specific genomic alterations is already the standard of care in several tumor types, *e.g.,* as suggested in the National Comprehensive Cancer Network (NCCN) guidelines for melanoma, colorectal cancer, and non-small cell lung cancer. In practice, implementation of these targeted therapies requires determining the status of the diagnostic marker in each eligible cancer patient. While this can be accomplished for the few, well-known mutations associated with treatment recommendations in the NCCN guidelines using individual assays or small next generation sequencing (NGS) panels, the growing number of actionable genomic alterations and increasing complexity of diagnostic classifiers necessitates a more comprehensive evaluation of each patient's cancer genome, epigenome, and/or transcriptome.

For instance, some evidence suggests that use of combination therapies where each component is matched to an actionable genomic alteration holds the greatest potential for treating individual cancers. To this point, a retroactive study of cancer patients treated with one or more therapeutic regimens revealed that patients who received therapies matched to a higher percentage of their genomic alterations experienced a greater frequency of stable disease (*e.g.,* a longer time to recurrence), longer time to treatment failure, and greater overall survival. Wheeler et al., Cancer Res., 76:3690-701 (2016). Thus, comprehensive evaluation of each cancer patient's genome, epigenome, and/or transcriptome should maximize the benefits provided by precision oncology, by facilitating more fine-tuned combination therapies, use of novel off-label drug indications, and/or tissue agnostic immunotherapy. *See,* for example, Schwaederle et al., J Clin Oncol., 33(32):3817-25 (2015); Schwaederle et al., JAMA Oncol., 2(11):1452-59 (2016); and Wheler et al., Cancer Res., 76(13):3690-701 (2016). Further, the use of comprehensive next generation sequencing analysis of cancer genomes facilitates better access and a larger patient pool for clinical trial enrollment. Coyne et al., Curr. Probl. Cancer, 41(3):182-93 (2017); and Markman, Oncology, 31(3):158, 168.

The use of large NGS genomic analysis is growing in order to address the need for more comprehensive characterization of an individual's cancer genome. See, for example, Fernandes et al., Clinics, 72(10):588-94. Recent studies indicate that of the patients for which large NGS genomic analysis is performed, 30-40% then receive clinical care based on the assay results, which is limited by at least the identification of actionable genomic alterations, the availability of medication for treatment of identified actionable genomic alterations, and the clinical condition of the subject. See, Ross et al., JAMA Oncol., 1(1):40-49 (2015); Ross et al., Arch. Pathol. Lab Med., 139:642-49 (2015); Hirshfield et al., Oncologist, 21(11):1315-25 (2016); and Groisberg et al., Oncotarget, 8:39254-67 (2017).

However, these large NGS genomic analyses are conventionally performed on solid tumor samples. For instance, each of the studies referenced in the paragraph above performed NGS analysis of FFPE tumor blocks from patients. Solid tissue biopsies remain the gold standard for diagnosis and identification of predictive biomarkers because they represent well-known and validated methodologies that provide a high degree of accuracy. Nevertheless, there are significant limitations to the use of solid tissue material for large NGS genomic analyses of cancers. For example, tumor biopsies are subject to sampling bias caused by spatial and/or temporal genetic heterogeneity, *e.g.*, between two regions of a single tumor and/or between different cancerous tissues (such as between primary and metastatic tumor sites or between two different primary tumor sites). Such intertumor or intratumor heterogeneity can cause sub-clonal or emerging mutations to be overlooked when using localized tissue biopsies, with the potential for sampling bias to be exacerbated over time as sub-clonal populations further evolve and/or shift in predominance.

Additionally, the acquisition of solid tissue biopsies often requires invasive surgical procedures, *e.g.*, when the primary tumor site is located at an internal organ. These procedures can be expensive, time consuming, and carry a significant risk to the patient, *e.g.,* when the patient's health is poor and may not be able to tolerate invasive medical procedures and/or the tumor is located in a particularly sensitive or inoperable location, such as in the brain or heart. Further, the amount of tissue, if any, that can be procured depends on multiple factors, including the location of the tumor, the size of the tumor, the fragility of the patient, and the risk of comorbidities related to biopsies, such as bleeding and infections. For instance, recent studies report that tissue samples in a majority of advanced non-small cell lung cancer patients are limited to small biopsies and cannot be obtained at all in up to 31% of patients. Ilie and Hofman, Transl. Lung Cancer Res., 5(4):420-23 (2016). Even when a tissue biopsy is obtained, the sample may be too scant for comprehensive testing.

Further, the method of tissue collection, preservation (*e.g.*, formalin fixation), and/or storage of tissue biopsies can result in sample degradation and variable quality DNA. This, in turn, leads to inaccuracies in downstream assays and analysis, including next-generation sequencing (NGS) for the identification of biomarkers. Ilie and Hofman, Transl Lung Cancer Res., 5(4):420-23 (2016).

In addition, the invasive nature of the biopsy procedure, the time and cost associated with obtaining the sample, and the compromised state of cancer patients receiving therapy render repeat testing of cancerous tissues impracticable, if not impossible. As a result, solid tissue biopsy analysis is not amenable to many monitoring schemes that would benefit cancer patients, such as disease progression analysis, treatment efficacy evaluation, disease recurrence monitoring, and other techniques that require data from several time points.

Cell-free DNA (cfDNA) has been identified in various bodily fluids, *e.g.,* blood serum, plasma, urine, *etc.* Chan et al., 2003, Ann. Clin. Biochem., 40(Pt 2):122-30. This cfDNA originates from necrotic or apoptotic cells of all types, including germline cells, hematopoietic cells, and diseased (*e.g.*, cancerous) cells. Advantageously, genomic alterations in cancerous tissues can be identified from cfDNA isolated from cancer patients. *See, e.g.,* Stroun et al., 1989, Oncology, 46(5):318-22; Goessl et al., 2000, Cancer Res., 60(21):5941-45; and Frenel et al., 2015, Clin. Cancer Res. 21(20):4586-96. Thus, one approach to overcoming the problems presented by the use of solid tissue biopsies described above is to analyze cell-free nucleic acids (*e.g.*, cfDNA) and/or nucleic acids in circulating tumor cells present in biological fluids, *e.g.,* via a liquid biopsy.

Specifically, liquid biopsies offer several advantages over conventional solid tissue biopsy analysis. For instance, because bodily fluids can be collected in a minimally invasive or non-invasive fashion, sample collection is simpler, faster, safer, and less expensive than solid tumor biopsies. Such methods require only small amounts of sample (*e.g.,* 10 mL or less of whole blood per biopsy) and reduce the discomfort and risk of complications experienced by patients during conventional tissue biopsies. In fact, liquid biopsy samples can be collected with limited or no assistance from medical professionals and can be performed at almost any location. Further, liquid biopsy samples can be collected from any patient, regardless of the location of their cancer, their overall health, and any previous biopsy collection. This allows for analysis of the cancer genome of patients from which a solid tumor sample cannot be easily and/or safely obtained. In addition, because cell-free DNA in the bodily fluids arise from many different types of tissues in the patient, the genomic alterations present in the pool of cell-free DNA are representative of various different clonal sub-populations of the cancerous tissue of the subject, facilitating a more comprehensive analysis of the cancerous genome of the subject than is possible from one or more sections of a single solid tumor sample.

Liquid biopsies also enable serial genetic testing prior to cancer detection, during the early stages of cancer progression, throughout the course of treatment, and during remission, *e.g.*, to monitor for disease recurrence. The ability to conduct serial testing via non-invasive liquid biopsies throughout the course of disease could prove beneficial for many patients, *e.g.*, through monitoring patient response to therapies, the emergence of new actionable genomic alterations, and/or drug-resistance alterations. These types of information allow medical professionals to more quickly tailor and update therapeutic regimens, *e.g.*, facilitating more timely intervention in the case of disease progression. See, *e.g.,* Ilie and Hofman, 2016, Transl. Lung Cancer Res. 5(4):420-23.

Nevertheless, while liquid biopsies are promising tools for improving outcomes using precision oncology, there are significant challenges specific to the use of cell-free DNA for evaluation of a subject's cancer genome. For instance, there is a highly variable signal-to-noise ratio from one liquid biopsy sample to the next. This occurs because cfDNA originates from a variety of different cells in a subject, both healthy and diseased. Depending on the stage and type of cancer in any particular subject, the fraction of cfDNA fragments originating from cancerous cells (the "tumor fraction" or "ctDNA fraction" of the sample/subject) can range from almost 0% to well over 50%. Other factors, including tumor type and mutation profile, can also impact the amount of DNA released from cancerous tissues. For instance, cfDNA clearance through the liver and kidneys is affected by a variety of factors, including renal dysfunction or other tissue damaging factors (e.g., chemotherapy, surgery, and/or radiotherapy).

The information disclosed in this Background section is only for enhancement of understanding of the general background of the invention and should not be taken as an acknowledgement or any form of suggestion that this information forms the prior art already known to a person skilled in the art.

**SUMMARY**

Given the above background, there is a need in the art for improved methods and systems for supporting clinical decisions in precision oncology using liquid biopsy assays. In particular, there is a need in the art for improved methods and systems for determining tumor mutational burden (TMB) based on a liquid biopsy assay. Tumor mutational burden (TMB), defined as the total number of somatic variations per defined region of a tumor genome, is a pantumor biomarker for immune checkpoint inhibitor (ICI) response in patients with advanced cancer. Without intending to be limited to any particular theory, the potential clinical benefit of this biomarker is founded upon the hypothesis that highly mutated tumors produce high-quality neoantigens that increase T-cell reactivity, which in turn leads to improved response to immune checkpoint blockade treatment. For instance, Aggrawal et al., 2023, "Assessment of Tumor Mutational Burden and Outcomes in Patients With Diverse Advanced Cancers Treated With Immunotherapy," JAMA Network Open 6(5): e2311181, found that high TMB non-small cell lung cancer (NSCLC), bladder, melanoma, and colorectal subjects each have higher 1-year survival probability than low TMB subjects with of the same cancer type. Accordingly, one aspect of the present disclosure provides a method of determining a liquid biopsy tumor mutational burden (ITMB) for a test subject.

In some such embodiments, at a computer system having one or more processors, and memory storing one or more programs for execution by the one or more processors, there is obtained, from a panel-enriched sequencing reaction, a plurality of nucleic acid sequences comprising a corresponding sequence for each cell-free DNA fragment in a first plurality of cell-free DNA fragments obtained from a liquid biopsy sample from the test subject. Each respective cell-free DNA fragment in the first plurality of cell-free DNA fragments corresponds to a respective probe sequence in a plurality of probe sequences used to enrich cell-free DNA fragments in the liquid biopsy sample in the panel-enriched sequencing reaction. In some embodiments, the plurality of probe sequences map to no more than 150 genes in the human genome.

In some embodiments, the panel-enriched sequencing reaction is performed at a read depth of at least 1,000X.

In some embodiments, the panel-enriched sequencing reaction uses a sequencing panel that enriches for from 50 genes to 150 genes.

In some embodiments, the plurality of probe sequences used to enrich cell-free DNA fragments in the liquid biopsy sample in the panel-enriched sequencing reaction collectively map to from 25 different genes to 150 different genes in a human reference genome.

In some embodiments, the panel-enriched sequencing reaction uses a sequencing panel that enriches for at least 10 genes listed in Table 1.

In some embodiments, the panel-enriched sequencing reaction uses a sequencing panel that enriches for at least 10 genes listed in List 1.

In some embodiments, the panel-enriched sequencing reaction uses a sequencing panel that enriches for at least 10 genes listed in List 2.

In some embodiments, the liquid biopsy sample is a blood sample. In some embodiments, the liquid biopsy same is blood, whole blood, plasma, serum, urine, cerebrospinal fluid, fecal material, saliva, sweat, tears, pleural fluid, pericardial fluid, or peritoneal fluid of the subject.

In some embodiments, the liquid biopsy sample is a cell-free sample, *e.g.,* a cell free blood sample.

There is determined, using the panel-enriched sequencing reaction, that a circulating tumor fraction (ctFE) is above a threshold ctFE value. In some such embodiments, the threshold ctFE value is 0.01.

Responsive to determining that the ctFE is above the threshold, the 1TMB for the test subject is calculate from the panel-enriched sequencing reaction.

In some embodiments, this calculating comprises determining a count of a plurality of genetic variants present in the plurality of nucleic acid sequences.

In some embodiments, the count of the plurality of genetic variants present in the plurality of nucleic acid sequences is a count of unique genetic variants present in the plurality of nucleic acid sequences that satisfy one or more qualifying criterion in a set of qualifying criteria.

In some such embodiments, a qualifying criterion in the set of qualifying criteria is a requirement that the respective genetic variant is a missense variant, a combination of a missense variant and a splice region variant, a frameshift variant, a stop loss variant, a splice acceptor variant, an in frame insertion variant, an in frame deletion variant, a combination of a frameshift variant and a splice region variant, a disruptive in frame insertion variant, or a disruptive in frame deletion variant.

In some embodiments, a qualifying criterion in the set of qualifying criteria is a requirement that the respective genetic variant has a variant allele frequency in the liquid biopsy sample that is greater than 0.005 (0.5%).

In some embodiments, a qualifying criterion in the set of qualifying criteria is a requirement that the respective genetic variant has a variant allele frequency in the liquid biopsy sample that is less than 1.0 (100%)

In some embodiments, a qualifying criterion in the set of qualifying criteria is a requirement that the respective genetic variant has a variant allele frequency (VAF) in the liquid biopsy sample that is any one of: (i) greater than 0.01 (1%) and less than 0.4 (40%), (ii) greater than 0.6 (60%) and less than 0.9 (90%), or (iii) greater than 0.4 (40%) and less than 0.60 (60%) with the proviso that |VAF - ctFE| / ctFE < 1, (iv) greater than 0.9 (90%) with the proviso that |VAF - ctFE| / ctFE < 1.

In some embodiments, a qualifying criterion in the set of qualifying criteria is selection of a genetic variant present in the plurality of nucleic acid sequences by a medical professional.

In some such embodiments, the calculating further comprises normalizing the count of the plurality of genetic variants present in the plurality of nucleic acid sequences by a coverage of the plurality of probe sequences. In some such embodiments, the coverage is from 0.1 megabases to 0.4 megabases. In some such embodiments, the coverage is from 0.15 megabases to 0.3 megabases.

In some embodiments, the 1TMB is reported for the test subject.

In some embodiments, the reporting further comprises, responsive to determining that the 1TMB satisfies a therapeutic threshold, reporting a matched therapeutic recommendation for the test subject.

In some embodiments, the reporting comprises comparing the 1TMB for the subject to a severity threshold and reporting a qualitative status of either 1TMB high (ITBM-H) or 1TMB low (ITMB-L) based on the comparing.

In some embodiments, the 1TMB for the test subject is reported only if the 1TMB satisfies a reporting threshold.

In some embodiments, an immunotherapeutic agent is administered to the test subject only if the ITMB for the test subject satisfies a therapeutic threshold.

In some embodiments, responsive to determining that the 1TMB satisfies a clinical trial threshold, reporting a matched clinical trial recommendation for the test subject.

In some embodiments, the method further comprises enrolling the test subject only if the 1TMB for the test subject satisfies a clinical trial threshold.

In some embodiments, the method further comprises using the 1TMB to identify a concordant 1TMB based on a predetermined correlation between (i) detection of somatic mutations in cell-free DNA from liquid biopsy sample from a cohort of training subjects and (ii) detection of somatic mutations in genomic DNA from solid tumor biopsy samples from the cohort of training subjects. Moreover, the reporting further comprises reporting the concordant ITMB.

In some embodiments, an electronic health record for the test subject is updated to include the ITMB for the test subject.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

## BRIEF DESCRIPTION OF THE DRAWINGS

Figures 1A, 1B, 1C and 1D collectively illustrate a block diagram of an example computing device for determining a liquid biopsy tumor mutational burden (ITMB) for a test subject, in accordance with some embodiments of the present disclosure.

Figure 2A illustrates an example workflow for generating a clinical report based on information generated from analysis of one or more patient specimens, in accordance with some embodiments of the present disclosure.

Figure 2B illustrates an example of a distributed diagnostic environment for collecting and evaluating patient data for the purpose of precision oncology, in accordance with some embodiments of the present disclosure.

Figure 3 provides an example flow chart of processes and features for liquid biopsy sample collection and analysis for use in precision oncology, in which dashed boxes represent optional elements, in accordance with some embodiments of the present disclosure.

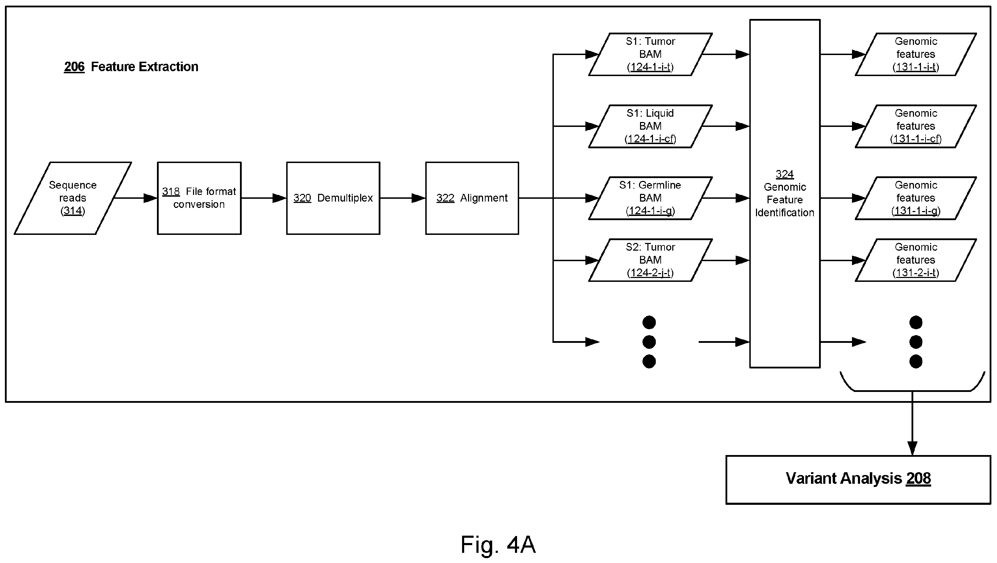

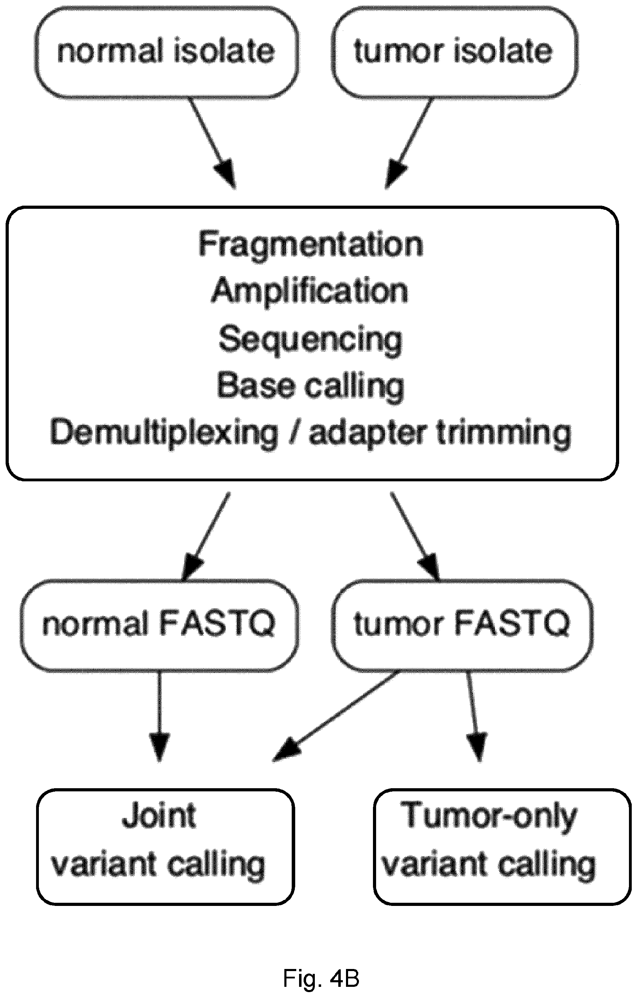

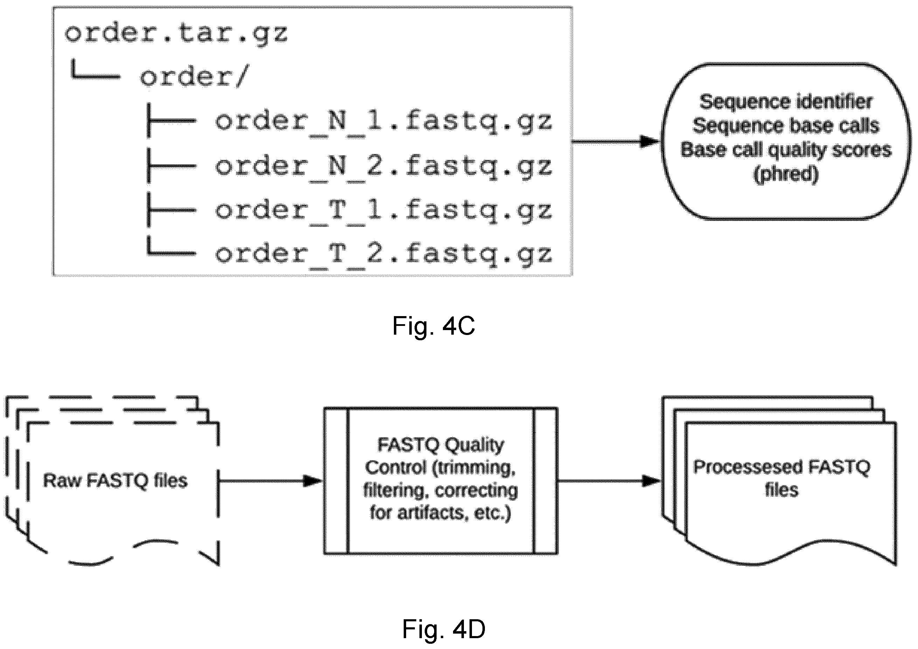

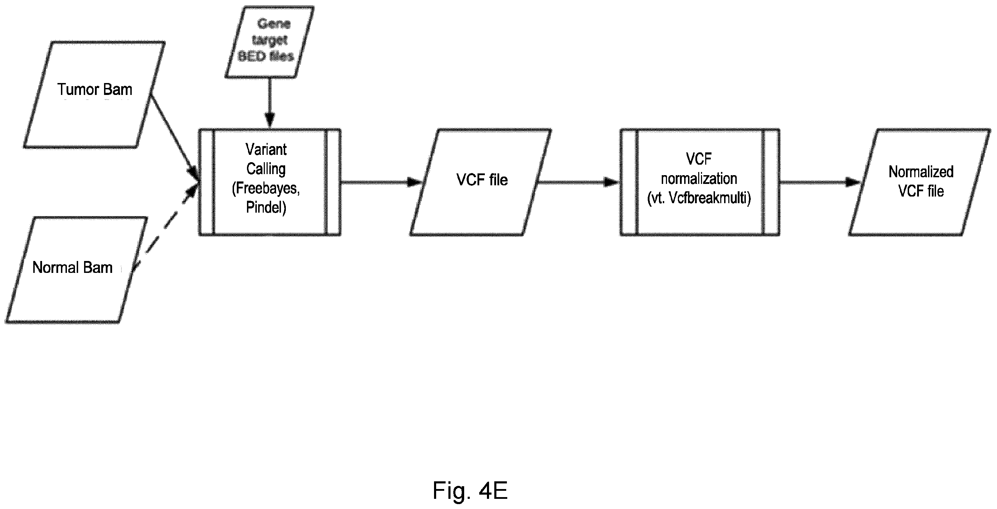

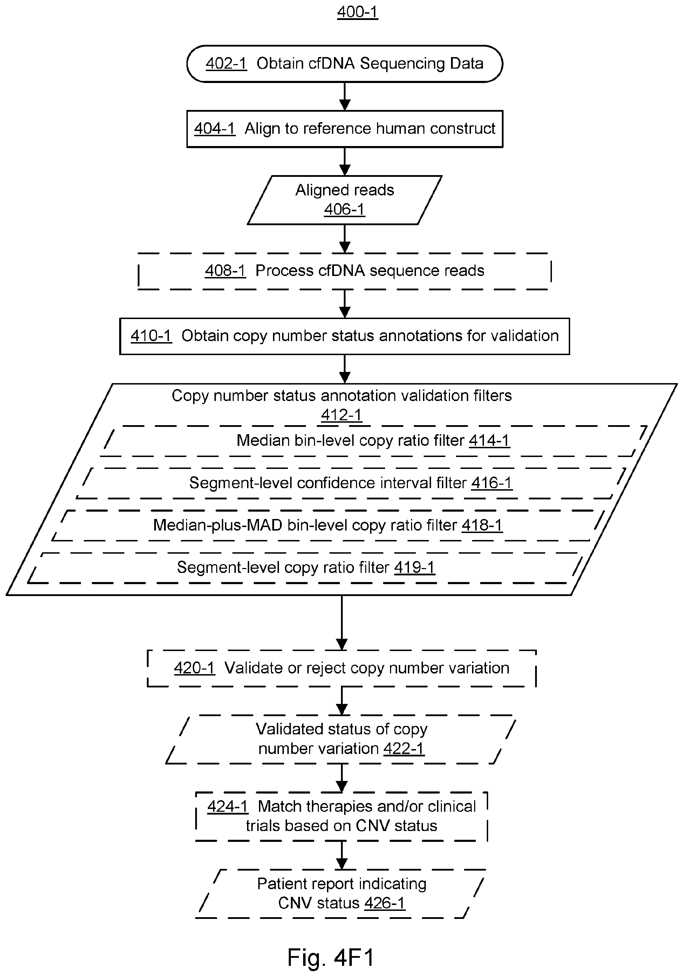

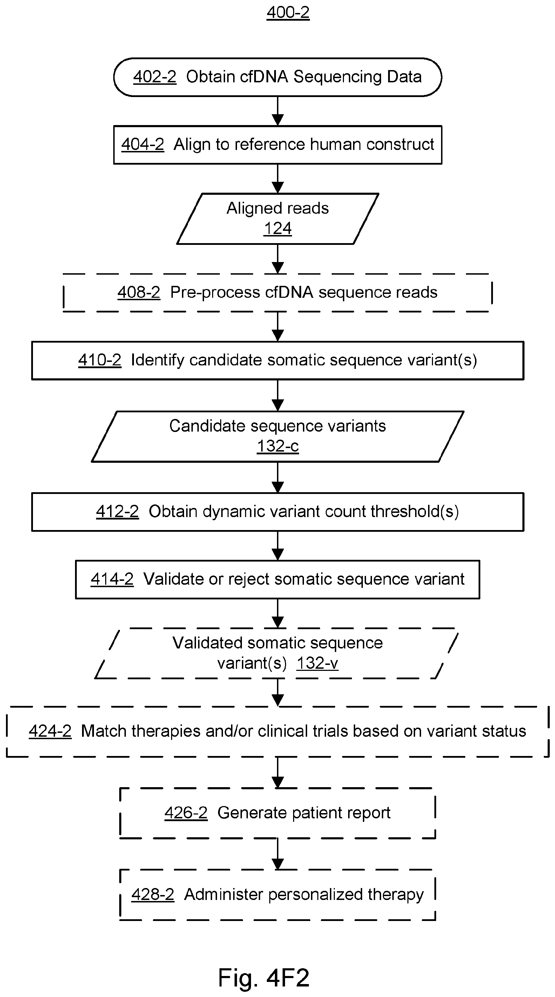

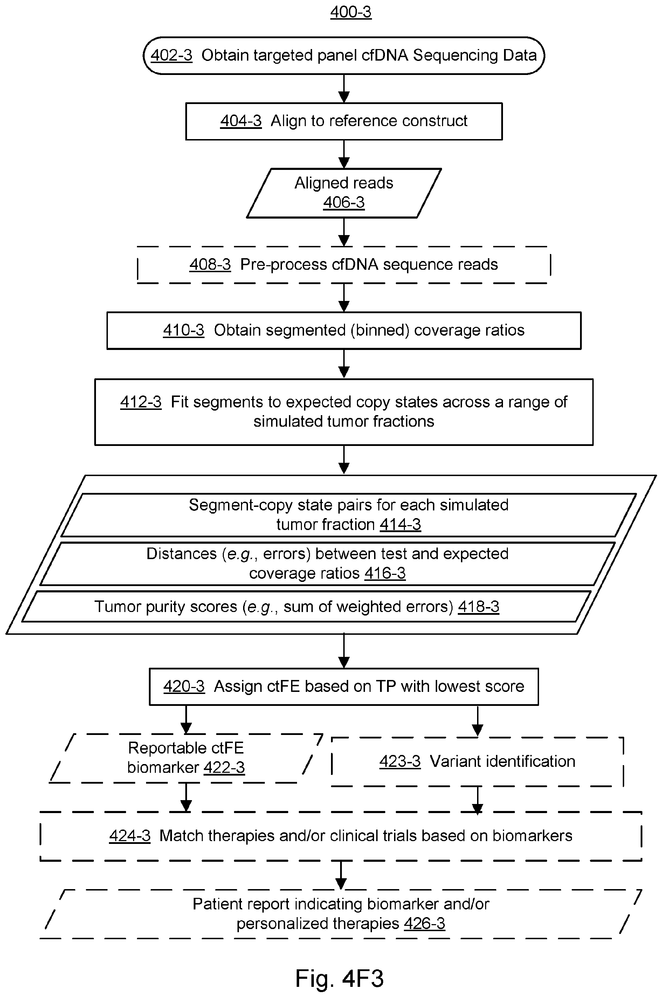

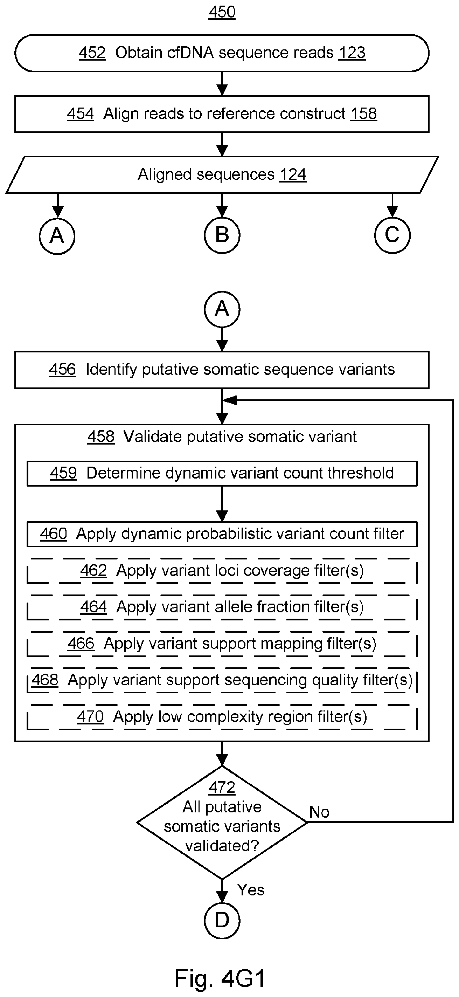

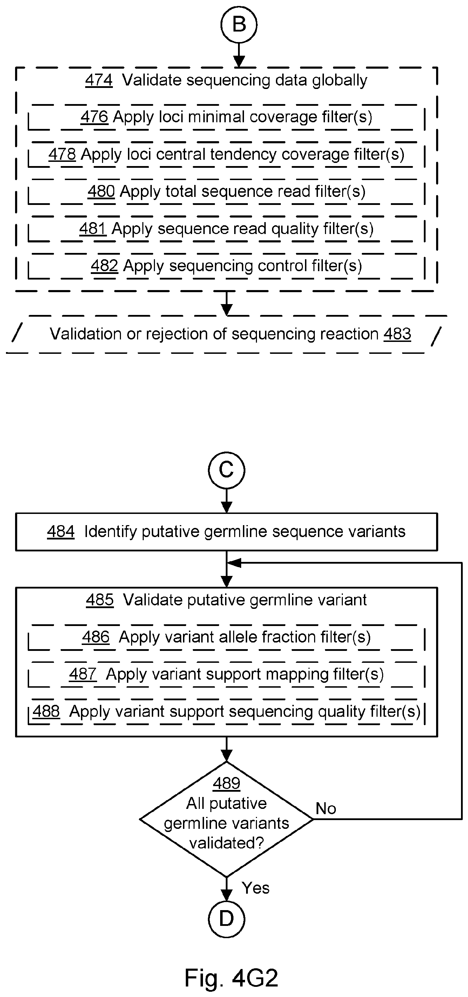

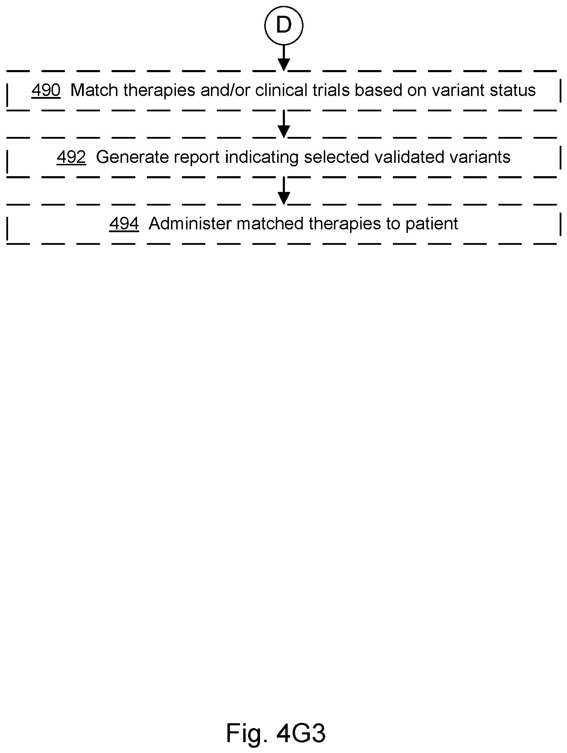

Figures 4A, 4B, 4C, 4D, 4E, 4F1, 4F2, 4F3, 4G1, 4G2, and 4G3 collectively illustrate an example bioinformatics pipeline for precision oncology. Figure 4A provides an overview flow chart of processes and features in a bioinformatics pipeline, in accordance with some embodiments of the present disclosure. Figure 4B provides an overview of a bioinformatics pipeline executed with either a liquid biopsy sample alone or a liquid biopsy sample and a matched normal sample. Figure 4C illustrates that paired end reads from tumor and normal isolates are zipped and stored separately under the same order identifier, in accordance with some embodiments of the present disclosure. Figure 4D illustrates quality correction for FASTQ files, in accordance with some embodiments of the present disclosure. Figure 4E illustrates processes for obtaining tumor and normal BAM alignment files. Figure 4F 1 provides a flow chart of a method for validating a copy number variation, in which dashed boxes represent optional portions of the method, in accordance with some embodiments of the present disclosure. Figure 4F2 provides a flow chart of a method for validating a somatic sequence variant in a test subject having a cancer condition, in which dashed boxes represent optional portions of the method, in accordance with some embodiments of the present disclosure. Figures 4G1, 4G2, and 4G3 illustrate a method of variant detection, in which dashed boxes represent optional portions of the method, in accordance with some embodiments of the present disclosure. Figure 4F3 provides an overview of a method for estimating the circulating tumor fraction for a liquid biopsy sample, based on targeted panel sequencing data, in which dashed boxes represent optional portions of the method, in accordance with some embodiments of the present disclosure.

Figures 5A, 5B, 5C, 5D, 5E, 5F, 5G, and 5H collectively illustrate results of a comparison between circulating tumor fraction estimate (ctFE) and variant allele fraction (VAF) using an Off-Target Tumor Estimation Routine (OTTER) method, in accordance with various embodiments of the present disclosure.

Figures 6A and 6B collectively illustrate results of evaluating ctFE and mutational landscape according to cancer type, in accordance with various embodiments of the present disclosure.

Figures 7A, 7B, and 7C collectively illustrate results of evaluating associations between ctFE and advanced disease states, in accordance with various embodiments of the present disclosure.

Figures 8A, 8B, and 8C collectively illustrate results of comparing ctFE with recent clinical response outcomes, in accordance with various embodiments of the present disclosure.

Figures 9A, 9B, 9C, 9D, and 9E collectively provide a flow chart of processes and features for determining tumor mutational burden from a liquid biopsy sample, in which dashed boxes represent optional portions of the method, in accordance with some embodiments of the present disclosure.

Figures 10A, 10B, 10C, 10D, 10E, 10F, 10G, 10H, 10I 10J, 10K, 10L, and 10M collectively illustrate example nucleic acids targeted for enrichment and variant detection using one or more probes, in accordance with some embodiments of the present disclosure.

Like reference numerals refer to corresponding parts throughout the several views of the drawings.

## DETAILED DESCRIPTION

*Introduction.*

Tumor mutational burden (TMB), which is a measure of the total number of somatic mutations in a cancer, has been used as a biomarker for identifying cancer patients that are most likely to respond favorably to immune checkpoint blockade (ICB) therapies. However, conventional liquid biopsy assays do not provide accurate determination of TMB, also referred to herein as a blood TMB (bTMB) or liquid biopsy TMB (lTMB). There have been a number of reasons suggested as to why bTMB determinations are particularly difficult, including the diluted nature of circulating tumor DNA (ctDNA), the use of small targeted-panels for sequencing cell-free DNA (cfDNA) that enrich genomic regions that are mutational hotspots. Thus, there is a need in the art for improved methods of determining bTMB from liquid biopsy assays, and particularly from liquid biopsy assays that use small targeting panels (e.g., targeting less than 250 genes and/or less than 1 Mb) for enriching genomic regions of interest.

Advantageously, disclosed herein are methods and systems that provide accurate determination of bTMB from small targeted-panel sequencing-based liquid biopsy assays. In some embodiments, the improved properties of the methods and systems described herein are based, at least in part, on the incorporation of a threshold circulating tumor fraction above which the bTMB is calculated. In some embodiments, the improved properties of the methods and systems described herein are based, at least in part, on the incorporation of filtering criteria for identifying which mutations detected in the liquid biopsy assay should be used for bTMB determination.

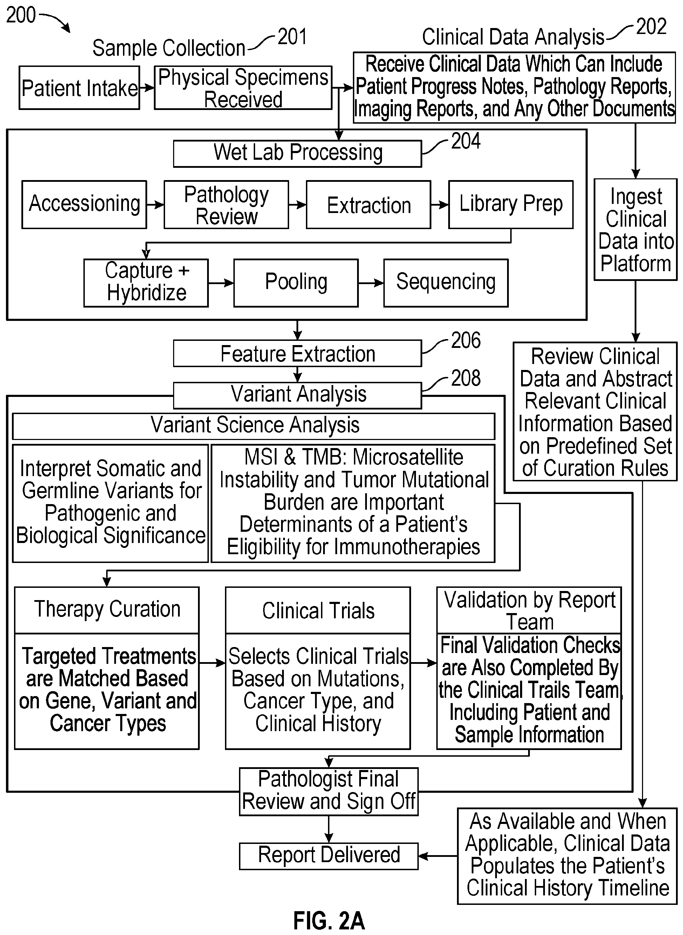

In one aspect, the disclosure provides methods, and corresponding systems and non-transitory computer readble medium (CRM) for executing such methods, for determining a blood tumor mutational burden (bTMB) for a test subject. In some embodiments, the methods, systems, and CRM described herein are incorporated within the framework of a liquid biopsy assay. For example, Figure 2A illustrates an example schema for combined wet lab and bioinformatic steps for a liquid biopsy analysis.

In some embodiments, the methods, systems, and CRM described herein obtain data from one or more different portions of the bioinformatic pipeline for the liquid biopsy assay. In some embodiments, the methods and systems described herein use somatic mutations identified in a separate workstream of the liquid biopsy assay. For example, Figures 4F2 and 4G illustrate methods 400-2 and 450, respectively, for identifying somatic sequence variants from cfDNA sequencing data using dynamic variant count thresholds. In some embodiments, such identified somatic variants are used in the determination of bTMB using the methods, systems, and CRM described herein. For more information on such methods for identifying somatic mutations from cfDNA samples see, for example, U.S. Patent No. 11,475,981, the disclosure of which is incorporated herein by reference, in its entirety, for all purposes.

Similarly, Figure 4F3 illustrates a method 400-3 for estimating the circulating tumor fraction of a liquid biopsy sample by matching simulated tumor fractions to copy number states generated from cfDNA sequencing data. In some embodiments, such circulating tumor fraction estimates (ctFE) are used in the methods, systems, and CRM described herein, *e.g.,* in the threshold determination of whether to determine bTMB. For more information on such methods for estimating the circulating tumor fraction of a liquid biopsy sample see, for example, U.S. Patent No. 11,211,147, the disclosure of which is incorporated herein by reference, in its entirety, for all purposes.

In one aspect, the disclosure provides methods, as well as systems and CRM for executing all or a portion of such methods, for obtaining, from a panel-enriched sequencing reaction, a plurality of nucleic acid sequences comprising a corresponding sequence for each cell-free DNA fragment in a first plurality of cell-free DNA fragments obtained from a liquid biopsy sample from the test subject. For example, sequencing data 122-1 described herein, *e.g.*, with respect to the sequencing 312 performed during wet lab portion 204 of the example liquid biopsy schema illustrated in Figure 2A.

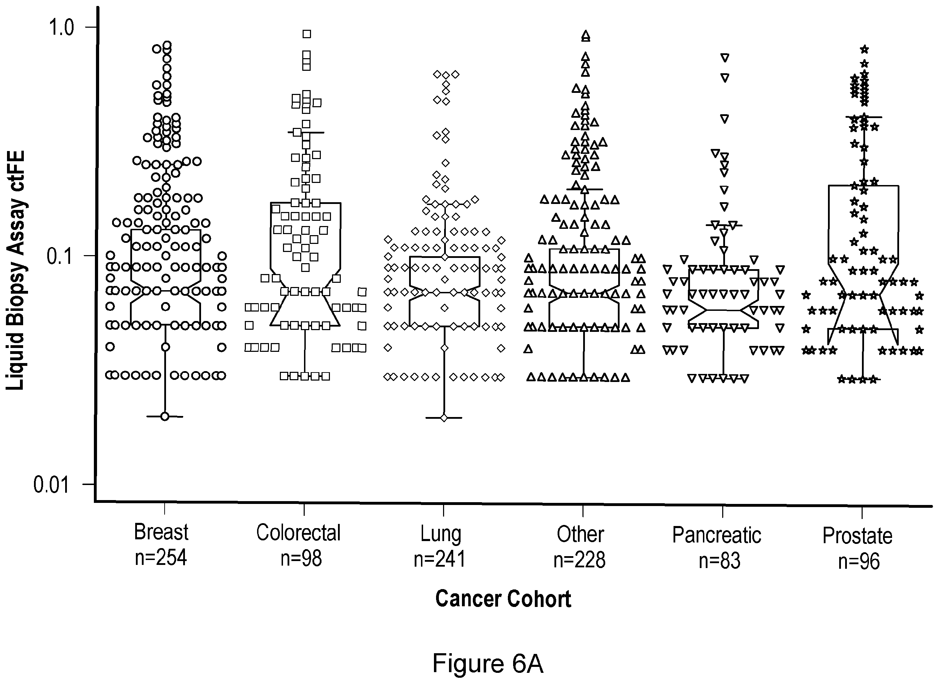

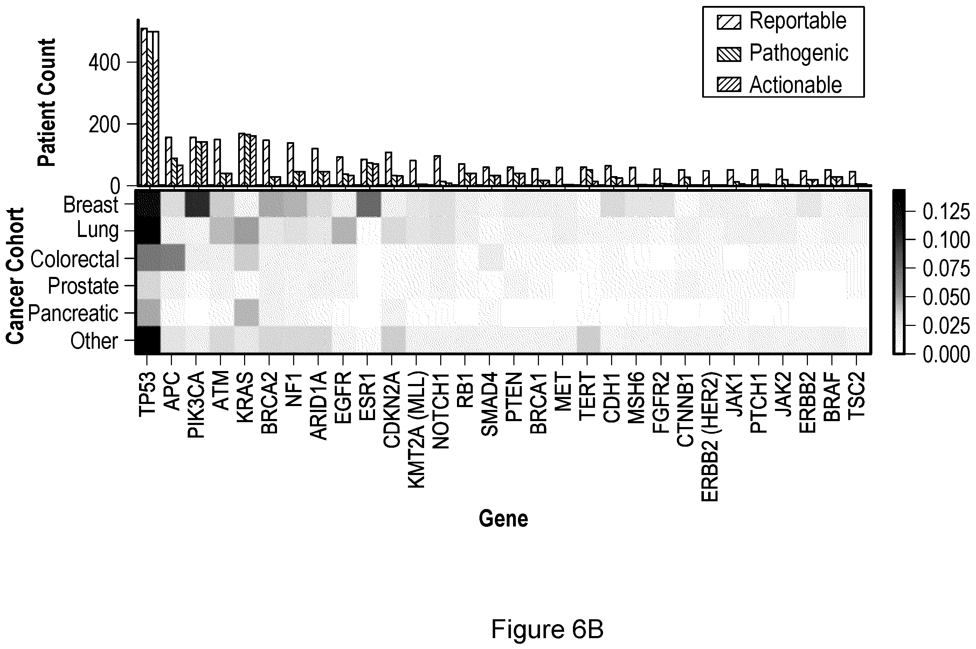

Each respective cell-free DNA fragment in the first plurality of cell-free DNA fragments corresponds to a respective probe sequence in a plurality of probe sequences used to enrich cell-free DNA fragments in the liquid biopsy sample in the panel-enriched sequencing reaction. Example gene sets targeted by such probes are described, for example, with reference to Table 1 provided herein, as well as Figure 6 in PCT Patent Application Publication Number WO 2023/164713, the disclosure of which is hereby incorporated by reference, in its entirety, for all purposes. In some embodiments, the plurality of probe sequences map to no more than 150 genes in the human genome.

The method also includes determining, using the panel-enriched sequencing reaction, that a circulating tumor fraction (ctFE) is above a threshold ctFE value. For example, in some embodiments, a circulating tumor fraction estimate prepared according to a method described herein, *e.g.*, with reference to the method 400-3 illustrated in Figure 4F3, is used in such a step to threshold the bTMB determination. Similarly, in some embodiments, a ctFE prepared according to a method described in U.S. Provisional Application Serial Number 18/930,786 entitled "ESTIMATION OF CIRCULATING TUMOR FRACTION USING OFF-TARGET READS OF TARGETED-PANEL SEQUENCING," filed October 31, 2023, the disclosure of which is hereby incorporated by reference, in its entirety, for all purposes, is used in such a step to threshold the bTMB determination.

The method also includes, responsive to determining that the ctFE is above the threshold, calculating the bTMB for the test subject from the panel-enriched sequencing reaction. In some embodiments, this includes determining a count of a plurality of genetic variants present in the plurality of nucleic acid sequences. In some embodiments, the plurality of genetic variants are validated somatic variants, *e.g.*, as determined according to a validation method described herein, *e.g.*, with respect to method 400-2 or 450 illustrated in Figures 4F2 and 4G, respectively. In some embodiments, the plurality of genetic variants are somatic variants validated using other methods, *e.g.*, as described in U.S. Patent No. 11,475,981. In some embodiments, the the calculating further comprises normalizing the count of the plurality of genetic variants present in the plurality of nucleic acid sequences by a coverage of the plurality of probe sequences, *e.g.*, a panel as described in Table 1 herein or in PCT Patent Application Publication Number WO 2023/164713. In some embodiments, the method also includes reporting the bTMB for the test subject.

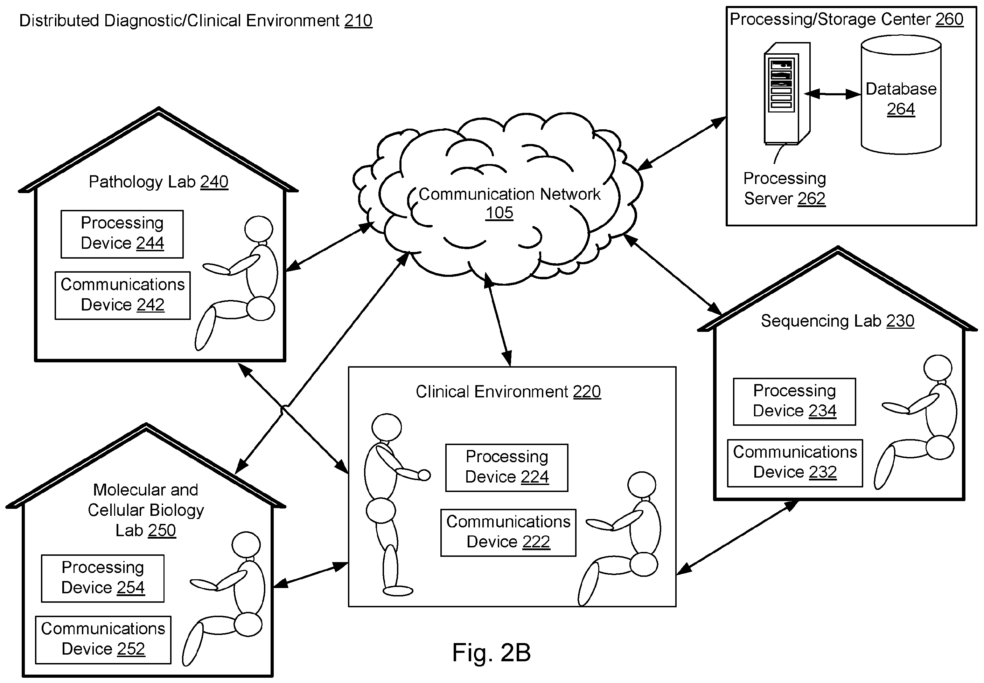

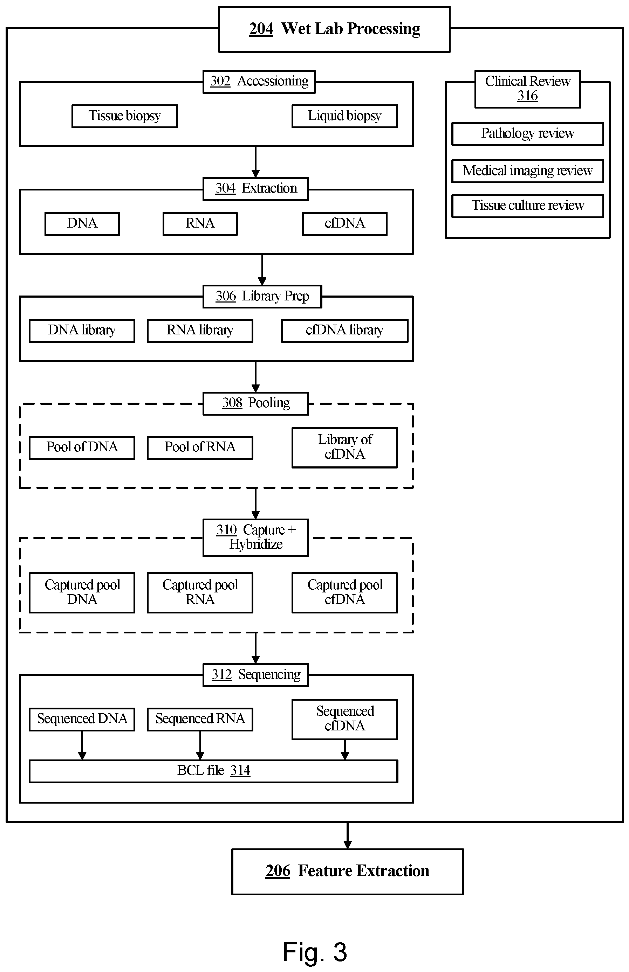

As described herein, in some embodiments, the methods described herein include one or more data collection steps, in addition to data analysis and downstream steps. For example, as described below, *e.g.*, with reference to Figures 2 and 3, in some embodiments, the methods include collection of a liquid biopsy sample and, optionally, one or more matching biological samples from the subject (*e.g.,* a matched cancerous and/or matched non-cancerous sample from the subject). Likewise, as described below, *e.g.*, with reference to Figures 2 and 3, in some embodiments, the methods include extraction of DNA from the liquid biopsy sample and, optionally, one or more matching biological samples from the subject (*e.g.,* a matched cancerous and/or matched non-cancerous sample from the subject). Similarly, as described below, *e.g.*, with reference to Figures 2 and 3, in some embodiments, the methods include nucleic acid sequencing of DNA from the liquid biopsy sample and, optionally, one or more matching biological samples from the subject (*e.g.,* a matched cancerous and/or matched non-cancerous sample from the subject).

However, in other embodiments, the methods described herein begin with obtaining nucleic acid sequencing results, *e.g.*, raw or collapsed sequence reads of DNA from a liquid biopsy sample and, optionally, one or more matching biological samples from the subject (*e.g.,* a matched cancerous and/or matched non-cancerous sample from the subject), from which the statistics needed for focal CNV validation (*e.g.*, bin-level sequence ratios, segment-level sequence ratios, and segment-level measures of dispersion) can be determined. For example, in some embodiments, sequencing data 122 for a patient 121 is accessed and/or downloaded over network 105 by system 100.

The identification of actionable genomic alterations in a patient's cancer genome is a difficult and computationally demanding problem. For instance, the determination of various prognostic metrics useful for precision oncology, such as variant allelic ratio, copy number variation, tumor mutational burden, microsatellite instability status, *etc.,* requires analysis of hundreds of millions to billions, of sequenced nucleic acid bases. An example of a typical bioinformatics pipeline established for this purpose includes at least five stages of analysis: assessment of the quality of raw next generation sequencing data, generation of collapsed nucleic acid fragment sequences and alignment of such sequences to a reference genome, detection of structural variants in the aligned sequence data, annotation of identified variants, and visualization of the data. See, Wadapurkar and Vyas, Informatics in Medicine Unlocked, 11:75-82 (2018), the content of which is hereby incorporated by reference, in its entirety, for all purposes. Each one of these procedures is computationally taxing in its own right.

For instance, the overall temporal and spatial computation complexity of simple global and local pairwise sequence alignment algorithms are quadratic in nature (*e.g.*, second order problems), that increase rapidly as a function of the size of the nucleic acid sequences (n and m) being compared. Specifically, the temporal and spatial complexities of these sequence alignment algorithms can be estimated as $O(mn)$, where O is the upper bound on the asymptotic growth rate of the algorithm, n is the number of bases in the first nucleic acid sequence, and m is the number of bases in the second nucleic acid sequence. See, Baichoo and Ouzounis, BioSystems, 156-157:72-85 (2017), the content of which is hereby incorporated by reference, in its entirety, for all purposes. Given that the human genome contains more than 3 billion bases, these alignment algorithms are extremely computationally taxing, especially when used to analyze next generation sequencing (NGS) data, which can generate more than 3 billion sequence reads per reaction.

This is particularly true when performed in the context of a liquid biopsy assay, because liquid biopsy samples contain a complex mixture of short DNA fragments originating from many different germline (*e.g.,* healthy) and diseased (*e.g.,* cancerous) tissues. Thus, the cellular origins of the sequence reads are unknown, and the sequence signals originating from cancerous cells, which may constitute multiple sub-clonal populations, must be computationally deconvoluted from signals originating from germline and hematopoietic origins, in order to provide relevant information about the subject's cancer. Thus, in addition to the computationally taxing processes required to align sequence reads to a human genome, there is a computation problem of determining whether a particular abnormal signal, *e.g.,* one or more sequence reads corresponding to a genomic alteration, (i) is not an artifact, and (ii) originated from a cancerous source in the subject. This is increasingly difficult during the early stages of cancer-when treatment is presumably most effective-when only small amounts of ctDNA are diluted by germline and hematopoietic DNA.

In addition to the computationally demanding problem of aligning sequencing data to a human reference genome, the method comprises dividing the plurality of aligned sequence reads into "bins" (*e.g.*, regions of a predefined span of base pairs corresponding to a reference genome), determining the copy ratio of each bin by calculating the differential read depths between experimental and reference samples, and grouping subsets of adjacent bins with shared copy ratios into segments. Grouping bins into segments divides each chromosome into regions of equal copy number that minimizes noise in the data. Such methods essentially perform a change-point or edge detection algorithm, which are either temporally limited or computationally intense. For example, in some embodiments, the segmentation is performed using circular binary segmentation. Circular binary segmentation calculates a statistic for each genomic position, where the statistic comprises a likelihood ratio for the null hypothesis (no change in copy ratio at the respective position) against the alternative (one change in copy ratio at the respective position), and where the null hypothesis is rejected if the statistic is greater than a predefined distribution threshold. Notably, in circular binary segmentation, the chromosome is assumed to be circularized, such that the calculation is performed recursively for each position (*e.g.*, each bin) around the circumference of the circle to identify all change-points across the length of the chromosome. Furthermore, for each position (*e.g.*, bin) under investigation, a reference distribution is generated using a permutation approach, where the copy ratios for the plurality of bins are randomized (typically 10,000 times). For some embodiments that utilize bins of approximately 100-150 bases long spanning a human reference genome of several billion bases, the number of permutations required to perform this recursive method contributes to a computationally intense procedure. See, for example, Olshen et al., Biostatistics 5, 4, 557-572 (2004), doi:10.1093/biostatistics/kxh008, which is hereby incorporated herein by reference in its entirety.

*Definitions.*

As used herein, the term "subject" refers to any living or non-living organism including, but not limited to, a human (*e.g.,* a male human, female human, fetus, pregnant female, child, or the like), a non-human mammal, or a non-human animal. Any human or non-human animal can serve as a subject, including but not limited to mammal, reptile, avian, amphibian, fish, ungulate, ruminant, bovine (*e.g.,* cattle), equine (*e.g.,* horse), caprine and ovine (*e.g.,* sheep, goat), swine (*e.g.,* pig), camelid (*e.g.,* camel, llama, alpaca), monkey, ape (*e.g.,* gorilla, chimpanzee), ursid (*e.g.,* bear), poultry, dog, cat, mouse, rat, fish, dolphin, whale and shark. In some embodiments, a subject is a male or female of any age (*e.g.,* a man, a woman, or a child).

As used herein, the terms "control," "control sample," "reference," "reference sample," "normal," and "normal sample" describe a sample from a non-diseased tissue. In some embodiments, such a sample is from a subject that does not have a particular condition (*e.g.,* cancer). In other embodiments, such a sample is an internal control from a subject, *e.g.,* who may or may not have the particular disease (*e.g.,* cancer), but is from a healthy tissue of the subject. For example, where a liquid or solid tumor sample is obtained from a subject with cancer, an internal control sample may be obtained from a healthy tissue of the subject, *e.g.,* a white blood cell sample from a subject without a blood cancer or a solid germline tissue sample from the subject. Accordingly, a reference sample can be obtained from the subject or from a database, *e.g.,* from a second subject who does not have the particular disease (*e.g.,* cancer).

As used herein the term "cancer," "cancerous tissue," or "tumor" refers to an abnormal mass of tissue in which the growth of the mass surpasses, and is not coordinated with, the growth of normal tissue, including both solid masses (*e.g.,* as in a solid tumor) or fluid masses (*e.g.,* as in a hematological cancer). A cancer or tumor can be defined as "benign" or "malignant" depending on the following characteristics: degree of cellular differentiation including morphology and functionality, rate of growth, local invasion and metastasis. A "benign" tumor can be well differentiated, have characteristically slower growth than a malignant tumor and remain localized to the site of origin. In addition, in some cases a benign tumor does not have the capacity to infiltrate, invade or metastasize to distant sites. A "malignant" tumor can be a poorly differentiated (anaplasia), have characteristically rapid growth accompanied by progressive infiltration, invasion, and destruction of the surrounding tissue. Furthermore, a malignant tumor can have the capacity to metastasize to distant sites. Accordingly, a cancer cell is a cell found within the abnormal mass of tissue whose growth is not coordinated with the growth of normal tissue. Accordingly, a "tumor sample" refers to a biological sample obtained or derived from a tumor of a subject, as described herein.

Non-limiting examples of cancer types include ovarian cancer, cervical cancer, uveal melanoma, colorectal cancer, chromophobe renal cell carcinoma, liver cancer, endocrine tumor, oropharyngeal cancer, retinoblastoma, biliary cancer, adrenal cancer, neural cancer, neuroblastoma, basal cell carcinoma, brain cancer, breast cancer, non-clear cell renal cell carcinoma, glioblastoma, glioma, kidney cancer, gastrointestinal stromal tumor, medulloblastoma, bladder cancer, gastric cancer, bone cancer, non-small cell lung cancer, thymoma, prostate cancer, clear cell renal cell carcinoma, skin cancer, thyroid cancer, sarcoma, testicular cancer, head and neck cancer (*e.g.,* head and neck squamous cell carcinoma), meningioma, peritoneal cancer, endometrial cancer, pancreatic cancer, mesothelioma, esophageal cancer, small cell lung cancer, Her2 negative breast cancer, ovarian serous carcinoma, HR+ breast cancer, uterine serous carcinoma, uterine corpus endometrial carcinoma, gastroesophageal junction adenocarcinoma, gallbladder cancer, chordoma, and papillary renal cell carcinoma.

As used herein, the terms "cancer state" or "cancer condition" refer to a characteristic of a cancer patient's condition, *e.g.,* a diagnostic status, a type of cancer, a location of cancer, a primary origin of a cancer, a cancer stage, a cancer prognosis, and/or one or more additional characteristics of a cancer (*e.g.,* tumor characteristics such as morphology, heterogeneity, size, *etc.*). In some embodiments, one or more additional personal characteristics of the subject are used further describe the cancer state or cancer condition of the subject, *e.g.,* age, gender, weight, race, personal habits (*e.g.,* smoking, drinking, diet), other pertinent medical conditions (*e.g.,* high blood pressure, dry skin, other diseases), current medications, allergies, pertinent medical history, current side effects of cancer treatments and other medications, *etc.*

As used herein, the term "liquid biopsy" sample refers to a liquid sample obtained from a subject that includes cell-free DNA. Examples of liquid biopsy samples include, but are not limited to, blood, whole blood, plasma, serum, urine, cerebrospinal fluid, fecal material, saliva, sweat, tears, pleural fluid, pericardial fluid, or peritoneal fluid of the subject. In some embodiments, a liquid biopsy sample is a cell-free sample, *e.g.,* a cell free blood sample. In some embodiments, a liquid biopsy sample is obtained from a subject with cancer. In some embodiments, a liquid biopsy sample is collected from a subject with an unknown cancer status, *e.g.,* for use in determining a cancer status of the subject. Likewise, in some embodiments, a liquid biopsy is collected from a subject with a non-cancerous disorder, *e.g.,* a cardiovascular disease. In some embodiments, a liquid biopsy is collected from a subject with an unknown status for a non-cancerous disorder, *e.g.,* for use in determining a non-cancerous disorder status of the subject.

As used herein, the term "cell-free DNA" and "cfDNA" interchangeably refer to DNA fragments that circulate in a subject's body (*e.g.,* bloodstream) and originate from one or more healthy cells and/or from one or more cancer cells.

These DNA molecules are found outside cells, in bodily fluids such as blood, whole blood, plasma, serum, urine, cerebrospinal fluid, fecal material, saliva, sweat, sweat, tears, pleural fluid, pericardial fluid, or peritoneal fluid of a subject, and are believed to be fragments of genomic DNA expelled from healthy and/or cancerous cells, *e.g.,* upon apoptosis and lysis of the cellular envelope.

As used herein, the term "locus" refers to a position (*e.g.,* a site) within a genome, *e.g.,* on a particular chromosome. In some embodiments, a locus refers to a single nucleotide position, on a particular chromosome, within a genome. In some embodiments, a locus refers to a group of nucleotide positions within a genome. In some instances, a locus is defined by a mutation (*e.g.,* substitution, insertion, deletion, inversion, or translocation) of consecutive nucleotides within a cancer genome. In some instances, a locus is defined by a gene, a sub-genic structure (*e.g.,* a regulatory element, exon, intron, or combination thereof), or a predefined span of a chromosome. Because normal mammalian cells have diploid genomes, a normal mammalian genome (*e.g.,* a human genome) will generally have two copies of every locus in the genome, or at least two copies of every locus located on the autosomal chromosomes, *e.g.,* one copy on the maternal autosomal chromosome and one copy on the paternal autosomal chromosome.

As used herein, the term "allele" refers to a particular sequence of one or more nucleotides at a chromosomal locus. In a haploid organism, the subject has one allele at every chromosomal locus. In a diploid organism, the subject has two alleles at every chromosomal locus.

As used herein, the term "base pair" or "bp" refers to a unit consisting of two nucleobases bound to each other by hydrogen bonds. Generally, the size of an organism's genome is measured in base pairs because DNA is typically double stranded. However, some viruses have single-stranded DNA or RNA genomes.

As used herein, the terms "genomic alteration," "mutation," and "variant" refer to a detectable change in the genetic material of one or more cells. A genomic alteration, mutation, or variant can refer to various type of changes in the genetic material of a cell, including changes in the primary genome sequence at single or multiple nucleotide positions, *e.g.,* a single nucleotide variant (SNV), a multi-nucleotide variant (MNV), an indel (*e.g.,* an insertion or deletion of nucleotides), a DNA rearrangement (*e.g.,* an inversion or translocation of a portion of a chromosome or chromosomes), a variation in the copy number of a locus (*e.g.,* an exon, gene, or a large span of a chromosome) (CNV), a partial or complete change in the ploidy of the cell, as well as in changes in the epigenetic information of a genome, such as altered DNA methylation patterns. In some embodiments, a mutation is a change in the genetic information of the cell relative to a particular reference genome, or one or more 'normal' alleles found in the population of the species of the subject. For instance, mutations can be found in both germline cells (*e.g.,* non-cancerous, 'normal' cells) of a subject and in abnormal cells (*e.g.,* pre-cancerous or cancerous cells) of the subject. As such, a mutation in a germline of the subject (*e.g.,* which is found in substantially all 'normal cells' in the subject) is identified relative to a reference genome for the species of the subject. However, many loci of a reference genome of a species are associated with several variant alleles that are significantly represented in the population of the subject and are not associated with a diseased state, *e.g.,* such that they would not be considered "mutations." By contrast, in some embodiments, a mutation in a cancerous cell of a subject can be identified relative to either a reference genome of the subject or to the subject's own germline genome. In certain instances, identification of both types of variants can be informative. For instance, in some instances, a mutation that is present in both the cancer genome of the subject and the germline of the subject is informative for precision oncology when the mutation is a so-called 'driver mutation," which contributes to the initiation and/or development of a cancer. However, in other instances, a mutation that is present in both the cancer genome of the subject and the germline of the subject is not informative for precision oncology, *e.g.,* when the mutation is a so-called 'passenger mutation," which does not contribute to the initiation and/or development of the cancer. Likewise, in some instances, a mutation that is present in the cancer genome of the subject but not the germline of the subject is informative for precision oncology, *e.g.,* where the mutation is a driver mutation and/or the mutation facilitates a therapeutic approach, *e.g.,* by differentiating cancer cells from normal cells in a therapeutically actionable way. However, in some instances, a mutation that is present in the cancer genome but not the germline of a subject is not informative for precision oncology, *e.g.,* where the mutation is a passenger mutation and/or where the mutation fails to differentiate the cancer cell from a germline cell in a therapeutically actionable way.

As used herein, the term "reference allele" refers to the sequence of one or more nucleotides at a chromosomal locus that is either the predominant allele represented at that chromosomal locus within the population of the species (*e.g.,* the "wild-type" sequence), or an allele that is predefined within a reference genome for the species.

As used herein, the term "variant allele" refers to a sequence of one or more nucleotides at a chromosomal locus that is either not the predominant allele represented at that chromosomal locus within the population of the species (*e.g.,* not the "wild-type" sequence), or not an allele that is predefined within a reference sequence construct (*e.g.,* a reference genome or set of reference genomes) for the species. In some instances, sequence isoforms found within the population of a species that do not affect a change in a protein encoded by the genome, or that result in an amino acid substitution that does not substantially affect the function of an encoded protein, are not variant alleles.

As used herein, the term "variant allele fraction," "VAF," "allelic fraction," or "AF" refers to the number of times a variant or mutant allele was observed (*e.g.,* a number of reads supporting a candidate variant allele) divided by the total number of times the position was sequenced (*e.g.,* a total number of reads covering a candidate locus).

As used herein, the term "germline variants" refers to genetic variants inherited from maternal and paternal DNA. Germline variants may be determined through a matched tumor-normal calling pipeline.

As used herein, the term "somatic variants" refers to variants arising as a result of dysregulated cellular processes associated with neoplastic cells, *e.g.,* a mutation. Somatic variants may be detected via subtraction from a matched normal sample.

As used herein, the term "single nucleotide variant" or "SNV" refers to a substitution of one nucleotide to a different nucleotide at a position (*e.g.*, site) of a nucleotide sequence, *e.g.,* a sequence read from an individual. A substitution from a first nucleobase X to a second nucleobase Y may be denoted as "X>Y." For example, a cytosine to thymine SNV may be denoted as "C>T."

As used herein, the term "insertions and deletions" or "indels" refers to a variant resulting from the gain or loss of DNA base pairs within an analyzed region.

As used herein, the term "copy number variation" or "CNV" refers to the process by which large structural changes in a genome associated with tumor aneuploidy and other dysregulated repair systems are detected. These processes are used to detect large scale insertions or deletions of entire genomic regions. CNV is defined as structural insertions or deletions greater than a certain base pair ("bp") in size, such as 500 bp.

As used herein, the term "gene fusion" refers to the product of large-scale chromosomal aberrations resulting in the creation of a chimeric protein. These expressed products can be non-functional, or they can be highly over or underactive. This can cause deleterious effects in cancer such as hyper-proliferative or anti-apoptotic phenotypes.

As used herein, the term "loss of heterozygosity" refers to the loss of one copy of a segment (*e.g.*, including part or all of one or more genes) of the genome of a diploid subject (*e.g.*, a human) or loss of one copy of a sequence encoding a functional gene product in the genome of the diploid subject, in a tissue, *e.g.*, a cancerous tissue, of the subject. As used herein, when referring to a metric representing loss of heterozygosity across the entire genome of the subject, loss of heterozygosity is caused by the loss of one copy of various segments in the genome of the subject. Loss of heterozygosity across the entire genome may be estimated without sequencing the entire genome of a subject, and such methods for such estimations based on gene panel targeting-based sequencing methodologies are described in the art. Accordingly, in some embodiments, a metric representing loss of heterozygosity across the entire genome of a tissue of a subject is represented as a single value, *e.g.,* a percentage or fraction of the genome. In some cases, a tumor is composed of various sub-clonal populations, each of which may have a different degree of loss of heterozygosity across their respective genomes. Accordingly, in some embodiments, loss of heterozygosity across the entire genome of a cancerous tissue refers to an average loss of heterozygosity across a heterogeneous tumor population. As used herein, when referring to a metric for loss of heterozygosity in a particular gene, *e.g.*, a DNA repair protein such as a protein involved in the homologous DNA recombination pathway (*e.g.*, BRCA1 or BRCA2), loss of heterozygosity refers to complete or partial loss of one copy of the gene encoding the protein in the genome of the tissue and/or a mutation in one copy of the gene that prevents translation of a full-length gene product, *e.g.,* a frameshift or truncating (creating a premature stop codon in the gene) mutation in the gene of interest. In some cases, a tumor is composed of various sub-clonal populations, each of which may have a different mutational status in a gene of interest. Accordingly, in some embodiments, loss of heterozygosity for a particular gene of interest is represented by an average value for loss of heterozygosity for the gene across all sequenced sub-clonal populations of the cancerous tissue. In other embodiments, loss of heterozygosity for a particular gene of interest is represented by a count of the number of unique incidences of loss of heterozygosity in the gene of interest across all sequenced sub-clonal populations of the cancerous tissue (*e.g.,* the number of unique frame-shift and/or truncating mutations in the gene identified in the sequencing data).

As used herein, the term "microsatellites" refers to short, repeated sequences of DNA. The smallest nucleotide repeated unit of a microsatellite is referred to as the "repeated unit" or "repeat unit." In some embodiments, the stability of a microsatellite locus is evaluated by comparing some metric of the distribution of the number of repeated units at a microsatellite locus to a reference number or distribution.

As used herein, the term "microsatellite instability" or "MSI" refers to a genetic hypermutability condition associated with various cancers that results from impaired DNA mismatch repair (MMR) in a subject. Among other phenotypes, MSI causes changes in the size of microsatellite loci, *e.g.,* a change in the number of repeated units at microsatellite loci, during DNA replication. Accordingly, the size of microsatellite repeats is varied in MSI cancers as compared to the size of the corresponding microsatellite repeats in the germline of a cancer subject. The term "Microsatellite Instability-High" or "MSI-H" refers to a state of a cancer (*e.g.*, a tumor) that has a significant MMR defect, resulting in microsatellite loci with significantly different lengths than the corresponding microsatellite loci in normal cells of the same individual. The term "Microsatellite Stable" or "MSS" refers to a state of a cancer (*e.g.*, a tumor) without significant MMR defects, such that there is no significant difference between the lengths of the microsatellite loci in cancerous cells and the lengths of the corresponding microsatellite loci in normal (*e.g.*, non-cancerous) cells in the same individual. The term "Microsatellite Equivocal" or "MSE" refers to a state of a cancer (*e.g.*, a tumor) having an intermediate microsatellite length phenotype, that cannot be clearly classified as MSI-H or MSS based on statistical cutoffs used to define those two categories.

As used herein, the term "gene product" refers to an RNA (*e.g.*, mRNA or miRNA) or protein molecule transcribed or translated from a particular genomic locus, *e.g.,* a particular gene. The genomic locus can be identified using a gene name, a chromosomal location, or any other genetic mapping metric.

As used herein, the terms "expression level," "abundance level," or simply "abundance" refers to an amount of a gene product, (an RNA species, *e.g.*, mRNA or miRNA, or protein molecule) transcribed or translated by a cell, or an average amount of a gene product transcribed or translated across multiple cells. When referring to mRNA or protein expression, the term generally refers to the amount of any RNA or protein species corresponding to a particular genomic locus, *e.g.,* a particular gene. However, in some embodiments, an expression level can refer to the amount of a particular isoform of an mRNA or protein corresponding to a particular gene that gives rise to multiple mRNA or protein isoforms. The genomic locus can be identified using a gene name, a chromosomal location, or any other genetic mapping metric.

As used herein, the term "ratio" refers to any comparison of a first metric X, or a first mathematical transformation thereof X' (*e.g.*, measurement of a number of units of a genomic sequence in a first one or more biological samples or a first mathematical transformation thereof) to another metric Y or a second mathematical transformation thereof Y' (*e.g.,* the number of units of a respective genomic sequence in a second one or more biological samples or a second mathematical transformation thereof) expressed as $X/Y$, $Y/X$, $\log_N(X/Y)$, $\log_N(Y/X)$, $X'/Y$, $Y/X'$, $\log_N(X/Y)$, or $\log_N(Y/X')$, $X/Y'$, $Y'/X$, $\log_N(X/Y')$, $\log_N(Y'/X)$, $X'/Y'$, $Y'/X'$, $\log_N(X'/Y')$, or $\log_N(Y'/X')$, where N is any real number greater than 1 and where example mathematical transformations of X and Y include, but are not limited to. raising X or Y to a power Z, multiplying X or Y by a constant Q, where Z and Q are any real numbers, and/or taking an M based logarithm of X and/or Y, where M is a real number greater than 1. In one non-limiting example, X is transformed to X' prior to ratio calculation by raising X by the power of two ($X^2$) and Y is transformed to Y' prior to ratio calculation by raising Y by the power of 3.2 ($Y^{3.2}$) and the ratio of X and Y is computed as $\log_2(X'/Y')$.

As used herein, the term "relative abundance" refers to a ratio of a first amount of a compound measured in a sample, *e.g.,* a gene product (an RNA species, *e.g.,* mRNA or miRNA, or protein molecule) or nucleic acid fragments having a particular characteristic (*e.g.*, aligning to a particular locus or encompassing a particular allele), to a second amount of a compound measured in a second sample. In some embodiments, relative abundance refers to a ratio of an amount of species of a compound to a total amount of the compound in the same sample. For instance, a ratio of the amount of mRNA transcripts encoding a particular gene in a sample (*e.g.*, aligning to a particular region of the exome) to the total amount of mRNA transcripts in the sample. In other embodiments, relative abundance refers to a ratio of an amount of a compound or species of a compound in a first sample to an amount of the compound of the species of the compound in a second sample. For instance, a ratio of a normalized amount of mRNA transcripts encoding a particular gene in a first sample to a normalized amount of mRNA transcripts encoding the particular gene in a second and/or reference sample.

As used herein, the terms "sequencing," "sequence determination," and the like refer to any biochemical processes that may be used to determine the order of biological macromolecules such as nucleic acids or proteins. For example, sequencing data can include all or a portion of the nucleotide bases in a nucleic acid molecule such as an mRNA transcript or a genomic locus.

As used herein, the term "genetic sequence" refers to a recordation of a series of nucleotides present in a subject's RNA or DNA as determined by sequencing of nucleic acids from the subject.

As used herein, the term "sequence reads" or "reads" refers to nucleotide sequences produced by any nucleic acid sequencing process described herein or known in the art. Reads can be generated from one end of nucleic acid fragments ("single-end reads") or from both ends of nucleic acid fragments (*e.g.*, paired-end reads, double-end reads). The length of the sequence read is often associated with the particular sequencing technology. High-throughput methods, for example, provide sequence reads that can vary in size from tens to hundreds of base pairs (bp). In some embodiments, the sequence reads are of a mean, median or average length of about 15 bp to 900 bp long (*e.g.,* about 20 bp, about 25 bp, about 30 bp, about 35 bp, about 40 bp, about 45 bp, about 50 bp, about 55 bp, about 60 bp, about 65 bp, about 70 bp, about 75 bp, about 80 bp, about 85 bp, about 90 bp, about 95 bp, about 100 bp, about 110 bp, about 120 bp, about 130, about 140 bp, about 150 bp, about 200 bp, about 250 bp, about 300 bp, about 350 bp, about 400 bp, about 450 bp, or about 500 bp. In some embodiments, the sequence reads are of a mean, median or average length of about 1000 bp, 2000 bp, 5000 bp, 10,000 bp, or 50,000 bp or more. Nanopore® sequencing, for example, can provide sequence reads that can vary in size from tens to hundreds to thousands of base pairs. Illumina® parallel sequencing, for example, can provide sequence reads that do not vary as much, for example, most of the sequence reads can be smaller than 200 bp. A sequence read (or sequencing read) can refer to sequence information corresponding to a nucleic acid molecule (*e.g.,* a string of nucleotides). For example, a sequence read can correspond to a string of nucleotides (*e.g.*, about 20 to about 150) from part of a nucleic acid fragment, can correspond to a string of nucleotides at one or both ends of a nucleic acid fragment, or can correspond to nucleotides of the entire nucleic acid fragment. A sequence read can be obtained in a variety of ways, *e.g.*, using sequencing techniques or using probes, *e.g.*, in hybridization arrays or capture probes, or amplification techniques, such as the polymerase chain reaction (PCR) or linear amplification using a single primer or isothermal amplification.

As used herein, the term "read segment" refers to any form of nucleotide sequence read including the raw sequence reads obtained directly from a nucleic acid sequencing technique or from a sequence derived therefrom, *e.g.*, an aligned sequence read, a collapsed sequence read, or a stitched sequence read.

As used herein, the term "read count" refers to the total number of nucleic acid reads generated, which may or may not be equivalent to the number of nucleic acid molecules generated, during a nucleic acid sequencing reaction.

As used herein, the term "read-depth," "sequencing depth," or "depth" can refer to a total number of unique nucleic acid fragments encompassing a particular locus or region of the genome of a subject that are sequenced in a particular sequencing reaction. Sequencing depth can be expressed as "Yx", *e.g.,* 50x, 100x, *etc.,* where "Y" refers to the number of unique nucleic acid fragments encompassing a particular locus that are sequenced in a sequencing reaction. In such a case, Y is necessarily an integer, because it represents the actual sequencing depth for a particular locus. Alternatively, read-depth, sequencing depth, or depth can refer to a measure of central tendency (*e.g.*, a mean or mode) of the number of unique nucleic acid fragments that encompass one of a plurality of loci or regions of the genome of a subject that are sequenced in a particular sequencing reaction. For example, in some embodiments, sequencing depth refers to the average depth of every locus across an arm of a chromosome, a targeted sequencing panel, an exome, or an entire genome. In such case, Y may be expressed as a fraction or a decimal, because it refers to an average coverage across a plurality of loci. When a mean depth is recited, the actual depth for any particular locus may be different than the overall recited depth. Metrics can be determined that provide a range of sequencing depths in which a defined percentage of the total number of loci fall. For instance, a range of sequencing depths within which 90% or 95%, or 99% of the loci fall. As understood by the skilled artisan, different sequencing technologies provide different sequencing depths. For instance, low-pass whole genome sequencing can refer to technologies that provide a sequencing depth of less than 5x, less than 4x, less than 3x, or less than 2x, *e.g.,* from about 0.5x to about 3x.

As used herein, the term "sequencing breadth" refers to what fraction of a particular reference exome (*e.g.*, human reference exome), a particular reference genome (*e.g.*, human reference genome), or part of the exome or genome has been analyzed. Sequencing breadth can be expressed as a fraction, a decimal, or a percentage, and is generally calculated as (the number of loci analyzed / the total number of loci in a reference exome or reference genome). The denominator of the fraction can be a repeat-masked genome, and thus 100% can correspond to all of the reference genome minus the masked parts. A repeat-masked exome or genome can refer to an exome or genome in which sequence repeats are masked (*e.g.*, sequence reads align to unmasked portions of the exome or genome). In some embodiments, any part of an exome or genome can be masked and, thus, sequencing breadth can be evaluated for any desired portion of a reference exome or genome. In some embodiments, "broad sequencing" refers to sequencing/analysis of at least 0.1% of an exome or genome.

As used herein, the terms "sequence ratio" and "coverage ratio" interchangeably refer to any measurement of a number of units of a genomic sequence in a first one or more biological samples (*e.g.,* a test and/or tumor sample) compared to the number of units of the respective genomic sequence in a second one or more biological samples (*e.g.,* a reference and/or control sample). In some embodiments, a sequence ratio is a copy ratio, a $\log_2$-transformed copy ratio (*e.g.,* $\log_2$ copy ratio), a coverage ratio, a base fraction, an allele fraction (*e.g.,* a variant allele fraction), and/or a tumor ploidy. In some embodiments sequence ratio is a $\log_N$-transformed copy ratio, where N is any real number greater than 1.

As used herein, the term "sequencing probe" refers to a molecule that binds to a nucleic acid with affinity that is based on the expected nucleotide sequence of the RNA or DNA present at that locus.

As used herein, the term "targeted panel" or "targeted gene panel" refers to a combination of probes for sequencing (*e.g.*, by next-generation sequencing) nucleic acids present in a biological sample from a subject (*e.g.,* a tumor sample, liquid biopsy sample, germline tissue sample, white blood cell sample, or tumor or tissue organoid sample), selected to map to one or more loci of interest on one or more chromosomes. An example set of loci/genes useful for precision oncology, *e.g.*, via solid or liquid biopsy assay, that can be analyzed using a targeted panel is described in Table 1. Another example set of loci/genes useful for precision oncology, *e.g.*, via solid or liquid biopsy assay, that can be analyzed using a targeted panel is described in Table 2. In some embodiments, in addition to loci that are informative for precision oncology, a targeted panel includes one or more probes for sequencing one or more of a loci associated with a different medical condition, a loci used for internal control purposes, or a loci from a pathogenic organism (*e.g.*, an oncogenic pathogen).

As used herein, the term, "reference exome" refers to any sequenced or otherwise characterized exome, whether partial or complete, of any tissue from any organism or pathogen that may be used to reference identified sequences from a subject. Typically, a reference exome will be derived from a subject of the same species as the subject whose sequences are being evaluated. Example reference exomes used for human subjects as well as many other organisms are provided in the on-line genome browser hosted by the National Center for Biotechnology Information ("NCBI"). An "exome" refers to the complete transcriptional profile of an organism or pathogen, expressed in nucleic acid sequences. As used herein, a reference sequence or reference exome often is an assembled or partially assembled exomic sequence from an individual or multiple individuals. In some embodiments, a reference exome is an assembled or partially assembled exomic sequence from one or more human individuals. The reference exome can be viewed as a representative example of a species' set of expressed genes. In some embodiments, a reference exome comprises sequences assigned to chromosomes.

As used herein, the term "reference genome" refers to any sequenced or otherwise characterized genome, whether partial or complete, of any organism or pathogen that may be used to reference identified sequences from a subject. Typically, a reference genome will be derived from a subject of the same species as the subject whose sequences are being evaluated. Exemplary reference genomes used for human subjects as well as many other organisms are provided in the on-line genome browser hosted by the National Center for Biotechnology Information ("NCBI") or the University of California, Santa Cruz (UCSC). A "genome" refers to the complete genetic information of an organism or pathogen, expressed in nucleic acid sequences. As used herein, a reference sequence or reference genome often is an assembled or partially assembled genomic sequence from an individual or multiple individuals. In some embodiments, a reference genome is an assembled or partially assembled genomic sequence from one or more human individuals. The reference genome can be viewed as a representative example of a species' set of genes. In some embodiments, a reference genome comprises sequences assigned to chromosomes. Exemplary human reference genomes include but are not limited to NCBI build 34 (UCSC equivalent: hg16), NCBI build 35 (UCSC equivalent: hg17), NCBI build 36.1 (UCSC equivalent: hg18), GRCh37 (UCSC equivalent: hg19), and GRCh38 (UCSC equivalent: hg38). For a haploid genome, there can be only one nucleotide at each locus. For a diploid genome, heterozygous loci can be identified; each heterozygous locus can have two alleles, where either allele can allow a match for alignment to the locus.

As used herein, the term "bioinformatics pipeline" refers to a series of processing stages used to determine characteristics of a subject's genome or exome based on sequencing data of the subject's genome or exome. A bioinformatics pipeline may be used to determine characteristics of a germline genome or exome of a subject and/or a cancer genome or exome of a subject. In some embodiments, the pipeline extracts information related to genomic alterations in the cancer genome of a subject, which is useful for guiding clinical decisions for precision oncology, from sequencing results of a biological sample, *e.g.,* a tumor sample, liquid biopsy sample, reference normal sample, *etc.,* from the subject. Certain processing stages in a bioinformatics may be 'connected,' meaning that the results of a first respective processing stage are informative and/or essential for execution of a second, downstream processing stage. For instance, in some embodiments, a bioinformatics pipeline includes a first respective processing stage for identifying genomic alterations that are unique to the cancer genome of a subject and a second respective processing stage that uses the quantity and/or identity of the identified genomic alterations to determine a metric that is informative for precision oncology, *e.g.,* a tumor mutational burden. In some embodiments, the bioinformatics pipeline includes a reporting stage that generates a report of relevant and/or actionable information identified by upstream stages of the pipeline, which may or may not further include recommendations for aiding clinical therapy decisions.

As used herein, the term "limit of detection" or "LOD" refers to the minimal quantity of a feature that can be identified with a particular level of confidence. Accordingly, level of detection can be used to describe an amount of a substance that must be present in order for a particular assay to reliably detect the substance. A level of detection can also be used to describe a level of support needed for an algorithm to reliably identify a genomic alteration based on sequencing data. For example, a minimal number of unique sequence reads to support identification of a sequence variant such as a SNV.

As used herein, the term "BAM File" or "Binary file containing Alignment Maps" refers to a file storing sequencing data aligned to a reference sequence *(e.g.,* a reference genome or exome). In some embodiments, a BAM file is a compressed binary version of a SAM (Sequence Alignment Map) file that includes, for each of a plurality of unique sequence reads, an identifier for the sequence read, information about the nucleotide sequence, information about the alignment of the sequence to a reference sequence, and optionally metrics relating to the quality of the sequence read and/or the quality of the sequence alignment. While BAM files generally relate to files having a particular format, for simplicity they are used herein to simply refer to a file, of any format, containing information about a sequence alignment, unless specifically stated otherwise.

As used herein, the term "measure of central tendency" refers to a central or representative value for a distribution of values. Non-limiting examples of measures of central tendency include an arithmetic mean, weighted mean, midrange, midhinge, trimean, geometric mean, geometric median, Winsorized mean, median, and mode of the distribution of values.

As used herein, the term "Positive Predictive Value" or "PPV" means the likelihood that a variant is properly called given that a variant has been called by an assay. PPV can be expressed as (number of true positives)/ (number of false positives + number of true positives).

As used herein, the term "assay" refers to a technique for determining a property of a substance, *e.g.,* a nucleic acid, a protein, a cell, a tissue, or an organ. An assay *(e.g., a* first assay or a second assay) can comprise a technique for determining the copy number variation of nucleic acids in a sample, the methylation status of nucleic acids in a sample, the fragment size distribution of nucleic acids in a sample, the mutational status of nucleic acids in a sample, or the fragmentation pattern of nucleic acids in a sample. Any assay known to a person having ordinary skill in the art can be used to detect any of the properties of nucleic acids mentioned herein. Properties of a nucleic acids can include a sequence, genomic identity, copy number, methylation state at one or more nucleotide positions, size of the nucleic acid, presence or absence of a mutation in the nucleic acid at one or more nucleotide positions, and pattern of fragmentation of a nucleic acid (*e.g.,* the nucleotide position(s) at which a nucleic acid fragments). An assay or method can have a particular sensitivity and/or specificity, and their relative usefulness as a diagnostic tool can be measured using ROC-AUC statistics.

As used herein, the term "classification" can refer to any number(s) or other characters(s) that are associated with a particular property of a sample. For example, in some embodiments, the term "classification" can refer to a type of cancer in a subject, a stage of cancer in a subject, a prognosis for a cancer in a subject, a tumor load, a presence of tumor metastasis in a subject, and the like. The classification can be binary (*e.g.*, positive or negative) or have more levels of classification (*e.g.*, a scale from 1 to 10 or 0 to 1). The terms "cutoff" and "threshold" can refer to predetermined numbers used in an operation. For example, a cutoff size can refer to a size above which fragments are excluded. A threshold value can be a value above or below which a particular classification applies. Either of these terms can be used in either of these contexts.

As used herein, the term "sensitivity" or "true positive rate" (TPR) refers to the number of true positives divided by the sum of the number of true positives and false negatives. Sensitivity can characterize the ability of an assay or method to correctly identify a proportion of the population that truly has a condition. For example, sensitivity can characterize the ability of a method to correctly identify the number of subjects within a population having cancer. In another example, sensitivity can characterize the ability of a method to correctly identify the one or more markers indicative of cancer.

As used herein, the term "specificity" or "true negative rate" (TNR) refers to the number of true negatives divided by the sum of the number of true negatives and false positives. Specificity can characterize the ability of an assay or method to correctly identify a proportion of the population that truly does not have a condition. For example, specificity can characterize the ability of a method to correctly identify the number of subjects within a population not having cancer. In another example, specificity characterizes the ability of a method to correctly identify one or more markers indicative of cancer.

As used herein, an "actionable genomic alteration" or "actionable variant" refers to a genomic alteration (*e.g.*, a SNV, MNV, indel, rearrangement, copy number variation, or ploidy variation), or value of another cancer metric derived from nucleic acid sequencing data (*e.g.,* a tumor mutational burden, MSI status, or tumor fraction), that is known or believed to be associated with a therapeutic course of action that is more likely to produce a positive effect in a cancer patient that has the actionable variant than in a similarly situated cancer patient that does not have the actionable variant. For instance, administration of EGFR inhibitors (*e.g.*, afatinib, erlotinib, gefitinib) is more effective for treating non-small cell lung cancer in patients with an EGFR mutation in exons 19/21 than for treating non-small cell lung cancer in patients that do not have an EGFR mutations in exons 19/21. Accordingly, an EGFR mutation in exon 19/21 is an actionable variant. In some instances, an actionable variant is only associated with an improved treatment outcome in one or a group of specific cancer types. In other instances, an actionable variant is associated with an improved treatment outcome in substantially all cancer types.

As used herein, a "variant of uncertain significance" or "VUS" refers to a genomic alteration (*e.g.*, a SNV, MNV, indel, rearrangement, copy number variation, or ploidy variation), or value of another cancer metric derived from nucleic acid sequencing data (*e.g.,* a tumor mutational burden, MSI status, or tumor fraction), whose impact on disease development/progression is unknown.

As used herein, a "benign variant" or "likely benign variant" refers to a genomic alteration (*e.g.*, a SNV, MNV, indel, rearrangement, copy number variation, or ploidy variation), or value of another cancer metric derived from nucleic acid sequencing data (*e.g.,* a tumor mutational burden, MSI status, or tumor fraction), that is known or believed to not contribute to disease development/progression.

As used herein, a "pathogenic variant" or "likely pathogenic variant" refers to a genomic alteration (*e.g.*, a SNV, MNV, indel, rearrangement, copy number variation, or ploidy variation), or value of another cancer metric derived from nucleic acid sequencing data (*e.g.,* a tumor mutational burden, MSI status, or tumor fraction), that is known or believed to contribute to disease development/progression.

As used herein, an "effective amount" or "therapeutically effective amount" is an amount sufficient to affect a beneficial or desired clinical result upon treatment. An effective amount can be administered to a subject in one or more doses. In terms of treatment, an effective amount is an amount that is sufficient to palliate, ameliorate, stabilize, reverse or slow the progression of the disease, or otherwise reduce the pathological consequences of the disease. The effective amount is generally determined by the physician on a case-by-case basis and is within the skill of one in the art. Several factors are typically taken into account when determining an appropriate dosage to achieve an effective amount. These factors include age, sex and weight of the subject, the condition being treated, the severity of the condition and the form and effective concentration of the therapeutic agent being administered.

The terminology used in the present disclosure is for the purpose of describing particular embodiments only and is not intended to be limiting of the invention. As used in the description of the invention and the appended claims, the singular forms "a", "an" and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. Furthermore, to the extent that the terms "including," "includes," "having," "has," "with," or variants thereof are used in either the detailed description and/or the claims, such terms are intended to be inclusive in a manner similar to the term "comprising."

As used herein, the term "if" may be construed to mean "when" or "upon" or "in response to determining" or "in response to detecting," depending on the context. Similarly, the phrase "if it is determined" or "if [a stated condition or event] is detected" may be construed to mean "upon determining" or "in response to determining" or "upon detecting [the stated condition or event]" or "in response to detecting [the stated condition or event]," depending on the context.

It will also be understood that, although the terms first, second, *etc.* may be used herein to describe various elements, these elements should not be limited by these terms. These terms are only used to distinguish one element from another. For example, a first subject could be termed a second subject, and, similarly, a second subject could be termed a first subject, without departing from the scope of the present disclosure. The first subject and the second subject are both subjects, but they are not the same subject. Furthermore, the terms "subject," "user," and "patient" are used interchangeably herein.

Reference will now be made in detail to embodiments, examples of which are illustrated in the accompanying drawings. In the following detailed description, numerous specific details are set forth in order to provide a thorough understanding of the present disclosure, including example systems, methods, techniques, instruction sequences, and computing machine program products that embody illustrative implementations. However, the illustrative discussions below are not intended to be exhaustive or to limit the implementations to the precise forms disclosed. Many modifications and variations are possible in view of the above teachings. The features described herein are not limited by the illustrated ordering of acts or events, as some acts can occur in different orders and/or concurrently with other acts or events.

The implementations provided herein are chosen and described in order to best explain the principles and their practical applications, to thereby enable others skilled in the art to best utilize the various embodiments with various modifications as are suited to the particular use contemplated. In some instances, well-known methods, procedures, components, circuits, and networks have not been described in detail so as not to unnecessarily obscure aspects of the embodiments. In other instances, it will be apparent to one of ordinary skill in the art that the present disclosure may be practiced without one or more of the specific details.

It will be appreciated that, in the development of any such actual implementation, numerous implementation-specific decisions are made in order to achieve the designer's specific goals, such as compliance with use case- and business-related constraints, and that these specific goals will vary from one implementation to another and from one designer to another. Moreover, it will be appreciated that though such a design effort might be complex and time-consuming, it will nevertheless be a routine undertaking of engineering for those of ordering skill in the art having the benefit of the present disclosure.

*Example System Embodiments.*

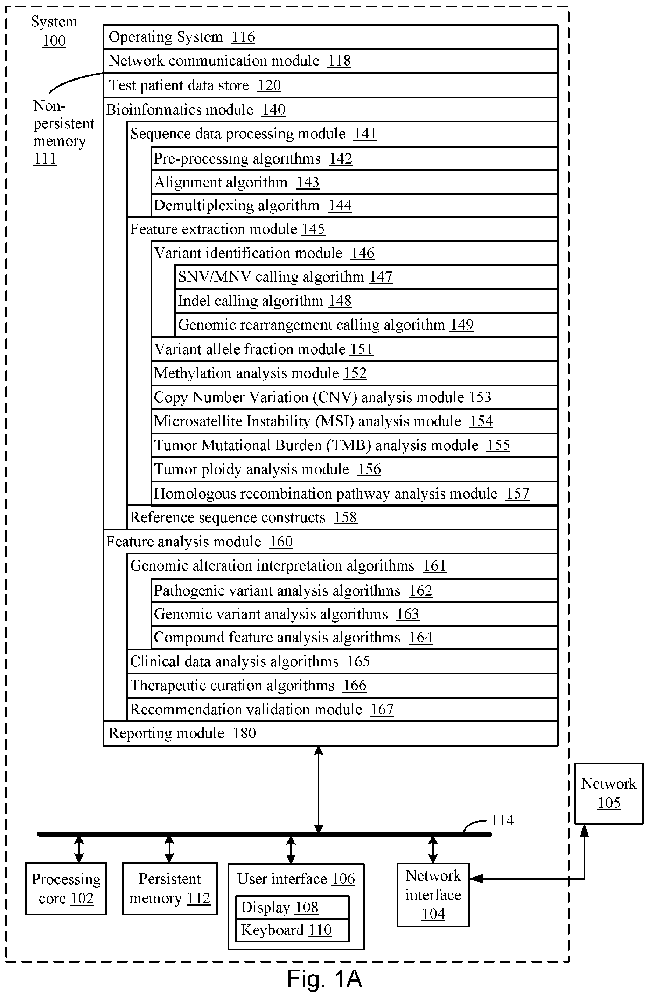

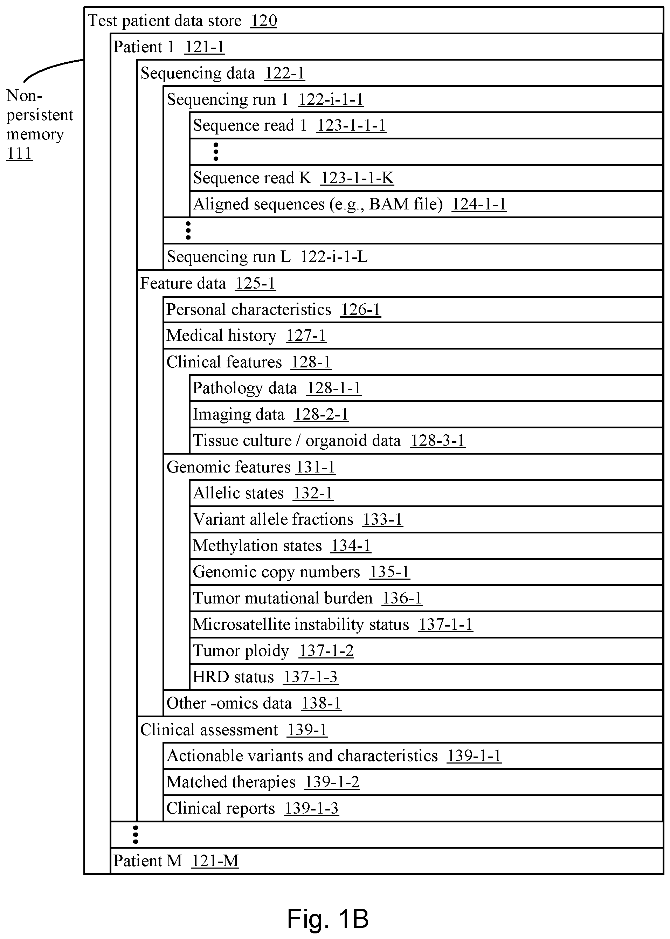

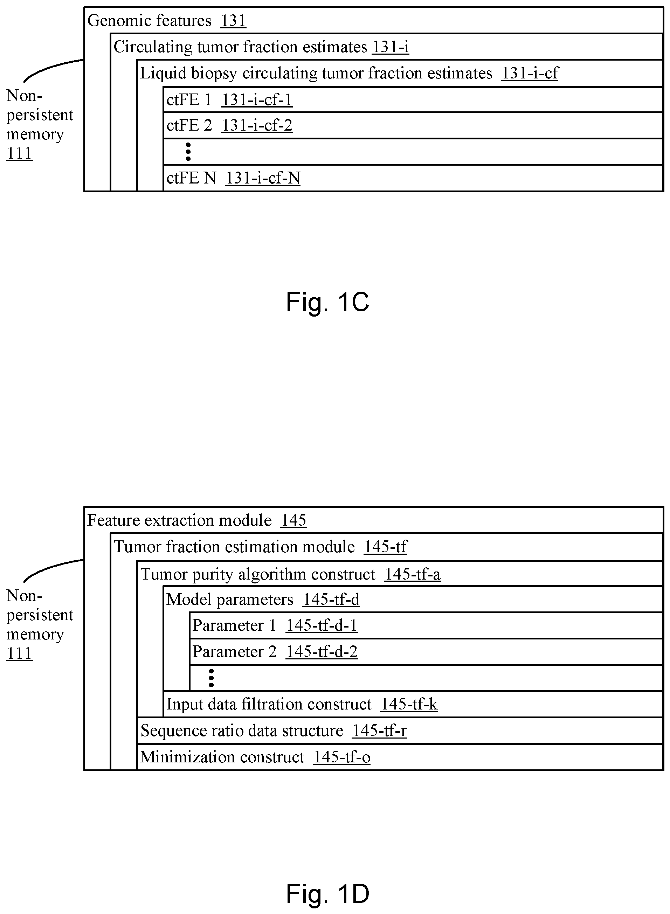

Now that an overview of some aspects of the present disclosure and some definitions used in the present disclosure have been provided, details of an exemplary system for providing clinical support for personalized cancer therapy using a liquid biopsy assay are now described in conjunction with Figures 1A, 1B, 1C, and 1D collectively illustrate the topology of an example system for providing clinical support for personalized cancer therapy using a liquid biopsy assay, in accordance with some embodiments of the present disclosure. Advantageously, the example system illustrated in Figures 1A, 1B, 1C, and 1D improves upon conventional methods for providing clinical support for personalized cancer therapy by determining accurate liquid biopsy tumor mutational burden (ITMB).

Figure 1A is a block diagram illustrating a system in accordance with some implementations. The device 100 in some implementations includes one or more processing units CPU(s) 102 (also referred to as processors), one or more network interfaces 104, a user interface 106, *e.g.,* including a display 108 and/or an input 110 (*e.g.,* a mouse, touchpad, keyboard, *etc.*)*,* a non-persistent memory 111, a persistent memory 112, and one or more communication buses 114 for interconnecting these components. The one or more communication buses 114 optionally include circuitry (sometimes called a chipset) that interconnects and controls communications between system components. The non-persistent memory 111 typically includes high-speed random access memory, such as DRAM, SRAM, DDR RAM, ROM, EEPROM, flash memory, whereas the persistent memory 112 typically includes CD-ROM, digital versatile disks (DVD) or other optical storage, magnetic cassettes, magnetic tape, magnetic disk storage or other magnetic storage devices, magnetic disk storage devices, optical disk storage devices, flash memory devices, or other non-volatile solid state storage devices. The persistent memory 112 optionally includes one or more storage devices remotely located from the CPU(s) 102. The persistent memory 112, and the non-volatile memory device(s) within the non-persistent memory 112, comprise non-transitory computer readable storage medium. In some implementations, the non-persistent memory 111 or alternatively the non-transitory computer readable storage medium stores the following programs, modules and data structures, or a subset thereof, sometimes in conjunction with the persistent memory 112:

- an operating system 116, which includes procedures for handling various basic system services and for performing hardware dependent tasks;
- a network communication module (or instructions) 118 for connecting the system 100 with other devices and/or a communication network 105;
- a test patient data store 120 for storing one or more collections of features from patients (*e.g.*, subjects);
- a bioinformatics module 140 for processing sequencing data and extracting features from sequencing data, *e.g.,* from liquid biopsy sequencing assays;
- a feature analysis module 160 for evaluating patient features, *e.g.*, genomic alterations, compound genomic features, and clinical features; and
- a reporting module 180 for generating and transmitting reports that provide clinical support for personalized cancer therapy.

Although Figures 1A, 1B, 1C, and 1D depict a "system 100," the figures are intended more as a functional description of the various features that may be present in computer systems than as a structural schematic of the implementations described herein. In practice, and as recognized by those of ordinary skill in the art, items shown separately could be combined and some items could be separated. Moreover, although Figure 1 depicts certain data and modules in non-persistent memory 111, some or all of these data and modules may be in persistent memory 112. For example, in various implementations, one or more of the above identified elements are stored in one or more of the previously mentioned memory devices and correspond to a set of instructions for performing a function described above. The above identified modules, data, or programs (*e.g.*, sets of instructions) need not be implemented as separate software programs, procedures, datasets, or modules, and thus various subsets of these modules and data may be combined or otherwise re-arranged in various implementations.

In some implementations, the non-persistent memory 111 optionally stores a subset of the modules and data structures identified above. Furthermore, in some embodiments, the memory stores additional modules and data structures not described above. In some embodiments, one or more of the above-identified elements is stored in a computer system, other than that of system 100, that is addressable by system 100 so that system 100 may retrieve all or a portion of such data when needed.

For purposes of illustration in Figure 1A, system 100 is represented as a single computer that includes all of the functionality for providing clinical support for personalized cancer therapy. However, while a single machine is illustrated, the term "system" shall also be taken to include any collection of machines that individually or jointly execute a set (or multiple sets) of instructions to perform any one or more of the methodologies discussed herein.

For example, in some embodiments, system 100 includes one or more computers. In some embodiments, the functionality for providing clinical support for personalized cancer therapy is spread across any number of networked computers and/or resides on each of several networked computers and/or is hosted on one or more virtual machines at a remote location accessible across the communications network 105. For example, different portions of the various modules and data stores illustrated in Figures 1A, 1B, 1C1, 1D1, 1C2, 1D2, 1E2, 1F2, 1C3, and 1D3 can be stored and/or executed on the various instances of a processing device and/or processing server/database in the distributed diagnostic environment 210 illustrated in Figure 2B (*e.g.*, processing devices 224, 234, 244, and 254, processing server 262, and database 264).

The system may operate in the capacity of a server or a client machine in client-server network environment, as a peer machine in a peer-to-peer (or distributed) network environment, or as a server or a client machine in a cloud computing infrastructure or environment. The system may be a personal computer (PC), a tablet PC, a set-top box (STB), a Personal Digital Assistant (PDA), a cellular telephone, a web appliance, a server, a network router, a switch or bridge, or any machine capable of executing a set of instructions (sequential or otherwise) that specify actions to be taken by that machine.

In another implementation, the system comprises a virtual machine that includes a module for executing instructions for performing any one or more of the methodologies disclosed herein. In computing, a virtual machine (VM) is an emulation of a computer system that is based on computer architectures and provides functionality of a physical computer. Some such implementations may involve specialized hardware, software, or a combination of hardware and software.

One of skill in the art will appreciate that any of a wide array of different computer topologies are used for the application and all such topologies are within the scope of the present disclosure.

*Test Patient Data Store (120).*

Referring to Figure 1B, in some embodiments, the system (*e.g.*, system 100) includes a patient data store 120 that stores data for patients 121-1 to 121-M (*e.g.*, cancer patients or patients being tested for cancer) including one or more sequencing data 122, feature data 125, and clinical assessments 139. These data are used and/or generated by the various processes stored in the bioinformatics module 140 and feature analysis module 160 of system 100, to ultimately generate a report providing clinical support for personalized cancer therapy of a patient. While the feature scope of patient data 121 across all patients may be informationally dense, an individual patient's feature set may be sparsely populated across the entirety of the collective feature scope of all features across all patients. That is to say, the data stored for one patient may include a different set of features that the data stored for another patient. Further, while illustrated as a single data construct in Figure 1B, different sets of patient data may be stored in different databases or modules spread across one or more system memories.

In some embodiments, sequencing data 122 from one or more sequencing reactions 122-i, including a plurality of sequence reads 123-i-1 to 123-i-K, is stored in the test patient data store 120. The data store may include different sets of sequencing data from a single subject, corresponding to different samples from the patient, *e.g.,* a tumor sample, liquid biopsy sample, tumor organoid derived from a patient tumor, and/or a normal sample, and/or to samples acquired at different times, *e.g.*, while monitoring the progression, regression, remission, and/or recurrence of a cancer in a subject. The sequence reads may be in any suitable file format, *e.g.,* BCL, FASTA, FASTQ, *etc.* In some embodiments, sequencing data 122 is accessed by a sequencing data processing module 141, which performs various pre-processing, genome alignment, and demultiplexing operations, as described in detail below with reference to bioinformatics module 140. In some embodiments, sequence data that has been aligned to a reference construct, *e.g.*, BAM file 124, is stored in test patient data store 120.

In some embodiments, the test patient data store 120 includes feature data 125, *e.g.,* that is useful for identifying clinical support for personalized cancer therapy. In some embodiments, the feature data 125 includes personal characteristics 126 of the patient, such as patient name, date of birth, gender, ethnicity, physical address, smoking status, alcohol consumption characteristic, anthropomorphic data, *etc.*

In some embodiments, the feature data 125 includes medical history data 127 for the patient, such as cancer diagnosis information (*e.g.*, date of initial diagnosis, date of metastatic diagnosis, cancer staging, tumor characterization, tissue of origin, previous treatments and outcomes, adverse effects of therapy, therapy group history, clinical trial history, previous and current medications, surgical history, *etc.*), previous or current symptoms, previous or current therapies, previous treatment outcomes, previous disease diagnoses, diabetes status, diagnoses of depression, diagnoses of other physical or mental maladies, and family medical history. In some embodiments, the feature data 125 includes clinical features 128, such as pathology data 128-1, medical imaging data 128-2, and tissue culture and/or tissue organoid culture data 128-3.

In some embodiments, yet other clinical features, such as previous laboratory testing results, are stored in the test patient data store 120. Medical history data 127 and clinical features may be collected from various sources, including at intake directly from the patient, from an electronic medical record (EMR) or electronic health record (EHR) for the patient, or curated from other sources, such as fields from various testing records (*e.g.,* genetic sequencing reports).

In some embodiments, the feature data 125 includes genomic features 131 for the patient. Non-limiting examples of genomic features include allelic states 132 (*e.g.,* the identity of alleles at one or more loci, support for wild type or variant alleles at one or more loci, support for SNVs/MNVs at one or more loci, support for indels at one or more loci, and/or support for gene rearrangements at one or more loci), allelic fractions 133 (*e.g.,* ratios of variant to reference alleles (or vice versa), methylation states 134 (*e.g.,* a distribution of methylation patterns at one or more loci and/or support for aberrant methylation patterns at one or more loci), genomic copy numbers 135 (*e.g.,* a copy number value at one or more loci and/or support for an aberrant (increased or decreased) copy number at one or more loci), tumor mutational burden 136 (*e.g.,* a measure of the number of mutations in the cancer genome of the subject), and microsatellite instability status 137 (*e.g.,* a measure of the repeated unit length at one or more microsatellite loci and/or a classification of the MSI status for the patient's cancer). In some embodiments, one or more of the genomic features 131 are determined by a nucleic acid bioinformatics pipeline, *e.g.,* as described in detail below with reference to Figure 4 (*e.g.,* Figures 4A-E, 4F1, 4F2, and 4F3). In particular, in some embodiments, the feature data 125 include genomic copy numbers 135 (*e.g.,* 135-1 for Patient 1 121-1) variant allele fractions 133, and/or circulating tumor fraction estimates 131-i, as determined using the improved methods for analyzing copy number variations (CNVs) using the copy number variation analysis module 153, validating somatic sequence variants, and/or determining circulating tumor fraction estimates, and as described in further detail below with reference to Figures 1 and 4 (*e.g.,* Figures 1C1, 1D1, 4F1; Figures 1C2, 1D2, and 4F2; and/or Figures 1C3, 1D3, and 4F3). In some embodiments, one or more of the genomic features 131 are obtained from an external testing source, *e.g.,* not connected to the bioinformatics pipeline as described below.

For example, referring to Figure 1C1, the one or more genomic features 131 include genomic copy numbers 135 comprising liquid biopsy genomic copy numbers 135-cf and optional tumor biopsy genomic copy numbers 135-t, in accordance with some embodiments of the present disclosure. In some embodiments, the liquid biopsy genomic copy numbers 135-cf are determined by a nucleic acid bioinformatics pipeline (*e.g.,* as described in detail below with reference to Figures 4A-E and 4F1) using a plurality of sequence reads 123 obtained from a sequencing of cell-free nucleic acids from a liquid biopsy sample. In some embodiments, the liquid biopsy genomic copy numbers comprise plurality of copy number annotations (*e.g.*, 135-cf-1, 135-cf-2,..., 135-cf-N), where each copy number annotation corresponds to a genomic target (*e.g.,* a gene or a region of a genome). In some embodiments, a copy number annotation comprises a qualitative status and/or a quantitative copy number. In some alternative embodiments, the optional tumor biopsy genomic copy numbers 135-t are determined by a nucleic acid bioinformatics pipeline using a plurality of sequence reads 123 obtained from a sequencing of nucleic acids from a tumor (*e.g.*, tissue) biopsy. In some embodiments, the optional tumor biopsy genomic copy numbers comprise a plurality of optional copy number annotations (*e.g.*, 135-1-t-1, 135-1-t-2,..., 135-1-t-O), where each copy number annotation corresponds to a genomic target (*e.g.,* a gene or a region of a genome).

Referring again to Figure 1B, in some embodiments, the feature data 125 further includes data 138 from other -omics fields of study. Non-limiting examples of -omics fields of study that may yield feature data useful for providing clinical support for personalized cancer therapy include transcriptomics, epigenomics, proteomics, metabolomics, metabonomics, microbiomics, lipidomics, glycomics, cellomics, and organoidomics.

In some embodiments, yet other features may include features derived from machine learning approaches, *e.g.*, based at least in part on evaluation of any relevant molecular or clinical features, considered alone or in combination, not limited to those listed above. For instance, in some embodiments, one or more latent features learned from evaluation of cancer patient training datasets improve the diagnostic and prognostic power of the various analysis algorithms in the feature analysis module 160.

The skilled artisan will know of other types of features useful for providing clinical support for personalized cancer therapy. The listing of features above is merely representative and should not be construed to be limiting.

In some embodiments, a test patient data store 120 includes clinical assessment data 139 for patients, *e.g.*, based on the feature data 125 collected for the subject. In some embodiments, the clinical assessment data 139 includes a catalogue of actionable variants and characteristics 139-1 (*e.g.,* genomic alterations and compound metrics based on genomic features known or believed to be targetable by one or more specific cancer therapies), matched therapies 139-2 (*e.g.,* the therapies known or believed to be particularly beneficial for treatment of subjects having actionable variants), and/or clinical reports 139-3 generated for the subject, *e.g.*, based on identified actionable variants and characteristics 139-1 and/or matched therapies 139-2.

In some embodiments, clinical assessment data 139 is generated by analysis of feature data 125 using the various algorithms of feature analysis module 160, as described in further detail below. In some embodiments, clinical assessment data 139 is generated, modified, and/or validated by evaluation of feature data 125 by a clinician, *e.g.*, an oncologist. For instance, in some embodiments, a clinician (*e.g.*, at clinical environment 220) uses feature analysis module 160, or accesses test patient data store 120 directly, to evaluate feature data 125 to make recommendations for personalized cancer treatment of a patient. Similarly, in some embodiments, a clinician (*e.g.*, at clinical environment 220) reviews recommendations determined using feature analysis module 160 and approves, rejects, or modifies the recommendations, *e.g.*, prior to the recommendations being sent to a medical professional treating the cancer patient.

*Bioinformatics Module (140).*

Referring again to Figure 1A, the system (*e.g.*, system 100) includes a bioinformatics module 140 that includes a feature extraction module 145 and optional ancillary data processing constructs, such as a sequence data processing module 141 and/or one or more reference sequence constructs 158 (*e.g.,* a reference genome, exome, or targeted-panel construct that includes reference sequences for a plurality of loci targeted by a sequencing panel).

In some embodiments, bioinformatics module 140 includes a sequence data processing module 141 that includes instructions for processing sequence reads, *e.g.*, raw sequence reads 123 from one or more sequencing reactions 122-i, prior to analysis by the various feature extraction algorithms, as described in detail below. In some embodiments, sequence data processing module 141 includes one or more pre-processing algorithms 142 that prepare the data for analysis. In some embodiments, the pre-processing algorithms 142 include instructions for converting the file format of the sequence reads from the output of the sequencer (*e.g.*, a BCL file format) into a file format compatible with downstream analysis of the sequences (*e.g.*, a FASTQ or FASTA file format). In some embodiments, the pre-processing algorithms 142 include instructions for evaluating the quality of the sequence reads (*e.g.,* by interrogating quality metrics like Phred score, base-calling error probabilities, Quality (Q) scores, and the like) and/or removing sequence reads that do not satisfy a threshold quality (*e.g.,* an inferred base call accuracy of at least 80%, at least 90%, at least 95%, at least 99%, at least 99.5%, at least 99.9%, or higher). In some embodiments, the pre-processing algorithms 142 include instructions for filtering the sequence reads for one or more properties, *e.g.*, removing sequences failing to satisfy a lower or upper size threshold or removing duplicate sequence reads.

In some embodiments, sequence data processing module 141 includes one or more alignment algorithms 143, for aligning pre-processed sequence reads 123 to a reference sequence construct 158, *e.g.,* a reference genome, exome, or targeted-panel construct. Many algorithms for aligning sequencing data to a reference construct are known in the art, for example, BWA, Blat, SHRiMP, LastZ, and MAQ. One example of a sequence read alignment package is the Burrows-Wheeler Alignment tool (BWA), which uses a Burrows-Wheeler Transform (BWT) to align short sequence reads against a large reference construct, allowing for mismatches and gaps. Li and Durbin, Bioinformatics, 25(14):1754-60 (2009), the content of which is incorporated herein by reference, in its entirety, for all purposes. Sequence read alignment packages import raw or pre-processed sequence reads 122, *e.g.,* in BCL, FASTA, or FASTQ file formats, and output aligned sequence reads 124, *e.g.,* in SAM or BAM file formats.

In some embodiments, sequence data processing module 141 includes one or more demultiplexing algorithms 144, for dividing sequence read or sequence alignment files generated from sequencing reactions of pooled nucleic acids into separate sequence read or sequence alignment files, each of which corresponds to a different source of nucleic acids in the nucleic acid sequencing pool. For instance, because of the cost of sequencing reactions, it is common practice to pool nucleic acids from a plurality of samples into a single sequencing reaction. The nucleic acids from each sample are tagged with a sample-specific and/or molecule-specific sequence tag (*e.g.,* a UMI), which is sequenced along with the molecule. In some embodiments, demultiplexing algorithms 144 sort these sequence tags in the sequence read or sequence alignment files to demultiplex the sequencing data into separate files for each of the samples included in the sequencing reaction.

Bioinformatics module 140 includes a feature extraction module 145, which includes instructions for identifying diagnostic features, *e.g.*, genomic features 131, from sequencing data 122 of biological samples from a subject, *e.g.*, one or more of a solid tumor sample, a liquid biopsy sample, or a normal tissue (*e.g.*, control) sample. For instance, in some embodiments, a feature extraction algorithm compares the identity of one or more nucleotides at a locus from the sequencing data 122 to the identity of the nucleotides at that locus in a reference sequence construct (*e.g.*, a reference genome, exome, or targeted-panel construct) to determine whether the subject has a variant at that locus. In some embodiments, a feature extraction algorithm evaluates data other than the raw sequence, to identify a genomic alteration in the subject, *e.g.*, an allelic ratio, a relative copy number, a repeat unit distribution, *etc.*

For instance, in some embodiments, feature extraction module 145 includes one or more variant identification modules that include instructions for various variant calling processes. In some embodiments, variants in the germline of the subject are identified, *e.g.*, using a germline variant identification module 146. In some embodiments, variants in the cancer genome, *e.g.,* somatic variants, are identified, *e.g.,* using a somatic variant identification module 150. While separate germline and somatic variant identification modules are illustrated in Figure 1A, in some embodiments they are integrated into a single module. In some embodiments, the variant identification module includes instructions for identifying one or more of nucleotide variants (*e.g.*, single nucleotide variants (SNV) and multi-nucleotide variants (MNV)) using one or more SNV/MNV calling algorithms (*e.g.*, algorithms 147 and/or 151), indels (*e.g.*, insertions or deletions of nucleotides) using one or more indel calling algorithms (*e.g.*, algorithms 148 and/or 152), and genomic rearrangements (*e.g.*, inversions, translocation, and fusions of nucleotide sequences) using one or more genomic rearrangement calling algorithms (*e.g.*, algorithms 149 and/or 153).

A SNV/MNV algorithm 147 may identify a substitution of a single nucleotide that occurs at a specific position in the genome. For example, at a specific base position, or locus, in the human genome, the C nucleotide may appear in most individuals, but in a minority of individuals, the position is occupied by an A. This means that there is a SNP at this specific position and the two possible nucleotide variations, C or A, are said to be alleles for this position. SNPs underlie differences in human susceptibility to a wide range of diseases (*e.g.*, sickle-cell anemia, β-thalassemia and cystic fibrosis result from SNPs). The severity of illness and the way the body responds to treatments are also manifestations of genetic variations. For example, a single-base mutation in the APOE (apolipoprotein E) gene is associated with a lower risk for Alzheimer's disease. A single-nucleotide variant (SNV) is a variation in a single nucleotide without any limitations of frequency and may arise in somatic cells. A somatic single-nucleotide variation (*e.g.*, caused by cancer) may also be called a single-nucleotide alteration. An MNP (Multiple-nucleotide polymorphisms) module may identify the substitution of consecutive nucleotides at a specific position in the genome.

An indel calling algorithm 148 may identify an insertion or deletion of bases in the genome of an organism classified among small genetic variations. While indels usually measure from 1 to 10 000 base pairs in length, a microindel is defined as an indel that results in a net change of 1 to 50 nucleotides. Indels can be contrasted with a SNP or point mutation. An indel inserts and/or deletes nucleotides from a sequence, while a point mutation is a form of substitution that replaces one of the nucleotides without changing the overall number in the DNA. Indels, being insertions and/or deletions, can be used as genetic markers in natural populations, especially in phylogenetic studies. Indel frequency tends to be markedly lower than that of single nucleotide polymorphisms (SNP), except near highly repetitive regions, including homopolymers and microsatellites.

A genomic rearrangement algorithm 149 may identify hybrid genes formed from two previously separate genes. It can occur as a result of translocation, interstitial deletion, or chromosomal inversion. Gene fusion can play an important role in tumorigenesis. Fusion genes can contribute to tumor formation because fusion genes can produce much more active abnormal protein than non-fusion genes. Often, fusion genes are oncogenes that cause cancer; these include BCR-ABL, TEL-AML1 (ALL with t(12 ; 21)), AML1-ETO (M2 AML with t(8 ; 21)), and TMPRSS2-ERG with an interstitial deletion on chromosome 21, often occurring in prostate cancer. In the case of TMPRSS2-ERG, by disrupting androgen receptor (AR) signaling and inhibiting AR expression by oncogenic ETS transcription factor, the fusion product regulates prostate cancer. Most fusion genes are found from hematological cancers, sarcomas, and prostate cancer. BCAM-AKT2 is a fusion gene that is specific and unique to high-grade serous ovarian cancer. Oncogenic fusion genes may lead to a gene product with a new or different function from the two fusion partners. Alternatively, a proto-oncogene is fused to a strong promoter, and thereby the oncogenic function is set to function by an upregulation caused by the strong promoter of the upstream fusion partner. The latter is common in lymphomas, where oncogenes are juxtaposed to the promoters of the immunoglobulin genes. Oncogenic fusion transcripts may also be caused by trans-splicing or read-through events. Since chromosomal translocations play such a significant role in neoplasia, a specialized database of chromosomal aberrations and gene fusions in cancer has been created. This database is called Mitelman Database of Chromosome Aberrations and Gene Fusions in Cancer.

In some embodiments, feature extraction module 145 includes instructions for identifying one or more complex genomic alterations (*e.g.*, features that incorporate more than a change in the primary sequence of the genome) in the cancer genome of the subject. For instance, in some embodiments, feature extraction module 145 includes modules for identifying one or more of copy number variation (*e.g.*, copy number variation analysis module 153), microsatellite instability status (*e.g.*, microsatellite instability analysis module 154), tumor mutational burden (*e.g.*, tumor mutational burden analysis module 155), tumor ploidy (*e.g.*, tumor ploidy analysis module 156), and homologous recombination pathway deficiencies (*e.g.*, homologous recombination pathway analysis module 157).

For example, referring to Figure 1D, in some embodiments, feature extraction module 145 comprises a tumor fraction estimation module 145-tf. In some embodiments, the tumor fraction estimation module 145-tf comprises a sequence ratio data structure 145-tf-r including a plurality of sequence ratios (*e.g.*, coverage ratios) obtained from a sequencing of a test liquid biopsy sample of a subject. In some embodiments, the sequence ratio data structure 145-tf-r includes the sequence ratios that are used as input to determine tumor fraction estimates for the test liquid biopsy sample. In some embodiments, the tumor fraction estimation module 145-tf also comprises a tumor purity algorithm construct 145-tf-a that executes, for example, a maximum likelihood estimation (*e.g.*, an expectation-maximization algorithm) to calculate an estimate of the circulating tumor fraction. The tumor purity algorithm construct 145-tf-a comprises an optional input data filtration construct 145-tf-k (*e.g.,* for removing one or more inputs passed from the sequence ratio data structure based on a minimum probe threshold or a position on a sex chromosome) and a plurality of model parameters 145-tf-d (*e.g.,* 145-tf-d-1, 145-tf-d-2,...) used for executing the algorithm. In some embodiments, model parameters include expected sequence ratios for a set of copy states at a given tumor purity; a distance (*e.g.*, an error) from a test sequence ratio to the closest expected sequence ratio at the given tumor purity; a minimum distance (*e.g.,* a minimum error) from a test sequence ratio to the closest expected sequence ratio at the given tumor purity (*e.g.,* an assigned test copy state selected from a minimal distance expected copy state); and/or a tumor purity score (*e.g.,* a sum of weighted errors).

In some embodiments, referring to Figure 1C, the tumor fraction estimation module 145-tf is used to obtain one or more circulating tumor fraction estimates 131-i that are included as feature data 125 in a test patient data store 120. For example, in some embodiments, a plurality of circulating tumor fraction estimates is obtained from a test liquid biopsy sample of a subject 131-i-cf(*e.g.*, 131-i-cf-1, 131-i-cf-2..., 131-i-cf-N). In some embodiments, the plurality of circulating tumor fraction estimates is obtained from a single patient at different collection times.

*Feature Analysis Module (160).*

Referring again to Figure 1A, the system (*e.g.*, system 100) includes a feature analysis module 160 that includes one or more genomic alteration interpretation algorithms 161, one or more optional clinical data analysis algorithms 165, an optional therapeutic curation algorithm 165, and an optional recommendation validation module 167. In some embodiments, feature analysis module 160 identifies actionable variants and characteristics 139-1 and corresponding matched therapies 139-2 and/or clinical trials using one or more analysis algorithms (*e.g.*, algorithms 162, 163, 164, and 165) to evaluate feature data 125. The identified actionable variants and characteristics 139-1 and corresponding matched therapies 139-2, which are optionally stored in test patient data store 120, are then curated by feature analysis module 160 to generate a clinical report 139-3, which is optionally validated by a user, *e.g.,* a clinician, before being transmitted to a medical professional, *e.g.,* an oncologist, treating the patient.

In some embodiments, the genomic alteration interpretation algorithms 161 include instructions for evaluating the effect that one or more genomic features 131 of the subject, *e.g.*, as identified by feature extraction module 145, have on the characteristics of the patient's cancer and/or whether one or more targeted cancer therapies may improve the clinical outcome for the patient. For example, in some embodiments, one or more genomic variant analysis algorithms 163 evaluate various genomic features 131 by querying a database, *e.g.*, a look-up-table ("LUT") of actionable genomic alterations, targeted therapies associated with the actionable genomic alterations, and any other conditions that should be met before administering the targeted therapy to a subject having the actionable genomic alteration. For instance, evidence suggests that depatuxizumab mafodotin (an anti-EGFR mAb conjugated to monomethyl auristatin F) has improved efficacy for the treatment of recurrent glioblastomas having EGFR focal amplifications. van den Bent et al., 2017, Cancer Chemother Pharmacol., 80(6):1209-17. Accordingly, the actionable genomic alteration LUT would have an entry for the focal amplification of the EGFR gene indicating that depatuxizumab mafodotin is a targeted therapy for glioblastomas (*e.g.*, recurrent glioblastomas) having a focal gene amplification. In some instances, the LUT may also include counter indications for the associated targeted therapy, *e.g.*, adverse drug interactions or personal characteristics that are counter-indicated for administration of the particular targeted therapy.

In some embodiments, a genomic alteration interpretation algorithm 161 determines whether a particular genomic feature 131 should be reported to a medical professional treating the cancer patient. In some embodiments, genomic features 131 (*e.g.,* genomic alterations and compound features) are reported when there is clinical evidence that the feature significantly impacts the biology of the cancer, impacts the prognosis for the cancer, and/or impacts pharmacogenomics, *e.g.*, by indicating or counter-indicating particular therapeutic approaches. For instance, a genomic alteration interpretation algorithm 161 may classify a particular CNV feature 135 as "Reportable," *e.g.*, meaning that the CNV has been identified as influencing the character of the cancer, the overall disease state, and/or pharmacogenomics, as "Not Reportable," *e.g.*, meaning that the CNV has not been identified as influencing the character of the cancer, the overall disease state, and/or pharmacogenomics, as "No Evidence," *e.g.*, meaning that no evidence exists supporting that the CNV is "Reportable" or "Not Reportable," or as "Conflicting Evidence," *e.g.*, meaning that evidence exists supporting both that the CNV is "Reportable" and that the CNV is "Not Reportable."

In some embodiments, the genomic alteration interpretation algorithms 161 include one or more pathogenic variant analysis algorithms 162, which evaluate various genomic features to identify the presence of an oncogenic pathogen associated with the patient's cancer and/or targeted therapies associated with an oncogenic pathogen infection in the cancer. For instance, RNA expression patterns of some cancers are associated with the presence of an oncogenic pathogen that is helping to drive the cancer. See, for example, U.S. Patent No. 11,043,304, the content of which is hereby incorporated by reference, in its entirety, for all purposes. In some instances, the recommended therapy for the cancer is different when the cancer is associated with the oncogenic pathogen infection than when it is not. Accordingly, in some embodiments, *e.g.*, where feature data 125 includes RNA abundance data for the cancer of the patient, one or more pathogenic variant analysis algorithms 162 evaluate the RNA abundance data for the patient's cancer to determine whether a signature exists in the data that indicates the presence of the oncogenic pathogen in the cancer. Similarly, in some embodiments, bioinformatics module 140 includes an algorithm that searches for the presence of pathogenic nucleic acid sequences in sequencing data 122. See, for example, U.S. Patent Application No. 17/800,492, entitled "SYSTEMS AND METHODS FOR DETECTING VIRAL DNA FROM SEQUENCING," filed August 17, 2022, the content of which is hereby incorporated by reference, in its entirety, for all purposes. Accordingly, in some embodiments, one or more pathogenic variant analysis algorithms 162 evaluates whether the presence of an oncogenic pathogen in a subject is associated with an actionable therapy for the infection. In some embodiments, system 100 queries a database, *e.g.*, a look-up-table ("LUT"), of actionable oncogenic pathogen infections, targeted therapies associated with the actionable infections, and any other conditions that should be met before administering the targeted therapy to a subject that is infected with the oncogenic pathogen. In some instances, the LUT may also include counter indications for the associated targeted therapy, *e.g.*, adverse drug interactions or personal characteristics that are counter-indicated for administration of the particular targeted therapy.

In some embodiments, the genomic alteration interpretation algorithms 161 include one or more multi-feature analysis algorithms 164 that evaluate a plurality of features to classify a cancer with respect to the effects of one or more targeted therapies. For instance, in some embodiments, feature analysis module 160 includes one or more classifiers trained against feature data, one or more clinical therapies, and their associated clinical outcomes for a plurality of training subjects to classify cancers based on their predicted clinical outcomes following one or more therapies.

In some embodiments, the classifier is implemented as an artificial intelligence engine and may include gradient boosting models, random forest models, neural networks (NN), regression models, Naive Bayes models, and/or machine learning algorithms (MLA). An MLA or a NN may be trained from a training data set that includes one or more features 125, including personal characteristics 126, medical history 127, clinical features 128, genomic features 131, and/or other -omic features 138. MLAs include supervised algorithms (such as algorithms where the features/classifications in the data set are annotated) using linear regression, logistic regression, decision trees, classification and regression trees, naive Bayes, nearest neighbor clustering; unsupervised algorithms (such as algorithms where no features/classification in the data set are annotated) using Apriori, means clustering, principal component analysis, random forest, adaptive boosting; and semi-supervised algorithms (such as algorithms where an incomplete number of features/classifications in the data set are annotated) using generative approach (such as a mixture of Gaussian distributions, mixture of multinomial distributions, hidden Markov models), low density separation, graph-based approaches (such as mincut, harmonic function, manifold regularization), heuristic approaches, or support vector machines.

NNs include conditional random fields, convolutional neural networks, attention based neural networks, deep learning, long short term memory networks, or other neural models where the training data set includes a plurality of tumor samples, RNA expression data for each sample, and pathology reports covering imaging data for each sample.

While MLA and neural networks identify distinct approaches to machine learning, the terms may be used interchangeably herein. Thus, a mention of MLA may include a corresponding NN or a mention of NN may include a corresponding MLA unless explicitly stated otherwise. Training may include providing optimized datasets, labeling these traits as they occur in patient records, and training the MLA to predict or classify based on new inputs. Artificial NNs are efficient computing models which have shown their strengths in solving hard problems in artificial intelligence. They have also been shown to be universal approximators, that is, they can represent a wide variety of functions when given appropriate parameters.

In some embodiments, system 100 includes a classifier training module that includes instructions for training one or more untrained or partially trained classifiers based on feature data from a training dataset. In some embodiments, system 100 also includes a database of training data for use in training the one or more classifiers. In other embodiments, the classifier training module accesses a remote storage device hosting training data. In some embodiments, the training data includes a set of training features, including but not limited to, various types of the feature data 125 illustrated in Figure 1B. In some embodiments, the classifier training module uses patient data 121, *e.g.,* when test patient data store 120 also stores a record of treatments administered to the patient and patient outcomes following therapy.

In some embodiments, feature analysis module 160 includes one or more clinical data analysis algorithms 165, which evaluate clinical features 128 of a cancer to identify targeted therapies which may benefit the subject. For example, in some embodiments, *e.g.*, where feature data 125 includes pathology data 128-1, one or more clinical data analysis algorithms 165 evaluate the data to determine whether an actionable therapy is indicated based on the histopathology of a tumor biopsy from the subject, *e.g.*, which is indicative of a particular cancer type and/or stage of cancer. In some embodiments, system 100 queries a database, *e.g.,* a look-up-table ("LUT"), of actionable clinical features (*e.g.,* pathology features), targeted therapies associated with the actionable features, and any other conditions that should be met before administering the targeted therapy to a subject associated with the actionable clinical features 128 (*e.g.*, pathology features 128-1). In some embodiments, system 100 evaluates the clinical features 128 (*e.g.*, pathology features 128-1) directly to determine whether the patient's cancer is sensitive to a particular therapeutic agent. Further details on example methods, systems, and algorithms for classifying cancer and identifying targeted therapies based on clinical data, such as pathology data 128-1, imaging data 138-2, and/or tissue culture/organoid data 128-3 are discussed, for example, in U.S. Patent Nos. 10,957,041; 10,957,445, 11,244,763; 11,848,107; and 11,145,416, the contents of which are hereby incorporated by reference, in their entireties, for all purposes.

In some embodiments, feature analysis module 160 includes a clinical trials module that evaluates test patient data 121 to determine whether the patient is eligible for inclusion in a clinical trial for a cancer therapy, *e.g.,* a clinical trial that is currently recruiting patients, a clinical trial that has not yet begun recruiting patients, and/or an ongoing clinical trial that may recruit additional patients in the future. In some embodiments, a clinical trial module evaluates test patient data 121 to determine whether the results of a clinical trial are relevant for the patient, *e.g.,* the results of an ongoing clinical trial and/or the results of a completed clinical trial. For instance, in some embodiments, system 100 queries a database, *e.g.,* a look-up-table ("LUT") of clinical trials, *e.g.,* active and/or completed clinical trials, and compares patient data 121 with inclusion criteria for the clinical trials, stored in the database, to identify clinical trials with inclusion criteria that closely match and/or exactly match the patient's data 121. In some embodiments, a record of matching clinical trials, *e.g.,* those clinical trials that the patient may be eligible for and/or that may inform personalized treatment decisions for the patient, are stored in clinical assessment database 139.

In some embodiments, feature analysis module 160 includes a therapeutic curation algorithm 166 that assembles actionable variants and characteristics 139-1, matched therapies 139-2, and/or relevant clinical trials identified for the patient, as described above. In some embodiments, a therapeutic curation algorithm 166 evaluates certain criteria related to which actionable variants and characteristics 139-1, matched therapies 139-2, and/or relevant clinical trials should be reported and/or whether certain matched therapies, considered alone or in combination, may be counter-indicated for the patient, *e.g.*, based on personal characteristics 126 of the patient and/or known drug-drug interactions. In some embodiments, the therapeutic curation algorithm then generates one or more clinical reports 139-3 for the patient. In some embodiments, the therapeutic curation algorithm generates a first clinical report 139-3-1 that is to be reported to a medical professional treating the patient and a second clinical report 139-3-2 that will not be communicated to the medical professional, but may be used to improve various algorithms within the system.

In some embodiments, feature analysis module 160 includes a recommendation validation module 167 that includes an interface allowing a clinician to review, modify, and approve a clinical report 139-3 prior to the report being sent to a medical professional, *e.g.*, an oncologist, treating the patient.

In some embodiments, each of the one or more feature collections, sequencing modules, bioinformatics modules (including, *e.g.*, alteration module(s), structural variant calling and data processing modules), classification modules and outcome modules are communicatively coupled to a data bus to transfer data between each module for processing and/or storage. In some alternative embodiments, each of the feature collection, alteration module(s), structural variant and feature store are communicatively coupled to each other for independent communication without sharing the data bus.

Further details on systems and exemplary embodiments of modules and feature collections are discussed in U.S. Patent No. 11,830,587 which is hereby incorporated herein by reference in its entirety.

*Example Methods.*

Now that details of a system 100 for providing clinical support for personalized cancer therapy, e.g., with improved determination of liquid biopsy tumor mutational burden, have been disclosed, details regarding processes and features of the system, in accordance with various embodiments of the present disclosure, are disclosed below. Specifically, example processes are described below with reference to Figures 3, 4, and 9 *(e.g.,* Figures 3, 4A-E, and 9A-9E). In some embodiments, such processes and features of the system are carried out by modules 118, 120, 140, 160, and/or 170, as illustrated in Figure 1A. Referring to these methods, the systems described herein (*e.g.*, system 100) include instructions for determining a liquid biopsy tumor mutational burden that are improved compared to conventional methods for determining a liquid biopsy tumor mutational burden.

*Figure 2B: Distributed Diagnostic and Clinical Environment.*

In some aspects, the methods described herein for providing clinical support for personalized cancer therapy are performed across a distributed diagnostic/clinical environment, e.g., as illustrated in Figure 2B. However, in some embodiments, the improved methods described herein for supporting clinical decisions in precision oncology using liquid biopsy assays (*e.g.*, by determining a liquid biopsy tumor mutational burden) are performed at a single location, *e.g.,* at a single computing system or environment, although ancillary procedures supporting the methods described herein, and/or procedures that make further use of the results of the methods described herein, may be performed across a distributed diagnostic/clinical environment.

Figure 2B illustrates an example of a distributed diagnostic/clinical environment 210. In some embodiments, the distributed diagnostic/clinical environment is connected via communication network 105. In some embodiments, one or more biological samples, e.g., one or more liquid biopsy samples, solid tumor biopsy, normal tissue samples, and/or control samples, are collected from a subject in clinical environment 220, *e.g.,* a doctor's office, hospital, or medical clinic, or at a home health care environment (not depicted). Advantageously, while solid tumor samples should be collected within a clinical setting, liquid biopsy samples can be acquired in a less invasive fashion and are more easily collected outside of a traditional clinical setting. In some embodiments, one or more biological samples, or portions thereof, are processed within the clinical environment 220 where collection occurred, using a processing device 224, *e.g.,* a nucleic acid sequencer for obtaining sequencing data, a microscope for obtaining pathology data, a mass spectrometer for obtaining proteomic data, *etc.* In some embodiments, one or more biological samples, or portions thereof are sent to one or more external environments, e.g., sequencing lab 230, pathology lab 240, and/or molecular biology lab 250, each of which includes a processing device 234, 244, and 254, respectively, to generate biological data 121 for the subject. Each environment includes a communications device 222, 232, 242, and 252, respectively, for communicating biological data 121 about the subject to a processing server 262 and/or database 264, which may be located in yet another environment, e.g., processing/storage center 260. Thus, in some embodiments, different portions of the systems and methods described herein are fulfilled by different processing devices located in different physical environments.

Accordingly, in some embodiments, a method for providing clinical support for personalized cancer therapy, e.g., with improved determination of liquid biopsy tumor mutational burden, is performed across one or more environments, as illustrated in Figure 2B. For instance, in some such embodiments, a liquid biopsy sample is collected at clinical environment 220 or in a home healthcare environment. The sample, or a portion thereof, is sent to sequencing lab 230 where raw sequence reads 123 of nucleic acids in the sample are generated by sequencer 234. The raw sequencing data 123 is communicated, e.g., from communications device 232, to database 264 at processing/storage center 260, where processing server 262 extracts features from the sequence reads by executing one or more of the processes in bioinformatics module 140, thereby generating genomic features 131 for the sample. Processing server 262 may then analyze the identified features by executing one or more of the processes in feature analysis module 160, thereby generating clinical assessment 139, including a clinical report 139-3. A clinician may access clinical report 139-3, *e.g.,* at processing/storage center 260 or through communications network 105, via recommendation validation module 167. After final approval, clinical report 139-3 is transmitted to a medical professional, e.g., an oncologist, at clinical environment 220, who uses the report to support clinical decision making for personalized treatment of the patient's cancer.

*Figure 2A: Example Workflow for Precision Oncology.*

Figure 2A is a flowchart of an example workflow 200 for collecting and analyzing data in order to generate a clinical report 139 to support clinical decision making in precision oncology. Advantageously, the methods described herein improve this process, for example, by improving various stages within feature extraction 206, including determining a liquid biopsy tumor mutational burden.

Briefly, the workflow begins with patient intake and sample collection 201, where one or more liquid biopsy samples, one or more tumor biopsy, and one or more normal and/or control tissue samples are collected from the patient *(e.g.,* at a clinical environment 220 or home healthcare environment, as illustrated in Figure 2B). In some embodiments, personal data 126 corresponding to the patient and a record of the one or more biological samples obtained *(e.g.,* patient identifiers, patient clinical data, sample type, sample identifiers, cancer conditions, *etc.)* are entered into a data analysis platform, *e.g.,* test patient data store 120. Accordingly, in some embodiments, the methods disclosed herein include obtaining one or more biological samples from one or more subjects, e.g., cancer patients. In some embodiments, the subject is a human, *e.g.,* a human cancer patient.

Sequence reads are then generated (312) from the sequencing library or pool of sequencing libraries. Sequencing data may be acquired by any methodology known in the art. For example, next generation sequencing (NGS) techniques such as sequencing-by-synthesis technology (Illumina), pyrosequencing (454 Life Sciences), ion semiconductor technology (Ion Torrent sequencing), single-molecule real-time sequencing (Pacific Biosciences), sequencing by ligation (SOLiD sequencing), nanopore sequencing (Oxford Nanopore Technologies), or paired-end sequencing. In some embodiments, massively parallel sequencing is performed using sequencing-by-synthesis with reversible dye terminators. In some embodiments, sequencing is performed using next generation sequencing technologies, such as short-read technologies. In other embodiments, long-read sequencing or another sequencing method known in the art is used.

Referring again to Figure 2A, nucleic acid sequencing data 122 generated from the one or more patient samples is then evaluated *(e.g.,* via variant analysis 206) in a bioinformatics pipeline, e.g., using bioinformatics module 140 of system 100, to identify genomic alterations and other metrics in the cancer genome of the patient. An example overview for a bioinformatics pipeline is described below with respect to Figure 4 *(e.g.,* Figure 4A-E, 4F1-3, and/or 4G1-3). Advantageously, in some embodiments, the present disclosure improves bioinformatics pipelines, like pipeline 206, by improving methods and systems for the validation of copy number variations, the validation of somatic sequence variants, and/or the determination of circulating tumor fraction estimates.

Figure 4A illustrates an example bioinformatics pipeline 206 *(e.g.,* as used for feature extraction in the workflows illustrated in Figures 2A and 3) for providing clinical support for precision oncology. As shown in Figure 4A, sequencing data 122 obtained from the wet lab processing 204 *(e.g.,* sequence reads 314) is input into the pipeline.

Figure 4A illustrates an example bioinformatics pipeline 206 *(e.g.,* as used for feature extraction in the workflows illustrated in Figures 2A and 3) for providing clinical support for precision oncology. As shown in Figure 4A, sequencing data 122 obtained from the wet lab processing 204 *(e.g.,* sequence reads 314) is input into the pipeline.

In various embodiments, the bioinformatics pipeline includes a circulating tumor DNA (ctDNA) pipeline for analyzing liquid biopsy samples. The pipeline may detect SNVs, INDELs, copy number amplifications/deletions and genomic rearrangements (for example, fusions). The pipeline may employ unique molecular index (UMI)-based consensus base calling as a method of error suppression as well as a Bayesian tri-nucleotide context-based position level error suppression. In various embodiments, it is able to detect variants having a 0.1%, 0.15%, 0.2%, 0.25%, 0.3%, 0.4%, or 0.5% variant allele fraction.

*Homologous Recombination Status (HRD).*

In some embodiments, analysis of aligned sequence reads, *e.g.,* in SAM or BAM format, includes analysis of whether the cancer is homologous recombination deficient (HRD status 137-3), using a homologous recombination pathway analysis module 157.

Homologous recombination (HR) is a normal, highly conserved DNA repair process that enables the exchange of genetic information between identical or closely related DNA molecules. It is most widely used by cells to accurately repair harmful breaks (e.g. damage) that occur on both strands of DNA. DNA damage may occur from exogenous (external) sources like UV light, radiation, or chemical damage; or from endogenous (internal) sources like errors in DNA replication or other cellular processes that create DNA damage. Double strand breaks are a type of DNA damage. Using poly (ADP-ribose) polymerase (PARP) inhibitors in patients with HRD compromises two pathways of DNA repair, resulting in cell death (apoptosis). The efficacy of PARP inhibitors is improved not only in ovarian cancers displaying germline or somatic BRCA mutations, but also in cancers in which HRD is caused by other underlying etiologies.

In some embodiments, HRD status can be determined by inputting features correlated with HRD status into a classifier trained to distinguish between cancers with homologous recombination pathway deficiencies and cancers without homologous recombination pathway deficiencies. For example, in some embodiments, the features include one or more of (i) a heterozygosity status for a first plurality of DNA damage repair genes in the genome of the cancerous tissue of the subject, (ii) a measure of the loss of heterozygosity across the genome of the cancerous tissue of the subject, (iii) a measure of variant alleles detected in a second plurality of DNA damage repair genes in the genome of the cancerous tissue of the subject, and (iv) a measure of variant alleles detected in the second plurality of DNA damage repair genes in the genome of the non-cancerous tissue of the subject. In some embodiments, all four of the features described above are used as features in an HRD classifier. More details about HRD classifiers using these and other features are described in U.S. Patent No. 10,975,445, the content of which is hereby incorporated by reference, in its entirety, for all purposes.

*Concurrent Testing*

Unless stated otherwise, as used herein, the term "concurrent" as it relates to assays refers to a period of time between zero and ninety days. In some embodiments, concurrent tests using different biological samples from the same subject (*e.g.*, two or more of a liquid biopsy sample, cancerous tissue-such as a solid tumor sample or blood sample for a blood-based cancer-and a non-cancerous sample) are performed within a period of time (*e.g.*, the biological samples are collected within the period of time) of from 0 days to 90 days. In some embodiments, concurrent tests using different biological samples from the same subject (*e.g.*, two or more of a liquid biopsy sample, cancerous tissue-such as a solid tumor sample or blood sample for a blood-based cancer-and a non-cancerous sample) are performed within a period of time (*e.g.,* the biological samples are collected within the period of time) of from 0 days to 60 days. In some embodiments, concurrent tests using different biological samples from the same subject (*e.g.*, two or more of a liquid biopsy sample, cancerous tissue-such as a solid tumor sample or blood sample for a blood-based cancer-and a non-cancerous sample) are performed within a period of time (*e.g.,* the biological samples are collected within the period of time) of from 0 days to 30 days. In some embodiments, concurrent tests using different biological samples from the same subject (*e.g.*, two or more of a liquid biopsy sample, cancerous tissue-such as a solid tumor sample or blood sample for a blood-based cancer-and a non-cancerous sample) are performed within a period of time (*e.g.,* the biological samples are collected within the period of time) of from 0 days to 21 days. In some embodiments, concurrent tests using different biological samples from the same subject (*e.g.*, two or more of a liquid biopsy sample, cancerous tissue-such as a solid tumor sample or blood sample for a blood-based cancer-and a non-cancerous sample) are performed within a period of time (*e.g.,* the biological samples are collected within the period of time) of from 0 days to 14 days. In some embodiments, concurrent tests using different biological samples from the same subject (*e.g.*, two or more of a liquid biopsy sample, cancerous tissue-such as a solid tumor sample or blood sample for a blood-based cancer-and a non-cancerous sample) are performed within a period of time (*e.g.,* the biological samples are collected within the period of time) of from 0 days to 7 days. In some embodiments, concurrent tests using different biological samples from the same subject (*e.g.*, two or more of a liquid biopsy sample, cancerous tissue-such as a solid tumor sample or blood sample for a blood-based cancer-and a non-cancerous sample) are performed within a period of time (*e.g.,* the biological samples are collected within the period of time) of from 0 days to 3 days.

In some embodiments, a liquid biopsy assay may be used concurrently with a solid tumor assay to return more comprehensive information about a patient's variants. For example, a blood specimen and a solid tumor specimen may be sent to a laboratory for evaluation. The solid tumor specimen may be analyzed using a bioinformatics pipeline to produce a solid tumor result. A solid tumor assay is described, for instance, in U.S. Patent No. 11,705,226, the content of which is hereby incorporated by reference, in its entirety, for all purposes. The cancer type of the solid tumor may include, for example, non small cell lung cancer, colorectal cancer, or breast cancer. Alterations identified in the tumor/matched normal result may include, for example, EGFR+ for non small cell lung cancer; HER2+ for breast cancer; or KRAS G12C for several cancers.

In some embodiments, a blood specimen may be divided into a first portion and a second portion. The first portion of the blood specimen and the solid tumor specimen may be analyzed using a bioinformatics pipeline to produce a tumor/matched normal result. The second portion of the blood specimen may be analyzed using a bioinformatics pipeline to produce a liquid biopsy result. For example, the blood specimen may be analyzed using at least an improvement in somatic variant identification, *e.g.,* as described herein in the section entitled "Variant Identification." For example, the blood specimen may be analyzed using an improvement in focal copy number identification, *e.g.,* as described herein in the section entitled "Copy Number Variation." For example, the blood specimen may be analyzed using an improvement in circulating tumor fraction determination, *e.g.,* as described above in the section entitled "Systems and Methods for Improved Circulating Tumor Fraction Estimates" and/or "Systems and Methods for Improved Validation of Somatic Sequence Variants."

Therapies may be identified for further consideration in response to receiving the tumor or tumor/matched normal result along with the liquid biopsy result. For example, if the results overall indicate that the patient has HER2+ breast cancer, neratinib may be identified along with the test results for further consideration by the ordering clinician.

The solid tumor or tumor/matched normal assay may be ordered concurrently; their results may be delivered concurrently; and they may be analyzed concurrently.

*Methods for Improved Determination of Tumor mutational Burden,*

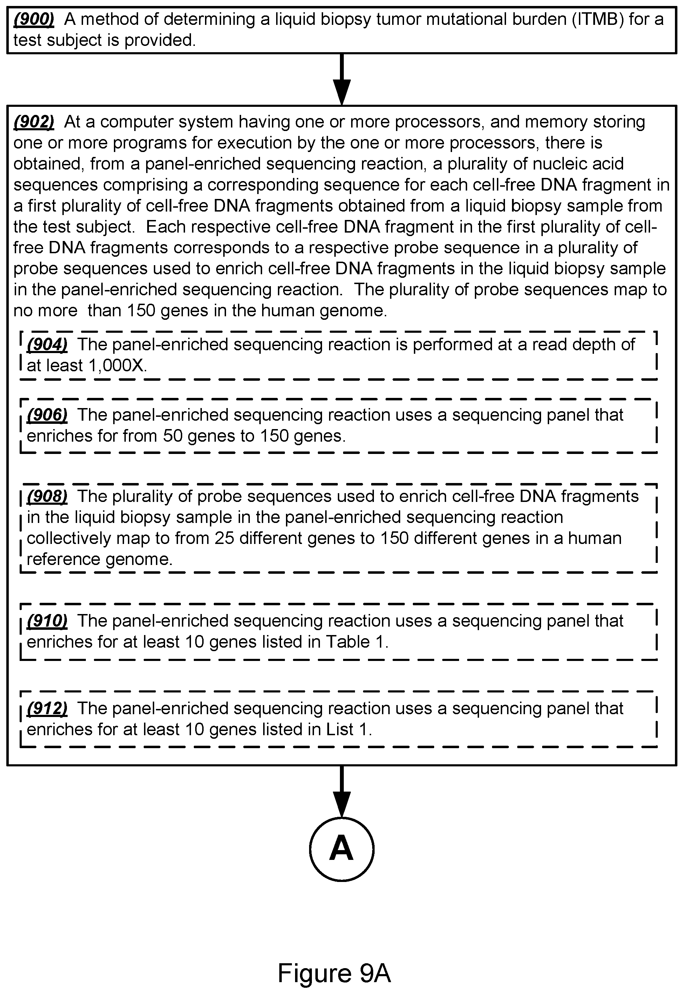

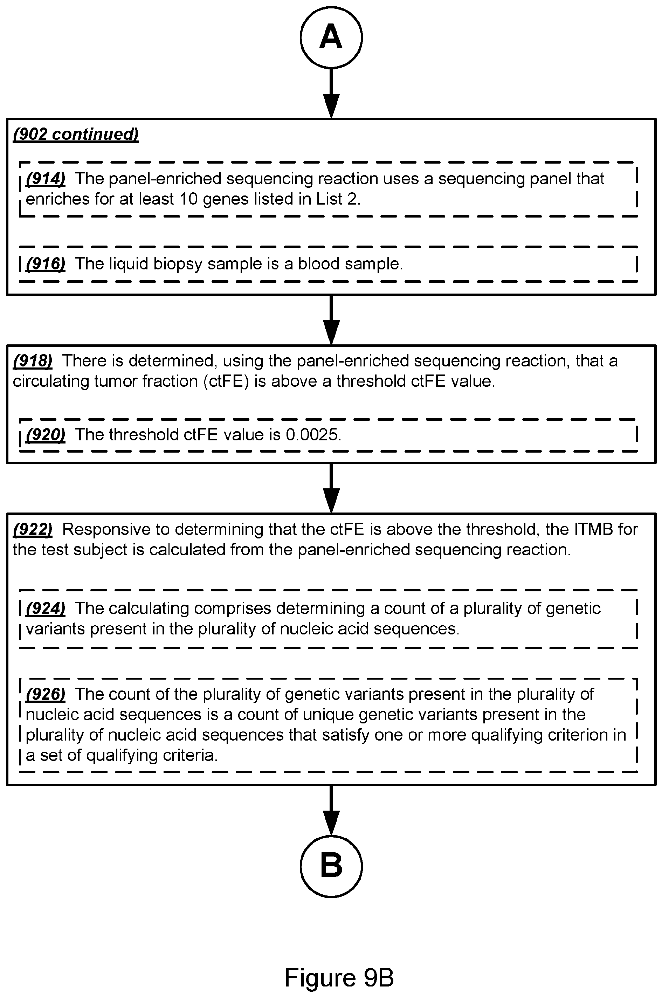

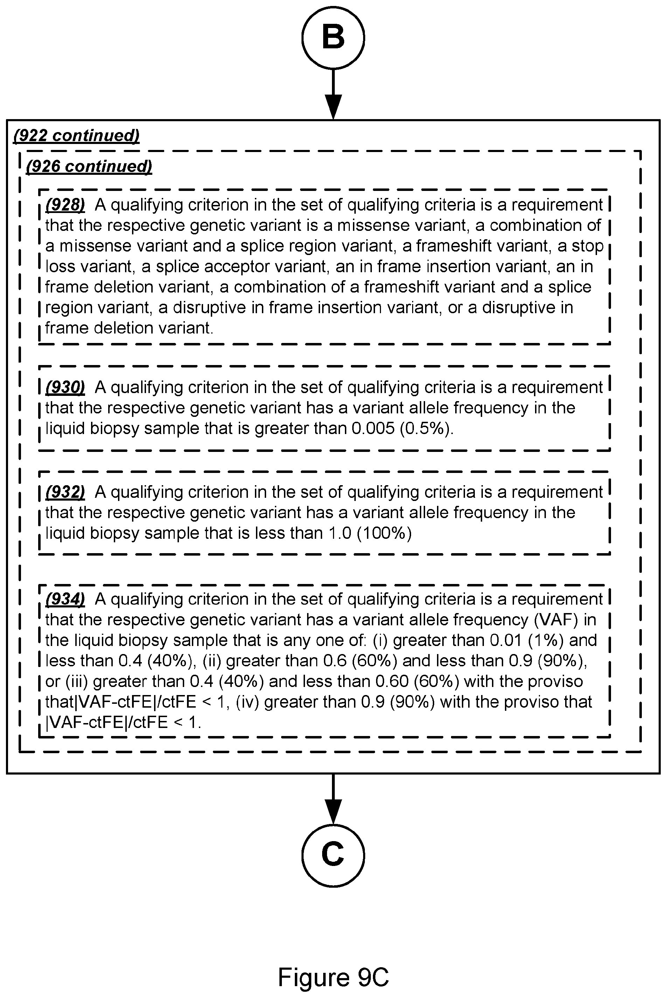

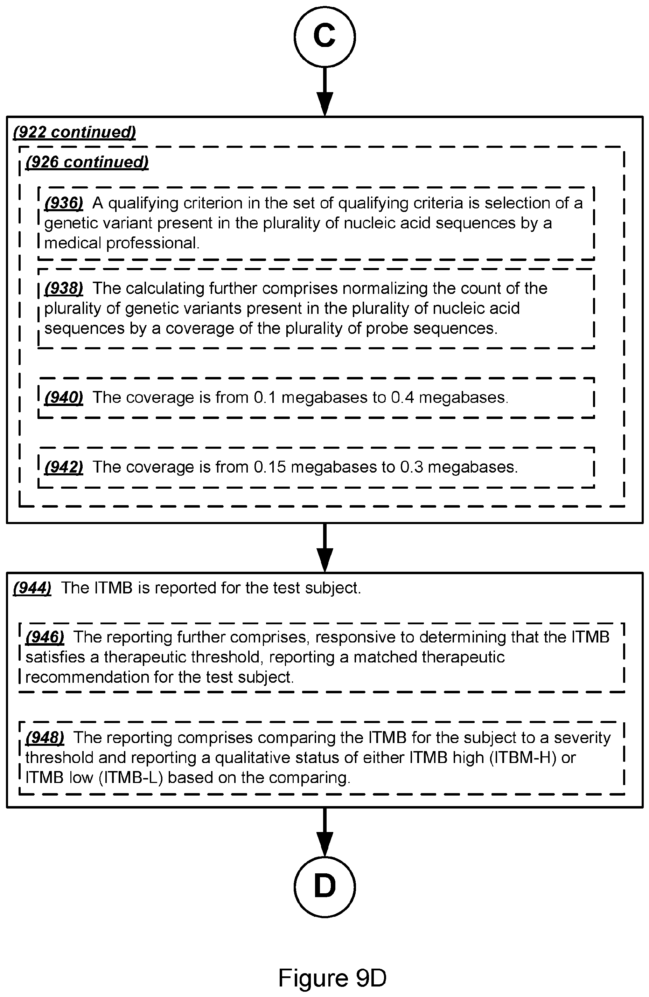

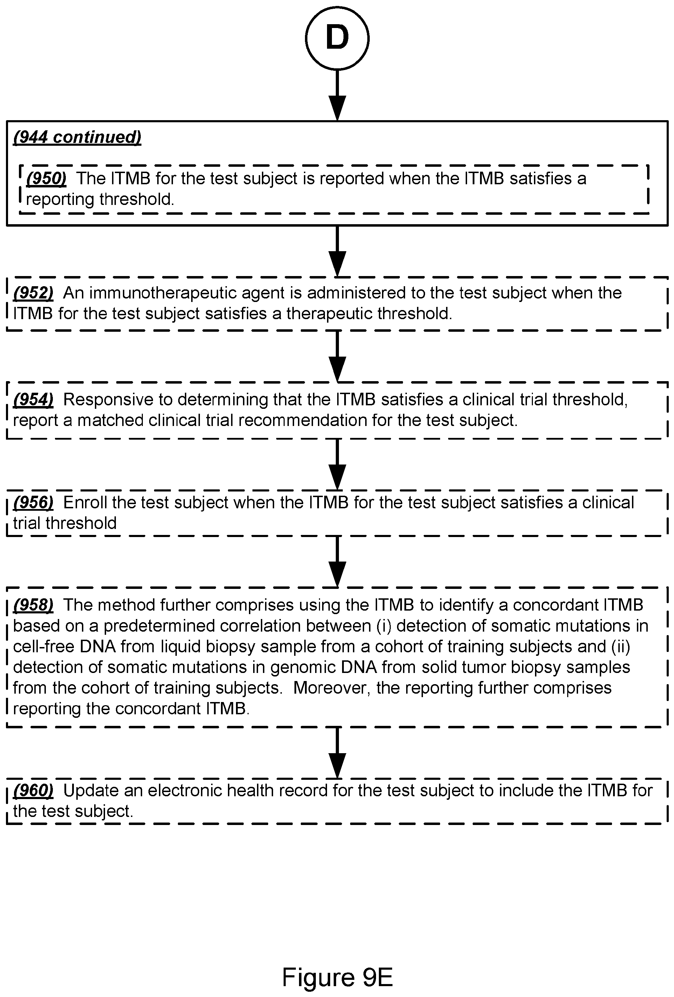

An overview of methods for providing clinical support for personalized cancer therapy is described above with reference to Figures 2-4 above. Below, systems and methods for improving determination of tumor mutational burden in a test subject, *e.g.,* within the context of the methods and systems described above, are described with reference to Figure 9.

Many of the embodiments described below, in conjunction with Figure 9, relate to analyses performed using sequencing data for cfDNA obtained from a liquid biopsy sample of a subject, *e.g.,* a cancer patient. Generally, these embodiments are independent and, thus, not reliant upon any particular DNA sequencing methods. However, in some embodiments, the methods described below include generating the sequencing data.

As described herein, in some embodiments, the methods described herein *(e.g.,* method 900 as illustrated in Figure 9) include one or more data collection steps, in addition to data analysis and downstream steps. For example, as described herein, *e.g.,* with reference to Figures 2 and 3, in some embodiments, the methods include collection of a liquid biopsy sample and, optionally, one or more matching biological samples from the subject *(e.g.,* a matched cancerous and/or matched non-cancerous sample from the subject). Likewise, as described herein, *e.g.,* with reference to Figures 2 and 3, in some embodiments, the methods include extraction of DNA from the liquid biopsy sample (cfDNA) and, optionally, one or more matching biological samples from the subject *(e.g.,* a matched cancerous and/or matched non-cancerous sample from the subject). Similarly, as herein, *e.g.,* with reference to Figures 2 and 3, in some embodiments, the methods include nucleic acid sequencing of DNA from the liquid biopsy (cfDNA) sample and, optionally, one or more matching biological samples from the subject *(e.g.,* a matched cancerous and/or matched non-cancerous sample from the subject). Advantageously, the methods and systems described herein allow for accurate classification of variant lineage as either somatic or hematopoietic based on sequencing data from only cfDNA fragments. Accordingly, in some embodiments, a matched cancerous and/or matched non-cancerous sample from the subject is not used in the methods described herein.

However, in other embodiments, the methods described herein begin with obtaining nucleic acid sequencing results, *e.g.,* raw or collapsed sequence reads of DNA from a liquid biopsy sample (cfDNA) and, optionally, one or more matching biological samples from the subject *(e.g.,* a matched cancerous and/or matched non-cancerous sample from the subject), from which the genomic features needed for detecting clonal hematopoiesis variants and/or solid tumor variants can be determined. For example, in some embodiments, sequencing data 122 for a patient 121 is accessed and/or downloaded over network 105 by system 100.

In some embodiments, the method further comprises isolating the plurality of cell-free nucleic acids from the liquid biopsy sample of the test subject prior to the sequencing. In some embodiments, the sequencing is multiplexed sequencing. In some embodiments, the sequencing is short-read sequencing or long-read sequencing.

Similarly, in some embodiments, the methods described herein begin with obtaining the genomic features needed for filtering of clonal hematopoiesis variants from a sequencing of a liquid biopsy sample and, optionally, one or more matching biological samples from the subject *(e.g.,* a matched cancerous and/or matched non-cancerous sample from the subject). For example, in some embodiments, (i) one or more fragment length metrics, (ii) a variant allele fraction for the candidate somatic variant and a ctFE for the liquid biopsy sample or one or more features determined from the variant allele fraction for the candidate somatic variant and the ctFE for the liquid biopsy sample, and (iii) one or more metrics of clonal hematopoiesis prevalence for the first nucleotide position, is accessed and/or downloaded over network 105 by system 100.

Figures 9A-9E collectively provide a flow chart of processes and features for using determining a liquid biopsy tumor mutational burden (ITMB) for a test subject cell-free DNA from a liquid biopsy assay of the test subject (block 902), in accordance with some embodiments of the present disclosure.

*Block 902.* Referring to block 902 in some embodiments, the method includes obtaining a corresponding nucleic acid sequence of each cell-free DNA (cfDNA) fragment in a plurality of DNA fragments (*e.g.*, cfDNA fragments), from a plurality of sequence reads of a sequencing reaction of the plurality of DNA fragments from one or more biological samples from a subject.

In some embodiments, the plurality of sequence reads is from a panel-enriched sequencing reaction that includes a first subset of sequence reads corresponding to cfDNA fragments targeted by one or more probes in a targeted enrichment panel. In some embodiments, each respective cell-free DNA fragment in the first plurality of cell-free DNA fragments corresponds to a respective probe sequence in a plurality of probe sequences used to enrich cell-free DNA fragments in the liquid biopsy sample in the panel-enriched sequencing reaction. In some embodiments, the plurality of probe sequences map to no more than 150 genes in the human genome.

With reference to Figure 2B, nucleic acid sequencing of one or more samples collected from the subject is performed, *e.g.,* at sequencing lab 230, during wet lab processing 204. An example workflow for nucleic acid sequencing is illustrated in Figure 3. In some embodiments, the one or more biological samples obtained at the sequencing lab 230 are accessioned (302), to track the sample and data through the sequencing process.

Next, nucleic acids, *e.g.,* RNA and/or DNA are extracted (304) from the one or more biological samples. Methods for isolating nucleic acids from biological samples are known in the art, and are dependent upon the type of nucleic acid being isolated *(e.g.,* cfDNA, DNA, and/or RNA) and the type of sample from which the nucleic acids are being isolated *(e.g.,* liquid biopsy samples, white blood cell buffy coat preparations, formalin-fixed paraffin-embedded (FFPE) solid tissue samples, and fresh frozen solid tissue samples). The selection of any particular nucleic acid isolation technique for use in conjunction with the embodiments described herein is well within the skill of the person having ordinary skill in the art, who will consider the sample type, the state of the sample, the type of nucleic acid being sequenced and the sequencing technology being used.

For instance, many techniques for DNA isolation, *e.g.,* genomic DNA isolation, from a tissue sample are known in the art, such as organic extraction, silica adsorption, and anion exchange chromatography. Likewise, many techniques for RNA isolation, *e.g.,* mRNA isolation, from a tissue sample are known in the art. For example, acid guanidinium thiocyanate-phenol-chloroform extraction (see, for example, Chomczynski and Sacchi, 2006, Nat Protoc, 1(2):581-85, which is hereby incorporated by reference herein), and silica bead/glass fiber adsorption (see, for example, Poeckh et al., 2008, Anal Biochem., 373(2):253-62, which is hereby incorporated by reference herein). The selection of any particular DNA or RNA isolation technique for use in conjunction with the embodiments described herein is well within the skill of the person having ordinary skill in the art, who will consider the tissue type, the state of the tissue, *e.g.,* fresh, frozen, formalin-fixed, paraffin-embedded (FFPE), and the type of nucleic acid analysis that is to be performed.

In some embodiments where the biological sample is a liquid biopsy sample, *e.g.,* a blood or blood plasma sample, and cfDNA is isolated from blood samples using commercially available reagents, including proteinase K, to generate a liquid solution of cfDNA.

In some embodiments, isolated DNA molecules are mechanically sheared to an average length using an ultrasonicator (for example, a Covaris ultrasonicator). In some embodiments, isolated nucleic acid molecules are analyzed to determine their fragment size, *e.g.,* through gel electrophoresis techniques and/or the use of a device such as a LabChip GX Touch. The skilled artisan will know of an appropriate range of fragment sizes, based on the sequencing technique being employed, as different sequencing techniques have differing fragment size requirements for robust sequencing. In some embodiments, quality control testing is performed on the extracted nucleic acids (*e.g.,* DNA and/or RNA), *e.g.,* to assess the nucleic acid concentration and/or fragment size. For example, sizing of DNA fragments provides valuable information used for downstream processing, such as determining whether DNA fragments require additional shearing prior to sequencing.

Wet lab processing 204 then includes preparing a nucleic acid library from the isolated nucleic acids (*e.g.,* cfDNA, DNA, and/or RNA). For example, in some embodiments, DNA libraries (*e.g.,* gDNA and/or cfDNA libraries) are prepared from isolated DNA from the one or more biological samples. In some embodiments, the DNA libraries are prepared using a commercial library preparation kit, *e.g.,* the KAPA Hyper Prep Kit, a New England Biolabs (NEB) kit, or a similar kit.

In some embodiments, during library preparation, adapters (*e.g.,* UDI adapters, such as Roche SeqCap dual end adapters, or UMI adapters such as full length or stubby Y adapters) are ligated onto the nucleic acid molecules. In some embodiments, the adapters include unique molecular identifiers (UMIs), which are short nucleic acid sequences (*e.g., 3-10* base pairs) that are added to ends of DNA fragments during adapter ligation. In some embodiments, UMIs are degenerate base pairs that serve as a unique tag that can be used to identify sequence reads originating from a specific DNA fragment. In some embodiments, *e.g.,* when multiplex sequencing will be used to sequence DNA from a plurality of samples (*e.g.,* from the same or different subjects) in a single sequencing reaction, a patient-specific index is also added to the nucleic acid molecules. In some embodiments, the patient specific index is a short nucleic acid sequence (*e.g.,* 3-20 nucleotides) that are added to ends of DNA fragments during library construction, that serve as a unique tag that can be used to identify sequence reads originating from a specific patient sample. Examples of identifier sequences are described, for example, in Kivioja et al., 2011, Nat. Methods 9(1):72-74 and Islam et al., 2014, Nat. Methods 11(2):163-66, the contents of which are hereby incorporated by reference, in their entireties, for all purposes.

In some embodiments, an adapter includes a PCR primer landing site, designed for efficient binding of a PCR or second-strand synthesis primer used during the sequencing reaction. In some embodiments, an adapter includes an anchor binding site, to facilitate binding of the DNA molecule to anchor oligonucleotide molecules on a sequencer flow cell, serving as a seed for the sequencing process by providing a starting point for the sequencing reaction. During PCR amplification following adapter ligation, the UMIs, patient indexes, and binding sites are replicated along with the attached DNA fragment. This provides a way to identify sequence reads that came from the same original fragment in downstream analysis.

In some embodiments, DNA libraries are amplified and purified using commercial reagents, (e.g., Axygen MAG PCR clean up beads). In some such embodiments, the concentration and/or quantity of the DNA molecules are then quantified using a fluorescent dye and a fluorescence microplate reader, standard spectrofluorometer, or filter fluorometer. In some embodiments, library amplification is performed on a device (*e.g.,* an Illumina C-Bot2) and the resulting flow cell containing amplified target-captured DNA libraries is sequenced on a next generation sequencer (*e.g.,* an Illumina HiSeq 4000 or an Illumina NovaSeq 6000) to a unique on-target depth selected by the user. In some embodiments, DNA library preparation is performed with an automated system, using a liquid handling robot (*e.g.,* a SciClone NGSx).

In some embodiments, where feature data 125 includes methylation states 132 for one or more genomic locations, nucleic acids isolated from the biological sample (*e.g.,* cfDNA) are treated to convert unmethylated cytosines to uracils, *e.g.,* prior to generating the sequencing library. Accordingly, when the nucleic acids are sequenced, all cytosines called in the sequencing reaction were necessarily methylated, since the unmethylated cytosines were converted to uracils and accordingly would have been called as thymidines, rather than cytosines, in the sequencing reaction. Commercial kits are available for bisulfite-mediated conversion of methylated cytosines to uracils, for instance, the EZ DNA Methylation™-Gold, EZ DNA Methylation™-Direct, and EZ DNA Methylation™-Lightning kit (available from Zymo Research Corp (Irvine, CA)). Commercial kits are also available for enzymatic conversion of methylated cytosines to uracils, for example, the APOBEC-Seq kit (available from NEBiolabs, Ipswich, MA).

In some embodiments, wet lab processing 204 includes pooling (308) DNA molecules from a plurality of libraries, corresponding to different samples from the same and/or different patients, to forming a sequencing pool of DNA libraries. When the pool of DNA libraries is sequenced, the resulting sequence reads correspond to nucleic acids isolated from multiple samples. The sequence reads can be separated into different sequence read files, corresponding to the various samples represented in the sequencing read based on the unique identifiers present in the added nucleic acid fragments. In this fashion, a single sequencing reaction can generate sequence reads from multiple samples. Advantageously, this allows for the processing of more samples per sequencing reaction.

In some embodiments, wet lab processing 204 includes enriching (310) a sequencing library, or pool of sequencing libraries, for target nucleic acids, e.g., nucleic acids encompassing loci that are informative for precision oncology and/or used as internal controls for the sequencing or bioinformatics processes. In some embodiments, enrichment is achieved by hybridizing target nucleic acids in the sequencing library to probes that hybridize to the target sequences, and then isolating the captured nucleic acids away from off-target nucleic acids that are not bound by the capture probes. Of course, some off-target nucleic acids will remain in the final sequencing pool.

In some embodiments, the plurality of sequence reads that is obtained from the above described sequencing includes at least 10,000 sequence reads, at least 50,000 sequence reads, at least 100,000 sequence reads, at least 500,000 sequence reads, at least 1 million sequence reads, at least 5 million sequence reads, at least 10 million sequence reads, or more. In some embodiments, the plurality of sequence reads includes no more than 1 billion sequence reads, no more than 500 million sequence reads, no more than 100 million sequence reads, no more than 50 million sequence reads, no more than 10 million sequence reads, no more than 5 million sequence reads, no more than 1 million sequence reads, or less. In some embodiments, the plurality of sequence reads is from 10,000 sequence reads to 1 billion sequence reads, from 10,000 sequence reads to 500 million sequence reads, from 10,000 sequence reads to 100 million sequence reads, from 10,000 sequence reads to 50 million sequence reads, from 10,000 sequence reads to 10 million sequence reads, from 10,000 sequence reads to 5 million sequence reads, or from 10,000 sequence reads to 1 million sequence reads. In some embodiments, the plurality of sequence reads is from 100,000 sequence reads to 1 billion sequence reads, from 100,000 sequence reads to 500 million sequence reads, from 100,000 sequence reads to 100 million sequence reads, from 100,000 sequence reads to 50 million sequence reads, from 100,000 sequence reads to 10 million sequence reads, from 100,000 sequence reads to 5 million sequence reads, or from 100,000 sequence reads to 1 million sequence reads. In some embodiments, the plurality of sequence reads is from 500,000 sequence reads to 1 billion sequence reads, from 500,000 sequence reads to 500 million sequence reads, from 500,000 sequence reads to 100 million sequence reads, from 500,000 sequence reads to 50 million sequence reads, from 500,000 sequence reads to 10 million sequence reads, from 500,000 sequence reads to 5 million sequence reads, or from 500,000 sequence reads to 1 million sequence reads. In some embodiments, the plurality of sequence reads is from 1 million sequence reads to 1 billion sequence reads, from 1 million sequence reads to 500 million sequence reads, from 1 million sequence reads to 100 million sequence reads, from 1 million sequence reads to 50 million sequence reads, from 1 million sequence reads to 10 million sequence reads, or from 1 million sequence reads to 5 million sequence reads.

In some embodiments, the plurality of DNA *(e.g.,* cfDNA) fragments includes at least 1000 DNA *(e.g.,* cfDNA) fragments, at least 5000 DNA *(e.g.,* cfDNA) fragments, at least 10,000 DNA *(e.g.,* cfDNA) fragments, at least 50,000 DNA *(e.g.,* cfDNA) fragments, at least 100,000 DNA *(e.g.,* cfDNA) fragments, at least 500,000 DNA *(e.g.,* cfDNA) fragments, at least 1 million DNA *(e.g.,* cfDNA) fragments, at least 5 million DNA *(e.g.,* cfDNA) fragments, or more. In some embodiments, the plurality of DNA *(e.g.,* cfDNA) fragments includes no more than no more than 100 million DNA *(e.g.,* cfDNA) fragments, no more than 50 million DNA *(e.g.,* cfDNA) fragments, no more than 10 million DNA *(e.g.,* cfDNA) fragments, no more than 5 million DNA *(e.g.,* cfDNA) fragments, no more than 1 million DNA *(e.g.,* cfDNA) fragments, no more than 500,000 DNA *(e.g.,* cfDNA) fragments, no more than 100,000 DNA (e.g., cfDNA) fragments or less. In some embodiments, the plurality of DNA *(e.g.,* cfDNA) fragments is from 1000 DNA *(e.g.,* cfDNA) fragments to 500 million DNA *(e.g.,* cfDNA) fragments, from 1000 DNA *(e.g.,* cfDNA) fragments to 100 million DNA *(e.g.,* cfDNA) fragments, from 1000 DNA *(e.g.,* cfDNA) fragments to 50 million DNA *(e.g.,* cfDNA) fragments, from 1000 DNA *(e.g.,* cfDNA) fragments to 10 million DNA *(e.g.,* cfDNA) fragments, from 1000 DNA *(e.g.,* cfDNA) fragments to 5 million DNA *(e.g.,* cfDNA) fragments, from 1000 DNA *(e.g.,* cfDNA) fragments to 1 million DNA *(e.g.,* cfDNA) fragments, from 1000 DNA *(e.g.,* cfDNA) fragments to 500,000 DNA *(e.g.,* cfDNA) fragments, from 1000 DNA *(e.g.,* cfDNA) fragments to 250,000 DNA *(e.g.,* cfDNA) fragments, or from 1000 DNA *(e.g.,* cfDNA) fragments to 100,000 DNA *(e.g.,* cfDNA) fragments. In some embodiments, the plurality of DNA (e.g., cfDNA) fragments is from 5000 DNA *(e.g.,* cfDNA) fragments to 500 million DNA *(e.g.,* cfDNA) fragments, from 5000 DNA *(e.g.,* cfDNA) fragments to 100 million DNA *(e.g.,* cfDNA) fragments, from 5000 DNA *(e.g.,* cfDNA) fragments to 50 million DNA *(e.g.,* cfDNA) fragments, from 5000 DNA *(e.g.,* cfDNA) fragments to 10 million DNA *(e.g.,* cfDNA) fragments, from 5000 DNA *(e.g.,* cfDNA) fragments to 5 million DNA *(e.g.,* cfDNA) fragments, from 5000 DNA *(e.g.,* cfDNA) fragments to 1 million DNA *(e.g.,* cfDNA) fragments, from 5000 DNA *(e.g.,* cfDNA) fragments to 500,000 DNA *(e.g.,* cfDNA) fragments, from 5000 DNA *(e.g.,* cfDNA) fragments to 250,000 DNA *(e.g.,* cfDNA) fragments, or from 5000 DNA *(e.g.,* cfDNA) fragments to 100,000 DNA *(e.g.,* cfDNA) fragments. In some embodiments, the plurality of DNA *(e.g.,* cfDNA) fragments is from 10,000 DNA *(e.g.,* cfDNA) fragments to 500 million DNA *(e.g.,* cfDNA) fragments, from 10,000 DNA *(e.g.,* cfDNA) fragments to 100 million DNA *(e.g.,* cfDNA) fragments, from 10,000 DNA *(e.g.,* cfDNA) fragments to 50 million DNA *(e.g.,* cfDNA) fragments, from 10,000 DNA *(e.g.,* cfDNA) fragments to 10 million DNA *(e.g.,* cfDNA) fragments, from 10,000 DNA *(e.g.,* cfDNA) fragments to 5 million DNA *(e.g.,* cfDNA) fragments, from 10,000 DNA *(e.g.,* cfDNA) fragments to 1 million DNA *(e.g.,* cfDNA) fragments, from 10,000 DNA *(e.g.,* cfDNA) fragments to 500,000 DNA *(e.g.,* cfDNA) fragments, from 10,000 DNA *(e.g.,* cfDNA) fragments to 250,000 DNA *(e.g.,* cfDNA) fragments, or from 10,000 DNA *(e.g.,* cfDNA) fragments to 100,000 DNA *(e.g.,* cfDNA) fragments. In some embodiments, the plurality of DNA *(e.g.,* cfDNA) fragments is from 25,000 DNA *(e.g.,* cfDNA) fragments to 500 million DNA *(e.g.,* cfDNA) fragments, from 25,000 DNA *(e.g.,* cfDNA) fragments to 100 million DNA *(e.g.,* cfDNA) fragments, from 25,000 DNA *(e.g.,* cfDNA) fragments to 50 million DNA *(e.g.,* cfDNA) fragments, from 25,000 DNA *(e.g.,* cfDNA) fragments to 10 million DNA *(e.g.,* cfDNA) fragments, from 25,000 DNA *(e.g.,* cfDNA) fragments to 5 million DNA *(e.g.,* cfDNA) fragments, from 25,000 DNA *(e.g.,* cfDNA) fragments to 1 million DNA *(e.g.,* cfDNA) fragments, from 25,000 DNA *(e.g.,* cfDNA) fragments to 500,000 DNA *(e.g.,* cfDNA) fragments, from 25,000 DNA *(e.g.,* cfDNA) fragments to 250,000 DNA *(e.g.,* cfDNA) fragments, or from 25,000 DNA *(e.g.,* cfDNA) fragments to 100,000 DNA *(e.g.,* cfDNA) fragments.

In some embodiments, the obtaining, accessioning, storing, preparing, processing and/or analyzing the biopsy sample from the test subject comprises any of the methods and/or embodiments described above in the present disclosure. In some embodiments, the sequencing reaction comprises any of the methods and/or embodiments described above in the present disclosure.

In some embodiments, all, or nearly all, of the aligned sequence reads are evaluated to identify candidate sequence variants *(e.g.,* candidate somatic sequence variants and/or candidate germline sequence variants). In other embodiments, a subset of the aligned sequence reads is evaluated to identify candidate sequence variants. For example, in one embodiment, targeted-panel sequencing reaction is used to generate sequencing data 122 and only sequence reads corresponding to the target panel (on-target reads) are evaluated to identify candidate sequence variants. In some embodiments, targeted-panel sequencing reaction is used to generate sequencing data 122 and a subset of sequence reads corresponding to a subset of the target panel are evaluated to identify candidate sequence variants. In some embodiments, a subset of the sequence reads corresponding to a subset of genes, regardless of whether the sequencing reaction is a targeted-panel sequencing reaction, a whole exome sequencing reaction, or a whole genome sequencing reaction, are evaluated to identify candidate sequence variants. In some embodiments, a subset of sequence reads corresponding to a defined set of regions within the genome, *e.g.,* one or more genes, one or more introns, one or more exons, one or more subregion of an intron and/or exon associated with cancer etiology, *etc.,* are evaluated to identify candidate sequence variants.

Alternatively, in some embodiments, regardless of what subset of aligned sequence reads are evaluated to identify candidate sequence variants, only a subset of candidate sequence variants is further validated. For example, in some embodiments, only candidate sequence variants corresponding to the target panel (on-target reads) are validated. Similarly, in some embodiments, only candidate sequence variants corresponding to a subset of the target panel are validated. Likewise, in some embodiments, only candidate sequence variants corresponding to a subset of genes, regardless of whether the sequencing reaction is a targeted-panel sequencing reaction, a whole exome sequencing reaction, or a whole genome sequencing reaction, are validated. Similarly, in some embodiments, only candidate variants corresponding to a defined set of regions within the genome, *e.g.,* one or more genes, one or more introns, one or more exons, one or more subregion of an intron and/or exon associated with cancer etiology, *etc.,* are validated.

In some embodiments, the enrichment is performed prior to pooling multiple nucleic acid sequencing libraries. However, in other embodiments, the enrichment is performed after pooling nucleic acid sequencing libraries, which has the advantage of reducing the number of enrichment assays that have to be performed.

In some embodiments, the enrichment is performed prior to generating a nucleic acid sequencing library. This has the advantage that fewer reagents are needed to perform both the enrichment (because there are fewer target sequences at this point, prior to library amplification) and the library production (because there are fewer nucleic acid molecules to tag and amplify after the enrichment). However, this raises the possibility of pull-down bias and/or that small variations in the enrichment protocol will result in less consistent results.

In some embodiments, nucleic acid libraries are pooled (two or more DNA libraries may be mixed to create a pool) and treated with reagents to reduce off-target capture, for example Human COT-1 and/or IDT xGen Universal Blockers. Pools may be dried in a vacufuge and resuspended. DNA libraries or pools may be hybridized to a probe set (for example, a probe set specific to a panel that includes loci from at least 100, 600, 1,000, *10,000, etc.* of the 19,000 known human genes) and amplified with commercially available reagents (for example, the KAPA HiFi HotStart ReadyMix). For example, in some embodiments, a pool is incubated in an incubator, PCR machine, water bath, or other temperature-modulating device to allow probes to hybridize. Pools may then be mixed with Streptavidin-coated beads or another means for capturing hybridized DNA-probe molecules, such as DNA molecules representing exons of the human genome and/or genes selected for a genetic panel.

Pools may be amplified and purified more than once using commercially available reagents, for example, the KAPA HiFi Library Amplification kit and Axygen MAG PCR clean up beads, respectively. The pools or DNA libraries may be analyzed to determine the concentration or quantity of DNA molecules, for example by using a fluorescent dye (for example, PicoGreen pool quantification) and a fluorescence microplate reader, standard spectrofluorometer, or filter fluorometer. In one example, the DNA library preparation and/or capture is performed with an automated system, using a liquid handling robot (for example, a SciClone NGSx).

In some embodiments, *e.g.,* where a whole genome sequencing method is used, nucleic acid sequencing libraries are not target-enriched prior to sequencing, in order to obtain sequencing data on substantially all of the competent nucleic acids in the sequencing library. Similarly, in some embodiments, *e.g.,* where a whole genome sequencing method will be used, nucleic acid sequencing libraries are not mixed, because of bandwidth limitations related to obtaining significant sequencing depth across an entire genome. However, in other embodiments, *e.g.,* where a low pass whole genome sequencing (LPWGS) methodology will be used, nucleic acid sequencing libraries can still be pooled, because very low average sequencing coverage is achieved across a respective genome, *e.g.,* between about 0.5x and about 5x.

In some embodiments, a plurality of nucleic acid probes *(e.g.,* a probe set) is used to enrich one or more target sequences in a nucleic acid sample *(e.g.,* an isolated nucleic acid sample or a nucleic acid sequencing library), *e.g.,* where one or more target sequences is informative for precision oncology. For instance, in some embodiments, one or more of the target sequences encompasses a locus that is associated with an actionable allele. That is, variations of the target sequence are associated with targeted therapeutic approaches. In some embodiments, one or more of the target sequences and/or a property of one or more of the target sequences is used in a classifier trained to distinguish two or more cancer states.

*Block 904.* Referring to block 904, in some embodiments, the panel-enriched sequencing reaction is performed at a read depth of at least 1,000X. In some embodiments, the panel-targeting sequencing is performed to an average on-target depth of at least 500x, at least 750x, at least 1000x, at least 2500x, at least 500x, at least 10,000x, or greater depth. In some embodiments, samples are further assessed for uniformity above a sequencing depth threshold *(e.g.,* 95% of all targeted base pairs at 300x sequencing depth). In some embodiments, the sequencing depth threshold is a minimum depth selected by a user or practitioner.

Advantageously, enriching for target sequences prior to sequencing nucleic acids significantly reduces the costs and time associated with sequencing, facilitates multiplex sequencing by allowing multiple samples to be mixed together for a single sequencing reaction, and significantly reduces the computation burden of aligning the resulting sequence reads, as a result of significantly reducing the total amount of nucleic acids analyzed from each sample. Accordingly, in some embodiments, a panel-enriched sequencing reaction is performed at a read depth of at least 1,000X. In some embodiments, a panel-enriched sequencing reaction is performed at a read depth of at least 100X, at least 500X, at least 1000X, at least 5000X, at least 10,000X, at least 50,000X, or greater. In some embodiments, a panel-enriched sequencing reaction is performed at a read depth of no more than 100,000X, no more than 50,000X, no more than 10,000X, no more than 5000X, or less. In some embodiments, a panel-enriched sequencing reaction is performed at a read depth of from 100X to 50,000X, from 100X to 10,000X, from 100X to 5000X, from 100X to 1000X, or from 100X to 500X. In some embodiments, a panel-enriched sequencing reaction is performed at a read depth of from 500X to 50,000X, from 500X to 10,000X, from 500X to 5000X, or from 500X to 1000X. In some embodiments, a panel-enriched sequencing reaction is performed at a read depth of from 1000X to 50,000X, from 1000X to 10,000X, or from 1000X to 5000X.

In some embodiments, a total cfDNA fragment sequencing reaction is performed at a read depth of at least 1X. In some embodiments, a panel-enriched sequencing reaction is performed at a read depth of at least 2X, at least 3X, at least 4X, at least 5X, at least 10X, at least 25X, at least 50X, at least 100X, at least 250X, or greater. In some embodiments, a total cfDNA fragment sequencing reaction is performed at a read depth of no more than 1000X, no more than 500X, no more than 100X, no more than 50X, or less. In some embodiments, a total cfDNA fragment sequencing reaction is performed at a read depth of from 1X to 500X, from 1X to 100X, or from 1X to 50X. In some embodiments, a total cfDNA fragment sequencing reaction is performed at a read depth of from 2.5X to 500X, from 2.5X to 100X, or from 2.5X to 50X. In some embodiments, a total cfDNA fragment sequencing reaction is performed at a read depth of from 5X to 500X, from 5X to 100X, or from 5X to 50X. In some embodiments, a total cfDNA fragment sequencing reaction is performed at a read depth of from 10X to 500X, from 10X to 100X, or from 10X to 50X.

*Blocks 906 and 908.* Referring to block 906, in some embodiments, the panel-enriched sequencing reaction uses a sequencing panel that enriches (includes probes for) for between 50 genes and 150 genes. Referring to block 908, in some embodiments, the plurality of probe sequences used to enrich cell-free DNA fragments in the liquid biopsy sample in the panel-enriched sequencing reaction collectively map to from 25 different genes to 150 different genes in a human reference genome. In some embodiments, the panel-enriched sequencing reaction uses a sequencing panel that enriches for between 50 genes and 150 genes, between 100 genes and 200 genes, between 150 genes and 300 genes, or between 250 genes and 500 genes. In some embodiments, panel-enriched sequencing reaction uses a sequencing panel that enriches for between 50 genes and 1000 genes, between 60 genes and 800 genes, between 70 genes and 700 genes, between 80 genes and 600 genes, or between 90 genes and 500 genes. In some embodiments, each of the genes that are enriched for in the sequencing panel are human.

In some embodiments, the plurality of probe sequences used to enrich cell-free DNA fragments in the liquid biopsy sample in the panel-enriched sequencing reaction collectively map to at least 25 different genes in a human reference genome. In some embodiments, the panel-enriched sequencing reaction uses a sequencing panel that enriches for at least 25 human genes, at least 50 human genes, at least 100 human genes, at least 250 human genes, at least 500 human genes, at least 1000 human genes, at least 2500 human genes, at least 5000 human genes, or more. In some embodiments, the panel-enriched sequencing reaction uses a sequencing panel that enriches for no more than 1,000 human genes, no more than 500 human genes, no more than 250 human genes, no more than 200 human genes, no more than 175 human genes, no more than 100 human genes, or less. In some embodiments, the panel-enriched sequencing reaction uses a sequencing panel that enriches for from 25 human genes to 1,000 human genes, from 25 human genes to 500 human genes, from 10 human genes to 250 human genes, from 10 human genes to 200 human genes, from 5 human genes to 150 human genes, or from 5 human genes to 100 human genes. In some embodiments, the panel-enriched sequencing reaction uses a sequencing panel that enriches for from 25 human genes to 400 human genes, from 30 human genes to 500 human genes, from 50 human genes to 300 human genes, from 5 human genes to 95 human genes, from 15 human genes to 130 human genes, or from 15 human genes to 165 human genes. In some embodiments, the panel-enriched sequencing reaction uses a sequencing panel that enriches for from 25 human genes to 600 human genes, from 40 human genes to 80 human genes, from 35 human genes to 95 human genes, from 45 human genes to 80 human genes, from 20 human genes to 80 human genes, or from 20 human genes to 120 human genes.

*Blocks 910 - 912.* Referring to block 910, in some embodiments, the panel-enriched sequencing reaction uses a sequencing panel that enriches for at least 10, 20, 30, 40, or 50 genes listed in Table 1. In some embodiments, the panel-enriched sequencing reaction uses a sequencing panel that enriches for at least 10 genes of which at least 10, 20, 30, 40, or 50 are genes listed in Table 1. In some embodiments, the panel-enriched sequencing reaction uses a sequencing panel that enriches for at most 100 genes of which at least 10, 20, 30, 40, or 50 are genes listed in Table 1. In some embodiments, the sequencing panel only enriches for genes in Table 1 whereas in other embodiments the sequencing panel enriches for some genes that are in Table 1 and some genes that are not in Table 1. In some embodiments, the panel-enriched sequencing reaction uses a sequencing panel that enriches between 25 different genes and 150 different genes listed in Table 1. In some embodiments, the panel-enriched sequencing reaction uses a sequencing panel that enriches for between 50 genes and 150 genes, between 100 genes and 200 genes, or between 150 genes and 300 genes listed in Table 1.

In some embodiments, the panel-enriched sequencing reaction uses a sequencing panel that enriches for at least 10, 20, 30, 40, or 50 genes listed in Table 2. In some embodiments, the panel-enriched sequencing reaction uses a sequencing panel that enriches for at least 10 genes of which at least 10, 20, 30, 40, or 50 are genes listed in Table 2. In some embodiments, the panel-enriched sequencing reaction uses a sequencing panel that enriches for at most 100 genes of which at least 10, 20, 30, 40, or 50 are genes listed in Table 2. In some embodiments, the sequencing panel only enriches for genes in Table 2 whereas in other embodiments the sequencing panel enriches for some genes that are in Table 2 and some genes that are not in Table 2. In some embodiments, the panel-enriched sequencing reaction uses a sequencing panel that enriches between 25 different genes and 150 different genes listed in Table 2. In some embodiments, the panel-enriched sequencing reaction uses a sequencing panel that enriches for between 50 genes and 150 genes, between 100 genes and 200 genes, or between 150 genes and 300 genes listed in Table 2.

In some embodiments, the probe set includes probes that target no more than 50 genes, 100 genes, 150 genes or 200 genes. In some such emboidments the probe set includes probes targeting one or more of the genes listed in Table 1. In some such embodiments, the probe set includes probes targeting at least 5 of the genes listed in Table 1. In some such embodiments, the probe set includes probes targeting at least 10 of the genes listed in Table 1. In some such embodiments, the probe set includes probes targeting at least 25 of the genes listed in Table 1. In some such embodiments, the probe set includes probes targeting at least 50 of the genes listed in Table 1. In some such embodiments, the probe set includes probes targeting at least 75 of the genes listed in Table 1. In some such embodiments, the probe set includes probes targeting at least 100 of the genes listed in Table 1. In some such embodiments, the probe set includes probes targeting all of the genes listed in Table 1.

In some embodiments, the probe set includes probes that target no more than 50 genes, 100 genes, 150 genes or 200 genes. In some such embodiments, the probe set includes probes targeting one or more of the genes listed in Table 2. In some such embodiments, the probe set includes probes targeting at least 5 of the genes listed in Table 2. In some such embodiments, the probe set includes probes targeting at least 10 of the genes listed in Table 2. In some such embodiments, the probe set includes probes targeting at least 25 of the genes listed in Table 2. In some such embodiments, the probe set includes probes targeting at least 50 of the genes listed in Table 2. In some such embodiments, the probe set includes probes targeting at least 75 of the genes listed in Table 2. In some such embodiments, the probe set includes probes targeting at least 100 of the genes listed in Table 2. In some such embodiments, the probe set includes probes targeting all of the genes listed in Table 2.

**Table 1.** An example panel of 105 genes.

| | | | | | | |
|---|---|---|---|---|---|---|
| ALK | B2M | ERRFI1 | IDH2 | MSH6 | PIK3R1 | SPOP |
| FGFR2 | BAP1 | ESR1 | JAK1 | MTOR | PMS2 | STK11 |
| FGFR3 | BRCA1 | EZH2 | JAK2 | MYCN | PTCH1 | TERT |
| NTRK1 | BRCA2 | FBXW7 | JAK3 | NF1 | PTEN | TP53 |
| RET | BTK | FGFR1 | KDR | NF2 | PTPN11 | TSC1 |
| ROS1 | CCND1 | FGFR4 | KEAP1 | NFE2L2 | RAD51C | TSC2 |
| BRAF | CCND2 | FLT3 | KIT | NOTCH1 | RAF1 | UGT1A1 |
| AKT1 | CCND3 | FOXL2 | KRAS | NPM1 | RB1 | VHL |
| AKT2 | CDH1 | GATA3 | MAP2K 1 | NRAS | RHEB | CCNE1 |
| APC | CDK4 | GNA11 | MAP2K2 | PALB2 | RHOA | CD274 |
| AR | CDK6 | GNAQ | MAPK1 | PBRM1 | RIT1 | EGFR |
| ARAF | CDKN2A | GNAS | MLH1 | PDCD1LG2 | RNF43 | ERBB2 |
| ARID1A | CTNNB1 | HNF1A | MPL | PDGFRA | SDHA | MET |
| ATM | DDR2 | HRAS | MSH2 | PDGFRB | SMAD4 | MYC |
| ATR | DPYD | IDH1 | MSH3 | PIK3CA | SMO | KMT2A |

**Table 2.** An example panel of 523 genes.

| | | | | | |
|---|---|---|---|---|---|
| BCC3 | CIC | FGFR4 | KLF4 | PBRM1 | SIRPA |
| ABL1 | CKS1B | FH | KLHL6 | PDCD1 | SLC34A2 |
| ABL2 | CREBBP | FHIT | KLLN | PDCD 1LG2 | SLC9A3R1 |
| ABRAXAS1 | CRKL | FLCN | KMT2A | PDGFRA | SLFN11 |
| ACVR1 | CSF1R | FLT1 | KMT2C | PDGFRB | SLIT2 |
| ACVR1B | CSF3R | FLT3 | KMT2D | PDK1 | SMAD2 |
| AJUBA | CTC1 | FLT4 | KRAS | PHGDH | SMAD3 |
| AKT1 | CTCF | FOLH1 | LATS1 | PHLPP1 | SMAD4 |
| AKT2 | CTLA4 | FOXA1 | LCK | PHLPP2 | SMARCA2 |
| AKT3 | CTNNA1 | FOXL2 | LMO1 | PIAS4 | SMARCA4 |
| ALK | CTNNB1 | FOXO1 | LRP1B | PIK3C2B | SMARCB1 |
| ALOX12B | CUL3 | FOXO3 | LTK | PIK3C2G | SMC1A |
| AMER1 | CUL4A | FOXP1 | LYN | PIK3CA | SMC3 |
| APC | CUX1 | FRS2 | LZTR1 | PIK3CB | SMO |
| APLNR | CXCR4 | FUBP1 | MAF | PIK3CD | SNCAIP |
| AR | CYLD | GABRA6 | MALT1 | PIK3CG | SOCS1 |
| ARAF | CYP17A1 | GALNT12 | MAP2K1 | PIK3R1 | SOS1 |
| ARFRP1 | CYSLTR2 | GATA1 | MAP2K2 | PIK3R2 | SOX2 |
| ARID1A | DAXX | GATA3 | MAP2K4 | PIM1 | SOX9 |
| ARID1B | DDB2 | GATA4 | MAP3K1 | PLCG1 | SPEN |
| ARID2 | DDR1 | GATA6 | MAP3K13 | PLCG2 | SPOP |
| ASNS | DDR2 | GID4 | MAP3K21 | PMS1 | SRC |
| ASXL1 | DDX3X | GLI2 | MAP3K7 | PMS2 | SRSF2 |

(continued)

| ATM | DDX41 | GNA11 | MAPK1 | POLA1 | STAG2 |
|------|--------|--------|--------|--------|--------|
| ATR | DEPTOR | GNA13 | MAPK3 | POLD1 | STAT3 |
| ATRX | DICER1 | GNAQ | MAX | POLE | STAT5B |
| AURKA | DIS3 | GNAS | MC1R | POLQ | STAT6 |
| AURKB | DNMT1 | GPC3 | MCL1 | POT1 | STK11 |
| AURKC | DNMT3A | GPS2 | MDM2 | PPARG | SUFU |
| AXIN1 | DOT1L | GREM1 | MDM4 | PPM1D | SUZ12 |
| AXIN2 | DPYD | GRIN2A | MED 12 | PPP2R1A | SYK |
| AXL | EBF1 | GRM3 | MEF2B | PPP2R2A | TBX3 |
| B2M | EED | GSK3B | MEN1 | PPP6C | TCF7L2 |
| BAP1 | EEF2 | GSTP1 | MERTK | PRDM1 | TEK |
| BARD1 | EGFR | H3F3A | MET | PREX2 | TERC |
| BAX | EGLN1 | HAVCR2 | MITF | PRKACA | TERT |
| BCL2 | EIF1AX | HDAC1 | MKNK1 | PRKAR1A | TET2 |
| BCL2L1 | ELF3 | HDAC2 | MLH1 | PRKCI | TFEB |
| BCL2L11 | EMSY | HGF | MLH3 | PRKN | TGFB1 |
| BCL2L2 | EP300 | HIF1A | MPL | PTCH1 | TGFBR1 |
| BCL6 | EPCAM | HIST1H3B | MRE11 | PTEN | TGFBR2 |
| BCLAF1 | EPHA2 | HLA-B | MS4A1 | PTK2 | TIGIT |
| BCOR | EPHA3 | HNF1A | MSH2 | PTPN11 | TIPARP |
| BCORL1 | EPHB1 | HNF1B | MSH3 | PTPN13 | TMEM127 |
| BCR | EPHB4 | HOXB13 | MSH6 | PTPRD | TMPRSS2 |
| BIRC3 | ERBB2 | HRAS | MST1R | PTPRO | TNFAIP3 |
| BLM | ERBB3 | HSD3B1 | MTAP | PTPRT | TNFRSF14 |
| BMPR1A | ERBB4 | HSP90AA1 | MTHFR | QKI | TNFRSF17 |
| BRAF | ERCC2 | HSPH1 | MTOR | RAC1 | TOP1 |
| BRCA1 | ERCC3 | ID3 | MUC16 | RAD21 | TOP2A |
| BRCA2 | ERCC4 | IDH1 | MUTYH | RAD50 | TP53 |
| BRD4 | ERCC6 | IDH2 | MYB | RAD51 | TP53BP1 |
| BRIP1 | ERG | IFNA21 | MYC | RAD51B | TP63 |
| BTG1 | ERRFI1 | IFNAR1 | MYCL | RAD51C | TRAF3 |
| BTG2 | ESR1 | IFNAR2 | MYCN | RAD51D | TRAF7 |
| BTK | ETNK1 | IFNG | MYD88 | RAD52 | TSC1 |
| CALR | ETV1 | IFNGR1 | NBN | RAD54L | TSC2 |
| CARD11 | ETV4 | IFNGR2 | NCOA2 | RAF1 | TSHR |
| CARM1 | ETV5 | IFNW1 | NCOR1 | RARA | TYMS |
| CASP8 | ETV6 | IGF1 | NF1 | RASA1 | TYRO3 |
| CBFB | EWSR1 | IGF1R | NF2 | RB1 | U2AF1 |
| CBL | EZH2 | IKBKE | NFE2L2 | RBM10 | UGT1A1 |
| CCND1 | EZR | IKZF1 | NFKBIA | RECQL4 | VEGFA |

(continued)

| CCND2 | FAM46C | IL10RA | NKX2-1 | REL | VHL |
|-------|--------|--------|--------|-----|-----|
| CCND3 | FANCA | IL32 | NOTCH1 | RET | VSIR |
| CCNE1 | FANCC | IL6R | NOTCH2 | RHEB | WEE1 |
| CD22 | FANCD2 | IL7R | NOTCH3 | RHOA | WNK1 |
| CD274 | FANCE | IMPDH1 | NOTCH4 | RICTOR | WRN |
| CD70 | FANCG | ING1 | NPM1 | RIT1 | WT1 |
| CD74 | FANCI | INPP4B | NQO1 | RNF43 | XBP1 |
| CD79A | FANCL | INSR | NRAS | ROS1 | XPA |
| CD79B | FANCM | IRF1 | NRG1 | RPS6KB1 | XPC |
| CDC73 | FAS | IRF2 | NSD1 | RPTOR | XPO1 |
| CDH1 | FAT1 | IRF4 | NSD2 | RRM1 | XRCC1 |
| CDK12 | FBXW7 | IRS2 | NSD3 | RSF1 | XRCC2 |
| CDK4 | FCGR2A | JAK1 | NT5C2 | RSPO2 | YEATS4 |
| CDK6 | FCGR3A | JAK2 | NTRK1 | RUNX1 | ZFHX3 |
| CDK8 | FGF10 | JAK3 | NTRK2 | RXRA | ZMYM3 |
| CDK9 | FGF12 | JUN | NTRK3 | SDC4 | ZNF217 |
| CDKN1A | FGF14 | KAT6A | NUTM1 | SDHA | ZNF703 |
| CDKN1B | FGF19 | KDM5A | P2RY8 | SDHAF2 | ZNF750 |
| CDKN2A | FGF23 | KDM5C | PAK1 | SDHB | ZNRF3 |
| CDKN2B | FGF3 | KDM5D | PALB2 | SDHC | ZRSR2 |
| CDKN2C | FGF4 | KDM6A | PALLD | SDHD | |
| CEBPA | FGF6 | KDR | PARP1 | SETBP1 | |
| CHD4 | FGFR1 | KEAP1 | PARP2 | SETD2 | |
| CHEK1 | FGFR2 | KEL | PARP3 | SF3B1 | |
| CHEK2 | FGFR3 | KIT | PAX5 | SGK1 | |

*Block 912.* Referring to block 912, in some embodiments, the panel-enriched sequencing reaction uses a sequencing panel that enriches for at least 10, 20, 30, 40, or 50 genes listed in List 1. In some embodiments, the panel-enriched sequencing reaction uses a sequencing panel that enriches between 25 different genes and 50 different genes listed in List 1. In some embodiments, the panel-enriched sequencing reaction uses a sequencing panel that enriches for between 5 genes and all the genes listed in List 1, between 10 genes and all the genes listed in List 1, or between 20 genes and all the genes listed in List 1.

In some embodiments, the probe set includes probes that target no more than 50 genes, 100 genes, 150 genes or 200 genes. In some such emboidments the probe set consists of or comprises probes targeting one or more of the genes in List 1. In some such embodiments, the probe set consists of or comprises probes targeting at least 5 of the genes listed in List 1. In some such embodiments, the probe set consists of or comprises probes targeting at least 10 of the genes in List 1. In some such embodiments, the probe set consists of or comprises probes targeting at least 25 of the genes in List 1. In some such embodiments, the probe set consists of or comprises probes targeting at least 50 of the genes listed in List 1. In some such embodiments, the probe set consists of or comprises probes targeting all of the genes in List 1.

List 1: AKT1 (14q32.33), ALK (2p23.2-23.1), APC (5q22.2), AR (Xq12), ARAF (Xp11.3), ARID1A (1p36.11), ATM (11q22.3), BRAF (7q34), BRCA1 (17q21.31), BRCA2 (13q13.1), CCND1 (11q13.3), CCND2 (12p13.32), CCNE1 (19q12), CDH1 (16q22.1), CDK4 (12q14.1), CDK6 (7q21.2), CDKN2A (9p21.3), CTNNB1 (3p22.1), DDR2 (1q23.3), EGFR (7p11.2), ERBB2 (17q12), ESR1 (6q25.1-25.2), EZH2 (7q36.1), FBXW7 (4q31.3), FGFR1 (8p11.23), FGFR2 (10q26.13), FGFR3 (4p16.3), GATA3 (10p14), GNA11 (19p13.3), GNAQ (9q21.2), GNAS (20q13.32), HNF1A (12q24.31), HRAS (11p15.5), IDH1 (2q34), IDH2 (15q26.1), JAK2 (9p24.1), JAK3 (19p13.11), KIT (4q12), KRAS (12p12.1), MAP2K1 (15q22.31), MAP2K2 (19p13.3), MAPK1 (22q11.22), MAPK3 (16p11.2), MET (7q31.2), MLH1 (3p22.2), MPL (1p34.2), MTOR (1p36.22), MYC (8q24.21), NF1 (17q11.2), NFE2L2 (2q31.2), NOTCH1 (9q34.3), NPM1 (5q35.1), NRAS (1p13.2), NTRK1 (1q23.1), NTRK3 (15q25.3), PDGFRA (4q12), PIK3CA (3q26.32), PTEN (10q23.31), PTPN11 (12q24.13), RAF1 (3p25.2), RB1 (13q14.2), RET (10q11.21), RHEB (7q36.1), RHOA (3p21.31), RIT1 (1q22), ROS1 (6q22.1), SMAD4 (18q21.2), SMO (7q32.1), STK11 (19p13.3), TERT (5p15.33), TP53 (17p13.1), TSC1 (9q34.13), and VHL (3p25.3).

*Block 914.* Referring to block 914, in some embodiments, the panel-enriched sequencing reaction uses a sequencing panel that enriches for at least 10, 20, 30, 40, or 50 genes listed in List 2. In some embodiments, the panel-enriched sequencing reaction uses a sequencing panel that enriches between 25 different genes and 50 different genes listed in List 2. In some embodiments, the panel-enriched sequencing reaction uses a sequencing panel that enriches for between 5 genes and all the genes listed in List 2, between 10 genes and all the genes listed in List 2, or between 20 genes and all the genes listed in List 2.

In some embodiments, the probe set includes probes that target no more than 50 genes, 100 genes, 150 genes or 200 genes. In some such embodiments the probe set consists of or comprises probes targeting one or more of the genes in List 2. In some such embodiments, the probe set consists of or comprises probes targeting at least 5 of the genes listed in List 2. In some such embodiments, the probe set consists of or comprises probes targeting at least 10 of the genes in List 2. In some such embodiments, the probe set consists of or comprises probes targeting at least 25 of the genes in List 2. In some such embodiments, the probe set consists of or comprises probes targeting at least 50 of the genes listed in List 2. In some such embodiments, the probe set consists of or comprises probes targeting all of the genes in List 2.

List 2: ABL1, ACVR1B, AKT1, AKT2, AKT3, ALK, ALOX12B, AMER1 (FAM123B), APC, AR, ARAF, ARFRP1, ARID1A, ASXL1, ATM, ATR, ATRX, AURKA, AURKB, AXIN1, AXL, BAP1, BARD1, BCL2, BCL2L1, BCL2L2, BCL6, BCOR, BCORL1, BRAF, BRCA1, BRCA2, BRD4, BRIP1, BTG1, BTG2, BTK, C11orf30 (EMSY), C17orf39 (GID4), CALR, CARD11, CASP8, CBFB, CBL, CCND1, CCND2, CCND3, CCNE1, CD22, CD274 (PD-L1), CD70, CD79A, CD79B, CDC73, CDH1, CDK12, CDK4, CDK6, CDK8, CDKN1A, CDKN1B, CDKN2A, CDKN2B, CDKN2C, CEBPA, CHEK1, CHEK2, CIC, CREBBP, CRKL, CSF1R, CSF3R, CTCF, CTNNA1, CTNNB1, CUL3, CUL4A, CXCR4, CYP17A1, DAXX, DDR1, DDR2, DIS3, DNMT3A, DOT1L, EED, EGFR, EP300, EPHA3, EPHB1, EPHB4, ERBB2, ERBB3, ERBB4, ERCC4, ERG, ERRFI1, ESR1, EZH2, FAM46C, FANCA, FANCC, FANCG, FANCL, FAS, FBXW7, FGF10, FGF12, FGF14, FGF19, FGF23, FGF3, FGF4, FGF6, FGFR1, FGFR2, FGFR3, FGFR4, FH, FLCN, FLT1, FLT3, FOXL2, FUBP1, GABRA6, GATA3, GATA4, GATA6, GNA11, GNA13, GNAQ, GNAS, GRM3, GSK3B, H3F3A, HDAC1, HGF, HNF1A, HRAS, HSD3B1, ID3, IDH1, IDH2, IGF1R, IKBKE, IKZF1, INPP4B, IRF2, IRF4, IRS2, JAK1, JAK2, JAK3, JUN, KDM5A, KDM5C, KDM6A, KDR, KEAP1, KEL, KIT, KLHL6, KMT2A, KMT2D (MLL2), KRAS, LTK, LYN, MAF, MAP2K1 (MEK1), MAP2K2 (MEK2), MAP2K4, MAP3K1, MAP3K13, MAPK1, MCL1, MDM2, MDM4, MED12, MEF2B, MEN1, MERTK, MET, MITF, MKNK1, MLH1, MPL, MRE11A, MSH2, MSH3, MSH6, MST1R, MTAP, MTOR, MUTYH, MYC, MYCL (MYCL1), MYCN, MYD88, NBN, NF1, NF2, NFE2L2, NFKBIA, NKX2-1, NOTCH1, NOTCH2, NOTCH3, NPM1, NRAS, NSD3 (WHSC1L1), NT5C2, NTRK1, NTRK2, NTRK3, P2RY8, PALB2, PARK2, PARP1, PARP2, PARP3, PAX5, PBRM1, PDCD1 (PD-1), PDCD1LG2 (PD-L2), PDGFRA, PDGFRB, PDK1, PIK3C2B, PIK3C2G, PIK3CA, PIK3CB, PIK3R1, PIM1, PMS2, POLD1, POLE, PPARG, PPP2R1A, PPP2R2A, PRDM1, PRKAR1A, PRKCI, PTCH1, PTEN, PTPN11, PTPRO, QKI, RAC1, RAD21, RAD51, RAD51B, RAD51C, RAD51D, RAD52, RAD54L, RAF1, RARA, RB1, RBM10, REL, RET, RICTOR, RNF43, ROS1, RPTOR, SDHA, SDHB, SDHC, SDHD, SETD2, SF3B1, SGK1, SMAD2, SMAD4, SMARCA4, SMARCB1, SMO, SNCAIP, SOCS1, SOX2, SOX9, SPEN, SPOP, SRC, STAG2, STAT3, STK11, SUFU, SYK, TBX3, TEK, TERC, TERT, TET2, ncRNA, TGFBR2, TIPARP, TNFAIP3, TNFRSF14, TP53, TSC1, TSC2, TYRO3, U2AF1, VEGFA, VHL, WHSC1, WT1, XPO1, XRCC2, ZNF217, and ZNF703.

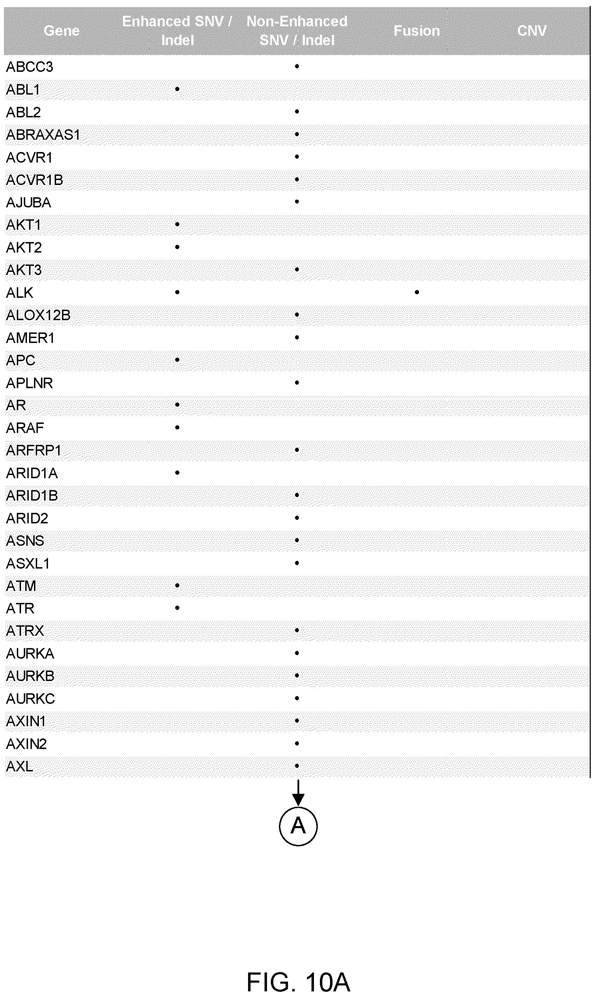

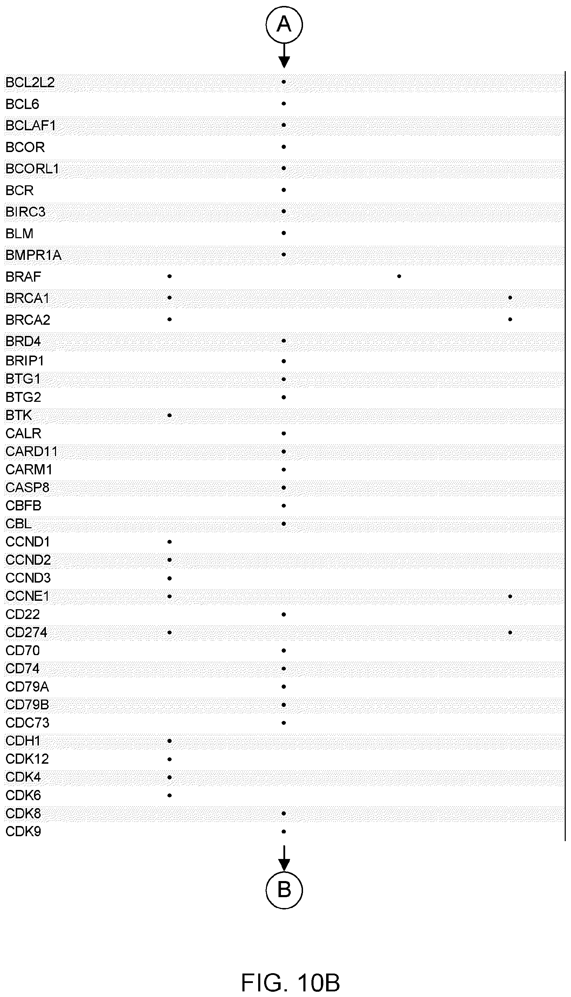

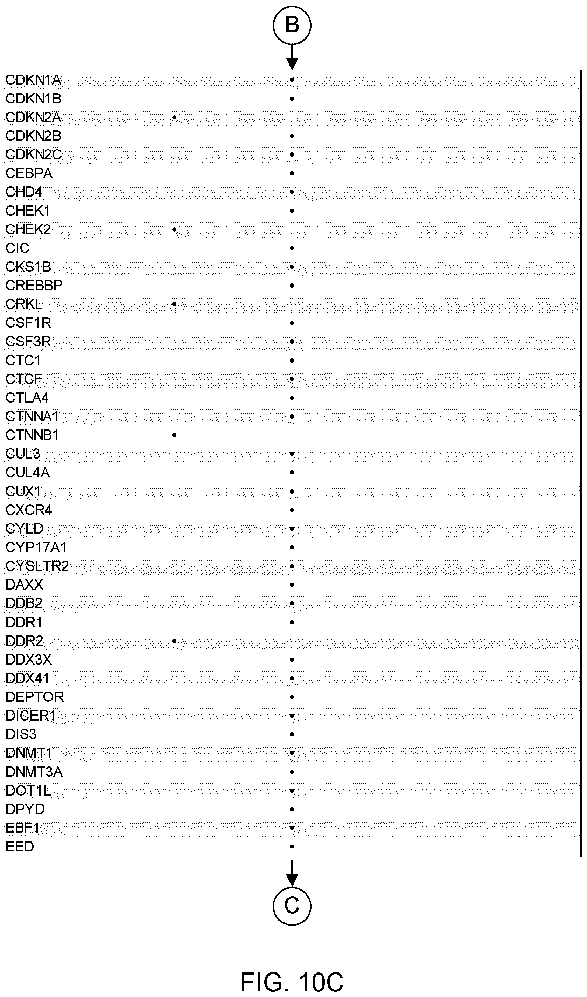

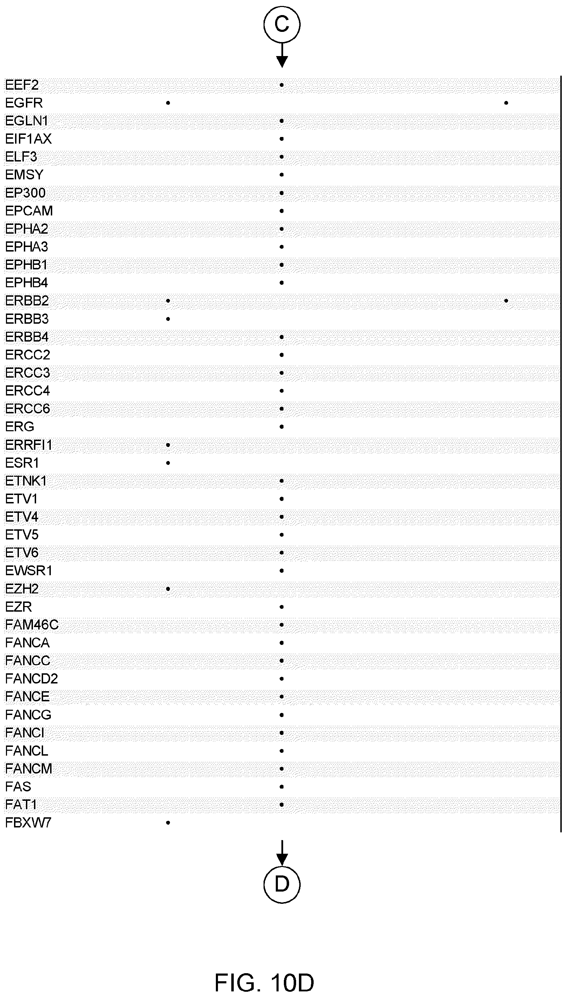

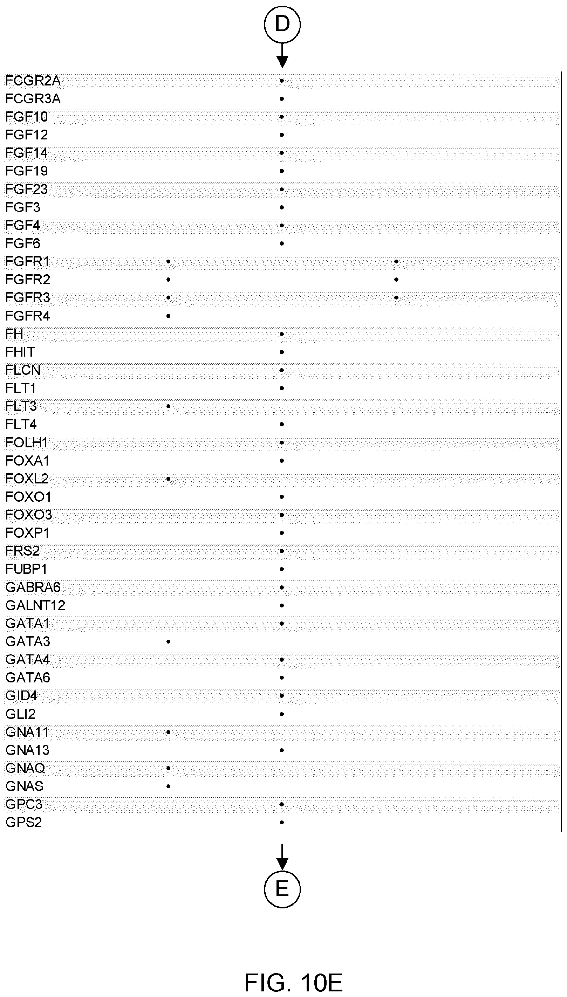

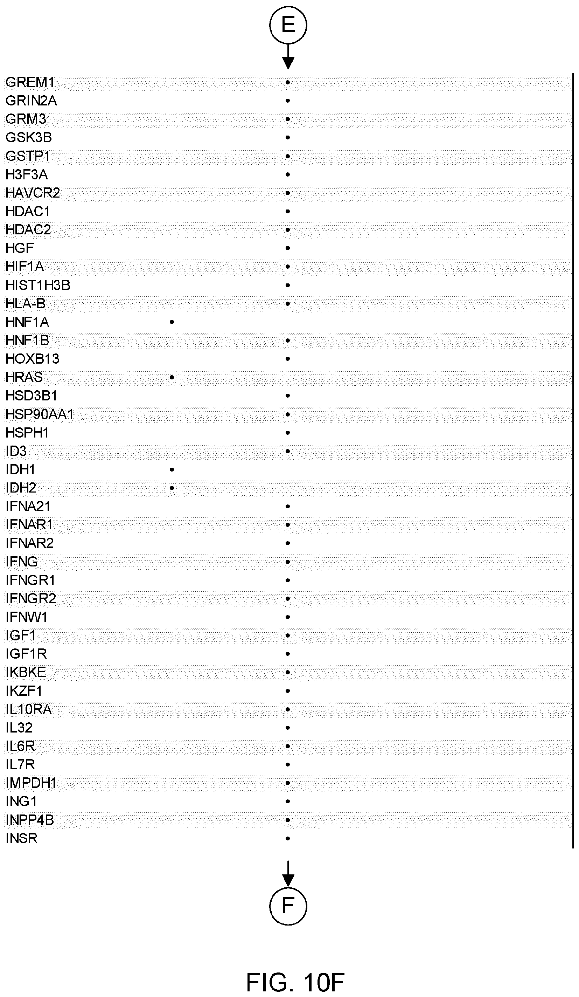

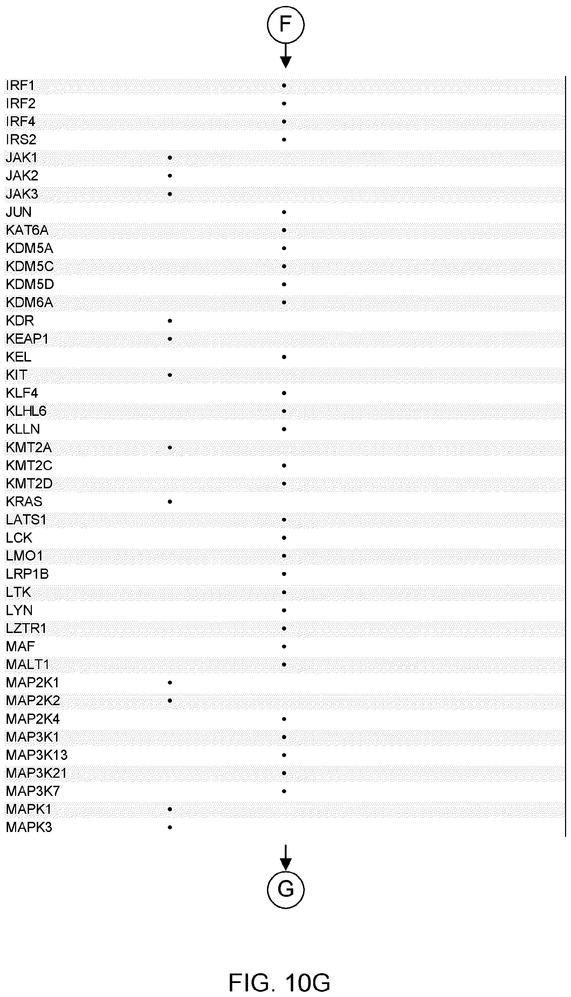

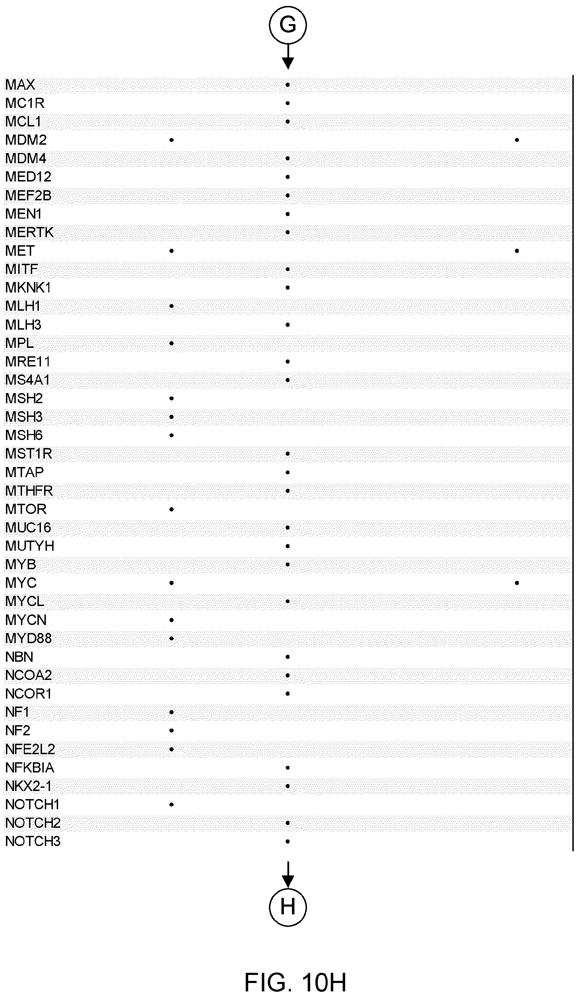

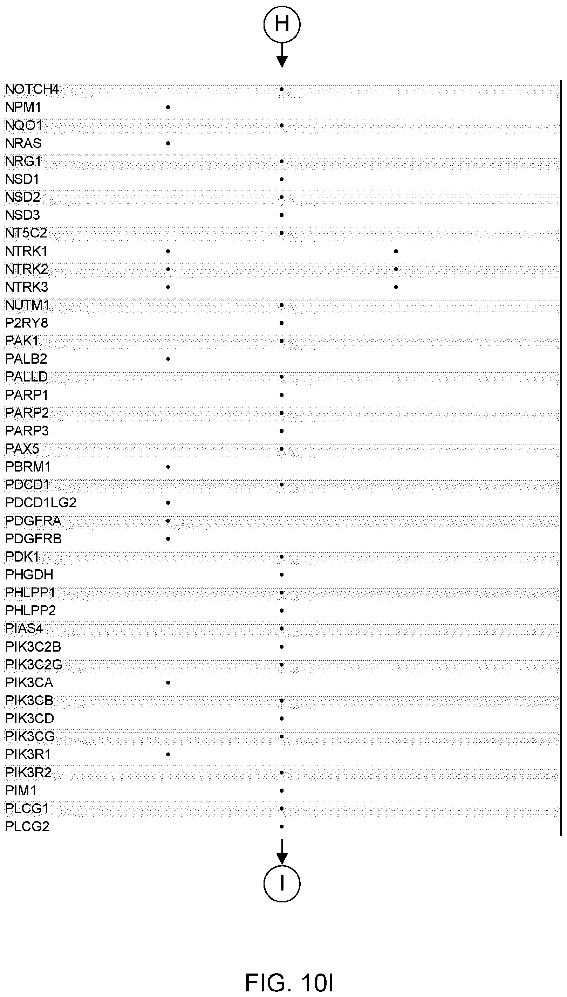

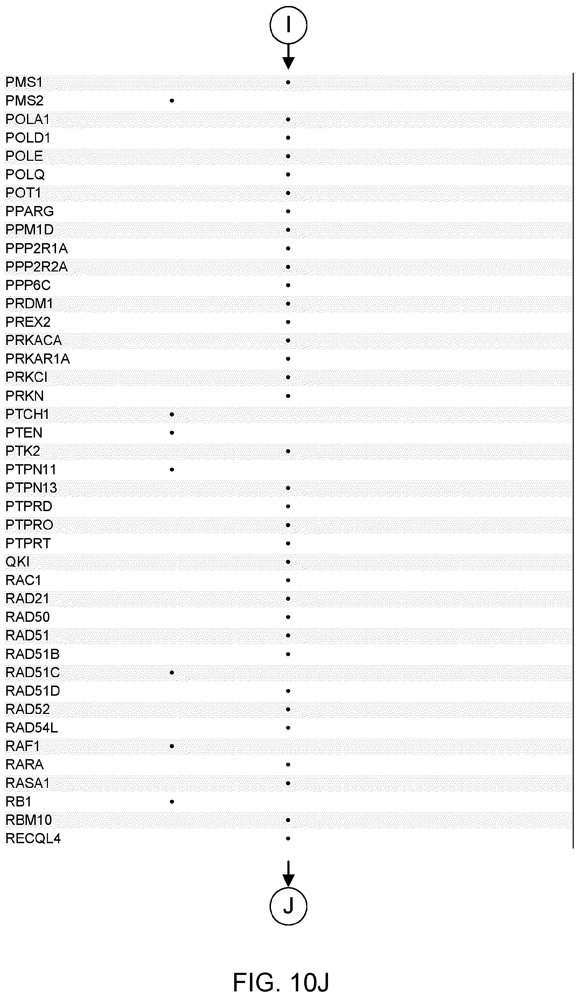

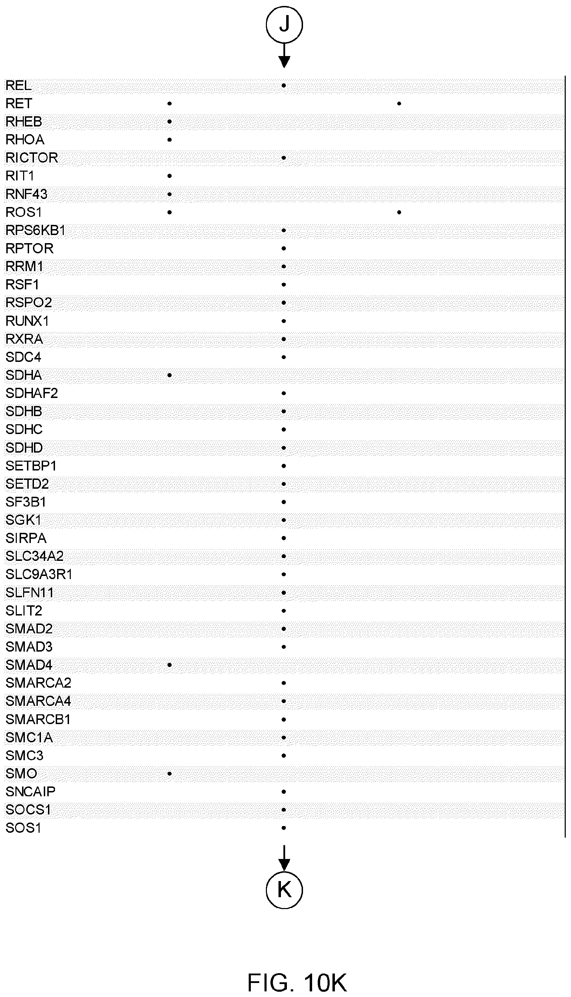

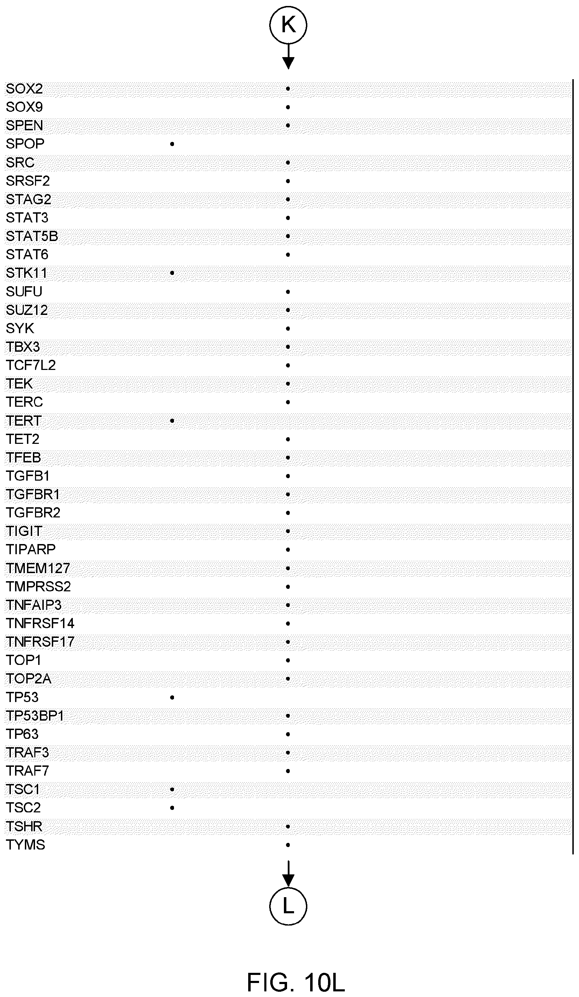

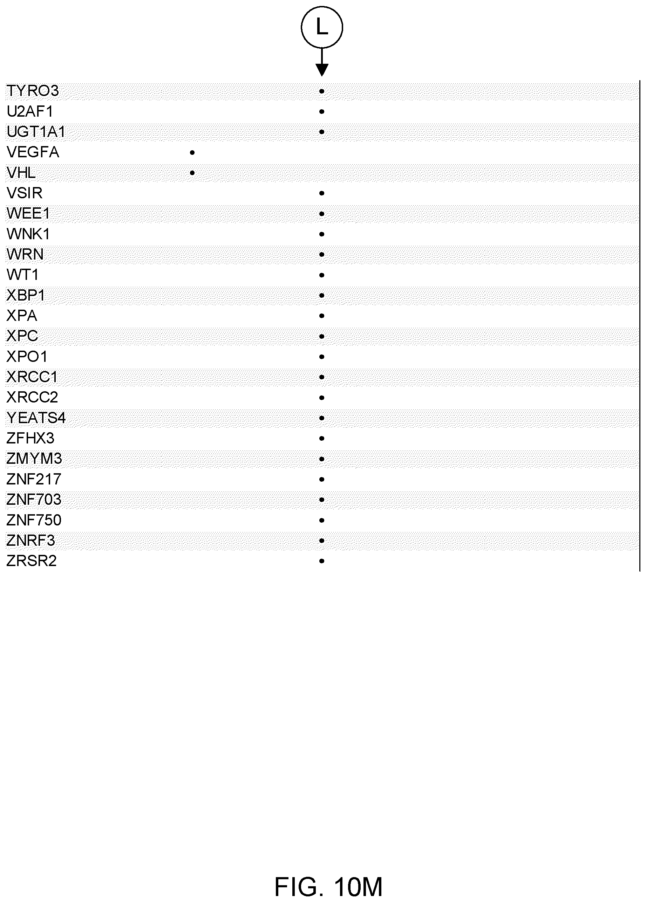

*Block 914.* Referring to block 914, in some embodiments, the panel-enriched sequencing reaction uses a sequencing panel that enriches for at least 10, 20, 30, 40, or 50 genes listed in Figure 10 (any combination of Figures 10A, 10B, 10C, 10D, 10E, 10F, 10G, 10H, 10I, 10j, 10K, 10L, and 10M). In some embodiments, the panel-enriched sequencing reaction uses a sequencing panel that enriches between 25 different genes and 50 different genes listed in Figure 10. In some embodiments, the panel-enriched sequencing reaction uses a sequencing panel that enriches for between 5 genes and all the genes listed in Figure 10, between 10 genes and all the genes listed in Figure 10, or between 20 genes and all the genes listed in Figure 10. While Figure 10 and List 2 provide the same genes, Figure 10 indicates, in preferred embodment, the type of variant that is such genes.

In some embodiments, the probe set includes probes that target no more than 50 genes, 100 genes, 150 genes or 200 genes. In some such emboidments the probe set consists of or comprises probes targeting one or more of the genes in Figure 10. In some such embodiments, the probe set consists of or comprises probes targeting at least 5 of the genes listed in Figure 10. In some such embodiments, the probe set consists of or comprises probes targeting at least 10 of the genes in Figure 10. In some such embodiments, the probe set consists of or comprises probes targeting at least 25 of the genes in Figure 10. In some such embodiments, the probe set consists of or comprises probes targeting at least 50 of the genes listed in Figure 10. In some such embodiments, the probe set consists of or comprises probes targeting all of the genes in Figure 10.

In some embodiments the panel-enriched sequencing reaction uses a sequencing panel that enriches for at least 10, 20, 30, 40, or 50 genes listed in any of Figures 10A, 10B, 10C, 10D, 10E, 10F, 10G, 10H, 10I, 10j, 10K, 10L, and 10M.

In some embodiments the panel-enriched sequencing reaction uses a sequencing panel that enriches for at least 20, 30, 40, 50, 60, 70, 80, 90, 100, 110, 120, 130, 140, 150, 160, 170, 180, 190, or 200 genes listed in any of Figures 10A, 10B, 10C, 10D, 10E, 10F, 10G, 10H, 10I, 10j, 10K, 10L, and 10M.

In some embodiments the panel-enriched sequencing reaction uses a sequencing panel that enriches for at most 20, 30, 40, 50, 60, 70, 80, 90, 100, 110, 120, 130, 140, 150, 160, 170, 180, 190, or 200 genes listed in any of Figures 10A, 10B, 10C, 10D, 10E, 10F, 10G, 10H, 10I, 10j, 10K, 10L, and 10M.

In some embodiments, the panel-enriched sequencing reaction uses a sequencing panel that enriches for between 25 different genes and 150 different genes listed in any of Figures 10A, 10B, 10C, 10D, 10E, 10F, 10G, 10H, 10I, 10j, 10K, 10L, and 10M. In some embodiments, the panel-enriched sequencing reaction uses a sequencing panel that enriches for between 50 genes and 150 genes, between 100 genes and 200 genes, or between 150 genes and 300 genes listed in any of Figures 10A, 10B, 10C, 10D, 10E, 10F, 10G, 10H, 10I, 10j, 10K, 10L, and 10M.

Generally, probes for enrichment of nucleic acids (e.g., cfDNA obtained from a liquid biopsy sample) include DNA, RNA, or a modified nucleic acid structure with a base sequence that is complementary to a locus of interest. For instance, a probe designed to hybridize to a locus in a cfDNA molecule can contain a sequence that is complementary to either strand, because the cfDNA molecules are double stranded. In some embodiments, each probe in the plurality of probes includes a nucleic acid sequence that is identical or complementary to at least 10, at least 11, at least 12, at least 13, at least 14, or at least 15 consecutive bases of a locus of interest. In some embodiments, each probe in the plurality of probes includes a nucleic acid sequence that is identical or complementary to at least 20, 25, 30, 40, 50, 75, 100, 150, 200, or more consecutive bases of a locus of interest.

Targeted panels provide several benefits for nucleic acid sequencing. For example, in some embodiments, algorithms for discriminating between, e.g., a first and second cancer condition can be trained on smaller, more informative data sets (e.g., fewer genes), which leads to more computationally efficient training of classifiers that discriminate between the first and second cancer states. Such improvements in computational efficiency, owing to the reduced size of the discriminating gene set, can advantageously either be used to speed up classifier training or be used to improve the performance of such classifiers (e.g., through more extensive training of the classifier).

In some embodiments, the gene panel is a whole-exome panel that analyzes the exomes of a biological sample. In some embodiments, the gene panel is a whole-genome panel that analyzes the genome of a specimen. In some preferred embodiments, the gene panel is optimized for use with liquid biopsy samples (e.g., to provide clinical decision support for solid tumors). See, for example, Table 1 above.

In some embodiments, the probes include additional nucleic acid sequences that do not share any homology to the loci of interest. For example, in some embodiments, the probes also include nucleic acid sequences containing an identifier sequence, e.g., a unique molecular identifier (UMI), e.g., that is unique to a particular sample or subject. Examples of identifier sequences are described, for example, in Kivioja et al., 2011, Nat. Methods 9(1), pp. 72-74 and Islam et al., 2014, Nat. Methods 11(2), pp. 163-66, which are incorporated by reference herein. Similarly, in some embodiments, the probes also include primer nucleic acid sequences useful for amplifying the nucleic acid molecule of interest, e.g., using PCR. In some embodiments, the probes also include a capture sequence designed to hybridize to an anti-capture sequence for recovering the nucleic acid molecule of interest from the sample.

Likewise, in some embodiments, the probes each include a non-nucleic acid affinity moiety covalently attached to nucleic acid molecule that is complementary to the locus of interest, for recovering the nucleic acid molecule of interest. Non-limited examples of non-nucleic acid affinity moieties include biotin, digoxigenin, and dinitrophenol. In some embodiments, the probe is attached to a solid-state surface or particle, e.g., a dipstick or magnetic bead, for recovering the nucleic acid of interest. In some embodiments, the methods described herein include amplifying the nucleic acids that bound to the probe set prior to further analysis, e.g., sequencing. Methods for amplifying nucleic acids, e.g., by PCR, are well known in the art.

Next-generation sequencing produces millions of short reads (e.g., sequence reads) for each biological sample. Accordingly, in some embodiments, the plurality of sequence reads obtained by next-generation sequencing of cfDNA molecules are DNA sequence reads. In some embodiments, the sequence reads have an average length of at least fifty nucleotides. In other embodiments, the sequence reads have an average length of at least 50, 60, 70, 80, 90, 100, 150, 200, 250, 300, or more nucleotides.

In some embodiments, sequencing is performed after enriching for nucleic acids (e.g., cfDNA, gDNA, and/or RNA) encompassing a plurality of predetermined target sequences, e.g., human genes and/or non-coding sequences associated with cancer. Advantageously, sequencing a nucleic acid sample that has been enriched for target nucleic acids, rather than all nucleic acids isolated from a biological sample, significantly reduces the average time and cost of the sequencing reaction. Accordingly, in some preferred embodiments, the methods described herein include obtaining a plurality of sequence reads of nucleic acids that have been hybridized to a probe set for hybrid-capture enrichment (e.g., of one or more genes listed in Table 1 or of one or more genes listed in Table 2, one or more genes listed in List 1, one or more genes listed in List 2, or one or more genes listed in Figure 10).

In some embodiments, panel-targeting sequencing is performed to an average on-target depth of at least 500x, at least 750x, at least 1000x, at least 2500x, at least 500x, at least 10,000x, or greater depth. In some embodiments, samples are further assessed for uniformity above a sequencing depth threshold *(e.g.,* 95% of all targeted base pairs at 300x sequencing depth). In some embodiments, the sequencing depth threshold is a minimum depth selected by a user or practitioner.

In some embodiments, the sequence reads are obtained by a whole genome or whole exome sequencing methodology. In some such embodiments, whole exome capture is performed with an automated system, using a liquid handling robot (for example, a SciClone NGSx). Whole genome sequencing, and to some extent whole exome sequencing, is typically performed at lower sequencing depth than smaller target-panel sequencing reactions, because many more loci are being sequenced. For example, in some embodiments, whole genome or whole exome sequencing is performed to an average sequencing depth of at least 3x, at least 5x, at least 10x, at least 15x, at least 20x, or greater. In some embodiments, low-pass whole genome sequencing (LPWGS) techniques are used for whole genome or whole exome sequencing. LPWGS is typically performed to an average sequencing depth of about 0.25x to about 5x, more typically to an average sequencing depth of about 0.5x to about 3x.

Because of the differences in the sequencing methodologies, data obtained from targeted-panel sequencing is better suited for certain analyses than data obtained from whole genome/whole exome sequencing, and vice versa. For instance, because of the higher sequencing depth achieved by targeted-panel sequencing, the resulting sequence data is better suited for the identification of variant alleles present at low allelic fractions in the sample, *e.g.,* less than 20%. By contrast, data generated from whole genome/whole exome sequencing is better suited for the estimation of genome-wide metrics, such as tumor mutational burden, because the entire genome is better represented in the sequencing data. Accordingly, in some embodiments, a nucleic acid sample, *e.g.,* a cfDNA, gDNA, or mRNA sample, is evaluated using both targeted-panel sequencing and whole genome/whole exome sequencing (*e.g.,* LPWGS).

In some embodiments, the raw sequence reads resulting from the sequencing reaction are output from the sequencer in a native file format, *e.g.,* a BCL file. In some embodiments, the native file is passed directly to a bioinformatics pipeline *(e.g.,* variant analysis 206), components of which are described in detail below. In other embodiments, pre-processing is performed prior to passing the sequences to the bioinformatics platform. For instance, in some embodiments, the format of the sequence read file is converted from the native file format *(e.g.,* BCL) to a file format compatible with one or more algorithms used in the bioinformatics pipeline *(e.g.,* FASTQ or FASTA). In some embodiments, the raw sequence reads are filtered to remove sequences that do not meet one or more quality thresholds. In some embodiments, raw sequence reads generated from the same unique nucleic acid molecule in the sequencing read are collapsed into a single sequence read representing the molecule, e.g., using UMIs as described above. In some embodiments, one or more of these pre-processing activities is performed within the bioinformatics pipeline itself.

In one example, a sequencer may generate a BCL file. A BCL file may include raw image data of a plurality of patient specimens which are sequenced. BCL image data is an image of the flow cell across each cycle during sequencing. A cycle may be implemented by illuminating a patient specimen with a specific wavelength of electromagnetic radiation, generating a plurality of images which may be processed into base calls via BCL to FASTQ processing algorithms which identify which base pairs are present at each cycle. The resulting FASTQ file includes the entirety of reads for each patient specimen paired with a quality metric, *e.g.,* in a range from 0 to 64 where a 64 is the best quality and a 0 is the worst quality. In embodiments where both a liquid biopsy sample and a normal tissue sample are sequenced, sequence reads in the corresponding FASTQ files may be matched, such that a liquid biopsy-normal analysis may be performed.

FASTQ format is a text-based format for storing both a biological sequence, such as a nucleotide sequence, and its corresponding quality scores. These FASTQ files are analyzed to determine what genetic variants or copy number changes are present in the sample. Each FASTQ file contains reads that may be paired-end or single reads, and may be short-reads or long-reads, where each read represents one detected sequence of nucleotides in a nucleic acid molecule that was isolated from the patient sample or a copy of the nucleic acid molecule, detected by the sequencer. Each read in the FASTQ file is also associated with a quality rating. The quality rating may reflect the likelihood that an error occurred during the sequencing procedure that affected the associated read. In some embodiments, the results of paired-end sequencing of each isolated nucleic acid sample are contained in a split pair of FASTQ files, for efficiency. Thus, in some embodiments, forward (Read 1) and reverse (Read 2) sequences of each isolated nucleic acid sample are stored separately but in the same order and under the same identifier.

In various embodiments, the bioinformatics pipeline may filter FASTQ data from the corresponding sequence data file for each respective biological sample. Such filtering may include correcting or masking sequencer errors and removing (trimming) low quality sequences or bases, adapter sequences, contaminations, chimeric reads, overrepresented sequences, biases caused by library preparation, amplification, or capture, and other errors.

While workflow 200 illustrates obtaining a biological sample, extracting nucleic acids from the biological sample, and sequencing the isolated nucleic acids, in some embodiments, sequencing data used in the improved systems and methods described herein (*e.g.,* which include improved methods for determining accurate circulating tumor fraction estimates) is obtained by receiving previously generated sequence reads, in electronic form.

In some embodiments, sequencing of the plurality of cell-free nucleic acids in the liquid biopsy sample of the subject is performed at a central laboratory or sequencing facility. In some such embodiments, the method comprises accessing one or more sequencing datasets and/or one or more auxiliary files, in electronic form, through a cloud-based interface. For example, a dataset can be obtained by performing a bioinformatics pipeline using tumor BAM files, normal BAM files, a human reference genome file, a target region BED file, a list of mappable regions of the genome, and/or a blacklist of recurrent problematic areas of the genome.

In some embodiments, the obtaining the dataset comprises accessing the dataset, in electronic form, through a cloud-based interface. For example, a dataset can comprise one or more outputs from a bioinformatics pipeline (*e.g.,* CNVkit outputs ".cns" and/or ".cnr").

Additional methods and embodiments for sequencing nucleic acids, including aligning and preprocessing sequence reads, are described in further detail above (see, Example Methods: Figure 2A: Example Workflow for Precision Oncology). Additional methods and embodiments for performing the presently disclosed methods at a distributed diagnostic and clinical environment are described in detail above (see, Example Methods: Figure 2B: Distributed Diagnostic and Clinical Environment). Other embodiments and/or any combinations, substitutions, additions or deletions thereof are possible, as will be apparent to one skilled in the art.

In some embodiments, the subject is a patient with a cancer. In some such embodiments, the cancer is a solid tumor cancer. In some embodiments, the cancer is ovarian cancer, cervical cancer, uveal melanoma, colorectal cancer, chromophobe renal cell carcinoma, liver cancer, endocrine tumor, oropharyngeal cancer, retinoblastoma, biliary cancer, adrenal cancer, neural, neuroblastoma, basal cell carcinoma, brain cancer, breast cancer, melanoma, non-clear cell renal cell carcinoma, glioblastoma, glioma, tumor of unknown origin, kidney cancer, gastrointestinal stromal tumor, medulloblastoma, bladder cancer, gastric cancer, bone cancer, non-small cell lung cancer, thymoma, low grade glioma, prostate cancer, clear cell renal cell carcinoma, skin cancer, thyroid cancer, sarcoma, testicular cancer, head and neck cancer, head and neck squamous cell carcinoma, meningioma, peritoneal cancer, endometrial cancer, pancreatic cancer, mesothelioma, esophageal cancer, small cell lung cancer, her2 negative breast cancer, solid tumor, ovarian serous carcinoma, hr+ breast cancer, uterine serous carcinoma, endometrial cancer, uterine corpus endometrial carcinoma, gastroesophageal junction adenocarcinoma, gallbladder cancer, chordoma, or papillary renal cell carcinoma. In some embodiments, the test subject is a patient in a clinical trial.

In some embodiments, the sequencing data is processed (*e.g.,* using sequence data processing module 141) to prepare it for genomic feature identification 385. For instance, in some embodiments as described above, the sequencing data is present in a native file format provided by the sequencer. Accordingly, in some embodiments, the system (*e.g.,* system 100) applies a pre-processing algorithm 142 to convert the file format (318) to one that is recognized by one or more upstream processing algorithms. For example, BCL file outputs from a sequencer can be converted to a FASTQ file format using the bcl2fastq or bcl2fastq2 conversion software (Illumina®). FASTQ format is a text-based format for storing both a biological sequence, such as nucleotide sequence, and its corresponding quality scores. These FASTQ files are analyzed to determine what genetic variants, copy number changes, *etc.,* are present in the sample.

In some embodiments, other preprocessing functions are performed, *e.g.,* filtering sequence reads 122 based on a desired quality, *e.g.,* size and/or quality of the base calling. In some embodiments, quality control checks are performed to ensure the data is sufficient for variant calling. For instance, entire reads, individual nucleotides, or multiple nucleotides that are likely to have errors may be discarded based on the quality rating associated with the read in the FASTQ file, the known error rate of the sequencer, and/or a comparison between each nucleotide in the read and one or more nucleotides in other reads that has been aligned to the same location in the reference genome. Filtering may be done in part or in its entirety by various software tools, for example, a software tool such as Skewer. See, Jiang et al., 2014, BMC Bioinformatics 15(182):1-12. FASTQ files may be analyzed for rapid assessment of quality control and reads, for example, by a sequencing data QC software such as AfterQC, Kraken, RNA-SeQC, FastQC, or another similar software program. For paired end reads, reads may be merged.

In some embodiments, when both a liquid biopsy sample and a normal tissue sample from the patient are sequenced, two FASTQ output files are generated, one for the liquid biopsy sample and one for the normal tissue sample. A 'matched' (e.g., panel-specific) workflow is run to jointly analyze the liquid biopsy-normal matched FASTQ files. When a matched normal sample is not available from the patient, FASTQ files from the liquid biopsy sample are analyzed in the 'tumor-only' mode. See, for example, Figure 4B. If two or more patient samples are processed simultaneously on the same sequencer flow cell, *e.g.,* a liquid biopsy sample and a normal tissue sample, a difference in the sequence of the adapters used for each patient sample barcodes nucleic acids extracted from both samples, to associate each read with the correct patient sample and facilitate assignment to the correct FASTQ file.

For efficiency, in some embodiments, the results of paired-end sequencing of each isolate are contained in a split pair of FASTQ files. Forward (Read 1) and reverse (Read 2) sequences of each tumor and normal isolate are stored separately but in the same order and under the same identifier. See, for example, Figure 4C. In various embodiments, the bioinformatics pipeline may filter FASTQ data from each isolate. Such filtering may include correcting or masking sequencer errors and removing (trimming) low quality sequences or bases, adapter sequences, contaminations, chimeric reads, overrepresented sequences, biases caused by library preparation, amplification, or capture, and other errors. See, for example, Figure 4D.

Similarly, in some embodiments, sequencing (312) is performed on a pool of nucleic acid sequencing libraries prepared from different biological samples, *e.g.,* from the same or different patients. Accordingly, in some embodiments, the system demultiplexes (320) the data *(e.g.,* using demultiplexing algorithm 144) to separate sequence reads into separate files for each sequencing library included in the sequencing pool, *e.g.,* based on UMI or patient identifier sequences added to the nucleic acid fragments during sequencing library preparation, as described above. In some embodiments, the demultiplexing algorithm is part of the same software package as one or more pre-processing algorithms 142. For instance, the bcl2fastq or bcl2fastq2 conversion software (Illumina®) include instructions for both converting the native file format output from the sequencer and demultiplexing sequence reads 122 output from the reaction.

The sequence reads are then aligned (322), *e.g.,* using an alignment algorithm 143, to a reference sequence construct 158, *e.g.,* a reference genome, reference exome, or other reference construct prepared for a particular targeted-panel sequencing reaction. For example, in some embodiments, individual sequence reads 123, in electronic form *(e.g.,* in FASTQ files), are aligned against a reference sequence construct for the species of the subject *(e.g.,* a reference human genome) by identifying a sequence in a region of the reference sequence construct that best matches the sequence of nucleotides in the sequence read. In some embodiments, the sequence reads are aligned to a reference exome or reference genome using known methods in the art to determine alignment position information. The alignment position information may indicate a beginning position and an end position of a region in the reference genome that corresponds to a beginning nucleotide base and end nucleotide base of a given sequence read. Alignment position information may also include sequence read length, which can be determined from the beginning position and end position. A region in the reference genome may be associated with a gene or a segment of a gene. Any of a variety of alignment tools can be used for this task.

For instance, local sequence alignment algorithms compare subsequences of different lengths in the query sequence (*e.g.,* sequence read) to subsequences in the subject sequence (*e.g.,* reference construct) to create the best alignment for each portion of the query sequence. In contrast, global sequence alignment algorithms align the entirety of the sequences, *e.g.,* end to end. Examples of local sequence alignment algorithms include the Smith-Waterman algorithm.

In some embodiments, the read mapping process starts by building an index of either the reference genome or the reads, which is then used to retrieve the set of positions in the reference sequence where the reads are more likely to align. Once this subset of possible mapping locations has been identified, alignment is performed in these candidate regions with slower and more sensitive algorithms. See, for example, Hatem et al., 2013, "Benchmarking short sequence mapping tools," BMC Bioinformatics 14: p. 184; and Flicek and Birney, 2009, "Sense from sequence reads: methods for alignment and assembly," Nat Methods 6(Suppl. 11), S6-S12, each of which is hereby incorporated by reference. In some embodiments, the mapping tools methodology makes use of a hash table or a Burrows-Wheeler transform (BWT). See, for example, Li and Homer, 2010, "A survey of sequence alignment algorithms for next-generation sequencing," Brief Bioinformatics 11, pp. 473-483, which is hereby incorporated by reference.

Other software programs designed to align reads include, for example, Novoalign (Novocraft, Inc.), Bowtie, Burrows Wheeler Aligner (BWA), and/or programs that use a Smith-Waterman algorithm. Candidate reference genomes include, for example, HG19, GRCh38, hg38, GRCh37, and/or other reference genomes developed by the Genome Reference Consortium. In some embodiments, the alignment generates a SAM file, which stores the locations of the start and end of each read according to coordinates in the reference genome and the coverage (number of reads) for each nucleotide in the reference genome.

For example, in some embodiments, each read of a FASTQ file is aligned to a location in the human genome having a sequence that best matches the sequence of nucleotides in the read. There are many software programs designed to align reads, for example, Novoalign (Novocraft, Inc.), Bowtie, Burrows Wheeler Aligner (BWA), programs that use a Smith-Waterman algorithm, *etc.* Alignment may be directed using a reference genome (for example, HG19, GRCh38, HG38, GRCh37, other reference genomes developed by the Genome Reference Consortium, *etc.)* by comparing the nucleotide sequences in each read with portions of the nucleotide sequence in the reference genome to determine the portion of the reference genome sequence that is most likely to correspond to the sequence in the read. In some embodiments, one or more SAM files are generated for the alignment, which store the locations of the start and end of each read according to coordinates in the reference genome and the coverage (number of reads) for each nucleotide in the reference genome. The SAM files may be converted to BAM files. In some embodiments, the BAM files are sorted, and duplicate reads are marked for deletion, resulting in de-duplicated BAM files.

In some embodiments, adapter-trimmed FASTQ files are aligned to the 19th edition of the human reference genome build (HG19). Following alignment, reads are grouped by alignment position and UMI family and collapsed into consensus sequences. Bases with insufficient quality or significant disagreement among family members (for example, when it is uncertain whether the base is an adenine, cytosine, guanine, *etc.)* may be replaced by N's to represent a wildcard nucleotide type. PHRED scores are then scaled based on initial base calling estimates combined across all family members. Following single-strand consensus generation, duplex consensus sequences are generated by comparing the forward and reverse oriented PCR products with mirrored UMI sequences. In various embodiments, a consensus can be generated across read pairs. Otherwise, single-strand consensus calls will be used. Following consensus calling, filtering is performed to remove low-quality consensus fragments. The consensus fragments are then re-aligned to the human reference genome using BWA. A BAM output file is generated after the re-alignment, then sorted by alignment position, and indexed.

In some embodiments, where both a liquid biopsy sample and a normal tissue sample are analyzed, this process produces a liquid biopsy BAM file *(e.g.,* Liquid BAM 124-1-i-cf) and a normal BAM file *(e.g.,* Germline BAM 124-1-i-g), as illustrated in Figure 4A. In various embodiments, BAM files may be analyzed to detect genetic variants and other genetic features, including single nucleotide variants (SNVs), copy number variants (CNVs), gene rearrangements, *etc.*

In some embodiments, the sequencing data is normalized, *e.g.,* to account for pull-down, amplification, and/or sequencing bias *(e.g.,* mappability, GC bias *etc.).*

In some embodiments, SAM files generated after alignment are converted to BAM files 124. Thus, after preprocessing sequencing data generated for a pooled sequencing reaction, BAM files are generated for each of the sequencing libraries present in the master sequencing pools. For example, as illustrated in Figure 4A, separate BAM files are generated for each of three samples acquired from subject 1 at time i *(e.g.,* tumor BAM 124-1-i-t corresponding to alignments of sequence reads of nucleic acids isolated from a solid tumor sample from subject 1, Liquid BAM 124-1-i-cf corresponding to alignments of sequence reads of nucleic acids isolated from a liquid biopsy sample from subject 1, and Germline BAM 124-1-i-g corresponding to alignments of sequence reads of nucleic acids isolated from a normal tissue sample from subject 1), and one or more samples acquired from one or more additional subjects at time j *(e.g.,* Tumor BAM 124-2-j-t corresponding to alignments of sequence reads of nucleic acids isolated from a solid tumor sample from subject 2). In some embodiments, BAM files are sorted, and duplicate reads are marked for deletion, resulting in de-duplicated BAM files. For example, tools like SamBAMBA mark and filter duplicate alignments in the sorted BAM files.

Many of the embodiments described below, in conjunction with Figure 4, relate to analyses performed using sequencing data from cfDNA of a cancer patient, e.g., obtained from a liquid biopsy sample of the patient. Generally, these embodiments are independent and, thus, not reliant upon any particular sequencing data generation methods, e.g., sample preparation, sequencing, and/or data pre-processing methodologies. However, in some embodiments, the methods described herein include one or more features 204 of generating sequencing data, as illustrated in Figures 2A and 3.

Alignment files prepared as described above (e.g., BAM files 124) are then passed to a feature extraction module 145, where the sequences are analyzed (324) to identify genomic alterations *(e.g.,* SNVs/MNVs, indels, genomic rearrangements, copy number variations, *etc.)* and/or determine various characteristics of the patient's cancer *(e.g.,* MSI status, TMB, tumor ploidy, HRD status, tumor fraction, tumor purity, methylation patterns, *etc.).* Many software packages for identifying genomic alterations are known in the art. Generally, these software packages identify variants in sorted SAM or BAM files 124, relative to one or more reference sequence constructs 158. The software packages then output a file *e.g.,* a raw VCF (variant call format), listing the variants *(e.g.,* genomic features 131) called and identifying their location relevant to the reference sequence construct *(e.g.,* where the sequence of the sample nucleic acids differ from the corresponding sequence in the reference construct). In some embodiments, system 100 digests the contents of the native output file to populate feature data 125 in test patient data store 120. In other embodiments, the native output file serves as the record of these genomic features 131 in test patient data store 120.

Generally, the systems described herein can employ any combination of available variant calling software packages and internally developed variant identification algorithms. In some embodiments, the output of a particular algorithm of a variant calling software is further evaluated, *e.g.,* to improve variant identification. Accordingly, in some embodiments, system 100 employs an available variant calling software package to perform some of all of the functionality of one or more of the algorithms shown in feature extraction module 145.

In some embodiments, as illustrated in Figure 1A, separate algorithms (or the same algorithm implemented using different parameters) are applied to identify variants unique to the cancer genome of the patient and variants existing in the germline of the subject. In other embodiments, variants are identified indiscriminately and later classified as either germline or somatic, e.g., based on sequencing data, population data, or a combination thereof. In some embodiments, variants are classified as germline variants, and/or non-actionable variants, when they are represented in the population above a threshold level, *e.g.,* as determined using a population database such as ExAC or gnomAD. For instance, in some embodiments, variants that are represented in at least 1% of the alleles in a population are annotated as germline and/or non-actionable. In other embodiments, variants that are represented in at least 2%, at least 3%, at least 4%, at least 5%, at least 7.5%, at least 10%, or more of the alleles in a population are annotated as germline and/or non-actionable. In some embodiments, sequencing data from a matched sample from the patient, *e.g.,* a normal tissue sample, is used to annotate variants identified in a cancerous sample from the subject. That is, variants that are present in both the cancerous sample and the normal sample represent those variants that were in the germline prior to the patient developing cancer and can be annotated as germline variants.

In various aspects, the detected genetic variants and genetic features are analyzed as a form of quality control. For example, a pattern of detected genetic variants or features may indicate an issue related to the sample, sequencing procedure, and/or bioinformatics pipeline (*e.g.,* example, contamination of the sample, mislabeling of the sample, a change in reagents, a change in the sequencing procedure and/or bioinformatics pipeline, *etc.).*

Figure 4E illustrates an example workflow for genomic feature identification (324). This particular workflow is only an example of one possible collection and arrangement of algorithms for feature extraction from sequencing data 124. Generally, any combination of the modules and algorithms of feature extraction module 145, *e.g.,* illustrated in Figure 1A, can be used for a bioinformatics pipeline, and particularly for a bioinformatics pipeline for analyzing liquid biopsy samples. For instance, in some embodiments, an architecture useful for the methods and systems described herein includes at least one of the modules or variant calling algorithms shown in feature extraction module 145. In some embodiments, an architecture includes at least 2, 3, 4, 5, 6, 7, 8, 9, 10, or more of the modules or variant calling algorithms shown in feature extraction module 145. Further, in some embodiments, feature extraction modules and/or algorithms not illustrated in Figure 1A find use in the methods and systems described herein.

In some embodiments, the methods and systems described herein use somatic mutations identified in a separate workstream of the liquid biopsy assay. For example, Figures 4F2 and 4G (4G1 - 4G3) illustrate methods 400-2 and 450, respectively, for identifying somatic sequence variants from cfDNA sequencing data using dynamic variant count thresholds. In some embodiments, such identified somatic variants are used in the determination of the 1TMB using the methods, systems, and CRM described herein. For more information on such methods for identifying somatic mutations from cfDNA samples see, for example, U.S. Patent No. 11,475,981, the disclosure of which is incorporated herein by reference, in its entirety, for all purposes.

In one aspect, the disclosure provides methods, as well as systems and CRM for executing all or a portion of such methods, for obtaining, from the panel-enriched sequencing reaction, a plurality of nucleic acid sequences comprising a corresponding sequence for each cell-free DNA fragment in a first plurality of cell-free DNA fragments obtained from a liquid biopsy sample from the test subject. For example, sequencing data 122-1 described herein, e.g., with respect to the sequencing 312 performed during wet lab portion 204 of the example liquid biopsy schema illustrated in Figure 2A.

In some embodiments, each respective cell-free DNA fragment in the first plurality of cell-free DNA fragments corresponds to a respective probe sequence in a plurality of probe sequences used to enrich cell-free DNA fragments in the liquid biopsy sample in the panel-enriched sequencing reaction. Example gene sets targeted by such probes are described, for example, with reference to Table 1, Table 2, List 1, List 2, and Figure 10 provided herein, as well as Figure 6 in PCT Patent Application Publication Number WO 2023/164713, the disclosure of which is hereby incorporated by reference, in its entirety, for all purposes. In some embodiments, the plurality of probe sequences map to no more than 150 genes in the human genome. In some embodiments, the plurality of probe sequences map to no more than 150 genes.

*Block 916.* Referring to block 916, in some embodiments, the liquid biopsy sample is a blood sample. For example, in some embodiments, the liquid biopsy sample comprises blood, whole blood, peripheral blood, plasma, serum, or lymph of the test subject. In some alternative embodiments, the liquid biopsy sample is any of the embodiments described above (see, Definitions: Liquid Biopsy and/or Example Methods: Figure 2A: Example Workflow for Precision Oncology).

In some embodiments the liquid biopsy sample is blood, whole blood, plasma, serum, urine, cerebrospinal fluid, fecal material, saliva, sweat, tears, pleural fluid, pericardial fluid, or peritoneal fluid of the subject. In some embodiments, the liquid biopsy sample is a cell-free sample, *e.g.,* a cell free blood sample. In some embodiments, the liquid biopsy sample is obtained from a subject with cancer. In some embodiments, the liquid biopsy sample is collected from a subject with an unknown cancer status. In some embodiments, the liquid biopsy is collected from a subject with a non-cancerous disorder, *e.g.,* a cardiovascular disease. In some embodiments, the liquid biopsy is collected from a subject with an unknown status for a non-cancerous disorder.

In some embodiments, one or more of the biological samples obtained from the patient are a biological liquid sample, also referred to as a liquid biopsy sample. In some embodiments, one or more of the biological samples obtained from the patient are selected from blood, plasma, serum, urine, vaginal fluid, fluid from a hydrocele *(e.g.,* of the testis), vaginal flushing fluids, pleural fluid, ascitic fluid, cerebrospinal fluid, saliva, sweat, tears, sputum, bronchoalveolar lavage fluid, discharge fluid from the nipple, aspiration fluid from different parts of the body *(e.g.,* thyroid, breast), *etc.* In some embodiments, the liquid biopsy sample includes blood and/or saliva. In some embodiments, the liquid biopsy sample is peripheral blood. In some embodiments, blood samples are collected from patients in commercial blood collection containers, *e.g.,* using a PAXGENE® Blood DNA Tubes. In some embodiments, saliva samples are collected from patients in commercial saliva collection containers, *e.g.,* using an ORAGENE® DNA Saliva Kit.

In some embodiments, the liquid biopsy sample has a volume of from about 1 mL to about 50 mL. For example, in some embodiments, the liquid biopsy sample has a volume of about 1 mL, about 2 mL, about 3 mL, about 4 mL, about 5 mL, about 6 mL, about 7 mL, about 8 mL, about 9 mL, about 10 mL, about 11 mL, about 12 mL, about 13 mL, about 14 mL, about 15 mL, about 16 mL, about 17 mL, about 18 mL, about 19 mL, about 20 mL, or greater.

Liquid biopsy samples include cell free nucleic acids, including cell-free DNA (cfDNA). As described above, cfDNA isolated from cancer patients includes DNA originating from cancerous cells, also referred to as circulating tumor DNA (ctDNA), cfDNA originating from germline (*e.g.,* healthy or non-cancerous) cells, and cfDNA originating from hematopoietic cells (*e.g.,* white blood cells). The relative proportions of cancerous and non-cancerous cfDNA present in a liquid biopsy sample varies depending on the characteristics (*e.g.,* the type, stage, lineage, genomic profile, *etc.)* of the patient's cancer. As used herein, the 'tumor burden' of the subject refers to the percentage cfDNA that originated from cancerous cells.

As described herein, cfDNA is a particularly useful source of biological data for various implementations of the methods and systems described herein, because it is readily obtained from various body fluids. Advantageously, use of bodily fluids facilitates serial monitoring because of the ease of collection, as these fluids are collectable by non-invasive or minimally invasive methodologies. This is in contrast to methods that rely upon solid tissue samples, such as biopsies, which often times require invasive surgical procedures. Further, because bodily fluids, such as blood, circulate throughout the body, the cfDNA population represents a sampling of many different tissue types from many different locations.

In some embodiments, a liquid biopsy sample is separated into two different samples. For example, in some embodiments, a blood sample is separated into a blood plasma sample, containing cfDNA, and a buffy coat preparation, containing white blood cells.

In some embodiments, a plurality of liquid biopsy samples is obtained from a respective subject at intervals over a period of time (*e.g.,* using serial testing). For example, in some such embodiments, the time between obtaining liquid biopsy samples from a respective subject is at least 1 day, at least 2 days, at least 1 week, at least 2 weeks, at least 1 month, at least 2 months, at least 3 months, at least 4 months, at least 6 months, or at least 1 year.

In some alternative embodiments, one or more biological samples collected from the patient are solid tissue samples, *e.g.,* a solid tumor sample or a solid normal tissue sample. Methods for obtaining solid tissue samples, *e.g.,* of cancerous and/or normal tissue are known in the art and are dependent upon the type of tissue being sampled. For example, bone marrow biopsies and isolation of circulating tumor cells can be used to obtain samples of blood cancers, endoscopic biopsies can be used to obtain samples of cancers of the digestive tract, bladder, and lungs, needle biopsies (*e.g.,* fine-needle aspiration, core needle aspiration, vacuum-assisted biopsy, and image-guided biopsy, can be used to obtain samples of subdermal tumors, skin biopsies, *e.g.,* shave biopsy, punch biopsy, incisional biopsy, and excisional biopsy, can be used to obtain samples of dermal cancers, and surgical biopsies can be used to obtain samples of cancers affecting internal organs of a patient. In some embodiments, a solid tissue sample is a formalin-fixed tissue (FFT). In some embodiments, a solid tissue sample is a macro-dissected formalin fixed paraffin embedded (FFPE) tissue. In some embodiments, a solid tissue sample is a fresh frozen tissue sample.

In some embodiments, a dedicated normal sample is collected from the patient, for co-processing with a liquid biopsy sample. Generally, the normal sample is of a non-cancerous tissue, and can be collected using any tissue collection means described above. In some embodiments, buccal cells collected from the inside of a patient's cheeks are used as a normal sample. Buccal cells can be collected by placing an absorbent material, e.g., a swab, in the subject's mouth and rubbing it against their cheek, *e.g.,* for at least 15 second or for at least 30 seconds. The swab is then removed from the patient's mouth and inserted into a tube, such that the tip of the tube is submerged into a liquid that serves to extract the buccal cells off of the absorbent material. An example of buccal cell recovery and collection devices is provided in U.S. Patent No. 9,138,205, the content of which is hereby incorporated by reference, in its entirety, for all purposes. In some embodiments, the buccal swab DNA is used as a source of normal DNA in circulating heme malignancies.

Referring to Figure 2, in some embodiments the biological samples collected from the patient are, optionally, sent to various analytical environments (*e.g.,* sequencing lab 230, pathology lab 240, and/or molecular biology lab 250) for processing (*e.g.,* data collection) and/or analysis (e.g., feature extraction). Wet lab processing 204 may include cataloguing samples (*e.g.,* accessioning), examining clinical features of one or more samples (*e.g.,* pathology review), and nucleic acid sequence analysis (*e.g.,* extraction, library prep, capture + hybridize, pooling, and sequencing). In some embodiments, the workflow includes clinical analysis of one or more biological samples collected from the subject, *e.g.,* at a pathology lab 240 and/or a molecular and cellular biology lab 250, to generate clinical features such as pathology features 128-3, imaging data 128-3, and/or tissue culture / organoid data 128-3.

In some embodiments, the pathology data 128-1 collected during clinical evaluation includes visual features identified by a pathologist's inspection of a specimen (*e.g.,* a solid tumor biopsy), *e.g.,* of stained H&E or IHC slides. In some embodiments, the sample is a solid tissue biopsy sample. In some embodiments, the tissue biopsy sample is a formalin-fixed tissue (FFT), *e.g.,* a formalin-fixed paraffin-embedded (FFPE) tissue. In some embodiments, the tissue biopsy sample is an FFPE or FFT block. In some embodiments, the tissue biopsy sample is a fresh-frozen tissue biopsy. The tissue biopsy sample can be prepared in thin sections (*e.g.,* by cutting and/or affixing to a slide), to facilitate pathology review (*e.g.,* by staining with immunohistochemistry stain for IHC review and/or with hematoxylin and eosin stain for H&E pathology review). For instance, analysis of slides for H&E staining or IHC staining may reveal features such as tumor infiltration, programmed death-ligand 1 (PD-L1) status, human leukocyte antigen (HLA) status, or other immunological features.

In some embodiments, a liquid sample (*e.g.,* blood) collected from the patient (*e.g.,* in EDTA-containing collection tubes) is prepared on a slide (*e.g.,* by smearing) for pathology review. In some embodiments, macrodissected FFPE tissue sections, which may be mounted on a histopathology slide, from solid tissue samples (*e.g.,* tumor or normal tissue) are analyzed by pathologists. In some embodiments, tumor samples are evaluated to determine, *e.g.,* the tumor purity of the sample, the percent tumor cellularity as a ratio of tumor to normal nuclei, *etc.* For each section, background tissue may be excluded or removed such that the section meets a tumor purity threshold, *e.g.,* where at least 20% of the nuclei in the section are tumor nuclei, or where at least 25%, 30%, 40%, 50%, 60%, 70%, 80%, 90%, or more of the nuclei in the section are tumor nuclei.

In some embodiments, pathology data 128-1 is extracted, in addition to or instead of visual inspection, using computational approaches to digital pathology, *e.g.,* providing morphometric features extracted from digital images of stained tissue samples. In some embodiments, pathology data 128-1 includes features determined using machine learning algorithms to evaluate pathology data collected as described above.

Further details on methods, systems, and algorithms for using pathology data to classify cancer and identify targeted therapies are discussed, for example, in U.S. Patent No. 10,957,041, 11,244,763, 11,848,107, and 11,145,416, the contents of which are each hereby incorporated by reference, in their entireties, for all purposes.

In some embodiments, imaging data 128-2 collected during clinical evaluation includes features identified by review of in-vitro and/or in-vivo imaging results (*e.g.,* of a tumor site), for example a size of a tumor, tumor size differentials over time (such as during treatment or during other periods of change). In some embodiments, imaging data 128-2 includes features determined using machine learning algorithms to evaluate imaging data collected as described above.

Further details on methods, systems, and algorithms for using medical imaging to classify cancer and identify targeted therapies are discussed, for example, in U.S. Patent No. 10,957,041, 11,244,763, 11,848,107, and 11,145,416, the contents of which are each hereby incorporated by reference, in their entireties, for all purposes.

In some embodiments, tissue culture / organoid data 128-3 collected during clinical evaluation includes features identified by evaluation of cultured tissue from the subject. For instance, in some embodiments, tissue samples obtained from the patients (*e.g.,* tumor tissue, normal tissue, or both) are cultured (*e.g.,* in liquid culture, solid-phase culture, and/or organoid culture) and various features, such as cell morphology, growth characteristics, genomic alterations, and/or drug sensitivity, are evaluated. In some embodiments, tissue culture / organoid data 128-3 includes features determined using machine learning algorithms to evaluate tissue culture / organoid data collected as described above. Examples of tissue organoid (*e.g.,* personal tumor organoid) culturing and feature extractions thereof are described in PCT publication No. WO2021/081253 and U.S. Patent No. 11,629,385, the contents of which are each hereby incorporated by reference, in their entireties, for all purposes.

In some embodiments, the method further comprises obtaining the liquid biopsy sample from a sample repository or database (*e.g.,* BioIVT, TSC Biosample Repository, BioLINCC, *etc.*). In some embodiments, the liquid biopsy sample is obtained from the subject at least 1 hour, at least 2 hours, at least 12 hours, at least 1 day, at least 2 days, at least 1 week, at least 1 month, or at least 1 year prior to processing and/or sequencing the liquid biopsy sample. In some such embodiments, the liquid biopsy sample is fresh, frozen, dried, and/or fixed. In some embodiments, the liquid biopsy sample is processed and/or sequenced at least 1 day, at least 2 days, at least 1 week, at least 1 month, or at least 1 year prior to obtaining the first dataset. For example, in some embodiments, the sequencing data for the liquid biopsy sample are obtained from a data repository (*e.g.,* GenBank, NCBI Assembly, DNA DataBank of Japan, European Nucleotide Archive, European Variation Archive, *etc.*).

*Block 918.* Referring to block 918, there is determined, using the panel-enriched sequencing reaction, that a circulating tumor fraction (ctFE) satisfies (*e.g.,* is above) a threshold ctFE value. A ctFE refers to the proportion of tumor-derived material in the bloodstream of a cancer subject. This material can include circulating tumor cells (CTCs), cell-free DNA (cfDNA), or other components shed by tumors into the fluids of a subject, such as their bloodstream. The ctFE provides information about the extent of tumor burden, disease progression, treatment response, and the presence of minimal residual disease. Monitoring changes in CTF over time can help in assessing the effectiveness of cancer therapies, detecting early signs of metastasis or recurrence, and guiding treatment decisions.

Figure 4F3 illustrates a method 400-3 for estimating the circulating tumor fraction of a liquid biopsy sample by matching simulated tumor fractions to copy number states generated from cfDNA sequencing data. In some embodiments, such circulating tumor fraction estimates (ctFE) are used in the methods, systems, and CRM described herein. For more information on such methods for estimating the circulating tumor fraction of a liquid biopsy sample see, for example, U.S. Patent No. 11,211,147, the disclosure of which is incorporated herein by reference, in its entirety, for all purposes.

In some embodiments, any of the methods used for calculating ctFE disclosed in U.S. Patent No. 11,211,147 are used.

In some embodiments, the ctFE is determined according to any of the methods described in U.S. Application Number 18/930,786, entitled "ESTIMATION OF CIRCULATING TUMOR FRACTION USING OFF-TARGET READS OF TARGETED-PANEL SEQUENCING," filed October 29, 2024, the disclosure of which is hereby incorporated by reference, in its entirety, for all purposes, and this ctFE value is used to threshold (gate) the bTMB determination (*e.g.,* only make the bTMB determination when the ctFE value satisfies a threshold).

In some embodiment, the ctFE is determined using targeted sequencing of at least 10, 20, 30, 40, 50, 60, 70, 80, 90 or all the genes in Table 1.

In some embodiment, the ctFE is determined using targeted sequencing of at least 10, 20, 30, 40, 50, 60, 70, 80, 90 or all the genes in Table 2.

In some embodiment, the ctFE is determined using targeted sequencing of at least 10, 20, 30, 40, 50, 60, 70, 80, 90 or all the genes in Table 1, where the oncologic targets in these genes are any combination of: single-nucleotide variants (SNVs), insertions/deletions (indels), copy number variants (CNVs), and gene rearrangements.

In some embodiments, the ctFE is determined according to any of the methods described in Finkle, 2021, "Validation of a liquid biopsy assay with molecular and clinical profiling of circulating tumor DNA," npj Precision Oncology 5(63), the disclosure of which is hereby incorporated by reference, in its entirety, for all purposes, and this ctFE value is used to threshold (gate) the bTMB determination (*e.g.,* only make the bTMB determination when the ctFE value satisfies a threshold).

In some embodiments, the method also includes determining, using the panel-enriched sequencing reaction, that the ctFE is above a threshold ctFE value. For example, in some embodiments, a circulating tumor fraction estimate prepared according to a method described herein, *e.g.,* with reference to the method 400-3 illustrated in Figure 4F3, is used in such a step to threshold, or gate, the bTMB determination. In other words, the bTMB determination is only made if the ctFE value calculated for the subject is above a threshold value.

*Block 920.* Referring to block 920, in some embodiments, the threshold ctFE value is 0.01. In some embodiments, the threshold ctFE value is 0.0025. In some embodiments, the threshold ctFE value is between 0.001 and 0.015. In some embodiments, the threshold ctFE value is between 0.005 and 0.015. In some embodiments, the threshold ctFE value is between 0.015 and 0.025. In some embodiments, the threshold ctFE value is between 0.025 and 0.035. In some embodiments, the threshold ctFE value is between 0.035 and 0.045. In some embodiments, the threshold ctFE value is between 0.045 and 0.055.

*Block 922.* Referring to block 922, responsive to determining that the ctFE is above the threshold, the liquid biopsy tumor mutational burden (ITMB) for the test subject is calculated from the panel-enriched sequencing reaction. As used herein, the 1TMB refers to the number of mutations in the cell-free DNA of a liquid biopsy sample that originate from tumor cells of the subject. In some embodiments, the 1TMB is calculated by determining the total number of mutations detected in the panel-enriched sequencing reaction, typically per megabase (Mb) of DNA. In some embodiments, the 1TMB is reported as mutations per megabase (mut/Mb).

In some embodiments, all nonsilent somatic coding variations such as missense, indel, and stop-loss variants with coverage greater than $\times$ 100 and an allelic fraction greater than 5% are included in the count of nonsynonymous variations.

*Block 924.* Referring to block 924, in some embodiments, the calculating comprises counting the plurality of genetic variants present in the plurality of nucleic acid sequences. In such counting, because the panel-enriched sequencing reaction enriched for those genes that are known to be mutated in cancer cells, any somatic mutations detected in the panel-enriched sequencing reaction are presumed to arise from the tumor and thus contribute to the count. In some embodiments, a check is made to be sure that the genetic variants present in the plurality of nucleic acid sequences are not germ line, and any germ line mutations are removed from the count. In some embodiments a check for whether such genetic variants are germ line is not performed. Rather metrics associated with variant allele frequency and other criteria, discussed below beginning with block 2026, are used to filter out genetic variants from the count.

*Block 926.* Referring to block 926, in some embodiments, the count of the plurality of genetic variants present in the plurality of nucleic acid sequences is a count of unique genetic variants present in the plurality of nucleic acid sequences that satisfy one or more qualifying criterion in a set of qualifying criteria. That is, only those genetic variants that satisfy one or more qualifying criterion in a set of qualifying criteria contribute to the of the plurality of genetic variants present in the plurality of nucleic acid sequences used in the 1TMB calculation.

*Block 928.* Referring to block 928, in some embodiments, a qualifying criterion in the set of qualifying criteria is a requirement that the respective genetic variant is a missense variant, a combination of a missense variant and a splice region variant, a frameshift variant, a stop loss variant, a splice acceptor variant, an in frame insertion variant, an in frame deletion variant, a combination of a frameshift variant and a splice region variant, a disruptive in frame insertion variant, or a disruptive in frame deletion variant.

In some embodiments the set of qualifying criteria consists of any 1, 2, 3, 4, 5, 6, 7, 8, 9 or all 10 of the qualifying criteria in the group consisting of (i) a requirement that the respective genetic variant is a missense variant, (ii) a combination of a missense variant and a splice region variant, (iii) a frameshift variant, (iv) a stop loss variant, (v) a splice acceptor variant, (vi) an in frame insertion variant, (vii) an in frame deletion variant, (viii) a combination of a frameshift variant and a splice region variant, (ix) a disruptive in frame insertion variant, and (x) a disruptive in frame deletion variant. In such embodiments, genetic variants that meet any of the qualifying criterion in the set of qualifying criteria are removed from the count of genetic variants.

In some embodiments, a qualifying criterion in the set of qualifying criteria is a requirement that the respective genetic variant is a missense variant. A missense variant in a nucleic acid sequence is a type of genetic mutation that results in a single nucleotide change within the nucleic acid sequence of a gene, leading to the gene to substitute one amino acid for another in the corresponding protein. This mutation occurs when a single nucleotide substitution alters the codon (a sequence of three nucleotides) of a gene, causing it to code for a different amino acid.

In some embodiments, a qualifying criterion in the set of qualifying criteria is a requirement that the respective genetic variant is a combination of a missense variant and a splice region variant. That is, the original nucleic acid molecule represented by sequence reads in the panel-enriched sequencing reaction contain both a missense variant and a splice region variant. A splice region variant is a type of genetic mutation that occurs in the non-coding regions of a gene, specifically in the sequences that are involved in the process of RNA splicing. RNA splicing is a step in the expression of genes, where introns (non-coding regions) are removed from the pre-mRNA and exons (coding regions) are joined together to form the mature mRNA transcript. Splice region variants can affect this splicing process, leading to alterations in the mRNA transcript and potentially impacting protein expression. Splice region variants occur within the intronic sequences flanking exon-intron boundaries. These regions contain conserved sequences, such as the splice donor site (5' splice site), splice acceptor site (3' splice site), and branch point sequence, which are essential for the splicing machinery to recognize and splice out introns accurately. Thus, a combination of a missense variant and a splice region variant refers to a scenario where a genetic mutation affecting both the coding region and the non-coding region (splice site) of a gene is present within the same allele in tumor cells.

In some embodiments, a qualifying criterion in the set of qualifying criteria is a requirement that the respective genetic variant is a frameshift variant. A frameshift variant is a type of genetic mutation that results from the insertion or deletion of a number of nucleotides in a DNA sequence that is not divisible by three. This insertion or deletion alters the reading frame (the way in which the sequence of nucleotides is read in groups of three, known as codons) of the gene during translation, causing a disruption in the normal protein-coding sequence.

In some embodiments, a qualifying criterion in the set of qualifying criteria is a requirement that the respective genetic variant is a stop loss variant. A stop loss variant, also known as a non-stop mutation or readthrough mutation, is a type of genetic mutation that affects the termination codon (stop codon) in the coding region of a gene. Stop codons are signals in the genetic code that instruct the ribosome to stop translation of the mRNA strand and release the newly synthesized protein. However, in the case of a stop loss variant, a mutation occurs that alters a stop codon to a codon encoding an amino acid, thereby extending the length of the protein encoded by a gene.

In some embodiments, a qualifying criterion in the set of qualifying criteria is a requirement that the respective genetic variant is a splice acceptor variant. A splice acceptor variant, also known as a splice site variant or a splice donor mutation, is a type of genetic mutation that affects the splice acceptor site at the boundary between an exon and an intron in a gene's DNA sequence. Splice acceptor sites are specific sequences of nucleotides located at the exon-intron boundary in a gene's DNA sequence. They play a role in the process of RNA splicing, which removes introns and joins exons together to form a mature mRNA transcript. The splice acceptor site marks the 3' end of an exon and signals the spliceosome to recognize and remove the intron during splicing. A splice acceptor variant occurs when a mutation disrupts or alters the consensus sequence of the splice acceptor site. This disruption can interfere with the recognition of the splice site by the spliceosome, leading to errors in the splicing process. The presence of a splice acceptor variant can result in aberrant splicing patterns, such as exon skipping, intron retention, or the activation of cryptic splice sites. These abnormal splicing events can produce mRNA transcripts with altered exon composition, leading to the synthesis of abnormal or truncated proteins.

In some embodiments, a qualifying criterion in the set of qualifying criteria is a requirement that the respective genetic variant is an in frame insertion variant. An in-frame insertion variant is a type of genetic mutation that involves the insertion of nucleotides into a DNA sequence in such a way that the reading frame of the gene remains intact. In other words, the number of nucleotides inserted is divisible by three, meaning that the insertion does not disrupt the triplet codon structure of the gene. The insertion of coding for such amino acids may alter the structure and function of the protein to varying degrees depending on the specific location and sequence of the insertion.

In some embodiments, a qualifying criterion in the set of qualifying criteria is a requirement that the respective genetic variant is an in frame deletion variant. An in-frame deletion variant is a type of genetic mutation that involves the deletion of a number of nucleotides from a DNA sequence in such a way that the reading frame of the gene remains intact. In other words, the number of nucleotides deleted is divisible by three, ensuring that the triplet codon structure of the gene is maintained. The deletion of coding for these amino acids may alter the structure and function of the protein encoded by the nucleic acid, to varying degrees depending on the specific location and sequence of the deletion.

In some embodiments, a qualifying criterion in the set of qualifying criteria is a requirement that the mutation be a combination of a frameshift variant and a splice region variant. The combination of a frameshift variant and a splice region variant in the same gene locus can result in a complex genetic alteration that affects both the coding and non-coding regions of the gene. The frameshift variant may lead to the production of a truncated protein with altered or loss-of-function, while the splice region variant may further exacerbate these effects by causing aberrant splicing and the generation of abnormal mRNA transcripts.

In some embodiments, a qualifying criterion in the set of qualifying criteria is a requirement that the mutation be a disruptive in frame insertion variant. A disruptive in-frame insertion variant is a type of genetic mutation that involves the insertion of nucleotides into a DNA sequence in a way that disrupts the normal reading frame of the gene encoded by the DNA sequence but still maintains the reading frame to some extent.

In some embodiments, a qualifying criterion in the set of qualifying criteria is a requirement that the mutation be a disruptive in frame deletion variant. A disruptive in-frame deletion variant is a type of genetic mutation that involves the deletion of nucleotides from a DNA sequence in a manner that maintains the reading frame of the gene but leads to significant changes in the protein sequence encoded by the gene and potentially affects its structure and function.

*Blocks 930* - 932. Referring to block 930, in some embodiments, a qualifying criterion in the set of qualifying criteria is a requirement that the respective genetic variant has a variant allele frequency in the liquid biopsy sample that is greater than 0.005 (0.5%). Referring to block 2032, in some embodiments, a qualifying criterion in the set of qualifying criteria is a requirement that the respective genetic variant has a variant allele frequency in the liquid biopsy sample that is less than 1.0 (100%). The variant allele frequency (VAF) for a respective genetic variant is calculated by dividing the number of unique DNA fragments represented by the plurality of nucleic acid sequences that contain the respective genetic variant by the total number of unique DNA fragments represented by the plurality of nucleic acid sequences that map to the locus of the respective genetic variant in the human genome. Thus, for example, consider the case in which the plurality of nucleic acid sequences include sequence reads for 13 unique DNA fragments that contain the genetic variant, where the genetic variant is to one of the genes that is enriched by the panel-enriched sequencing reaction. Moreover, in this example the plurality of nucleic acid sequences include sequence reads for 1000 unique DNA fragments that map to the locus of the genetic variant. In this instance, the variant allele frequency for this genetic variant would be 13 / 1000 or 0.013.

*Block 934.* Referring to block 934, in some embodiments, a qualifying criterion in the set of qualifying criteria is a requirement that the respective genetic variant has a variant allele frequency (VAF) in the liquid biopsy sample that is any one of: (i) greater than 0.01 (1%) and less than 0.4 (40%), (ii) greater than 0.6 (60%) and less than 0.9 (90%), (iii) greater than 0.4 (40%) and less than 0.60 (60%) with the proviso that |VAF-ctFE| / ctFE < 1, or (iv) greater than 0.9 (90%) with the proviso that |VAF-ctFE| / ctFE < 1.

Here it is understood that a VAF represents the proportion of DNA molecules in a sample that carry a specific genetic variant. It ranges from 0 (no variant) to 1 (100% of the DNA carries the variant).

This filter seeks to retain genetic variants with certain VAF ranges. The filter has four criteria for inclusion. Satisfaction of any one of the criterion causes a variant to contribute toward the variant counts. Those variants that do not satisfy any of these criteria do not contribute to the variant count. As such, these criteria are retention criteria.

Criterion (i): variants with VAF between 1% and 40% are retained. Without intending to be limited by any particular theory, this range may represent variants that occur at relatively low frequencies, potentially indicating subclonal tumor populations or rare mutations.

Criterion (ii): variants with VAF between 60% and 90% are retained. Without intending to be limited to any particular theory, variants in this range may be frequent enough to potentially represent clonal events but are not at a frequency suggesting near-homozygosity. These variants may correspond to mutations present in a significant proportion of tumor cells.

Criterion (iii): variants with VAF between 40% and 60% are retained if:

$$\frac{|VAF - ctFE|}{ctFE} < 1$$

This condition ensures the VAF of the variant is meaningfully close to the reference value (*ctFE*) but not too far off. For example, if *VAF* = 50% and *ctFE* = 48% then:

$$\frac{|50 - 48|}{48} = \frac{2}{48} \approx 0.042$$

Since this is less than 1, the variant is retained.

Criterion (iv): variants with VAF greater than 90% are retained if:

$$\frac{|VAF - ctFE|}{ctFE} < 1$$

This criterion retains variants that are at very high frequencies (likely homozygous or clonal) if they align closely with the reference (ctFE).

In some embodiments, a qualifying criterion in the set of qualifying criteria is a requirement that the respective genetic variant has a variant allele frequency (VAF) in the liquid biopsy sample that is any one of: (i) greater than 0.0025 and less than 0.4 (40%), (ii) greater than 0.6 (60%) and less than 0.9 (90%), (iii) greater than 0.4 (40%) and less than 0.60 (60%) with the proviso that |VAF-ctFE| / ctFE < 1, or (iv) greater than 0.9 (90%) with the proviso that |VAF-ctFE| / ctFE < 1.

In some embodiments, a qualifying criterion in the set of qualifying criteria is a requirement that the respective genetic variant has a variant allele frequency (VAF) in the liquid biopsy sample that is any one of: (i) greater than 0.0025 and less than A, (ii) greater than B and less than C, (iii) greater than A and less than B with the proviso that |VAF-ctFE| / ctFE < 1, or (iv) greater than C with the proviso that |VAF-ctFE| / ctFE < 1. In some such embodiments A is a value between 30% and 50%, B is a value between 55% and 70% and C is a value between 80% and 95%.

In some embodiments, a qualifying criterion in the set of qualifying criteria is a requirement that the respective genetic variant has a variant allele frequency (VAF) in the liquid biopsy sample that is any one of: (i) greater than D and less than A, (ii) greater than B and less than C, (iii) greater than A and less than B with the proviso that |VAF-ctFE| / ctFE < 1, or (iv) greater than C with the proviso that |VAF-ctFE| / ctFE < 1. In some such embodiments A is a value between 30% and 50%, B is a value between 55% and 70% and C is a value between 80% and 95%, and D is a value between 0.0002 and 0.02.

In some embodiments, a qualifying criterion in the set of qualifying criteria is a requirement that the respective genetic variant has a variant allele frequency (VAF) in the liquid biopsy sample that is any one of: (i) greater than D and less than A, (ii) greater than B and less than C, (iii) greater than A and less than B with the proviso that |VAF-ctFE| / ctFE < P, or (iv) greater than C with the proviso that |VAF-ctFE| / ctFE < Q. In some such embodiments A is a value between 30% and 50%, B is a value between 55% and 70% and C is a value between 80% and 95%, D is a value between 0.0002 and 0.02, P is a value between 0.8 and 1.2 and Q, which is the same as or different than P, is a value between 0.8 and Q.

Criterion (i): variants with VAF between D and A are retained, where D is selected from the range 0.0002 to 0.02 and A is selected from the range 30% to 50%.

Criterion (ii): variants with VAF between B and C are retained, were B is selected from the range 55% to 70% and C is selected from the rante 80% to 95%.

Criterion (iii): variants with VAF between A and B are retained if:

$$\frac{|VAF - ctFE|}{ctFE} < \text{P}$$

where P is a value selected from the range 0.8 to 1.2.

Criterion (iv): variants with VAF greater than C are retained if:

$$\frac{|VAF - ctFE|}{ctFE} < \text{Q}$$

where Q is a value selected from the range 0.8 to 1.2 independent of the value of P.

*Block 936.* Referring to block 936, in some embodiments, a qualifying criterion in the set of qualifying criteria is selection of a genetic variant present in the plurality of nucleic acid sequences by a medical professional. In other words, a medical professional specifically flagged the genetic variant as being one that should contribute to the ITMB.

*Block 2038.* Referring to block 938, in some embodiments, the calculating further comprises normalizing the count of the plurality of genetic variants present in the plurality of nucleic acid sequences by the coverage of the plurality of probe sequences. In some such embodiments, the count of the plurality of genetic variants is normalized by dividing the count of the plurality of genetic variants by the total size of the panel associated with the panel-enriched sequencing reaction. For example, if the panel-enriched sequencing reaction includes probes that collectively map to 3 megabases of the human genome, the ITMB is computed by dividing the count of the plurality of genetic variants (that satisfy an applicable qualifying criterion in the set of qualifying criteria) by 3 Mb.

*Blocks 940 - 942.* Referring to block 940, in some embodiments, the coverage of the panel-enriched sequencing reaction is from 0.1 megabases to 0.4 megabases. Referring to block 2042, in some embodiments, the coverage of the panel-enriched sequencing reaction is from 0.15 megabases to 0.3 megabases. In some embodiments, the coverage of the panel-enriched sequencing reaction is between 0.1 megabases and 15 megabases. In some embodiments, the coverage of the panel-enriched sequencing reaction is between 0.2 megabases and 30 megabases. In some embodiments, the coverage of the panel-enriched sequencing reaction is at least 0.2 megabases, 0.3 megabases, 0.4 megabases, 0.5 megabases, 0.6 megabases, or 0.7 megabases.

*Block 944.* Referring to block 944, in some embodiments, the ITMB is reported for the test subject. Examples of suitable reports are described in conjunction with Figure 1 above, e.g., clinical assessment 139-1, clinical report 139-1-3, and reporting module 180.

In some embodiments, the method further comprises generating a report *(e.g.,* for use by a physician) comprising the ITMB for the biological sample of the respective test subject. In some such embodiments, the generated report further comprises matched therapies (e.g., treatments and/or clinical trials) based on the ITMB status of the sample.

In some embodiments, the method further comprises disease screening and/or monitoring over a plurality of time points. For example, in some embodiments, the method is used for monitoring disease progression and/or recurrence after treatment, for assessing the efficacy of a treatment, and/or for performing comparative studies using liquid biopsy samples and matched solid tissue samples.

When a subject is determined to have a high tumor mutational burden (TMB), it suggests that their tumor has a large number of genetic mutations. This has clinical implications, particularly in terms of treatment with immune checkpoint inhibitors (ICIs), such as anti-PD-1/PD-L1 or anti-CTLA-4 therapies. A high TMB often correlates with increased tumor neoantigens, making the cancer more likely to respond to immunotherapy. Thus, in some embodiments, when the methods of the present disclosure determine that a cancer subject has a high TMB, the subject is treated with an immune checkpoint inhibitor (ICIs), such as anti-PD-1/PD-L1 or anti-CTLA-4 therapy. In particular, in some embodiments, when the methods of the present disclosure determine that a cancer subject has a high TMB, the subject is treated with pembrolizumab (Keytruda). In some embodiments, when the methods of the present disclosure determine that a cancer subject has a high TMB, the subject is treated with an immune checkpoint inhibitor in addition to the cancer drug(s) applicable to the underlying cancer.

High TMB is a biomarker for the potential effectiveness of ICIs. Drugs like pembrolizumab (Keytruda) are FDA-approved for tumors with high TMB ($\geq$10 mutations per megabase) in a tumor-agnostic manner, meaning across different cancer types.

In some embodiments, when the methods of the present disclosure determine that a cancer subject has a high TMB, further testing is performed, such as PD-L1 expression and immune infiltrate, in order to develop a comprehensive cancer treatment strategy.

In some embodiments, when a subject is treated with an immune checkpoint inhibitor the subject is further closely monitored for response and potential immune-related adverse effects (irAEs), which can affect organs like the liver, skin, lungs, and endocrine glands.

In some embodiments, the cancer is lung cancer, metastatic melanoma, colorectal cancer, head and neck cancers, bladder cancer, endometrial cancer, or a cancer of unknown primary, each of which has been shown to be responsive to ICIs when the cancer is high MSI (MSI-H).

In some embodiments, the systems and methods of the present disclosure determine that a subject has high TMB colon cancer. High TMB is often found in mismatch repair-deficient (dMMR) or microsatellite instability-high (MSI-H) colorectal cancers, which respond well to pembrolizumab and nivolumab. Accordingly, in some embodiments, the subject is treated with pembrolizumab and/or nivolumab when the systems and methods of the present disclosure determine that a subject has high TMB colon cancer.

In some embodiments, the methods described herein include generating a clinical report 139-3 *(e.g.,* a patient report), providing clinical support for personalized cancer therapy, and/or using the information curated from sequencing of a liquid biopsy sample, as described above. In some embodiments, the report is provided to a patient, physician, medical personnel, or researcher in a digital copy (for example, a JSON object, a pdf file, or an image on a website or portal), a hard copy (for example, printed on paper or another tangible medium). A report object, such as a JSON object, can be used for further processing and/or display. For example, information from the report object can be used to prepare a clinical laboratory report for return to an ordering physician. In some embodiments, the report is presented as text, as audio (for example, recorded or streaming), as images, or in another format and/or any combination thereof.

The report includes information related to the specific characteristics of the patient's cancer, *e.g.,* ITMB, detected genetic variants, epigenetic abnormalities, associated oncogenic pathogenic infections, and/or pathology abnormalities. In some embodiments, other characteristics of a patient's sample and/or clinical records are also included in the report. For example, in some embodiments, the clinical report includes information on clinical variants, *e.g.,* one or more of copy number variants *(e.g.,* for actionable genes CCNE1, CD274(PD-L1), EGFR, ERBB2(HER2), MET, MYC, BRCA1, and/or BRCA2), fusions, translocations, and/or rearrangements (e.g., in actionable genes ALK, ROS1, RET, NTRK1, FGFR2, FGFR3, NTRK2 and/or NTRK3), pathogenic single nucleotide polymorphisms, insertion-deletions (e.g., somatic/tumor and/or germline/normal), therapy biomarkers, microsatellite instability status, and/or tumor mutational burden.

In some embodiments, the results are used to design cell-based studies of the patient's biology, e.g., tumor organoid experiments. For example, an organoid may be genetically engineered to have the same characteristics as the specimen and may be observed after exposure to a therapy to determine whether the therapy can reduce the growth rate of the organoid, and thus may be likely to reduce the growth rate of cancer in the patient associated with the specimen. Similarly, in some embodiments, the results are used to direct studies on tumor organoids derived directly from the patient. An example of such experimentation is described in U.S. Provisional Patent Application No. 62/944,292, filed December 5, 2019, the content of which is hereby incorporated by reference, in its entirety, for all purposes.

As illustrated in Figure 2A, in some embodiments, a clinical report is checked for final validation, review, and sign-off by a medical practitioner (e.g., a pathologist). The clinical report is then sent for action *(e.g.,* for precision oncology applications).

*Longitudinal Reporting.* In various embodiments, a report may include and/or compare the results of multiple liquid biopsy tests and/or solid tumor tests (for example, multiple tests associated with the same patient). The results of multiple liquid biopsy tests and/or solid tumor tests may be displayed on a portal in a variety of configurations that may be selected and/or customized by the viewer. The tests may have been performed at different times, and the samples on which the tests were performed may have been collected at different times.

*Download result.* Clinical and/or molecular data associated with a patient (for example, information that would be included in the report), may be aggregated and made available via the portal. Any portion of the report data may be available for download (for example, as a CSV file) by the physician and/or patient. In various embodiments, the data may include data related to genetic variants, RNA expression levels, immunotherapy markers (including MSI and TMB), RNA fusions, *etc.* In one embodiment, if a physician or medical facility has ordered multiple tests (all tests may be associated with the same patient or tests may be associated with multiple patients), results associated with more than one test may be aggregated into a single file for downloading.

*Block 946.* Referring to block 946, in some embodiments, the reporting further comprises, responsive to determining that the ITMB satisfies a therapeutic threshold, reporting a matched therapeutic recommendation for the test subject. Such a therapeutic threshold is necessarily application dependent. For instance, it will vary depending on the type of cancer the test is intended to monitor, the stage(s) of cancer the test is intended to monitor, and/or the identity of genes included in the sequencing panel for the panel-enriched sequencing reaction, to name a few nonlimiting variables that may affect the value of the therapeutic threshold.

*Block 948.* Referring to block 948, in some embodiments, the reporting comprises comparing the ITMB for the subject to a severity threshold and reporting a qualitative status of either ITMB high (ITBM-H) or ITMB low (ITMB-L) based on the comparing.

*Block 950.* Referring to block 950, in some embodiments, the ITMB for the test subject is reported only if the ITMB satisfies a reporting threshold. Such a reporting threshold is necessarily application dependent. For instance, it will vary depending on the type of cancer the test is intended to monitor, stage(s) of cancer the test is intended to monitor, and/or the identity of genes included in the sequencing panel for the panel-enriched sequencing reaction , to name a few nonlimiting variables that may affect the value of the reporting threshold.

*Block 952.* Referring to block 952, in some embodiments, an immunotherapeutic agent is administered to the test subject when the ITMB for the test subject satisfies a therapeutic threshold. Such a therapeutic threshold is necessarily application dependent. For instance, it will vary depending on the type of cancer the test is intended to monitor, the stage(s) of cancer the test is intended to monitor, and/or the identity of genes included in the sequencing panel for the panel-enriched sequencing reaction, to name a few nonlimiting variables that may affect the value of the therapeutic threshold.

*Block 954.* Referring to block 954, in some embodiments, responsive to determining that the ITMB satisfies a clinical trial threshold (associated with a clinical trial), a matched clinical trial recommendation is reported for the test subject. In other words, the test subject is recommended for the clinical trial. Such a clinical trial threshold is necessarily application dependent. For instance, it will vary depending on the type of cancer the clinical trial is intended for, the stage(s) of cancer the clinical trial is intended for, and/or the identity of genes included in the sequencing panel for the panel-enriched sequencing reaction, to name a few nonlimiting variables that may affect the value of the clinical trial threshold.

In some embodiments, a clinical report 139-3 includes information about clinical trials for which the patient is eligible, therapies that are specific to the patient's cancer, and/or possible therapeutic adverse effects associated with the specific characteristics of the patient's cancer, *e.g.,* the patient's genetic variations, epigenetic abnormalities, associated oncogenic pathogenic infections, and/or pathology abnormalities, or other characteristics of the patient's sample and/or clinical records. For example, in some embodiments, the clinical report includes such patient information and analysis metrics, including cancer type and/or diagnosis, variant allele fraction, patient demographic and/or institution, matched therapies (*e.g.,* FDA approved and/or investigational), matched clinical trials, variants of unknown significance (VUS), genes with low coverage, panel information, specimen information, details on reported variants, patient clinical history, status and/or availability of previous test results, and/or version of bioinformatics pipeline.

In some embodiments, the results included in the report, and/or any additional results (for example, from the bioinformatics pipeline), are used to query a database of clinical data, for example, to determine whether there is a trend showing that a particular therapy was effective or ineffective in treating (e.g., slowing or halting cancer progression), and/or adverse effects of such treatments in other patients having the same or similar characteristics.

*Block 956.* Referring to block 956, in some embodiments, the test subject is enrolled (in a respective clinical trial) only if the ITMB for the test subject satisfies a clinical trial threshold (associated with the respective clinical trial) . Such a clinical trial threshold is necessarily application dependent. For instance, it will vary depending on the type of cancer the clinical trial is intended for, the stage(s) of cancer the clinical trial is intended for, and/or the identity of genes included in the sequencing panel for the panel-enriched sequencing reaction, to name a few nonlimiting variables that may affect the value of the clinical trial threshold.

*Block 958.* Referring to block 958, in some embodiments, the method further comprises using the ITMB to identify a concordant ITMB based on a predetermined correlation (*e.g.*, a correlation of at least 0.3, at least 0.4, at least 0.5, at least 0.6, at least 0.7, or at least 0.8) between (i) detection of somatic mutations in cell-free DNA from liquid biopsy sample from a cohort of training subjects and (ii) detection of somatic mutations in genomic DNA from solid tumor biopsy samples from the cohort of training subjects. In other words, a correlation is established between the tumor mutational burden detected through sequencing of liquid biopsy samples and the tumor mutational burden detected through sequencing of solid tumor samples based on matched samples from a cohort of training subjects (that is, a liquid biopsy sample and a solid tumor sample is sequenced for each member of the training cohort). For instance, in some embodiments, the correlation is an average correlation across the plurality of training subjects or a correlation modeled using individual correlations for each training subject. Moreover, the reporting further comprises reporting the concordant ITMB.

*Block 960.* Referring to block 960, in some embodiments, an electronic health record for the test subject is updated to include the ITMB for the test subject.

*Subjects and Biological Samples.*

In some embodiments, the liquid biopsy sample corresponds to a matched tumor sample (*e.g.,* a solid tumor sample obtained from the test subject). For example, in some embodiments, the method further comprises obtaining a second dataset that is determined from a sequencing of a plurality of cell-free nucleic acids in a matched tumor sample of the test subject. In some embodiments, the matched tumor sample is obtained from the test subject concurrently with the liquid biopsy sample. In some embodiments, the matched tumor sample is obtained from the test subject at a different time point from the obtaining the liquid biopsy sample. In some embodiments, the matched tumor sample is any of the embodiments described above (see, Example Methods: Figure 2A: Example Workflow for Precision Oncology). In some embodiments, the method further comprises obtaining the matched tumor sample from a sample repository or database (e.g., BioIVT, TSC Biosample Repository, BioLINCC, *etc.).* In some embodiments, the matched tumor sample is obtained from the test subject at least 1 hour, at least 2 hours, at least 12 hours, at least 1 day, at least 2 days, at least 1 week, at least 1 month, or at least 1 year prior to obtaining the liquid biopsy sample. In some such embodiments, the matched tumor sample is fresh, frozen, dried, and/or fixed. In some embodiments, the matched tumor sample is processed and/or sequenced at least 1 day, at least 2 days, at least 1 week, at least 1 month, or at least 1 year prior to obtaining the second dataset. For example, in some such embodiments, the sequencing data for the plurality of nucleic acids in the matched tumor sample are obtained from a data repository (e.g., GenBank, NCBI Assembly, DNA DataBank of Japan, European Nucleotide Archive, European Variation Archive, *etc.).*

In some embodiments, the one or more reference samples are non-cancerous samples. In some embodiments, the one or more reference samples is a matched normal sample *(e.g.,* a normal sample obtained from the test subject). In some embodiments, the matched normal sample is obtained from the test subject concurrently with the liquid biopsy sample. In some embodiments, the matched normal sample is obtained from the test subject at a different time point from the obtaining the liquid biopsy sample. In some embodiments, the matched normal sample is any of the embodiments described above (see, Example Methods: Figure 2A: Example Workflow for Precision Oncology).

In some alternative embodiments, the one or more reference samples comprise a pool of normal (e.g., non-cancerous) samples obtained from a plurality of control subjects *(e.g.,* healthy subjects). In some such embodiments, the method further comprises obtaining the one or more reference samples from a sample repository or database (e.g., BioIVT, TSC Biosample Repository, BioLINCC, *etc.).* In some embodiments, the one or more reference samples include liquid biopsy samples comprising a plurality of cell-free nucleic acids and/or solid tissue samples comprising a plurality of nucleic acids. In some embodiments, the one or more reference samples are processed and/or sequenced at least 1 day, at least 2 days, at least 1 week, at least 1 month, or at least 1 year prior to obtaining the first dataset. For example, in some such embodiments, the sequencing data for the one or more reference samples are obtained from a data repository (e.g., GenBank, NCBI Assembly, DNA DataBank of Japan, European Nucleotide Archive, European Variation Archive, *etc.).*

In some embodiments, the cell-free nucleic acids *(e.g.,* in the first liquid biopsy sample of the test subject and the one or more reference samples) comprise circulating tumor DNA (ctDNA). In some embodiments, the method further comprises isolating the plurality of cell-free nucleic acids from the liquid biopsy sample of the test subject prior to the sequencing. In some embodiments, the sequencing is multiplexed sequencing. In some embodiments, the sequencing is short-read sequencing or long-read sequencing.

In some embodiments, the sequencing is a panel-enriched sequencing reaction. In some such embodiments, the sequencing reaction is performed at a read depth of 100X or more, 250X or more, 500X or more, 1000X or more, 2500X or more, 5000X or more, 10,000X or more, 20,000X or more, or 30,000X or more. In some embodiments, the sequencing panel comprises 1 or more, 10 or more, 20 or more, 50 or more, 100 or more, 150 or more, 200 or more, 300 or more, 500 or more, or 1000 or more genes. In some embodiments, the sequencing panel comprises one or more genes listed in Table 1.

In some embodiments, the sequencing panel includes at least 2, 3, 4, 5, 10, 15, 20, 25, 30, 40, 50, 60, 70, 80, 90, 100, or all of the genes listed in Table 1. In some embodiments, the sequencing panel comprises one or more genes selected from the group consisting of MET, EGFR, ERBB2, CD274, CCNE1, MYC, BRCA1 and BRCA2. In some embodiments, the sequencing panel includes at least 2, 3, 4, 5, 6, 7, or all 8 of MET, EGFR, ERBB2, CD274, CCNE1, MYC, BRCA1 and BRCA2. In some embodiments, the sequencing reaction is a whole exome sequencing reaction.

In some embodiments, the obtaining the first dataset further comprises aligning a plurality of sequence reads, obtained from a sequencing of the plurality of cell-free nucleic acids in the first liquid biopsy sample of the test subject, to the human reference genome.

In some embodiments, on average, each respective bin in the plurality of bins has two or more, three or more, five or more, ten or more, fifteen or more, twenty or more, fifty or more, one hundred or more, five hundred or more, one thousand or more, ten thousand or more, or 100,000 or more sequence reads in the plurality of sequence reads mapping onto the portion of the reference genome corresponding to the respective bin, where each such sequence read uniquely represents a different molecule in the plurality of cell-free nucleic acids in the liquid biopsy sample. For instance, in some embodiments, the plurality of cell-free nucleic acids in the liquid biopsy sample are sequenced with a sequencing methodology that makes use of unique molecular identifier (UMIs) for each cell-free nucleic acid in the liquid biopsy sample and each sequence read in the plurality of sequence reads has a unique UMI. In such embodiments, sequence reads with the same UMI are bagged (collapsed) into a single sequence read bearing the UMI.

*Longitudinal Testing*

In some embodiments, one or more liquid biopsy assays described herein may be used to analyze specimens from a patient taken over the course of the patient's treatment. For example, a blood specimen may be obtained periodically and/or upon indication of response to therapy, disease relapse, and/or disease progression. In some embodiments, the one or more liquid biopsy assays may be used on a specimen collected from the patient each month, every two months, every three months, every four months, every five months, every 6-12 months, and so forth. In some embodiments, the longitudinal use of liquid biopsy assays may be used to track clonal evolution to identify resistance mutations. In some embodiments, the longitudinal use of liquid biopsy assays may be used to track evolution of mutations, such as EGFR or APC mutations.

In some embodiments, longitudinal use of liquid biopsy assays may be used to detect emerging therapy resistance mechanisms. In some embodiments, longitudinal use of liquid biopsy assays may be used to detect AR gene alterations. In some embodiments, longitudinal use of liquid biopsy assays may be used to detect WNT pathway alterations in mCRPC associated with resistance to enzalutimide and abiraterone. In some embodiments, longitudinal use of liquid biopsy assays may be used to detect ER mutations, such as ER mutations associated with resistance to endocrine therapy in breast cancer. In some embodiments, longitudinal use of liquid biopsy assays may be used to detect EGFR mutations responsible for anti-EGFR therapy resistance (*e.g.*, T790M) in NSCLC. In some embodiments, longitudinal use of liquid biopsy assays may be used to detect KRAS, NRAS, MET, ERBB2, FLT3, or EGFR mutations associated with primary or acquired resistance to EGFR inhibitors in colorectal cancer. In some embodiments, longitudinal use of liquid biopsy assays may be used to assess gene alterations from tumor cells shed by primary tumor and metastatic sites.

In some embodiments the one or more blood specimens may be collected from the patient in a home-based environment. For example, the blood specimens may be collected by a mobile phlebotomist.

For example, a first blood specimen, a second blood specimen, and a third blood specimen may be collected from a patient during the course of treatment.

The present disclosure also provides a computer system comprising one or more processors and a non-transitory computer-readable medium including computer-executable instructions that, when executed by the one or more processors, cause the processors to perform any of the methods and embodiments disclosed herein.

The present disclosure also provides a non-transitory computer-readable storage medium having stored thereon program code instructions that, when executed by a processor, cause the processor to perform any of the methods and embodiments disclosed herein.

*Variant Characterization.*

In some embodiments, a predicted functional effect and/or clinical interpretation for one or more identified variants is curated by using information from variant databases. In some embodiments, a weighted-heuristic model is used to characterize each variant.

In some embodiments, identified clinical variants are labeled as "potentially actionable", "biologically relevant", "variants of unknown significance (VUSs)", or "benign". Potentially actionable alterations are protein-altering variants with an associated therapy based on evidence from the medical literature. Biologically relevant alterations are protein-altering variants that may have functional significance or have been observed in the medical literature but are not associated with a specific therapy. Variants of unknown significance (VUSs) are protein-altering variants exhibiting an unclear effect on function and/or without sufficient evidence to determine their pathogenicity. In some embodiments, benign variants are not reported. In some embodiments, variants are identified through aligning the patient's DNA sequence to the human genome reference sequence version hg19 (GRCh37). In some embodiments, actionable and biologically relevant somatic variants are provided in a clinical summary during report generation.

For instance, in some embodiments, variant classification and reporting is performed, where detected variants are investigated following criteria from known evolutionary models, functional data, clinical data, literature, and other research endeavors, including tumor organoid experiments. In some embodiments, variants are prioritized and classified based on known gene-disease relationships, hotspot regions within genes, internal and external somatic databases, primary literature, and other features of somatic drivers. Variants can be added to a patient (or sample, for example, organoid sample) report based on recommendations from the AMP/ASCO/CAP guidelines. Additional guidelines may be followed. Briefly, pathogenic variants with therapeutic, diagnostic, or prognostic significance may be prioritized in the report. Non-actionable pathogenic variants may be included as biologically relevant, followed by variants of uncertain significance. Translocations may be reported based on features of known gene fusions, relevant breakpoints, and biological relevance. Evidence may be curated from public and private databases or research and presented as 1) consensus guidelines 2) clinical research, or 3) case studies, with a link to the supporting literature. Germline alterations may be reported as secondary findings in a subset of genes for consenting patients. These may include genes recommended by the American College of Medical Genetics and Genomics (ACMG) and additional genes associated with cancer predisposition or drug resistance.

It should be understood that the examples given above are illustrative and do not limit the uses of the systems and methods described herein in combination with a digital and laboratory health care platform.

The results of the bioinformatics pipeline may be provided for report generation 208. Report generation may comprise variant science analysis, including the interpretation of variants (including somatic and germline variants as applicable) for pathogenic and biological significance. The variant science analysis may also estimate microsatellite instability (MSI) or tumor mutational burden. Targeted treatments may be identified based on gene, variant, and cancer type, for further consideration and review by the ordering physician. In some aspects, clinical trials may be identified for which the patient may be eligible, based on mutations, cancer type, and/or clinical history. Subsequent validation may occur, after which the report may be finalized for sign-out and delivery. In some embodiments, a first or second report may include additional data provided through a clinical dataflow 202, such as patient progress notes, pathology reports, imaging reports, and other relevant documents. Such clinical data is ingested, reviewed, and abstracted based on a predefined set of curation rules. The clinical data is then populated into the patient's clinical history timeline for report generation.

Further details on clinical report generation are disclosed in US Patent Application No. 16/789,363 (PCT/US20/180002), filed February 12, 2020, which is hereby incorporated herein by reference in its entirety.

*Stand-alone Device Integration.*

Hardware devices incorporating one or more embodiments as described herein may be implemented. In one example, a hardware device may record progress notes or other documents, automatically converting recorded audio into features and storing them in a structured format with respect to a patient. In another example, a hardware device may broadcast a response containing one or more analytical results, patient features, or reports as described in any of the embodiments above. For more information see, for example, PCT Publication No. WO 2021/168146, the disclosure of which is incorporated herein by reference, in its entirety, for all purposes.

*Specific Embodiments of the Disclosure.*

In some aspects, the systems and methods disclosed herein may be used to support clinical decisions for personalized treatment of cancer. For example, in some embodiments, the methods described herein identify actionable genomic variants and/or genomic states with associated recommended cancer therapies. In some embodiments, the recommended treatment is dependent upon whether or not the subject has a particular actionable variant and/or genomic status. Recommended treatment modalities can be therapeutic drugs and/or assignment to one or more clinical trials. Generally, current treatment guidelines for various cancers are maintained by various organizations, including the National Cancer Institute and Merck & Co., in the Merck Manual.

In some embodiments, the methods described herein further includes assigning therapy and/or administering therapy to the subject based on the identification of an actionable genomic variant and/or genomic state, *e.g.*, based on whether or not the subject's cancer will be responsive to a particular personalized cancer therapy regimen. For example, in some embodiments, when the subject's cancer is classified as having a first actionable variant and/or genomic state, the subject is assigned or administered a first personalized cancer therapy that is associated with the first actionable variant and/or genomic state, and when the subject's cancer is classified as having a second actionable variant and/or genomic state, the subject is assigned or administered a second personalized cancer therapy that is associated with the second actionable variant. Assignment or administration of a therapy or a clinical trial to a subject is thus tailored for treatment of the actionable variants and/or genomic states of the cancer patient.

*Examples.*

*Example 1 - The Cancer Genome Atlas (TCGA).*

The Cancer Genome Atlas (TCGA) is a publicly available dataset comprising more than two petabytes of genomic data for over 11,000 cancer patients, including clinical information about the cancer patients, metadata about the samples (*e.g.*, the weight of a sample portion, *etc.*) collected from such patients, histopathology slide images from sample portions, and molecular information derived from the samples (*e.g.*, mRNA/miRNA expression, protein expression, copy number, *etc.*). The TCGA dataset includes data on 33 different cancers: breast (breast ductal carcinoma, bread lobular carcinoma) central nervous system (glioblastoma multiforme, lower grade glioma), endocrine (adrenocortical carcinoma, papillary thyroid carcinoma, paraganglioma & pheochromocytoma), gastrointestinal (cholangiocarcinoma, colorectal adenocarcinoma, esophageal cancer, liver hepatocellular carcinoma, pancreatic ductal adenocarcinoma, and stomach cancer), gynecologic (cervical cancer, ovarian serous cystadenocarcinoma, uterine carcinosarcoma, and uterine corpus endometrial carcinoma), head and neck (head and neck squamous cell carcinoma, uveal melanoma), hematologic (acute myeloid leukemia, Thymoma), skin (cutaneous melanoma), soft tissue (sarcoma), thoracic (lung adenocarcinoma, lung squamous cell carcinoma, and mesothelioma), and urologic (chromophobe renal cell carcinoma, clear cell kidney carcinoma, papillary kidney carcinoma, prostate adenocarcinoma, testicular germ cell cancer, and urothelial bladder carcinoma).

*Example 2 - Method of Validating a Liquid Biopsy Assay.*

*Conducting sample collection, storage, nucleic acid isolation, and library preparation.*

To validate a liquid biopsy assay in accordance with some embodiments of the present disclosure, 188 unique specimens were sequenced. These unique specimens included 10 blood specimens purchased from BioIVT, 56 residual plasma samples, 39 whole-blood samples, 4 cfDNA reference standards set in synthetic plasma (Horizon Discovery's Multiplex I cfDNA Reference Standards HD812, HD813, HD814, HD815), and 2 cfDNA reference standard isolates (Horizon Discovery's Structural Multiplex cfDNA reference standard HD786, and 100% Multiplex I Wild Type Reference Standard HD776). Furthermore, an additional 55 blood samples with matched tumor samples were utilized to compare the liquid biopsy and solid tumor tests, and 375 blood samples were sequenced for low-pass whole-genome sequencing (LPWGS) analysis. Sequence data from an additional 1,000 patient samples that were previously sequenced were utilized for retrospective and clinical analyses. All blood was received in Cell-free DNA BCT® blood collection tubes (Streck). Plasma was prepared immediately after accessioning and stored at -80 °C until later nucleic acid extraction and library preparation. At this time, cfDNA was isolated from plasma using the Qiagen QIAamp MinElute ccfDNA Midi Kit (QIAGEN), conducted according to instructions provided by the manufacturer. Automated library preparation was performed on a SciClone NGSx (Perkin Elmer). All cfDNA samples were normalized with molecular grade water to a maximum of 50 microliters ($\mu$L).

*Conducting the liquid biopsy sequencing assay.*

The liquid biopsy assay utilized New England BioLab's NEBNEXT® ULTRA™ II DNA Library Prep Kit for ILLUMINA®, IDT's xGen CS Adapters, unique molecular indices (UMI), and 96 pairs of barcodes to prepare cfDNA sequencing libraries with unique sample identifiers (IDs). Each sample was ligated to a dual unique index. The dual unique index enables multiplexed sequencing of up to 7 patients and 1 positive control per SP NovaSeq flow cell, 16 patients and 1 positive control per S1 NovaSeq flow cell, 34 patients and 1 positive control per S2 NovaSeq flow cell, and 84 patients and 1 positive control per S4 NovaSeq flow cell. The library preparation protocol is optimized for greater than or equal to 20 nanograms (ng) cfDNA input to maximize mutation detection sensitivity. The final library was sequenced on an Illumina NovaSeq sequencer. Furthermore, analysis was performed using a bioinformatics pipeline and analysis server.

*The bioinformatics pipeline.*

Adapter-trimmed FASTQ files are aligned to the nineteenth edition of the human reference genome build HG19.

Following alignment, reads were grouped by alignment position and UMI family, and collapsed into consensus sequences. Bases with insufficient quality or significant disagreement among family members were reverted to N's. Phred scores were scaled based on initial base calling estimates combined across all family members. Following single-strand consensus sequence generation, duplex consensus sequences were generated by comparing the forward and reverse oriented PCR products with mirrored UMI sequences. Consensus sequences were re-aligned to the human reference genome using BWA. BAM files are generated and indexed after the re-alignment.

SNV and indel variants were detected using methods disclosed in Lai et al., 2016, "VarDict: a novel and versatile variant caller for next-generation sequencing in cancer research," Nucleic Acids Res, (44), pg. 108. SNVs were called down to 0.1% VAF for specified hotspot target regions and 0.25% VAF at all other base positions across the panel. Indels were called down to 0.5% VAF for variants within specific regions of interest. Any indels outside of these regions were called down to 5% VAF. All SNVs and indels were then sorted, deduplicated, normalized, and annotated accordingly. Following annotation, variants were classified as germline, somatic, or uncertain using a Bayesian model based on prior expectations informed by various internal and external databases of germline and cancer variants. Uncertain variants are treated as somatic for filtering and reporting purposes. Following classification, variants were filtered based on a plurality of quality metrics including coverage, VAF, strand bias, and genomic complexity. Additionally, variants were filtered with a Bayesian tri-nucleotide context-based model with position level background error rates estimated from a pool of process matched healthy controls. Furthermore, known artifactual variants were removed.

Copy number variants (CNVs) were analyzed utilizing CNVkit and a CNV annotation and filtering algorithm provided by the present disclosure. Talevich et al., 2016, "CNVkit: Genome-Wide Copy Number Detection and Visualization from Targeted DNA Sequencing," PLoS Comput Biol, (12), pg. 1004873. This CNVkit provides genomic region binning, coverage calculation, bias correction, normalization to a reference pool, segmentation, and visualization. The $\log_2$ ratios between the tumor sample and a pool of process matched healthy samples from the CNVkit output were annotated and filtered using statistical models, such that the amplification status (e.g., amplified or not-amplified) of each gene is predicted and non-focal amplifications are removed.

Rearrangements were detected using methods disclosed in Chiang et al., 2015, "SpeedSeq: ultra-fast personal genome analysis and interpretation," Nat Methods, (12), pg. 966. Briefly, FASTQ files were aligned to hg19 using BWA. Split reads mapped to multiple positions and read pairs mapped to discordant positions were identified and separated, then utilized to detect gene rearrangements by LUMPY. Layer et al., 2014, "LUMPY: a probabilistic framework for structural variant discovery," Genome Biol, (15), pg. 84. Fusions were then filtered according to the number of supporting reads.

Predicted functional effect and clinical interpretation for each variant was curated by automated software using information from both internal and external databases. A weighted-heuristic model was used, which has logic-based recommendations from the AMP/ASCO/CAP/ClinGen Somatic working group and ACMG guidelines. Li et al., 2017, "Standards and Guidelines for the Interpretation and Reporting of Sequence Variants in Cancer: A Joint Consensus Recommendation of the Association for Molecular Pathology, American Society of Clinical Oncology, and College of American Pathologists," The Journal of molecular diagnostics, (19), pg. 4; Kalia et al., 2017, "Recommendations for reporting of secondary findings in clinical exome and genome sequencing, 2016 update (ACMG SF v2.0): a policy statement of the American College of Medical Genetics and Genomics," Genetics in Medicine, (19), pg. 249.

The relative frequency and distribution are determined for any read containing repetitive sequences to detect microsatellite instability. To predict the probability of an unstable locus, a k-nearest neighbors model (with k = 100) was utilized along with normalized percent lower, mean lower, and mean log-likelihood metrics. The percentage of unstable loci was calculated from the probabilities of each sample, with greater than 50% unstable loci considered microsatellite instability-high (MSI-H).

*The validation approach.*

The present disclosure conducted extensive validation studies to establish robust technical perform of the liquid biopsy assay. Limit of detection (LOD) was determined by assessing analytical sensitivity in reference standards with 5%, 1%, 0.5%, 0.25%, and 0.1% VAF generated from the Horizon Discovery reference set. The Horizon Discovery set includes 160 bp cfDNA fragments from human cell lines in an artificial plasma matrix to closely resemble cfDNA extracted from human plasma. VAFs of SNVs and indels, including EGFR (ΔE746 - A750), EGFR (V769 - D770insASV), EGFR A767_V769dup, EGFR (L858R), EGFR (T790M), KRAS (G12D), NRAS (A59T), NRAS (Q61K), AKT1 E17K, PIK3CA (E545K), and GNA11 Q209L, and CNVs and rearrangements, including CCDC6/RET, SLC34A2/ROS1, MET, MYC, and MYCN, were measured in reference samples by the liquid biopsy assay of the present disclosure. Each measurement was conducted with a minimum of three replicates at 10 ng, 30 ng, and 50 ng of DNA. Sensitivity was determined by the number of detected variants divided by the total number of variants present in the reference samples. Samples with an on-target rate of less than 30% were excluded from the instant analysis, and MET (4.5 copies) was included in CNV sensitivity determinations. Sensitivity of greater than 90% was considered reliable detection.

Analytical specificity was determined using 44 normal samples titrated at 1%, 2.5%, or 5% from a wild-type cfDNA reference standard with a list of confirmed true-negative SNVs, indels, CNVs and rearrangements. Specificity was determined by the number of known true-negative variants divided by the number of true-negative variants plus false-positive variants identified by the liquid biopsy assay.

To assess inter-instrument concordance between the sequencing instruments, 10 patient libraries were sequenced on each instrument (3 NovaSeqs). Variants seen below the lower limit of detection (LLOD) (0.25% for SNVs and 0.50% for indels) were excluded from concordance analysis.

To establish analytical accuracy, the results of 40 validation samples were compared to the results of an orthogonal reference method (Roche's AVENIO ctDNA assay). Analytical accuracy was determined by the number of detected variants divided by the total number of variants present in the sample. Variants that were off-target or below LLOD (0.25% for SNVs and 0.5% for indels) were excluded from the instant analysis.

*Conducting digital droplet polymerase chain reaction (ddPCR).*

Five variants were validated on the ddPCR platform: KRAS G12D (Integrated DNA Technologies, IDT, published sequences); TERT promoter mutations c.-124C>T (C228T) & c.-146C>T (C250T) (Thermo Fisher Scientific); and TP53 p.R273H and TP53 p.R175H (Thermo Fisher Scientific). Each amplification reaction was performed in 25 µL and contained 1X Genotyping Master Mix (Thermo Fisher Scientific), 1X droplet stabilizer (RainDance), 1X of primer/probe mixture for TERT and TP53 (for KRAS: 800 nM of each primer and 500 nM of each probe) plus template. To improve the lower limit of detection, 4-cycle amplification was conducted prior to droplet generation. Amplification for KRAS was conducted using the cycling conditions of: 1 cycle of 95°C (0.6°C/s ramp) for 10 minutes, 4 cycles of 95°C (0.6°C/s ramp) for 15 seconds and 60°C for 2 minutes, followed by 1 cycle of 98°C (0.6°C/s ramp) for 10 minutes. Cycling conditions for the TP53 variants were the same as those for KRAS with the exception of the annealing and extension temperature, which was set at 55°C for 2 minutes. Amplification for TERT followed Thermo Fisher's recommendation as follows: 1 cycle of 96°C (1.6°C/s ramp) for 10 minutes, 4 cycles of 98°C (1.6°C/s ramp) for 30 seconds and 55°C for 2 minutes, followed by 1 cycle of 55°C (1.6°C/s ramp) for 2 minutes. Accordingly, droplets generated on the RainDance Source, and amplification performed following the above cycling conditions with cycle numbers of 45 for both KRAS and TP53, and 54 for TERT. Furthermore, droplets were analyzed on a RainDance Sense droplet reader. Additionally, RainDrop Analyst II v1.1.0 analysis software was utilized to acquire and analyze data.

*The concordance between liquid biopsy and solid tumor assays.*

Matched liquid biopsy and solid tumor sample pairs (n = 55) were used to determine analytical sensitivity and specificity. Solid tumor and matched normal samples obtained from peripheral blood buffy coat were analyzed with the solid tumor assay, and corresponding blood plasma samples were analyzed with the liquid biopsy assay of the present disclosure. Only variants in the reportable range of both the solid tumor and liquid biopsy panels were included in these analyses (*e.g.*, genes in the liquid biopsy gene panel is a subset of genes in the solid tumor gene panel). Germline, intronic, and synonymous variants identified in the solid tumor assay and the liquid biopsy assay were excluded from analysis with the exception of intronic splice variants. To determine analytical sensitivity, the number of variants called in both the liquid biopsy assay and the solid tumor assay (*e.g.*, true positives) was divided by the sum of true positives and those called only in the solid tumor assay. To determine analytical specificity the number of positions reported in neither the liquid biopsy assay nor the solid tumor assay (*e.g.*, true negatives) was divided by the sum of true negatives and variants only called in the liquid biopsy assay.

To improve variant calling in the liquid biopsy assay, a strategy that dynamically determines local sequence errors using Bayes Theorem and the likelihood ratio test was developed. The dynamic threshold was determined using a sample-specific error rate, the error rate from healthy control samples, and from a reference cohort of solid tumor samples. Accordingly, the method of the present disclosure was conducted on 55 matched liquid biopsy/solid tumor tissue samples, with variants detected in the solid tumor assay as the source of truth. Using sensitivity thresholds defined by the LOD analysis, fixed post-test-odds (*e.g.,* equal to the P(post-test) / [1 - P(post-test)]), as well as pre-test-odds. The Pre-test-odds were determined using historical data from the solid tumor assay with an equation identical to the post-test-odds calculation). Accordingly, the following formula was determined based on the above: specificity = 1 - pre-test-odds * sensitivity / post-test-odds

The specificity was input to a beta-binomial function and yielded the minimum number of alternate alleles to call a variant at a particular depth. The pre-test-odds metric was specific to individual cancer cohorts and individual genes, allowing for cancer-specific pre-test-odds to be applied to individual exons.

*Conducting low-pass whole genome sequencing and analysis.*

Blood samples from 375 patients were sequenced using low-pass whole-genome sequencing (LPWGS) across four flow cells. Sequencing coverage metrics for these samples were determined using Picard CollectWgsMetrics. The tumor fraction and ploidy values for each sample were estimated using ichorCNA with a specific reference panel of 47 normal samples. Adalsteinsson et al., (2017), "Scalable whole-exome sequencing of cell-free DNA reveals high concordance with metastatic tumors" Nat Commun, (8), pg. 1324. Reported variants from the corresponding liquid biopsy analysis of each sample were utilized to assess the accuracy of the tumor fraction estimates.

*Determining estimation of circulating tumor fraction.*

Circulating tumor fraction estimate (ctFE) was determined using a novel method, Off-Target Tumor Estimation Routine (OTTER), from off-target reads uniformly distributed across the human reference genome. As described above, the CNVkit was conducted on each sample, and segments were assigned via circular binary segmentation (CBS). Olshen et al., 2004, "Circular binary segmentation for the analysis of array-based DNA copy number data," Biostatistics, (5), pg. 557. Segments were then fit to integer copy states via an expectation-maximization algorithm using the sum of squared error of the segment $\log_2$ ratios (*e.g.,* normalized to genomic interval size) to expected ratios given a putative copy state and tumor purity. Estimates were confirmed by comparing results against LPWGS of the original patient isolate. As such, results are shown using randomly selected, de-identified samples.

*Clinical profiling of liquid biopsy patients.*

De-identified molecular and abstracted clinical data were evaluated in a cohort of 1,000 patients randomly selected from a specific reference clinicogenomic database. All data were de-identified in accordance with the Health Insurance Portability and Accountability Act (HIPAA). Dates used for analyses were relative to the first liquid biopsy sequencing date of each patient, and year of the first sequencing date was randomly off-set. Variants included in the analyses were those classified as pathogenic or likely pathogenic, and further divided into actionable if matched to diagnostic, prognostic or therapeutic evidence or biologically relevant. Outcomes were determined according to the most recent clinical response noted in patient records. The study protocol was submitted to the Advarra Institutional Review Board (IRB), which determined the research was exempt from IRB oversight and approved a waiver of HIPAA authorization for this study.

*Example 3 - Results of Validating Liquid Biopsy Assay.*

*Liquid biopsy validation summary.*

The liquid biopsy oncology assay is a 105-gene hybrid capture NGS panel designed to detect actionable somatic variant targets in plasma. The liquid biopsy assay detects mutations in four variant classes, including: single nucleotide variants (SNVs) and insertion-deletions (indels) in all 105 genes, copy number variants (CNVs) in 6 genes, and chromosomal rearrangements in 7 genes. To validate the liquid biopsy assay, a total of 188 samples were sequenced. The runs generated an average of 261.7 M $\pm$ 40.7 M total reads with 130.7 M $\pm$ 20.3 M read pairs and a unique median read depth of 4999.128 $\pm$ 1288.843. The average percent of mapped reads across all runs was 99.876% $\pm$ 0.0078.

Analytical sensitivity for all SNVs, indels, CNVs, and rearrangements targeted in the reference samples was provided. SNVs were reliably detected at greater than or equal to 0.25% VAF with 30 ng of input DNA (93.75% [45/48] sensitivity), indels at greater than or equal to 0.5% VAF with 30 ng (95.83% [23/24] sensitivity), CNVs at greater than or equal to 0.5% VAF with 10 ng (100.00% [8/8] sensitivity), and rearrangements at greater than or equal to 1% VAF with 30 ng (90% [9/10] sensitivity). Analytical specificity was provided in which 100% for SNVs, indels, and rearrangements; and 96.2% for CNVs on samples with greater than or equal to 0.25% VAF with 30 ng of input DNA.

Accordingly, intra-assay and inter-assay concordance between the replicates in the present disclosure was 100% for SNVs, indicating a high degree of repeatability and reproducibility. Moreover, the inter-instrument concordance was 96.70% for SNVs and 100% for indels, with a combined concordance of 96.83% across instruments. Additionally, interfering substances including genomic DNA, ethanol, and isopropanol did not cause a change in the detection of variants. Concordance between controls and samples with interfering substances was high (*e.g.,* 100%) among samples that passed filtering, and were above the LOD.

*The accuracy of the liquid biopsy assay compared to orthogonal assays.*

To evaluate analytical accuracy, the present disclosure compared the liquid biopsy assay to the Roche AVENIO ctDNA assay. In 30 ng cfDNA samples analyzed by liquid biopsy assay and AVENIO cfDNA assay (n = 40), sensitivity for SNVs, indels, CNVs and rearrangements was 94.8%, 100%, 100%, and 100%, respectively. In the 6 SNVs that were not detected, 5 were called but filtered out due to insufficient evidence. In 10 ng samples, sensitivity for SNV, indel, CNV, and rearrangements was 91.9%, 100%, 80%, and 100%, respectively. Of the 7 SNVs that were not detected, 6 were present in sequencing data but filtered out due to insufficient evidence.

To further validate the liquid biopsy assay results, patients with reported variants KRAS G12D (n = 12), TERT c.-124 (n = 7), TERT c.-146 (n = 5), TP53 R273H (n = 7), and TP53 R175H (n = 7) were selected for analysis by ddPCR. Liquid biopsy NGS VAF was compared with ddPCR VAF to determine concordance. Accordingly, 100% PPV and a high correlation between ddPCR results and liquid biopsy VAF ($R^2$ = 0.892), as well as individual variants such as KRAS G12D ($R^2$ = 0.970). These results indicate the liquid biopsy assay of the present disclosure can be used to accurately identify hotspot mutations. Results of an inter-assay comparison between liquid biopsy, ddPCR, and solid tumor results for patients samples with selected variants (n = 38) analyzed by ddPCR and compared with liquid biopsy variant allele fraction (VAF) resulted in high correlation overall ($R^2$ = 0.892). An inter-assay comparison between liquid biopsy, ddPCR and solid tumor results for patient samples with individual variants such as KRAS G12D was also performed (n = 12, $R^2$ = 0.970).

*The concordance between liquid biopsy and solid tumor tissue assay.*

Comparisons between analytical sensitivity and specificity in matched solid tumor and liquid biopsy tests from 55 patients were determined. Since solid tumor matched samples include both tumor tissue and buffy coat (*e.g.*, normal comparator), a specific classification strategy was utilized to determine and exclude germline variants from the analysis. Beaubier et al., 2019, "Clinical validation of the xT next-generation targeted oncology sequencing assay," Onctotarget, 10(24), pg. 2384. Removing intronic and synonymous variants, benign and likely benign variants, as well as variants below the LOD for solid tumor and liquid biopsy assays resulted in 145 concordant SNVs, 20 concordant indels, and 11 concordant CNVs. 66 SNVs, 11 indels, and 8 CNVs were identified that were reported in the solid tumor assay but not the liquid biopsy assay, as well as 209 SNVs, 14 indels, and 7 CNVs that were reported in the liquid biopsy assay but not the solid tumor assay. Accordingly, the specificity of the liquid biopsy assay was 100.00% for SNVs and indels and 96.67% for CNVs. A Bayesian dynamic filtering methodology was utilized to further reduce discordance by 11.45%, improving the specificity of variant calling in the liquid biopsy assay. The overall sensitivity of the liquid biopsy assay compared to the solid tumor assay was 68.18% for SNVs and indels and 57.89% for CNVs. When limiting analysis to clinically actionable targets, 107 concordant variants and 37 discordant, for a sensitivity of 74.31%, were reported.

Furthermore, comparisons between the sample classification of reportable variants between matched samples with liquid biopsy and solid tumor testing were determined. Variants were considered CH variants if found in the plasma as well as in the solid tumor normal sample but were not present at levels consistent with germline variation. Accordingly, this classification of germline and CH variants in liquid biopsy is possible with a corresponding solid tumor assay or a germline sequencing analysis from the buffy coat. Notably, two samples have a large number of variants only detected in liquid biopsy, many of which are at low VAFs. These samples were subsequently determined to have very high tumor mutational burdens (TMBs) in their corresponding solid tumor analyses. Accordingly, the large number of liquid biopsy variants at low VAFs and high TMBs suggest that these tumors may be more heterogeneous and that some variants are more easily detected in blood.

Also, liquid biopsy validation samples were utilized to assess microsatellite instability in patients whose MSI status was previously confirmed by a specific reference clinically validated solid tumor MSI test or immunohistochemistry. The liquid biopsy assay reported MSI-H status in 37.5% (6/16) of orthogonally confirmed MSI-H patients at 100% (6/6) positive predictive value. Accordingly, comparisons between the solid tumor and liquid biopsy assays demonstrate the strengths of the liquid biopsy assay and the added value of using multiple assays to detect genomic drivers of cancer.

*OTTER, a novel method for estimating tumor fraction.*

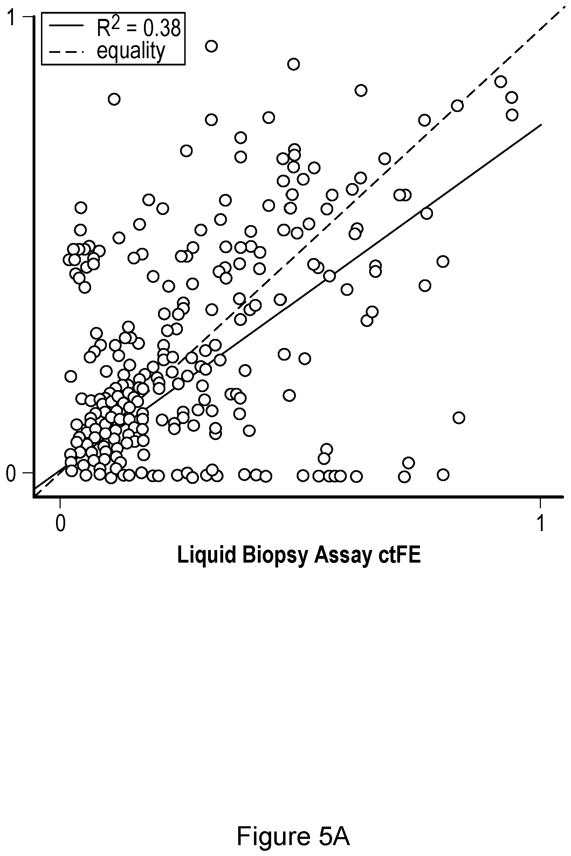

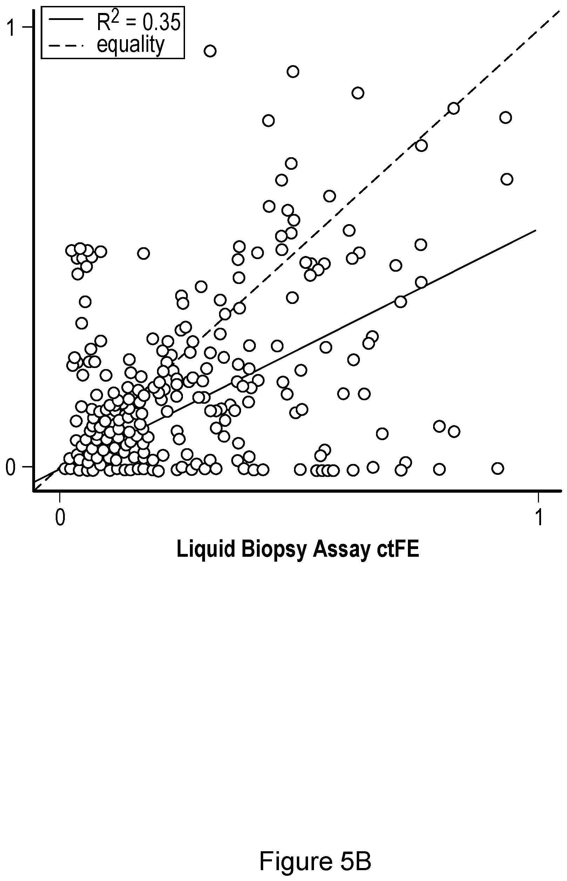

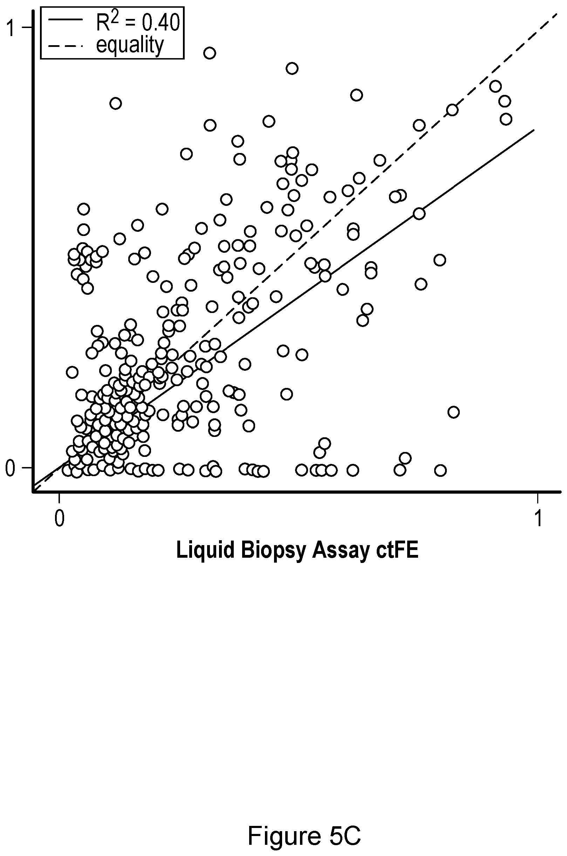

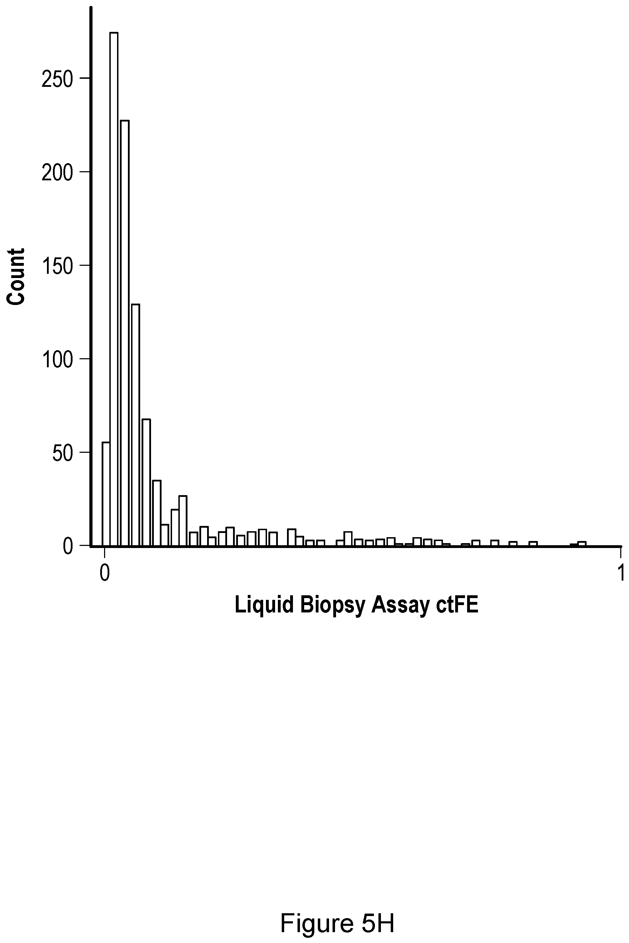

An accurate measure of tumor fraction can provide an improved understanding of variants identified through liquid biopsy testing. In the present disclosure, a novel method, Off-Target Tumor Estimation Routine (OTTER), for determining a more accurate circulating tumor fraction estimate (ctFE) was developed. Referring to Figures 5A and 5B, comparisons between OTTER ctFE with VAFs from 1,000 random patient samples across cancer types were determined, such that liquid biopsy ctFE correlates with max pathogenic VAF and median VAF. Referring to Figures 5C through 5F, removing germline variants and amplified regions from these analyses further increased the correlation. Plausible liquid biopsy ctFE estimates were expected to be greater than or equal to the maximal somatic VAF in a sample that was not on an amplified region. Overall, after removing germline variants and variants on amplified regions, 90.8% of median VAFs are less than or equal to the corresponding liquid biopsy ctFEs. Referring to Figure 5H, the distribution of liquid biopsy ctFE for the liquid biopsy 1,000 cohort is provided, in which the median ctFE was 0.07 with a mean ctFE of 0.12.

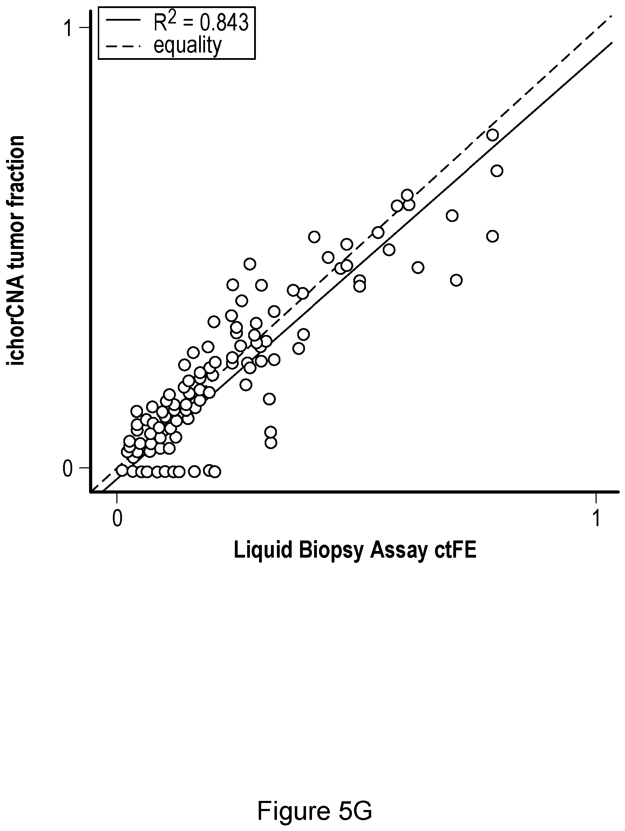

In addition to VAF, LPWGS is increasingly utilized to estimate tumor fractions and thought to be a more accurate measure than VAF. Adalsteinsson *et al.*, 2017; Chen et al., 2019, "Next-generation sequencing in liquid biopsy: cancer screening and early detection," Hum Genomics, (13), pg. 34. Referring to Figure 5G, comparisons between LPWGS ichorCNA-predicted circulating tumor fraction to the OTTER ctFE in matched patient samples (n = 375) determined a strong correlation between methods ($R^2$ = 0.843, *P* = 4.71e-152). Accordingly, this correlation indicates that OTTER ctFEs are highly concordant with estimates using LPWGS but can be determined directly from the targeted-panel sequencing without requiring additional sequencing.

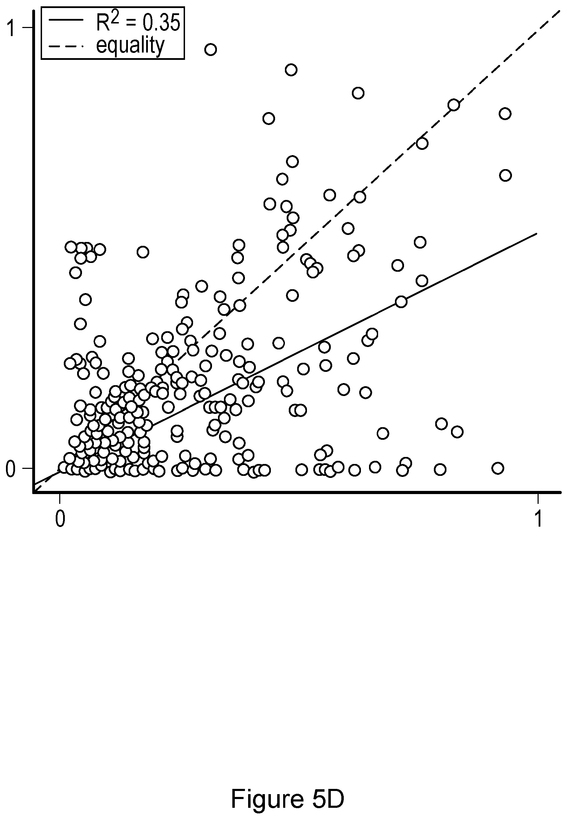

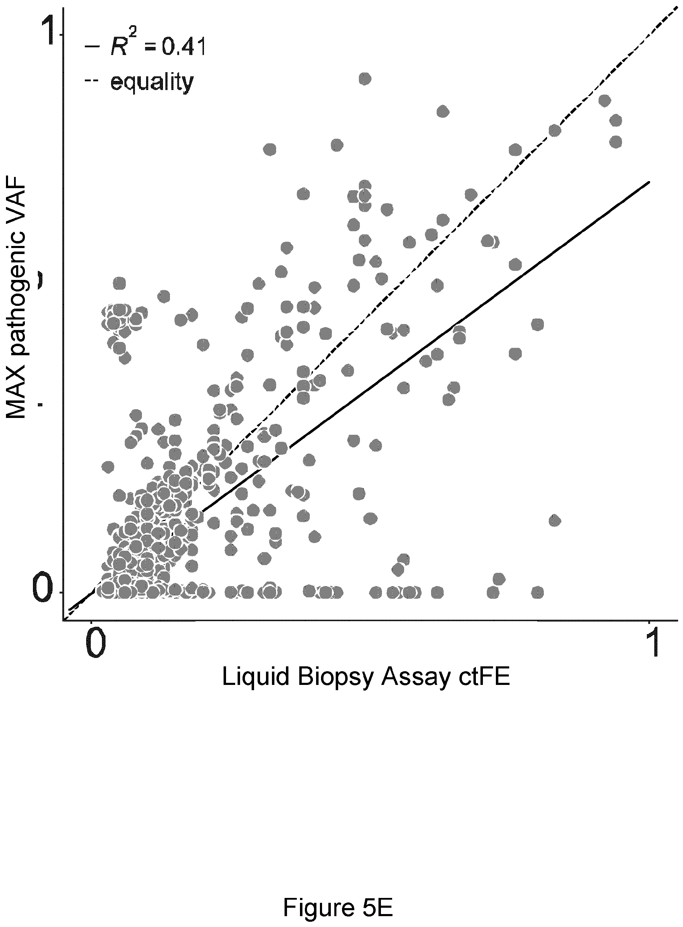

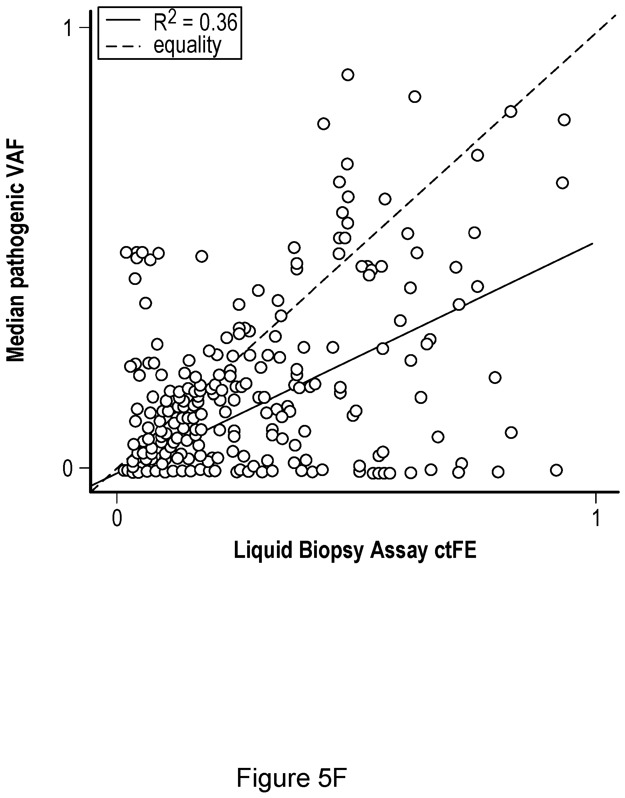

Specifically, Figure 5A illustrates results from circulating tumor fraction estimate (ctFE) and variant allele fraction (VAF) in which ctFE of liquid biopsy-sequenced patients (n = 1,000) was correlated with max pathogenic VAF ($R^2$ = 0.38). Figure 5B illustrates results from ctFE and VAF in which ctFE of liquid biopsy-sequenced patients (n = 1,000) was correlated with medium VAF ($R^2 = 0.35$). Figure 5C illustrates results from ctFE and VAF in which ctFE of liquid biopsy-sequenced patients (n = 1,000) in which germline variants were removed, increasing the correlation with max pathogenic VAF ($R^2 = 0.40$). Figure 5D illustrates results from ctFE and VAF in which ctFE of liquid biopsy-sequenced patients (n = 1,000) in which germline variants were removed, without increasing the correlation with medium VAF ($R^2 = 0.35$). Figure 5E illustrates results from ctFE and VAF in which ctFE of liquid biopsy-sequenced patients (n = 1,000) in which amplified regions from these analyses were removed, increasing the correlation with max pathogenic VAF ($R^2 = 0.41$). Figure 5F illustrates results from ctFE and VAF in which ctFE of liquid biopsy-sequenced patients (n = 1,000) in which amplified regions from these analyses were removed, increasing the correlation with medium VAF ($R^2 = 0.36$). Figure 5G illustrates results from ctFE and VAF in which ctFE of liquid biopsy-sequenced patients (n = 1,000) in which samples that also underwent low-pass whole genome sequencing (LPWGS, n = 375), a strong correlation between LPWGS-predicted tumor fraction and ctFE ($R^2 = 0.843$) is found. Furthermore, Figure 5G illustrates results from ctFE and VAF in which ctFE of liquid biopsy-sequenced patients (n = 1,000) and the overall distribution of ctFE across the cohort (median ctFE = 0.07, mean ctFE = 0.12, and standard deviation = 0.15).

*Retrospective clinical profiling of the liquid biopsy assay against a 1,000-subject cohort.*

To evaluate the clinical utility of the liquid biopsy, de-identified molecular and clinical data from 1,000 samples across cancer types were selected for clinical profiling. This included 55.7% female and 44.3% male patients, with a median age of 66 years, and interquartile range of 15. This cohort included patients from 24 cancer categories, with breast (n = 254), colorectal (n = 98), lung (n = 241), pancreatic (n = 83), and prostate (n = 96) being the most common. Referring to Figure 6A, the median ctFE predicted by OTTER was 0.07 for all cancer types, with the exception of prostate, which was 0.06. Referring to Figure 6B, in this cohort, 8,099 mutations were reported, of which 2,732 were pathogenic, and 2,238 were clinically actionable. Specifically, Figure 6A illustrates circulating tumor fraction estimate (ctFE) and mutational landscape by cancer type, in which median ctFE among the most common cancer types was 0.07, with the exception of prostate (ctFE = 0.06). Figure 6B illustrates circulating tumor fraction estimate (ctFE) and mutational landscape by cancer type, in which variants are categorized as reportable, pathogenic, or actionable. Across all patients, the most commonly mutated gene was TP53. The heatmap was normalized within rows to depict the most prevalent variants detected for each common cancer type in the cohort (breast n = 254, colorectal n = 98, lung n = 241, pancreatic n = 83, and prostate n = 96).

Accordingly, the most frequently mutated gene in the liquid biopsy 1,000 cohort was TP53 (51.1% of patients). The most commonly mutated genes were TP53, PIK3CA, ESR1, BRCA2, NF1, ATM and APC in breast cancer, TP53, EGFR, ATM and KRAS in lung cancer, and TP53, APC, and KRAS in colorectal cancer. These findings were consistent with existing literature on commonly mutated genes in each cancer type and suggest the liquid biopsy test accurately detects variants of interest to the broader cancer community. *See,* van Helden *et al,* 2019; Dal Maso *et al.,* 2019; Savli et al., 2019, "TP53, EGFR and PIK3CA gene variations observed as prominent biomarkers in breast and lung cancer by plasma cell-free DNA genomic testing," J Biotechnol, (300), pg. 87; Cheng et al., 2019, "Liquid Biopsy Detects Relapse Five Months Earlier than Regular Clinical Follow-Up and Guides Targeted Treatment in Breast Cancer," Case Rep Oncol Med, pg. 6545298; Keup et al., 2019, "Targeted deep sequencing revealed variants in cell-free DNA of hormone receptor-positive metastatic breast cancer patients," Cell Mol Life Sci, print.; Li et al., 2019, "Genomic profiling of cell-free circulating tumor DNA in patients with colorectal cancer and its fidelity to the genomics of the tumor biopsy," J Gastrointest Oncol, (10), pg. 831.

*Advanced disease is associated with higher estimated tumor fraction.*

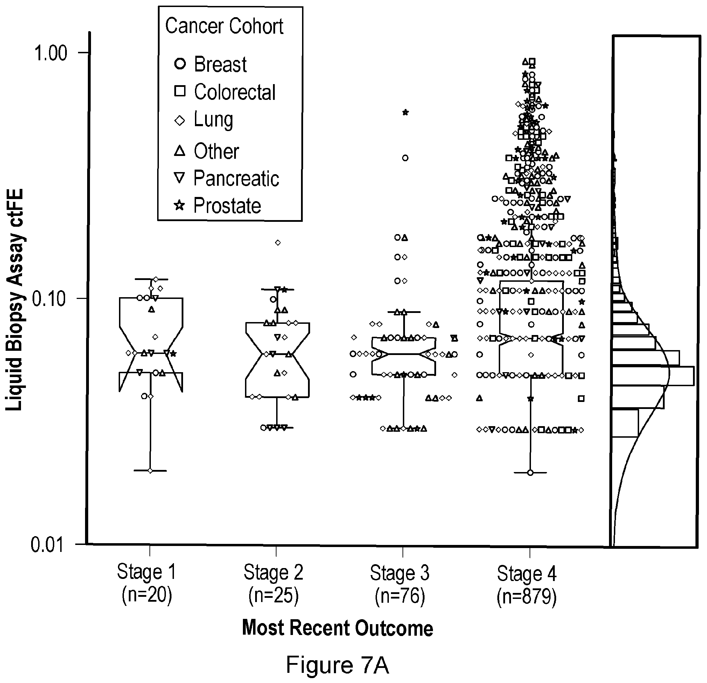

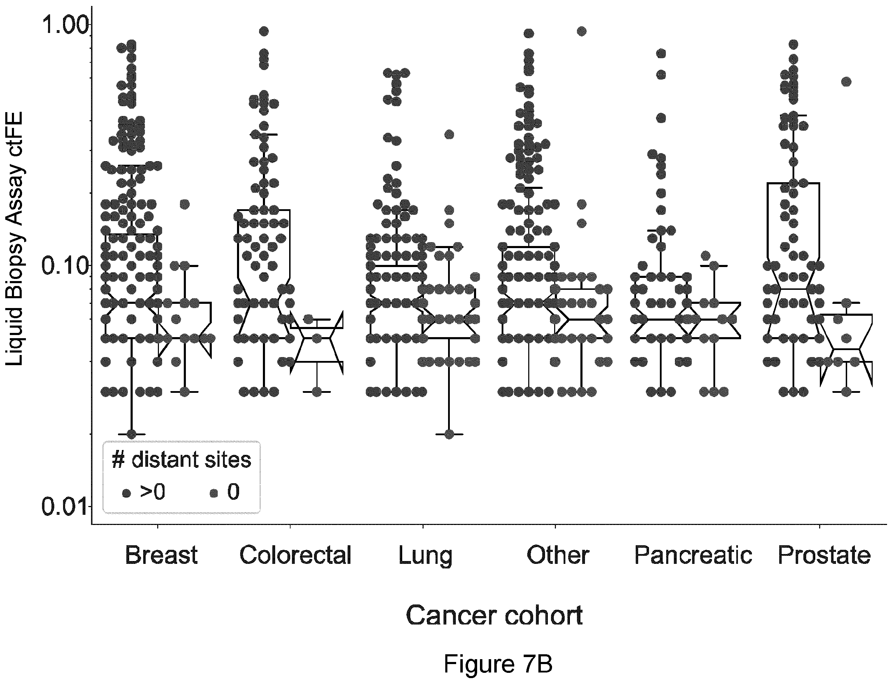

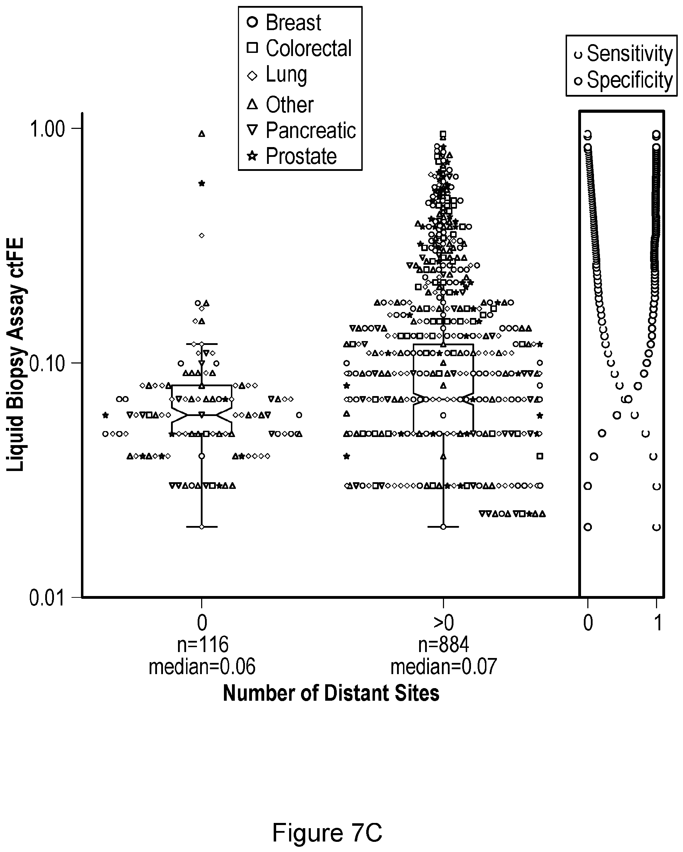

A goal of liquid biopsy assays of the present disclosure was to more efficiently monitor treatment response and predict disease progression in patients over time. To establish proof of concept, the association of ctFE with advanced disease states was investigated. Accordingly, referring to Figure 7A, a significant difference in ctFE between stages (P = 2.97e-5) was determined. However, since the majority of patients had advanced disease at the time of testing, more early stage samples are necessary to further verify these findings. Referring to Figure 7B, ctFE in patients with metastatic disease was evaluated to determined that ctFE increases when distant sites are affected. Indeed, referring to Figure 7C, patients with no metastatic lesions had a significantly lower ctFE than patients with one or more distant sites (P = 4.77e-7), further highlighting the potential of ctFE for disease monitoring. Specifically, Figure 7A illustrates circulating tumor fraction estimate (cfTE) according to stage and number of distant metastases among the liquid biopsy 1,000 cohort, in which there was a significant difference in ctFE between stages (Kruskal-Wallis P = 2.97e-5). Accordingly, patients with stage 4 cancer (n = 879, median ctFE = 0.07) had a higher ctFE than those with stages 1 (n = 20, median ctFE = 0.06), 2 (n = 25, median ctFE = 0.06), or 3 (n = 76, median ctFE = 0.06). Figures 7B and 7C illustrate that ctFE increased with the number of metastatic distant sites (Mann-Whitney U test P = 7.57e-7), and there was a significant difference in ctFE between patients with no metastatic lesions (n = 116) and those with 1 or more distant sites affected (n = 884, Mann-Whitney U test P = 2.12e-5). The sensitivity and specificity shown to the right-hand side of the Figure 7C represent the probability that a binary metastasis status prediction is correct at a given ctFE threshold. Accordingly, the model predicts metastasis with greater confidence at higher ctFE.

*Estimated tumor fraction correlates with response to treatment.*

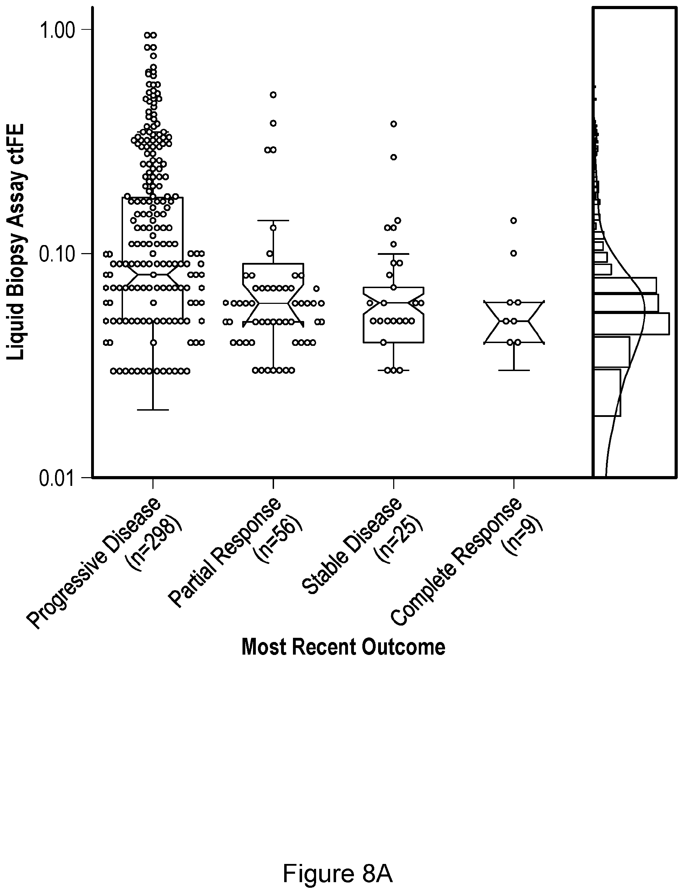

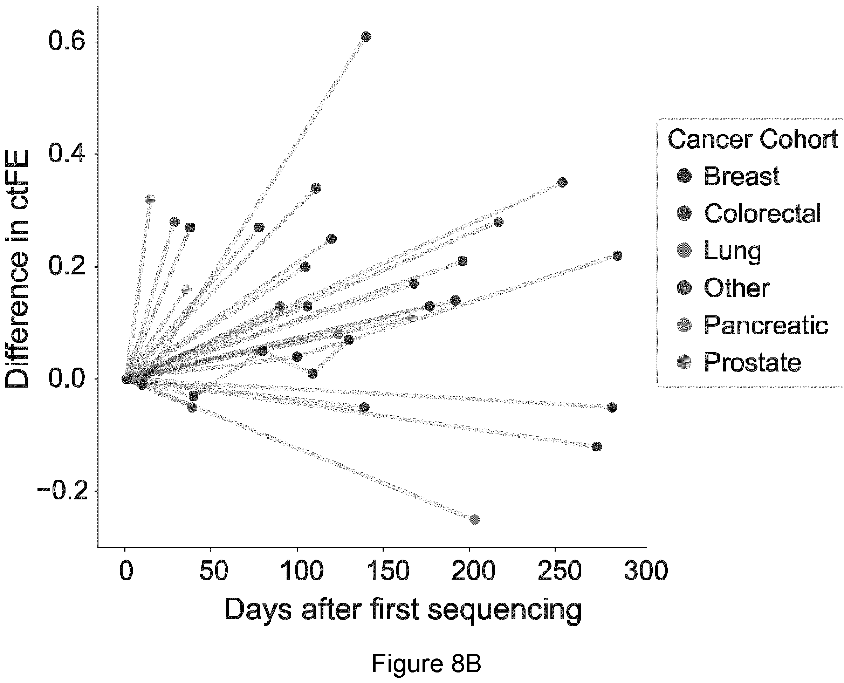

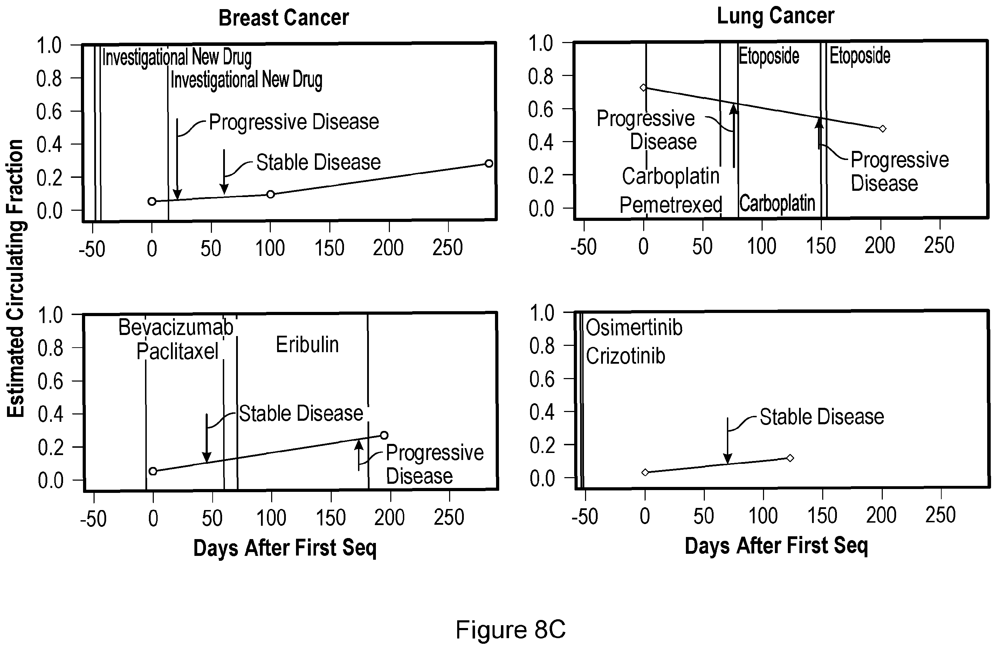

To determine how ctFE changes in response to treatment, comparisons between ctFE with the most recent clinical response outcome were determined. Accordingly, referring to Figure 8A, patients classified as having complete response were determined to have a significantly lower median ctFE of 0.05, compared to 0.06, 0.06, and 0.08 in patients with stable disease, partial response, and progressive disease, respectively. Additionally, referring to Figure 8B, patients with multiple liquid biopsy tests were determined to have large differences in ctFE between test dates. For example, referring to Figure 8C, one breast cancer case had a ctFE of 0.05 at initial liquid biopsy testing. After treatment with bevacizumab and paclitaxel, clinical notes indicate the patient was classified as having stable disease. Eribulin treatment was started shortly after, but the patient was later diagnosed with progressive disease. A second liquid biopsy test, which was performed approximately 200 days after the initial liquid biopsy test, revealed a ctFE of 0.26, which supports the progressive disease diagnosis. Alternatively, in a breast cancer patient with progressive disease who was treated with investigational new drug therapies, the patient's status was updated to stable disease shortly after the first liquid biopsy test, which revealed a ctFE of 0.05. Approximately 100 days later, the patient's second liquid biopsy test revealed a ctFE of 0.09. The patient likely received no further treatment before the third liquid biopsy test, which revealed a ctFE of 0.27, suggesting this patient's disease had progressed. Specifically, Figure 8A illustrates circulating tumor fraction estimate (cfTE) and abstracted clinical outcomes in a sub-cohort of the liquid biopsy 1000 (n = 388) in which patients with complete response (n = 9, ctFE = 0.05) exhibited lower ctFE than those with progressive disease (n = 298, ctFE = 0.08), partial response (n = 56, ctFE = 0.06), or stable disease (n = 25, ctFE = 0.06). Figure 8B illustrates that ctFE was also assessed temporally among a few randomly selected patients with multiple liquid biopsy tests throughout the course of treatment (n = 26), with most patients showing large differences in ctFE between test dates. Figure 8C illustrates four exemplary cases highlighting the utility of ctFE in relation to treatment course and disease status.

In the case of a lung cancer patient who underwent multiple rounds of treatment, including carboplatin, pemetrexed, and etoposide, a decrease in ctFE between liquid biopsy tests (0.72 to 0.47) was determined. However, the ctFE was still extremely high after treatment, making progressive disease likely. Indeed, the patient was classified as having progressive disease by their oncologist shortly before the second liquid biopsy test date. Alternatively, a patient who had undergone treatment with osimertinib and crizotinib approximately 50 days before the first liquid biopsy test showed very little change in ctFE between test dates (0.3-0.11) and was classified as stable shortly before the second liquid biopsy test. Referring to Figures 7A through 8C, while conclusions about the larger population based on these individual cases cannot be determined, the changes in ctFE in response to treatment was consistent with the above analyses showing that higher ctFEs were associated with advanced disease. Additionally, these results illustrate how serial testing can be beneficial for precision oncology in individual patients.

While liquid biopsy is a promising tool for improving outcomes in precision oncology, there are challenges that must be overcome before it can replace large panel NGS tissue genotyping. For example, in early stage disease, when treatments have much higher success rates, many patients have low ctDNA fractions that may be below the LOD for liquid biopsies, limiting clinical utility because of the risk of false negatives. Bettegowda et al., 2014, "Detection of circulating tumor DNA in early- and late-stage human malignancies," Sci Transl Med, (6), pg. 224; Xue et al., 2019, "Early detection and monitoring of cancer in liquid biopsy: advances and challenges," Expert Rev Mol Diagn, (19), pg. 273; Hennigan et al., 2019, "Low Abundance of Circulating Tumor DNA in Localized Prostate Cancer," JCO Precis Oncol, (3), print; Abbosh et al., 2018, "Early stage NSCLC - challenges to implementing ctDNA-based screening and MRD detection," Nat Rev Clin Oncol, (15), pg. 577. Consequently, most studies to date have focused on late stage patients for assay validation and research. Furthermore, while validation studies of existing liquid biopsy assays have shown high sensitivity and specificity, few studies have corroborated results with orthogonal methods, or between NGS testing platforms. Cheng et al., 2019, "Clinical Validation of a Cell-Free DNA Gene Panel," J Mol Diagn, (21), pg. 632; Hanibuchi et al., 2019, "Development, validation, and comparison of gene analysis methods for detecting EGFR mutation from non-small cell lung cancer patients-derived circulating free DNA," Oncotarget, (10), pg. 3654; Van Laar et al., 2018, "Development and validation of a plasma-based melanoma biomarker suitable for clinical use," Br J Cancer, (118), pg. 857; Odegaard et al., 2018, "Validation of a Plasma-Based Comprehensive Cancer Genotyping Assay Utilizing Orthogonal Tissue- and Plasma-Based Methodologies," Clin Cancer Res, (24), pg. 3539; Clark et al., 2018, "Analytical Validation of a Hybrid Capture-Based Next-Generation Sequencing Clinical Assay for Genomic Profiling of Cell-Free Circulating Tumor DNA," J Mol Diagn, (20), pg. 686; Plagnol et al., 2018, "Analytical validation of a next generation sequencing liquid biopsy assay for high sensitivity broad molecular profiling," PLoS One, (13), pg. 0193802. Kuderer *et al.* compared commercially available liquid and tissue NGS platforms and found only 22% concordance in genetic alterations. Kuderer et al., 2017, "Comparison of 2 Commercially Available Next-Generation Sequencing Platforms in Oncology," JAMA Oncol, (3), pg. 996. Other reports of liquid biopsy based studies are limited by comparison to non-comprehensive tissue testing algorithms including Sanger sequencing, small NGS hotspot panels, PCR and FISH, which may not contain all NCCN guideline genes in their reportable range, thus suffering in comparison to a more comprehensive liquid biopsy assay. Leighl *et al.,* 2019. Since the 105 gene liquid biopsy assay is a subset of the 648 gene solid tumor tissue-based assay, the concordance data presented herein (74.31% for actionable variants) represents a direct comparison to a comprehensive NGS test which includes the entire reportable range of the liquid biopsy assay. Beaubier et al., 2019, "Integrated genomic profiling expands clinical options for patients with cancer," Nat Biotechnol, (37), pg. 1351. While this concordance is high relative previous reports, 25.69% of actionable variants would have been missed if only one of the tests were performed. Thus, liquid biopsies provide the greatest value to patients when used in combination with standard tissue genotyping. Furthermore, having both tests enabled additional analyses to exclude germline and CH variants, significantly improving specificity.

Accordingly, the systems and methods of the present disclosure provide analytical and clinical validation of the liquid biopsy assay. The systems and methods of the present disclosure provide high accuracy compared to orthogonal methods, including tissue biopsy, Avenio liquid biopsy, ddPCR, and LPWGS. The systems and methods of the present disclosure also provide improvements upon existing methodologies for estimating circulating tumor fraction. Notably, in combination with real-world clinical data, the systems and methods of the present disclosure demonstrate the value and suitability of liquid biopsy testing for monitoring disease progression, predicting objective measures of response, and assessing treatment outcomes. As such, the results obtained through validating the systems and methods of the present disclosure strongly support utilizing the liquid biopsy assay in routine monitoring of cancer patients with advanced disease.

*Example 4 - A retrospective analysis on the prognostic value of Off- Target Tumor Estimation Routine, a novel circulating tumor fraction (ctFE) calculation, in patients with advanced prostate cancer.*

Prostate-specific antigen (PSA) is a biomarker for monitoring tumor burden and treatment response. However, due to multiple variable factors (e.g., variation in PSA production by prostate cancer (PC) cells, PSA level variation between patients, and PSA level variation during the course of the disease), non-invasive biomarkers are needed for better prognostication and assessing therapeutic response. We recently developed the Off-Target Tumor Estimation Routine method, described herein, which calculates circulating tumor fraction estimates (ctFE) using on- and off-target reads from a targeted-panel liquid biopsy assay (DNA-Seq of 105 genes at 5,000x depth in circulating tumor DNA [ctDNA] from peripheral blood samples). Here, the prognostic value of ctFE for advanced PC patients undergoing liquid biopsy testing was analyzed.

A total of 108 NGS results from 80 patients treated at Ben Taub Hospital (BTH) with locally advanced, biochemically recurrent or metastatic prostate cancer were retrospectively analyzed. The ctFE was calculated for all patients using this method, which evaluates the copy state of regions across the genome. Survival analysis was based on a 6-month follow-up. For prognostic analysis, the highest ctFE was used for each patient with >1 xF result. Patients were classified as: 1. Low (ctFE-L: ctFE < 0.02); 2. High (ctFE-H: ctFE $\geq$ 0.02); or 3. Converters (ctFE-H to L: ctFE drop below 0.02 during follow-up). In 16 metastatic PC patients receiving first-line androgen deprivation therapy (1LADT, augmented with abiraterone/prednisone), pre-treatment and on-treatment ctFE data as well as clinical follow-up (median: 12 months) were examined.

Results: 65/80 (81%) patients were classified as ctFE-L. Of these, 64 (98%) were alive at the 6-month follow-up, and one was deceased due to a non-PC-related cause. 15/80 (19%) patients had a least one ctFE-H estimate. Of these, 7 (47%) were deceased due to PC-related causes within 6 months (range: 2-172 days, median: 15 days), while the remaining 8 (53%) showed ctFE-H to -L conversion in response to treatment and were alive at the 6-month follow-up. Among 16 metastatic PC patients, 1LADT lowered ctFE in 12 patients; of these, 10 patients continued responding to treatment during the follow-up period. The 4 patients whose ctFE did not drop became castration-resistant during this period.

Conclusions: The data suggest that ctFE may predict PC patient overall survival. ctFE-L status is associated with patient survival at 6-month follow-up. Conversely, ctFE-H status was associated with death unless the implementation of a new active treatment can convert the patient to ctFE-L upon rechecking. Changes in ctFE may also correlate with response to 1LADT. This study illustrates the potential of using ctFE as a tool for PC prognostication.

## REFERENCES CITED AND ALTERNATIVE EMBODIMENTS

All references cited herein are incorporated herein by reference in their entirety and for all purposes to the same extent as if each individual publication or patent or patent application was specifically and individually indicated to be incorporated by reference in its entirety for all purposes.

Log$_2$ transformed copy ratios, log$_2$ copy ratios, log$_2$-transformed depths, log$_2$-transformed read depths, log$_2$ depths, corrected log$_2$ depths, log$_2$ ratios, log$_2$ read depths, and log$_2$ depth correction values have been discussed herein by way of example. In each instance where such a term is used, it will be appreciated that log base 2 is presented by way of example only and that the present disclosure is not so limited. Indeed, logarithms to any base N may be used, (*e.g.*, where N is a positive number greater than 1 for instance), and thus the present disclosure fully supports log$_N$ transformed copy ratios, log$_N$ copy ratios, log$_N$-transformed depths, log$_N$-transformed read depths, log$_N$ depths, corrected log$_N$ depths, log$_N$ ratios, log$_N$ read depths, and log$_N$ depth correction values as respective substitutes for log$_2$ transformed copy ratios, log$_2$ copy ratios, log$_2$-transformed depths, log$_2$-transformed read depths, log$_2$ depths, corrected log$_2$ depths, log$_2$ ratios, log$_2$ read depths, and log$_2$ depth correction values.

The present invention can be implemented as a computer program product that comprises a computer program mechanism embedded in a non-transitory computer readable storage medium. These program modules can be stored on a CD-ROM, DVD, magnetic disk storage product, USB key, or any other non-transitory computer readable data or program storage product.

Many modifications and variations of this invention can be made without departing from its spirit and scope, as will be apparent to those skilled in the art. The specific embodiments described herein are offered by way of example only. The embodiments were chosen and described in order to best explain the principles of the invention and its practical applications, to thereby enable others skilled in the art to best utilize the invention and various embodiments with various modifications as are suited to the particular use contemplated. The invention is to be limited only by the terms of the appended claims, along with the full scope of equivalents to which such claims are entitled.

**Disclosed Items:**

1. A method of determining a liquid biopsy tumor mutational burden (lTMB) for a test subject comprising:
at a computer system having one or more processors, and memory storing one or more programs for execution by the one or more processors:

> A) obtaining, from a panel-enriched sequencing reaction, a plurality of nucleic acid sequences comprising a corresponding sequence for each cell-free DNA fragment in a first plurality of cell-free DNA fragments obtained from a liquid biopsy sample from the test subject, wherein each respective cell-free DNA fragment in the first plurality of cell-free DNA fragments corresponds to a respective probe sequence in a plurality of probe sequences used to enrich cell-free DNA fragments in the liquid biopsy sample in the panel-enriched sequencing reaction; and
> B) determining, using the panel-enriched sequencing reaction, that a circulating tumor fraction (ctFE) is above a threshold ctFE value;
> C) responsive to determining that the ctFE is above the threshold, calculating the lTMB for the test subject from the panel-enriched sequencing reaction; and
> D) reporting the lTMB for the test subject.

2. The method according to item 1, wherein the panel-enriched sequencing reaction is performed at a read depth of at least 500X.

3. The method of item 1 or 2, wherein the panel-enriched sequencing reaction uses a sequencing panel that enriches for from 50 genes to 150 genes.

4. The method of item 1 or 2, wherein the plurality of probe sequences used to enrich cell-free DNA fragments in the liquid biopsy sample in the panel-enriched sequencing reaction collectively map to from 25 different genes to 150 different genes in a human reference genome.

5. The method of any one of items 1-4, wherein the panel-enriched sequencing reaction uses a sequencing panel that enriches for at least 10 genes listed in Table 1.

6. The method of any one of items 1-5, wherein the panel-enriched sequencing reaction uses a sequencing panel that enriches for at least 10 genes listed in List 1.

7. The method of any one of items 1-5, wherein the panel-enriched sequencing reaction uses a sequencing panel that enriches for at least 10 genes listed in List 2.

8. The method of any one of items 1-5, wherein the calculating C) comprises determining a count of a plurality of genetic variants present in the plurality of nucleic acid sequences.

9. The method of item 8, wherein the calculating C) further comprises normalizing the count of the plurality of genetic variants present in the plurality of nucleic acid sequences by a coverage of the plurality of probe sequences.

10. The method of item 9, wherein the coverage is from 0.1 megabases to 0.4 megabases.

11. The method of item 9, wherein the coverage is from 0.15 megabases to 0.3 megabases.

12. The method of any one of items 8-11, wherein the count of the plurality of genetic variants present in the plurality of nucleic acid sequences is a count of unique genetic variants present in the plurality of nucleic acid sequences that satisfy one or more qualifying criterion in a set of qualifying criteria.

13. The method of item 12, wherein a qualifying criterion in the set of qualifying criteria is a requirement that the respective genetic variant is a missense variant, a combination of a missense variant and a splice region variant, a frameshift variant, a stop lost variant, a splice acceptor variant, an in frame insertion variant, an in frame deletion variant, a combination of a frameshift variant and a splice region variant, a disruptive in frame insertion variant, or a disruptive in frame deletion variant.

14. The method of item 12 or 13, wherein a qualifying criterion in the set of qualifying criteria is a requirement that the respective genetic variant has a variant allele frequency in the liquid biopsy sample that is greater than 0.005 (0.5%).

15. The method of any one of items 12-14, wherein a qualifying criterion in the set of qualifying criteria is a requirement that the respective genetic variant has a variant allele frequency in the liquid biopsy sample that is less than 1.0 (100%).

16. The method of item 12 or 13, wherein a qualifying criterion in the set of qualifying criteria is a requirement that the respective genetic variant has a variant allele frequency (VAF) in the liquid biopsy sample that is any one of:

(i) greater than 0.01 (1%) and less than 0.4 (40%),
(ii) greater than 0.6 (60%) and less than 0.9 (90%),
(iii) greater than 0.4 (40%) and less than 0.60 (60%) with the proviso that

$$\frac{|VAF-ctFE|}{ctFE} < 1,$$

or
(iv) greater than 0.9 (90%) with the proviso that

$$\frac{|VAF-ctFE|}{ctFE} < 1.$$

17. The method of any one of items 1-16, wherein the threshold ctFE value is 0.0025.

18. The method of any one of items 1-17, wherein the liquid biopsy sample is a blood sample.

19. The method of any one of items 1-18, wherein the method further comprises:

using the ITMB to identify a concordant ITMB based on a predetermined correlation between (i) detection of somatic mutations in cell-free DNA from a liquid biopsy sample from each training subject in a cohort of training subjects and (ii) detection of somatic mutations in genomic DNA from solid tumor biopsy samples from the cohort of training subjects; and
the reporting D) further comprises reporting the concordant ITMB.

20. The method of any one of items 1-19, wherein the reporting D) further comprises, responsive to determining that the ITMB satisfies a therapeutic threshold, reporting a matched therapeutic recommendation for the test subject.

21. The method of any one of items 1-20, further comprising, administering an immunotherapeutic agent to the test subject when the ITMB for the test subject satisfies a therapeutic threshold.

22. The method of any one of items 1-21, wherein the reporting D) further comprises, responsive to determining that the ITMB satisfies a clinical trial threshold, reporting a matched clinical trial recommendation for the test subject.

23. The method of any one of items 1-22, further comprising, enrolling the test subject when the ITMB for the test subject satisfies a clinical trial threshold.

24. The method of any one of items 1-23, wherein the reporting D) comprises comparing the ITMB for the subject to a severity threshold and reporting a qualitative status of either ITMB high (ITMB-H) or ITMB low (ITMB-L) based on the comparing.

25. The method of any one of items 1-24, wherein the ITMB for the test subject is reported only if the ITMB satisfies a reporting threshold.

26. The method of any one of items 1-25, further comprising updating an electronic health record for the test subject to include the ITMB for the test subject.

27. The method of any one of items 12-16, wherein a qualifying criterion in the set of qualifying criteria is selection of a genetic variant present in the plurality of nucleic acid sequences by a medical professional.

28. The method of any one of items 1-27, wherein the plurality of probe sequences map to no more than 150 genes in the human genome

29. A computer system comprising:

one or more processors; and
a non-transitory computer-readable medium including computer-executable instructions that, when executed by the one or more processors, cause the processors to perform a method according to any one of items 1-28.

30. A non-transitory computer-readable storage medium having stored thereon program code instructions that, when executed by a processor, cause the processor to perform the method according to any one of items 1-28.

**Claims**

1. A method of determining a liquid biopsy tumor mutational burden (ITMB) for a test subject comprising:
   at a computer system having one or more processors, and memory storing one or more programs for execution by the one or more processors:

   A) obtaining, from a panel-enriched sequencing reaction, a plurality of nucleic acid sequences comprising a corresponding sequence for each cell-free DNA fragment in a first plurality of cell-free DNA fragments obtained from a liquid biopsy sample from the test subject, wherein each respective cell-free DNA fragment in the first plurality of cell-free DNA fragments corresponds to a respective probe sequence in a plurality of probe sequences used to enrich cell-free DNA fragments in the liquid biopsy sample in the panel-enriched sequencing reaction; and
   B) determining, using the panel-enriched sequencing reaction, that a circulating tumor fraction (ctFE) is above a threshold ctFE value;
   C) responsive to determining that the ctFE is above the threshold, calculating the ITMB for the test subject from the panel-enriched sequencing reaction; and
   D) reporting the ITMB for the test subject.

2. The method according to claim 1, wherein the panel-enriched sequencing reaction is performed at a read depth of at least 500X, or
   wherein the panel-enriched sequencing reaction uses a sequencing panel that enriches for from 50 genes to 150 genes.

3. The method of claim 1, wherein the plurality of probe sequences used to enrich cell-free DNA fragments in the liquid biopsy sample in the panel-enriched sequencing reaction collectively map to from 25 different genes to 150 different genes in a human reference genome.

4. The method of any one of claims 1-3, wherein the panel-enriched sequencing reaction uses a sequencing panel that enriches for at least 10 genes listed in Table 1, List 1, or List 2.

5. The method of any one of claims 1-4, wherein the calculating C) comprises determining a count of a plurality of genetic variants present in the plurality of nucleic acid sequences and wherein the calculating C) optionally further comprises normalizing the count of the plurality of genetic variants present in the plurality of nucleic acid sequences by a coverage of the plurality of probe sequences, optionally wherein the coverage is from 0.1 megabases to 0.4 megabases, or from 0.15 megabases to 0.3 megabases.

6. The method of claim 5, wherein the count of the plurality of genetic variants present in the plurality of nucleic acid sequences is a count of unique genetic variants present in the plurality of nucleic acid sequences that satisfy one or more qualifying criterion in a set of qualifying criteria.

7. The method of claim 6, wherein a qualifying criterion in the set of qualifying criteria is a requirement that the respective genetic variant is a missense variant, a combination of a missense variant and a splice region variant, a frameshift variant, a stop lost variant, a splice acceptor variant, an in frame insertion variant, an in frame deletion variant, a combination of a frameshift variant and a splice region variant, a disruptive in frame insertion variant, a disruptive in frame deletion variant, a requirement that the respective genetic variant has a variant allele frequency in the liquid biopsy sample that is greater than 0.005 (0.5%), or a selection of a genetic variant present in the plurality of nucleic acid sequences by a medical professional.

8. The method of claim 6 or 7, wherein a qualifying criterion in the set of qualifying criteria is a requirement that the respective genetic variant has a variant allele frequency (VAF) in the liquid biopsy sample that is any one of:

   (i) greater than 0.01 (1%) and less than 0.4 (40%),
   (ii) greater than 0.6 (60%) and less than 0.9 (90%),
   (iii) greater than 0.4 (40%) and less than 0.60 (60%) with the proviso that

$$\frac{|VAF-ctFE|}{ctFE} < 1,$$

   or
   (iv) greater than 0.9 (90%) with the proviso that

$$\frac{|VAF-ctFE|}{ctFE} < 1.$$

9. The method of any one of claims 1-8, wherein the liquid biopsy sample is a blood sample.

10. The method of any one of claims 1-9, wherein the method further comprises:
    using the ITMB to identify a concordant ITMB based on a predetermined correlation between (i) detection of somatic mutations in cell-free DNA from a liquid biopsy sample from each training subject in a cohort of training subjects and (ii) detection of somatic mutations in genomic DNA from solid tumor biopsy samples from the cohort of training subjects; and the reporting D) further comprises reporting the concordant ITMB or wherein the reporting D) further comprises, responsive to determining that the ITMB satisfies a therapeutic threshold, reporting a matched therapeutic recommendation for the test subject.

11. The method of any one of claims 1-10, the method further comprising administering an immunotherapeutic agent to the test subject when the ITMB for the test subject satisfies a therapeutic threshold.

12. The method of any one of claims 1-11, wherein

    (i) the reporting D) further comprises, responsive to determining that the ITMB satisfies a clinical trial threshold, reporting a matched clinical trial recommendation for the test subject,
    (ii) the method further comprises enrolling the test subject when the ITMB for the test subject satisfies a clinical trial threshold, or
    (iii) the reporting D) comprises comparing the ITMB for the subject to a severity threshold and reporting a qualitative status of either ITMB high (ITMB-H) or ITMB low (ITMB-L) based on the comparing.

13. The method of any one of claims 1-12, wherein the ITMB for the test subject is reported only if the ITMB satisfies a reporting threshold or wherein the method further comprises updating an electronic health record for the test subject to include the ITMB for the test subject.

**14.** A computer system comprising:

one or more processors; and
a non-transitory computer-readable medium including computer-executable instructions that, when executed by the one or more processors, cause the processors to perform a method according to any one of claims 1-13.

**15.** A non-transitory computer-readable storage medium having stored thereon program code instructions that, when executed by a processor, cause the processor to perform the method according to any one of claims 1-13.

System
100

| Operating System 116 |
| Network communication module 118 |
| Test patient data store 120 |

Non-persistent memory 111

| Bioinformatics module 140 |
| Sequence data processing module 141 |
| Pre-processing algorithms 142 |
| Alignment algorithm 143 |
| Demultiplexing algorithm 144 |
| Feature extraction module 145 |
| Variant identification module 146 |
| SNV/MNV calling algorithm 147 |
| Indel calling algorithm 148 |
| Genomic rearrangement calling algorithm 149 |
| Variant allele fraction module 151 |
| Methylation analysis module 152 |
| Copy Number Variation (CNV) analysis module 153 |
| Microsatellite Instability (MSI) analysis module 154 |
| Tumor Mutational Burden (TMB) analysis module 155 |
| Tumor ploidy analysis module 156 |
| Homologous recombination pathway analysis module 157 |
| Reference sequence constructs 158 |
| Feature analysis module 160 |
| Genomic alteration interpretation algorithms 161 |
| Pathogenic variant analysis algorithms 162 |
| Genomic variant analysis algorithms 163 |
| Compound feature analysis algorithms 164 |
| Clinical data analysis algorithms 165 |
| Therapeutic curation algorithms 166 |
| Recommendation validation module 167 |
| Reporting module 180 |

114

| Processing core 102 | Persistent memory 112 | User interface 106 | Network interface 104 |
| | | Display 108 | |
| | | Keyboard 110 | |

Network 105

Fig. 1A

Non-persistent memory 111

| Test patient data store 120 |
|---|
| Patient 1 121-1 |
| Sequencing data 122-1 |
| Sequencing run 1 122-i-1-1 |
| Sequence read 1 123-1-1-1 |
| ⋮ |
| Sequence read K 123-1-1-K |
| Aligned sequences (e.g., BAM file) 124-1-1 |
| ⋮ |
| Sequencing run L 122-i-1-L |
| Feature data 125-1 |
| Personal characteristics 126-1 |
| Medical history 127-1 |
| Clinical features 128-1 |
| Pathology data 128-1-1 |
| Imaging data 128-2-1 |
| Tissue culture / organoid data 128-3-1 |
| Genomic features 131-1 |
| Allelic states 132-1 |
| Variant allele fractions 133-1 |
| Methylation states 134-1 |
| Genomic copy numbers 135-1 |
| Tumor mutational burden 136-1 |
| Microsatellite instability status 137-1-1 |
| Tumor ploidy 137-1-2 |
| HRD status 137-1-3 |
| Other -omics data 138-1 |
| Clinical assessment 139-1 |
| Actionable variants and characteristics 139-1-1 |
| Matched therapies 139-1-2 |
| Clinical reports 139-1-3 |
| ⋮ |
| Patient M 121-M |

Fig. 1B

Non-persistent memory 111

| Genomic features 131 | | | |
|---|---|---|---|
| | Circulating tumor fraction estimates 131-i | | |
| | | Liquid biopsy circulating tumor fraction estimates 131-i-cf | |
| | | | ctFE 1 131-i-cf-1 |
| | | | ctFE 2 131-i-cf-2 |
| | | | ⋮ |
| | | | ctFE N 131-i-cf-N |

# Fig. 1C

Non-persistent memory 111

| Feature extraction module 145 | | | |
|---|---|---|---|
| | Tumor fraction estimation module 145-tf | | |
| | | Tumor purity algorithm construct 145-tf-a | |
| | | | Model parameters 145-tf-d |
| | | | Parameter 1 145-tf-d-1 |
| | | | Parameter 2 145-tf-d-2 |
| | | | ⋮ |
| | | Input data filtration construct 145-tf-k | |
| | Sequence ratio data structure 145-tf-r | | |
| | Minimization construct 145-tf-o | | |

# Fig. 1D

200

Sample Collection — 201

Clinical Data Analysis — 202

| Patient Intake | → | Physical Specimens Received | → | Receive Clinical Data Which Can Include Patient Progress Notes, Pathology Reports, Imaging Reports, and Any Other Documents |

Wet Lab Processing — 204

| Accessioning | → | Pathology Review | → | Extraction | → | Library Prep |

| Capture + Hybridize | → | Pooling | → | Sequencing |

Ingest Clinical Data into Platform

Feature Extraction — 206

Variant Analysis — 208

Variant Science Analysis

| Interpret Somatic and Germline Variants for Pathogenic and Biological Significance | MSI & TMB: Microsatellite Instability and Tumor Mutational Burden are Important Determinants of a Patient's Eligibility for Immunotherapies |

Review Clinical Data and Abstract Relevant Clinical Information Based on Predefined Set of Curation Rules

| Therapy Curation | Clinical Trials | Validation by Report Team |
| Targeted Treatments are Matched Based on Gene, Variant and Cancer Types | Selects Clinical Trials Based on Mutations, Cancer Type, and Clinical History | Final Validation Checks are Also Completed By the Clinical Trails Team Including Patient and Sample Information |

Pathologist Final Review and Sign Off

Report Delivered ← As Available and When Applicable, Clinical Data Populates the Patient's Clinical History Timeline

**FIG. 2A**

70

Distributed Diagnostic/Clinical Environment <u>210</u>

Fig. 2B

EP 4 600 963 A1

Fig. 3

Fig. 4A

Fig. 4B

```
order.tar.gz
└── order/
    ├── order_N_1.fastq.gz
    ├── order_N_2.fastq.gz
    ├── order_T_1.fastq.gz
    └── order_T_2.fastq.gz
```

Sequence identifier
Sequence base calls
Base call quality scores
(phred)

Fig. 4C

Raw FASTQ files → FASTQ Quality Control (trimming, filtering, correcting for artifacts, etc.) → Processesed FASTQ files

Fig. 4D

Fig. 4E

EP 4 600 963 A1

400-1

402-1 Obtain cfDNA Sequencing Data

404-1 Align to reference human construct

Aligned reads
406-1

408-1 Process cfDNA sequence reads

410-1 Obtain copy number status annotations for validation

Copy number status annotation validation filters
412-1

Median bin-level copy ratio filter 414-1

Segment-level confidence interval filter 416-1

Median-plus-MAD bin-level copy ratio filter 418-1

Segment-level copy ratio filter 419-1

420-1 Validate or reject copy number variation

Validated status of copy
number variation 422-1

424-1 Match therapies and/or clinical
trials based on CNV status

Patient report indicating
CNV status 426-1

Fig. 4F1

400-2

```
( 402-2  Obtain cfDNA Sequencing Data )
                    │
                    ▼
  ┌─────────────────────────────────────┐
  │ 404-2  Align to reference human construct │
  └─────────────────────────────────────┘
                    │
                    ▼
        / Aligned reads
          124        /
                    │
                    ▼
  ┌ ─ ─ ─ ─ ─ ─ ─ ─ ─ ─ ─ ─ ─ ─ ─ ─ ┐
  │  408-2  Pre-process cfDNA sequence reads  │
  └ ─ ─ ─ ─ ─ ─ ─ ─ ─ ─ ─ ─ ─ ─ ─ ─ ┘
                    │
                    ▼
  ┌─────────────────────────────────────┐
  │ 410-2  Identify candidate somatic sequence variant(s) │
  └─────────────────────────────────────┘
                    │
                    ▼
      / Candidate sequence variants
        132-c                    /
                    │
                    ▼
  ┌─────────────────────────────────────┐
  │ 412-2  Obtain dynamic variant count threshold(s) │
  └─────────────────────────────────────┘
                    │
                    ▼
  ┌─────────────────────────────────────┐
  │ 414-2  Validate or reject somatic sequence variant │
  └─────────────────────────────────────┘
                    │
                    ▼
    / Validated somatic sequence
      variant(s)  132-v        /
                    │
                    ▼
  ┌ ─ ─ ─ ─ ─ ─ ─ ─ ─ ─ ─ ─ ─ ─ ─ ─ ┐
  │ 424-2  Match therapies and/or clinical trials based on variant status │
  └ ─ ─ ─ ─ ─ ─ ─ ─ ─ ─ ─ ─ ─ ─ ─ ─ ┘
                    │
                    ▼
      ┌ ─ ─ ─ ─ ─ ─ ─ ─ ─ ─ ─ ─ ┐
      │  426-2  Generate patient report │
      └ ─ ─ ─ ─ ─ ─ ─ ─ ─ ─ ─ ─ ┘
                    │
                    ▼
      ┌ ─ ─ ─ ─ ─ ─ ─ ─ ─ ─ ─ ─ ┐
      │ 428-2  Administer personalized therapy │
      └ ─ ─ ─ ─ ─ ─ ─ ─ ─ ─ ─ ─ ┘
```

Fig. 4F2

400-3

402-3 Obtain targeted panel cfDNA Sequencing Data

404-3 Align to reference construct

Aligned reads
406-3

408-3 Pre-process cfDNA sequence reads

410-3 Obtain segmented (binned) coverage ratios

412-3 Fit segments to expected copy states across a range of simulated tumor fractions

Segment-copy state pairs for each simulated tumor fraction 414-3

Distances (e.g., errors) between test and expected coverage ratios 416-3

Tumor purity scores (e.g., sum of weighted errors) 418-3

420-3 Assign ctFE based on TP with lowest score

Reportable ctFE biomarker 422-3

423-3 Variant identification

424-3 Match therapies and/or clinical trials based on biomarkers

Patient report indicating biomarker and/or personalized therapies 426-3

Fig. 4F3

<u>450</u>

( <u>452</u> Obtain cfDNA sequence reads <u>123</u> )

↓

<u>454</u> Align reads to reference construct <u>158</u>

↓

Aligned sequences <u>124</u>

↓ ↓ ↓

(A) (B) (C)

(A)

↓

<u>456</u> Identify putative somatic sequence variants

↓

<u>458</u> Validate putative somatic variant

<u>459</u> Determine dynamic variant count threshold

↓

<u>460</u> Apply dynamic probabilistic variant count filter

<u>462</u> Apply variant loci coverage filter(s)

<u>464</u> Apply variant allele fraction filter(s)

<u>466</u> Apply variant support mapping filter(s)

<u>468</u> Apply variant support sequencing quality filter(s)

<u>470</u> Apply low complexity region filter(s)

↓

<u>472</u>
All putative
somatic variants
validated? — No

↓ Yes

(D)

Fig. 4G1

B

**474** Validate sequencing data globally

**476** Apply loci minimal coverage filter(s)

**478** Apply loci central tendency coverage filter(s)

**480** Apply total sequence read filter(s)

**481** Apply sequence read quality filter(s)

**482** Apply sequencing control filter(s)

Validation or rejection of sequencing reaction **483**

C

**484** Identify putative germline sequence variants

**485** Validate putative germline variant

**486** Apply variant allele fraction filter(s)

**487** Apply variant support mapping filter(s)

**488** Apply variant support sequencing quality filter(s)

**489** All putative germline variants validated?

No

Yes

D

Fig. 4G2

(D)

490  Match therapies and/or clinical trials based on variant status

492  Generate report indicating selected validated variants

494  Administer matched therapies to patient

Fig. 4G3

Figure 5A

Figure 5B

Figure 5C

Figure 5D

Figure 5E

Figure 5F

Figure 5G

Figure 5H

Figure 6A

EP 4 600 963 A1

Figure 6B

Figure 7A

EP 4 600 963 A1

Figure 7B

EP 4 600 963 A1

Figure 7C

Figure 8A

Figure 8B

EP 4 600 963 A1

Figure 8C

EP 4 600 963 A1

**(900)** A method of determining a liquid biopsy tumor mutational burden (ITMB) for a test subject is provided.

**(902)** At a computer system having one or more processors, and memory storing one or more programs for execution by the one or more processors, there is obtained, from a panel-enriched sequencing reaction, a plurality of nucleic acid sequences comprising a corresponding sequence for each cell-free DNA fragment in a first plurality of cell-free DNA fragments obtained from a liquid biopsy sample from the test subject. Each respective cell-free DNA fragment in the first plurality of cell-free DNA fragments corresponds to a respective probe sequence in a plurality of probe sequences used to enrich cell-free DNA fragments in the liquid biopsy sample in the panel-enriched sequencing reaction. The plurality of probe sequences map to no more than 150 genes in the human genome.

**(904)** The panel-enriched sequencing reaction is performed at a read depth of at least 1,000X.

**(906)** The panel-enriched sequencing reaction uses a sequencing panel that enriches for from 50 genes to 150 genes.

**(908)** The plurality of probe sequences used to enrich cell-free DNA fragments in the liquid biopsy sample in the panel-enriched sequencing reaction collectively map to from 25 different genes to 150 different genes in a human reference genome.

**(910)** The panel-enriched sequencing reaction uses a sequencing panel that enriches for at least 10 genes listed in Table 1.

**(912)** The panel-enriched sequencing reaction uses a sequencing panel that enriches for at least 10 genes listed in List 1.

(A)

Figure 9A

**A**

**(902 continued)**

**(914)** The panel-enriched sequencing reaction uses a sequencing panel that enriches for at least 10 genes listed in List 2.

**(916)** The liquid biopsy sample is a blood sample.

**(918)** There is determined, using the panel-enriched sequencing reaction, that a circulating tumor fraction (ctFE) is above a threshold ctFE value.

**(920)** The threshold ctFE value is 0.0025.

**(922)** Responsive to determining that the ctFE is above the threshold, the ITMB for the test subject is calculated from the panel-enriched sequencing reaction.

**(924)** The calculating comprises determining a count of a plurality of genetic variants present in the plurality of nucleic acid sequences.

**(926)** The count of the plurality of genetic variants present in the plurality of nucleic acid sequences is a count of unique genetic variants present in the plurality of nucleic acid sequences that satisfy one or more qualifying criterion in a set of qualifying criteria.

**B**

Figure 9B

100

**B**

**(922 continued)**

**(926 continued)**

**(928)** A qualifying criterion in the set of qualifying criteria is a requirement that the respective genetic variant is a missense variant, a combination of a missense variant and a splice region variant, a frameshift variant, a stop loss variant, a splice acceptor variant, an in frame insertion variant, an in frame deletion variant, a combination of a frameshift variant and a splice region variant, a disruptive in frame insertion variant, or a disruptive in frame deletion variant.

**(930)** A qualifying criterion in the set of qualifying criteria is a requirement that the respective genetic variant has a variant allele frequency in the liquid biopsy sample that is greater than 0.005 (0.5%).

**(932)** A qualifying criterion in the set of qualifying criteria is a requirement that the respective genetic variant has a variant allele frequency in the liquid biopsy sample that is less than 1.0 (100%)

**(934)** A qualifying criterion in the set of qualifying criteria is a requirement that the respective genetic variant has a variant allele frequency (VAF) in the liquid biopsy sample that is any one of: (i) greater than 0.01 (1%) and less than 0.4 (40%), (ii) greater than 0.6 (60%) and less than 0.9 (90%), or (iii) greater than 0.4 (40%) and less than 0.60 (60%) with the proviso that|VAF-ctFE|/ctFE < 1, (iv) greater than 0.9 (90%) with the proviso that |VAF-ctFE|/ctFE < 1.

**C**

**Figure 9C**

C

**(922 continued)**

**(926 continued)**

**(936)** A qualifying criterion in the set of qualifying criteria is selection of a genetic variant present in the plurality of nucleic acid sequences by a medical professional.

**(938)** The calculating further comprises normalizing the count of the plurality of genetic variants present in the plurality of nucleic acid sequences by a coverage of the plurality of probe sequences.

**(940)** The coverage is from 0.1 megabases to 0.4 megabases.

**(942)** The coverage is from 0.15 megabases to 0.3 megabases.

**(944)** The ITMB is reported for the test subject.

**(946)** The reporting further comprises, responsive to determining that the ITMB satisfies a therapeutic threshold, reporting a matched therapeutic recommendation for the test subject.

**(948)** The reporting comprises comparing the ITMB for the subject to a severity threshold and reporting a qualitative status of either ITMB high (ITBM-H) or ITMB low (ITMB-L) based on the comparing.

D

Figure 9D

**D**

**(944 continued)**

(950) The ITMB for the test subject is reported when the ITMB satisfies a reporting threshold.

(952) An immunotherapeutic agent is administered to the test subject when the ITMB for the test subject satisfies a therapeutic threshold.

(954) Responsive to determining that the ITMB satisfies a clinical trial threshold, report a matched clinical trial recommendation for the test subject.

(956) Enroll the test subject when the ITMB for the test subject satisfies a clinical trial threshold

(958) The method further comprises using the ITMB to identify a concordant ITMB based on a predetermined correlation between (i) detection of somatic mutations in cell-free DNA from liquid biopsy sample from a cohort of training subjects and (ii) detection of somatic mutations in genomic DNA from solid tumor biopsy samples from the cohort of training subjects. Moreover, the reporting further comprises reporting the concordant ITMB.

(960) Update an electronic health record for the test subject to include the ITMB for the test subject.

**Figure 9E**

| Gene | Enhanced SNV / Indel | Non-Enhanced SNV / Indel | Fusion | CNV |
|---|---|---|---|---|
| ABCC3 | | • | | |
| ABL1 | • | | | |
| ABL2 | | • | | |
| ABRAXAS1 | | • | | |
| ACVR1 | | • | | |
| ACVR1B | | • | | |
| AJUBA | | • | | |
| AKT1 | • | | | |
| AKT2 | • | | | |
| AKT3 | | • | | |
| ALK | • | | • | |
| ALOX12B | | • | | |
| AMER1 | | • | | |
| APC | • | | | |
| APLNR | | • | | |
| AR | • | | | |
| ARAF | • | | | |
| ARFRP1 | | • | | |
| ARID1A | • | | | |
| ARID1B | | • | | |
| ARID2 | | • | | |
| ASNS | | • | | |
| ASXL1 | | • | | |
| ATM | • | | | |
| ATR | • | | | |
| ATRX | | • | | |
| AURKA | | • | | |
| AURKB | | • | | |
| AURKC | | • | | |
| AXIN1 | | • | | |
| AXIN2 | | • | | |
| AXL | | • | | |

(A)

FIG. 10A

(A)

| Gene | | | |
|---|---|---|---|
| BCL2L2 | | • | |
| BCL6 | | • | |
| BCLAF1 | | • | |
| BCOR | | • | |
| BCORL1 | | • | |
| BCR | | • | |
| BIRC3 | | • | |
| BLM | | • | |
| BMPR1A | | • | |
| BRAF | • | | • |
| BRCA1 | • | | • |
| BRCA2 | • | | • |
| BRD4 | | • | |
| BRIP1 | | • | |
| BTG1 | | • | |
| BTG2 | | • | |
| BTK | • | | |
| CALR | | • | |
| CARD11 | | • | |
| CARM1 | | • | |
| CASP8 | | • | |
| CBFB | | • | |
| CBL | | • | |
| CCND1 | • | | |
| CCND2 | • | | |
| CCND3 | • | | |
| CCNE1 | • | | • |
| CD22 | | • | |
| CD274 | • | | • |
| CD70 | | • | |
| CD74 | | • | |
| CD79A | | • | |
| CD79B | | • | |
| CDC73 | | • | |
| CDH1 | • | | |
| CDK12 | • | | |
| CDK4 | • | | |
| CDK6 | • | | |
| CDK8 | | • | |
| CDK9 | | • | |

(B)

FIG. 10B

(B)

| | | |
|---|---|---|
| CDKN1A | | • |
| CDKN1B | | • |
| CDKN2A | • | |
| CDKN2B | | • |
| CDKN2C | | • |
| CEBPA | | • |
| CHD4 | | • |
| CHEK1 | | • |
| CHEK2 | • | |
| CIC | | • |
| CKS1B | | • |
| CREBBP | | • |
| CRKL | • | |
| CSF1R | | • |
| CSF3R | | • |
| CTC1 | | • |
| CTCF | | • |
| CTLA4 | | • |
| CTNNA1 | | • |
| CTNNB1 | • | |
| CUL3 | | • |
| CUL4A | | • |
| CUX1 | | • |
| CXCR4 | | • |
| CYLD | | • |
| CYP17A1 | | • |
| CYSLTR2 | | • |
| DAXX | | • |
| DDB2 | | • |
| DDR1 | | • |
| DDR2 | • | |
| DDX3X | | • |
| DDX41 | | • |
| DEPTOR | | • |
| DICER1 | | • |
| DIS3 | | • |
| DNMT1 | | • |
| DNMT3A | | • |
| DOT1L | | • |
| DPYD | | • |
| EBF1 | | • |
| EED | | • |

(C)

FIG. 10C

FIG. 10D

(D)

| | | |
|---|---|---|
| FCGR2A | | • |
| FCGR3A | | • |
| FGF10 | | • |
| FGF12 | | • |
| FGF14 | | • |
| FGF19 | | • |
| FGF23 | | • |
| FGF3 | | • |
| FGF4 | | • |
| FGF6 | | • |
| FGFR1 | • | • |
| FGFR2 | • | • |
| FGFR3 | • | • |
| FGFR4 | • | |
| FH | | • |
| FHIT | | • |
| FLCN | | • |
| FLT1 | | • |
| FLT3 | • | |
| FLT4 | | • |
| FOLH1 | | • |
| FOXA1 | | • |
| FOXL2 | • | |
| FOXO1 | | • |
| FOXO3 | | • |
| FOXP1 | | • |
| FRS2 | | • |
| FUBP1 | | • |
| GABRA6 | | • |
| GALNT12 | | • |
| GATA1 | | • |
| GATA3 | • | |
| GATA4 | | • |
| GATA6 | | • |
| GID4 | | • |
| GLI2 | | • |
| GNA11 | • | |
| GNA13 | | • |
| GNAQ | • | |
| GNAS | • | |
| GPC3 | | • |
| GPS2 | | • |

(E)

FIG. 10E

(E)

| | |
|---|---|
| GREM1 | • |
| GRIN2A | • |
| GRM3 | • |
| GSK3B | • |
| GSTP1 | • |
| H3F3A | • |
| HAVCR2 | • |
| HDAC1 | • |
| HDAC2 | • |
| HGF | • |
| HIF1A | • |
| HIST1H3B | • |
| HLA-B | • |
| HNF1A | • |
| HNF1B | • |
| HOXB13 | • |
| HRAS | • |
| HSD3B1 | • |
| HSP90AA1 | • |
| HSPH1 | • |
| ID3 | • |
| IDH1 | • |
| IDH2 | • |
| IFNA21 | • |
| IFNAR1 | • |
| IFNAR2 | • |
| IFNG | • |
| IFNGR1 | • |
| IFNGR2 | • |
| IFNW1 | • |
| IGF1 | • |
| IGF1R | • |
| IKBKE | • |
| IKZF1 | • |
| IL10RA | • |
| IL32 | • |
| IL6R | • |
| IL7R | • |
| IMPDH1 | • |
| ING1 | • |
| INPP4B | • |
| INSR | • |

(F)

FIG. 10F

(F)

| Gene | Left | Right |
|------|------|-------|
| IRF1 | | • |
| IRF2 | | • |
| IRF4 | | • |
| IRS2 | | • |
| JAK1 | • | |
| JAK2 | • | |
| JAK3 | • | |
| JUN | | • |
| KAT6A | | • |
| KDM5A | | • |
| KDM5C | | • |
| KDM5D | | • |
| KDM6A | | • |
| KDR | • | |
| KEAP1 | • | |
| KEL | | • |
| KIT | • | |
| KLF4 | | • |
| KLHL6 | | • |
| KLLN | | • |
| KMT2A | • | |
| KMT2C | | • |
| KMT2D | | • |
| KRAS | • | |
| LATS1 | | • |
| LCK | | • |
| LMO1 | | • |
| LRP1B | | • |
| LTK | | • |
| LYN | | • |
| LZTR1 | | • |
| MAF | | • |
| MALT1 | | • |
| MAP2K1 | • | |
| MAP2K2 | • | |
| MAP2K4 | | • |
| MAP3K1 | | • |
| MAP3K13 | | • |
| MAP3K21 | | • |
| MAP3K7 | | • |
| MAPK1 | • | |
| MAPK3 | • | |

(G)

FIG. 10G

(G)

| Gene | | | |
|------|---|---|---|
| MAX | | • | |
| MC1R | | • | |
| MCL1 | | • | |
| MDM2 | • | | • |
| MDM4 | | • | |
| MED12 | | • | |
| MEF2B | | • | |
| MEN1 | | • | |
| MERTK | | • | |
| MET | • | | • |
| MITF | | • | |
| MKNK1 | | • | |
| MLH1 | • | | |
| MLH3 | | • | |
| MPL | • | | |
| MRE11 | | • | |
| MS4A1 | | • | |
| MSH2 | • | | |
| MSH3 | • | | |
| MSH6 | • | | |
| MST1R | | • | |
| MTAP | | • | |
| MTHFR | | • | |
| MTOR | • | | |
| MUC16 | | • | |
| MUTYH | | • | |
| MYB | | • | |
| MYC | • | | • |
| MYCL | | • | |
| MYCN | • | | |
| MYD88 | • | | |
| NBN | | • | |
| NCOA2 | | • | |
| NCOR1 | | • | |
| NF1 | • | | |
| NF2 | • | | |
| NFE2L2 | • | | |
| NFKBIA | | • | |
| NKX2-1 | | • | |
| NOTCH1 | • | | |
| NOTCH2 | | • | |
| NOTCH3 | | • | |

(H)

FIG. 10H

(H)

| | | |
|---|---|---|
| NOTCH4 | | • |
| NPM1 | • | |
| NQO1 | | • |
| NRAS | • | |
| NRG1 | | • |
| NSD1 | | • |
| NSD2 | | • |
| NSD3 | | • |
| NT5C2 | | • |
| NTRK1 | • | • |
| NTRK2 | • | • |
| NTRK3 | • | • |
| NUTM1 | | • |
| P2RY8 | | • |
| PAK1 | | • |
| PALB2 | • | |
| PALLD | | • |
| PARP1 | | • |
| PARP2 | | • |
| PARP3 | | • |
| PAX5 | | • |
| PBRM1 | • | |
| PDCD1 | | • |
| PDCD1LG2 | • | |
| PDGFRA | • | |
| PDGFRB | • | |
| PDK1 | | • |
| PHGDH | | • |
| PHLPP1 | | • |
| PHLPP2 | | • |
| PIAS4 | | • |
| PIK3C2B | | • |
| PIK3C2G | | • |
| PIK3CA | • | |
| PIK3CB | | • |
| PIK3CD | | • |
| PIK3CG | | • |
| PIK3R1 | • | |
| PIK3R2 | | • |
| PIM1 | | • |
| PLCG1 | | • |
| PLCG2 | | • |

(I)

FIG. 10I

( I )

| | | |
|---|---|---|
| PMS1 | | • |
| PMS2 | • | |
| POLA1 | | • |
| POLD1 | | • |
| POLE | | • |
| POLQ | | • |
| POT1 | | • |
| PPARG | | • |
| PPM1D | | • |
| PPP2R1A | | • |
| PPP2R2A | | • |
| PPP6C | | • |
| PRDM1 | | • |
| PREX2 | | • |
| PRKACA | | • |
| PRKAR1A | | • |
| PRKCI | | • |
| PRKN | | • |
| PTCH1 | • | |
| PTEN | • | |
| PTK2 | | • |
| PTPN11 | • | |
| PTPN13 | | • |
| PTPRD | | • |
| PTPRO | | • |
| PTPRT | | • |
| QKI | | • |
| RAC1 | | • |
| RAD21 | | • |
| RAD50 | | • |
| RAD51 | | • |
| RAD51B | | • |
| RAD51C | • | |
| RAD51D | | • |
| RAD52 | | • |
| RAD54L | | • |
| RAF1 | • | |
| RARA | | • |
| RASA1 | | • |
| RB1 | • | |
| RBM10 | | • |
| RECQL4 | | • |

( J )

FIG. 10J

(J)

| | | | |
|---|---|---|---|
| REL | | • | |
| RET | • | | • |
| RHEB | • | | |
| RHOA | • | | |
| RICTOR | | • | |
| RIT1 | • | | |
| RNF43 | • | | |
| ROS1 | • | | • |
| RPS6KB1 | | • | |
| RPTOR | | • | |
| RRM1 | | • | |
| RSF1 | | • | |
| RSPO2 | | • | |
| RUNX1 | | • | |
| RXRA | | • | |
| SDC4 | | • | |
| SDHA | • | | |
| SDHAF2 | | • | |
| SDHB | | • | |
| SDHC | | • | |
| SDHD | | • | |
| SETBP1 | | • | |
| SETD2 | | • | |
| SF3B1 | | • | |
| SGK1 | | • | |
| SIRPA | | • | |
| SLC34A2 | | • | |
| SLC9A3R1 | | • | |
| SLFN11 | | • | |
| SLIT2 | | • | |
| SMAD2 | | • | |
| SMAD3 | | • | |
| SMAD4 | • | | |
| SMARCA2 | | • | |
| SMARCA4 | | • | |
| SMARCB1 | | • | |
| SMC1A | | • | |
| SMC3 | | • | |
| SMO | • | | |
| SNCAIP | | • | |
| SOCS1 | | • | |
| SOS1 | | • | |

(K)

FIG. 10K

(K)

| | | |
|---|---|---|
| SOX2 | | • |
| SOX9 | | • |
| SPEN | | • |
| SPOP | • | |
| SRC | | • |
| SRSF2 | | • |
| STAG2 | | • |
| STAT3 | | • |
| STAT5B | | • |
| STAT6 | | • |
| STK11 | • | |
| SUFU | | • |
| SUZ12 | | • |
| SYK | | • |
| TBX3 | | • |
| TCF7L2 | | • |
| TEK | | • |
| TERC | | • |
| TERT | • | |
| TET2 | | • |
| TFEB | | • |
| TGFB1 | | • |
| TGFBR1 | | • |
| TGFBR2 | | • |
| TIGIT | | • |
| TIPARP | | • |
| TMEM127 | | • |
| TMPRSS2 | | • |
| TNFAIP3 | | • |
| TNFRSF14 | | • |
| TNFRSF17 | | • |
| TOP1 | | • |
| TOP2A | | • |
| TP53 | • | |
| TP53BP1 | | • |
| TP63 | | • |
| TRAF3 | | • |
| TRAF7 | | • |
| TSC1 | • | |
| TSC2 | • | |
| TSHR | | • |
| TYMS | | • |

(L)

FIG. 10L

| Gene | | L |
|---|---|---|
| TYRO3 | | • |
| U2AF1 | | • |
| UGT1A1 | | • |
| VEGFA | • | |
| VHL | • | |
| VSIR | | • |
| WEE1 | | • |
| WNK1 | | • |
| WRN | | • |
| WT1 | | • |
| XBP1 | | • |
| XPA | | • |
| XPC | | • |
| XPO1 | | • |
| XRCC1 | | • |
| XRCC2 | | • |
| YEATS4 | | • |
| ZFHX3 | | • |
| ZMYM3 | | • |
| ZNF217 | | • |
| ZNF703 | | • |
| ZNF750 | | • |
| ZNRF3 | | • |
| ZRSR2 | | • |

FIG. 10M

Europäisches
Patentamt

European
Patent Office

Office européen
des brevets

# EUROPEAN SEARCH REPORT

**Application Number**

EP 25 15 7250

## DOCUMENTS CONSIDERED TO BE RELEVANT

| Category | Citation of document with indication, where appropriate, of relevant passages | Relevant to claim | CLASSIFICATION OF THE APPLICATION  (IPC) |
|---|---|---|---|
| X | KIM EDWARD S. ET AL: "Blood-based tumor mutational burden as a biomarker for atezolizumab in non-small cell lung cancer: the phase 2 B-F1RST trial", NATURE MEDICINE(AUTHOR MANUSCRIPT ), vol. 28, no. 5, 14 April 2022 (2022-04-14), pages 939-945, XP093289713, New York ISSN: 1078-8956, DOI: 10.1038/s41591-022-01754-x Retrieved from the Internet: URL:https://www.nature.com/articles/s41591-022-01754-x.pdf> * abstractM section "Methods"; p. 939, 941 * | 1-15 | INV. G16B20/20 C12Q1/6886 |
| X | US 2019/025308 A1 (CUMMINGS CRAIG ANTHONY [US] ET AL) 24 January 2019 (2019-01-24) * claims 1,3,34 * | 1 | |
| | | | TECHNICAL FIELDS SEARCHED        (IPC) |
| | | | G16B C12Q |

The present search report has been drawn up for all claims

| Place of search | Date of completion of the search | Examiner |
|---|---|---|
| Munich | 25 June 2025 | Heidrich, Alexander |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 25 15 7250

This annex lists the patent family members relating to the patent documents cited in the above-mentioned European search report.
The members are as contained in the European Patent Office EDP file on
The European Patent Office is in no way liable for these particulars which are merely given for the purpose of information.

25-06-2025

| Patent document cited in search report | Publication date | Patent family member(s) | | Publication date |
|---|---|---|---|---|
| US 2019025308 A1 | 24-01-2019 | AU | 2018304458 A1 | 06-02-2020 |
| | | AU | 2022200109 A1 | 03-02-2022 |
| | | CA | 3069469 A1 | 24-01-2019 |
| | | CN | 111492245 A | 04-08-2020 |
| | | EP | 3655034 A1 | 27-05-2020 |
| | | IL | 271888 A | 27-02-2020 |
| | | JP | 2020527351 A | 10-09-2020 |
| | | JP | 2023103258 A | 26-07-2023 |
| | | KR | 20200093518 A | 05-08-2020 |
| | | KR | 20240006698 A | 15-01-2024 |
| | | TW | 201908730 A | 01-03-2019 |
| | | US | 2019025308 A1 | 24-01-2019 |
| | | US | 2024288430 A1 | 29-08-2024 |
| | | WO | 2019018757 A1 | 24-01-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63552644
- US 11475981 B
- US 11211147 B
- WO 2023164713 A
- US 93078623
- US 11043304 B
- US 80049222
- US 10957041 B
- US 10957445 B
- US 11244763 B
- US 11848107 B
- US 11145416 B

- US 11830587 B
- US 10975445 B
- US 11705226 B
- US 9138205 B
- WO 2021081253 A
- US 11629385 B
- US 93078624
- US 62944292
- US 789363
- US 20180002 W
- WO 2021168146 A

**Non-patent literature cited in the description**

- **RADOVICH et al.** *Oncotarget*, 2016, vol. 7 (35), 56491-500
- **TSIMBERIDOU et al.** *ASCO*, 2018
- **WHEELER et al.** *Cancer Res.*, 2016, vol. 76, 3690-701
- **SCHWAEDERLE et al.** *J Clin Oncol.*, 2015, vol. 33 (32), 3817-25
- **SCHWAEDERLE et al.** *JAMA Oncol.*, 2016, vol. 2 (11), 1452-59
- **WHELER et al.** *Cancer Res.*, 2016, vol. 76 (13), 3690-701
- **COYNE et al.** *Curr. Probl. Cancer*, 2017, vol. 41 (3), 182-93
- **MARKMAN.** *Oncology*, vol. 31 (3), 158, 168
- **FERNANDES et al.** *Clinics*, vol. 72 (10), 588-94
- **ROSS et al.** *JAMA Oncol.*, 2015, vol. 1 (1), 40-49
- **ROSS et al.** *Arch. Pathol. Lab Med.*, 2015, vol. 139, 642-49
- **HIRSHFIELD et al.** *Oncologist*, 2016, vol. 21 (11), 1315-25
- **GROISBERG et al.** *Oncotarget*, 2017, vol. 8, 39254-67
- **ILIE ; HOFMAN.** *Transl. Lung Cancer Res.*, 2016, vol. 5 (4), 420-23
- **ILIE ; HOFMAN.** *Transl Lung Cancer Res.*, 2016, vol. 5 (4), 420-23
- **CHAN et al.** *Ann. Clin. Biochem.*, 2003, vol. 40 (2), 122-30
- **STROUN et al.** *Oncology*, 1989, vol. 46 (5), 318-22

- **GOESSL et al.** *Cancer Res.*, 2000, vol. 60 (21), 5941-45
- **FRENEL et al.** *Clin. Cancer Res.*, 2015, vol. 21 (20), 4586-96
- **AGGRAWAL et al.** Assessment of Tumor Mutational Burden and Outcomes in Patients With Diverse Advanced Cancers Treated With Immunotherapy. *JAMA Network Open*, 2023, vol. 6 (5), e2311181
- **WADAPURKAR ; VYAS.** *Informatics in Medicine Unlocked*, 2018, vol. 11, 75-82
- **BAICHOO ; OUZOUNIS.** *BioSystems*, 2017, vol. 156-157, 72-85
- **OLSHEN et al.** *Biostatistics*, 2004, vol. 5 (4), 557-572
- **LI ; DURBIN.** *Bioinformatics*, 2009, vol. 25 (14), 1754-60
- **BENT et al.** *Cancer Chemother Pharmacol.*, 2017, vol. 80 (6), 1209-17
- **CHOMCZYNSKI ; SACCHI.** *Nat Protoc*, 2006, vol. 1 (2), 581-85
- **POECKH et al.** *Anal Biochem.*, 2008, vol. 373 (2), 253-62
- **KIVIOJA et al.** *Nat. Methods*, 2011, vol. 9 (1), 72-74
- **ISLAM et al.** *Nat. Methods*, 2014, vol. 11 (2), 163-66
- **JIANG et al.** *BMC Bioinformatics*, 2014, vol. 15 (182), 1-12
- **HATEM et al.** Benchmarking short sequence mapping tools. *BMC Bioinformatics*, 2013, vol. 14, 184

- **FLICEK** ; **BIRNEY**. Sense from sequence reads: methods for alignment and assembly. *Nat Methods*, 2009, vol. 6 (11), S6-S12
- **LI** ; **HOMER**. A survey of sequence alignment algorithms for next-generation sequencing. *Brief Bioinformatics*, 2010, vol. 11, 473-483
- **LAI et al.** VarDict: a novel and versatile variant caller for next-generation sequencing in cancer research. *Nucleic Acids Res*, 2016, vol. 44, 108
- **TALEVICH et al.** CNVkit: Genome-Wide Copy Number Detection and Visualization from Targeted DNA Sequencing. *PLoS Comput Biol*, 2016, vol. 12, 1004873
- **CHIANG et al.** SpeedSeq: ultra-fast personal genome analysis and interpretation. *Nat Methods*, 2015, vol. 12, 966
- **LUMPY. LAYER et al.** LUMPY: a probabilistic framework for structural variant discovery. *Genome Biol*, 2014, vol. 15, 84
- **LI et al.** Standards and Guidelines for the Interpretation and Reporting of Sequence Variants in Cancer: A Joint Consensus Recommendation of the Association for Molecular Pathology, American Society of Clinical Oncology, and College of American Pathologists. *The Journal of molecular diagnostics*, 2017, vol. 19, 4
- **KALIA et al.** Recommendations for reporting of secondary findings in clinical exome and genome sequencing, 2016 update (ACMG SF v2.0): a policy statement of the American College of Medical Genetics and Genomics. *Genetics in Medicine*, 2017, vol. 19, 249
- **ADALSTEINSSON et al.** Scalable whole-exome sequencing of cell-free DNA reveals high concordance with metastatic tumors. *Nat Commun*, 2017, vol. 8, 1324
- **OLSHEN et al.** Circular binary segmentation for the analysis of array-based DNA copy number data. *Biostatistics*, 2004, vol. 5, 557
- **BEAUBIER et al.** Clinical validation of the xT next-generation targeted oncology sequencing assay. *Onctotarget*, 2019, vol. 10 (24), 2384
- **CHEN et al.** Next-generation sequencing in liquid biopsy: cancer screening and early detection. *Hum Genomics*, 2019, vol. 13, 34
- **SAVLI et al.** TP53, EGFR and PIK3CA gene variations observed as prominent biomarkers in breast and lung cancer by plasma cell-free DNA genomic testing. *J Biotechnol*, 2019, vol. 300, 87
- **CHENG et al.** Liquid Biopsy Detects Relapse Five Months Earlier than Regular Clinical Follow-Up and Guides Targeted Treatment in Breast Cancer. *Case Rep Oncol Med*, 2019, 6545298
- **KEUP et al.** Targeted deep sequencing revealed variants in cell-free DNA of hormone receptor-positive metastatic breast cancer patients. *Cell Mol Life Sci, print.*, 2019
- **LI et al.** Genomic profiling of cell-free circulating tumor DNA in patients with colorectal cancer and its fidelity to the genomics of the tumor biopsy. *J Gastrointest Oncol*, 2019, vol. 10, 831
- **BETTEGOWDA et al.** Detection of circulating tumor DNA in early- and late-stage human malignancies. *Sci Transl Med*, 2014, vol. 6, 224
- **XUE et al.** Early detection and monitoring of cancer in liquid biopsy: advances and challenges. *Expert Rev Mol Diagn*, 2019, vol. 19, 273
- **HENNIGAN et al.** Low Abundance of Circulating Tumor DNA in Localized Prostate Cancer. *JCO Precis Oncol*, 2019, vol. 3
- **ABBOSH et al.** Early stage NSCLC - challenges to implementing ctDNA-based screening and MRD detection. *Nat Rev Clin Oncol*, 2018, vol. 15, 577
- **CHENG et al.** Clinical Validation of a Cell-Free DNA Gene Panel. *J Mol Diagn*, 2019, vol. 21, 632
- **HANIBUCHI et al.** Development, validation, and comparison of gene analysis methods for detecting EGFR mutation from non-small cell lung cancer patients-derived circulating free DNA. *Oncotarget*, 2019, vol. 10, 3654
- **VAN LAAR et al.** Development and validation of a plasma-based melanoma biomarker suitable for clinical use. *Br J Cancer*, 2018, vol. 118, 857
- **ODEGAARD et al.** Validation of a Plasma-Based Comprehensive Cancer Genotyping Assay Utilizing Orthogonal Tissue- and Plasma-Based Methodologies. *Clin Cancer Res*, 2018, vol. 24, 3539
- **CLARK et al.** *J Mol Diagn*, 2018, vol. 20, 686, Analytical Validation of a Hybrid Capture-Based Next-Generation Sequencing Clinical Assay for Genomic Profiling of Cell-Free Circulating Tumor DNA
- **PLAGNOL et al.** Analytical validation of a next generation sequencing liquid biopsy assay for high sensitivity broad molecular profiling. *PLoS One*, 2018, vol. 13, 0193802
- **KUDERER et al.** Comparison of 2 Commercially Available Next-Generation Sequencing Platforms in Oncology. *JAMA Oncol*, 2017, vol. 3, 996
- **BEAUBIER et al.** Integrated genomic profiling expands clinical options for patients with cancer. *Nat Biotechnol*, 2019, vol. 37, 1351